# EUROPEAN PATENT APPLICATION

(11) **EP 1 574 569 A1**
(43) Date of publication of application: **14.09.2005**
(21) Application number: 03780880.5
(22) Date of filing: 18.12.2003
(51) Int. Cl.: C12N 9/90, C12N 9/99, C12N 15/09, C12Q 1/533, C07D 401/06, G06F 17/30, G06F 17/50, G01N 33/50

(54) **THREE-DIMENSIONAL STRUCTURE OF LIPOCALIN-TYPE PROSTAGLANDIN D SYNTHASE AND UTILIZATION OF THE SAME**

(30) Priority: 19.12.2002 JP 2002368112
(71) Applicant: RIKEN, Saitama 351-0198 (JP); OSAKA BIOSCIENCE INSTITUTE, Osaka-Fu 565-0874 (JP)
(72) Inventor: MIYANO, Masashi c/o RIKEN, Harima Institute, Sayo-gun, Hyogo 679-5148 (JP); YAMAMOTO, Masaki c/o RIKEN, Harima Institute, Sayo-gun, Hyogo 679-5148 (JP); KUMASAKA, Takashi c/o RIKEN, Harima Institute, Sayo-gun, Hyogo 679-5148 (JP); AGO, Hideo c/o RIKEN, Harima Institute, Sayo-gun, Hyogo 679-5148 (JP); URADE, Y. 503, Granshitio-Shimogamo-Shikisaikan,, Kyoto-shi, Kyoto 606-0804 (JP); IRIKURA, Daisuke, Suita-shi, Osaka 565-0874 (JP); ARITAKE, Kosuke, Kawanishi-shi, Hyogo 666-0143 (JP)
(74) Representative: Baldock, Sharon Claire
(86) International application number: PCT/JP2003/016233
(87) International publication number: WO 2004/056992

(57) **Abstract**

Crystals of lipocalin-type prostaglandin D synthase are produced and analyzed by X-ray difratometry to give the three dimensional structure of enzyme based on which an inhibitor of the enzyme can be designed and selected.

## Description

### TECHNICAL FIELD

The present invention relates to the three dimensional structure of lipocalin-type prostaglandin D synthase (may be referred to as "L-PGDS" hereinafter). The present invention also relates to a method for selecting an inhibitor of L-PGDS using the three dimensional structure.

### BACKGROUND ART

Prostaglandin (PG) D₂ is actively produced in various tissues and involved in many physiological phenomena. In central nervous system, PGD₂ accelerates non-rapid eye movement (NONREM) sleep and regulates nociceptive reaction. PGD₂ is also actively produced by mast cell, basophil, and T helper type-2 cell, and controls allergy reaction via DP and CRTH2 receptors. PGD₂ is converted to J series of prostaglandin such as 9α , 11β - PGF₂ and PGJ₂, Δ¹² - PGJ₂, and 15-deoxy- Δ^{12,14} - PGJ₂. These prostaglandins have quite different properties in biological system. 15-Deoxy- Δ^{12,14} - PGJ₂ acts as a ligand of PPAR γ i.e., nuclear receptor involved in the differentiation of adipocyte, macrophage, and monocyte, preventing NF- κ B and AP-1 dependent gene expression.

Arachidonate cascade starts from PGH₂ synthase (cyclooxygenase, COX) and the enzyme produces PGH₂ from arachidonic acid provided from lipid bilayer of cell membrane. PGH₂ having 9,11-endoperoxide is unstable and spontaneously decomposes to be isomerized mainly to PGE₂ having 9-keto and 11-hydroxy group. PGD₂ having 9-hydroxy and 11-keto group is a redio isomer of PGE₂, and is specifically produced from PGH₂ by PGD syntase (PGDS, Prostaglandin H₂ D-isomerase [EC5.3.99.2]).

There are two types of PGDSs genetically different from each other. One is hemopoietic enzyme (hemopoietic PGD Synthase; H-PGDS) and the other is lipokalin-type enzyme (lipokalin-type PGD Synthase; L-PGDS)(Y. Urade and O. Hayaishi, Vitamins Hormones 58:89-120 (2000)). H-PGDS has molecular weight of 26 KDa and is a glutathione-dependent enzyme. It is mainly localized in antigen-presenting cell and mast cell. H-PGDS belongs to sigma class of glutathione S transferase (GST) as assessed by evolutional and crystallographic analysis. On the other hand, L-PGDS has molecular weight of 26 KDa, which is identical with that of H-PGDS, but is glutathione-independent enzyme and quite differs from H-PGDS in amino acid sequence, gene structure, evolutional origin and cellular localization.

L-PGDS is a member of a lipokalin gene family composed of various secretory proteins and localized in choroid plexus, arachnoid membrane, and oligodendroglia of central nervous system (Y.Urade and O.Hayaishi, Biochem. Biophys. Acta, 1482:259-271(2000)). L-PGDS gene knock out mice lack allodynia induced by γ-aminobutyric acid and touch-evoked pain (Eguchi et al., Proc. Natl. Acad. Sci. USA, 96:726-730 (1998)), and have small NONREM sleep rebound after sleep deprivation (Eguchi et al., The 3rd International Conference on Oxygen and Life, Kyoto, Vol. 1233C:429-433(2002)). Megalocardia induced by loading high fat food is low in the gene knockout mice. Human L-PGDS overexpressing mice exhibit an excessive amount of NONREM sleep after the algetic stimulation, which occurs simultaneously with the increase of PGD₂ in brain (Pinzar et al., Proc. Natl. Acad. Sci. USA, 97:4903-4907(2000)). L-PGDS is considered to contribute to the control of algetic appearance and NONREM sleep by producing PGD₂ in central nervous system. In addition, allergic airway inflammation is exalted in human L-PGDS overexpressing mouse (Fujitani et al., J. Immunol, 168:443-449(2002)). The exaltation of L-PGDS gene expression is observed in the brain of patients with neurodegenerative disease such as multiple sclerosis (Chabas et al., Science, 294:1731-1735(2001), Thaisacks disease, and Sandhoff disease (Myerowitz et al., Hum. Mol. Genet, 11:1343-1350 (2002))). The expression of L-PGDS is observed in arteriosclerosis plaque of coronary artery in stable angina pectoris disease (Eguchi et al., Proc. Natl. Acad. Sci. USA, 94:14689-14694(1997)).

Therefore, If an inhibitor of L-PGDS can be found, the inhibitor can be expected to be used as new types of medicines such as allodynia, sleep controlling agent, antiallergic agent, anti-neurodegenerative agent, anti-arteriosclecrosis agent and anti-megalocardia agent.

### DISCLOSURE OF INVENTION

The present invention has an object to clarify three-dimensional structure of L-PGDS and to provide a method for designing and seeking an inhibitor of L-PGDS using the three-dimensional structure.

The present invention provides a crystal of lipocalin-type prostaglandin D synthase derived from mouse. Lipocalin-type prostaglandin D synthase derived from mouse has an amino acid sequence of SEQ ID NO:1. Since amino acids from sites 1 to 24 are a signal sequence, it is preferred that the protein excluding this portion is used. In addition, mouse L-PGDS contains three cystein residues at sites 65, 89, and 186. Accordingly, it is preferred for resolving crystal structure that, when L-PGDS is produced by recombinant method, cystein at site 65 is replaced by alanine to avoid the formation of disulfide bond other than native disulfide bond between cystein at sites 89 and 186. L-PGDS in which such substitution is made and the signal peptide is excluded may refer to as "native-type Cys⁶⁵Ala L-PGDS" or merely as "native-type L-PGDS" hereinafter.

This native-type L-PGDS crystal has a space group P2₁2₁2₁ of orthorhombic system and the size of unit cell is a=46.2±0.5Å, b=66.8±0.71Å, and c=105.3±1.OÅ. There are two molecules of L-PGDS in crystallographic asymmetric unit.

Another crystal is produced from L-PGDS in which methionine at sites 64, 94, and 145 are replaced by seleno methionine in addition to exclusion of signal peptide and Cys⁶⁵Ala substitution (may refer to as "Se-Met-type L-PGDS" hereinafter).

This Se-Met-type L-PGDS crystal has a space group C222₁ of orthorhombic system and the size of unit cell is a=45.7±0.5Å, b=66.8±0.7Å, and c=104.5±1.0Å. There is one molecule of L-PGDS in crystallographic asymmetric unit.

The present invention is related to lipocalin-type prostaglandin D synthase having the three-dimensional structure represented by the structure coordinates of Table 2. The structure coordinates were
obtained by X-ray structure analysis of "native-type L-PGDS" as described above.

The present invention is related to lipocalin-type prostaglandin D synthase having the three-dimensional structure represented by the structure coordinates of Table 3. The structure coordinates were obtained by X-ray structure analysis of "Se-Met-type L-PGDS" as described above.

Crystal structures of native-type Cys⁶⁵Ala L-PGDS and Se-Met-type L-PGDS are presumed to be identical with wild type L-PGDS by the following reasons:
(i) The catalytic mechanism of PGDS reaction can be reasonably explained by the crystal structure of native-type Cys⁶⁵Ala L-PGDS;
(ii) As apparent from the interaction with PGH₂, the side chain of cystein residue at site 65 is exposed to the surface of inner cavity of the protein, and is not involved in the formation of disulfide bond which is an element of the protein skeleton in wild type L-PGDS having PGDS activity;
(iii) The fact that even Cys^{89,189}Ala variant not having disulfide bond of protein skeleton has enzyme activity which is a phenotype of the structure suggests that the barrel structure of L-PGDS itself is strong, and that the structure of native-type Cys⁶⁵Ala L-PGDS is not significantly changed by the amino acid substitution;
(iv) The three dimensional structure of Se-Met-type L-PGDS is very similar with that of native-type Cys⁶⁵Ala L-PGDS.

Table 2 and 3 represent the three-dimensional structure coordinates according to the format of Protein Data Bank (http://rcsb. org/pdb/, USA). "ATOM" at the first column indicates that it is atom constituting protein; the second column indicates the atom number sequentially numbered from the first amino acid constituting the protein; the third column indicates the atom type constituting the protein. For example, Cacarbon atom is represented by CA; amido nitrogen atom is represented by N; carbonyl carbon atom is represented by C; and carbonyl oxygen atom is represented by O,; the fourth column indicates the amino acid residue by three letter notation; the fifth column indicates the class of molecule; the sixth column indicates the amino acid number; the seventh, eighth, and ninth columns indicate coordinates of the atom (in Å for X-axis, Y-axis, and Z-axis directions in the order); the tenth column indicates the occupancy of the atom (in the present invention 1.00 for all atoms); and the eleventh column indicates the temperature factor of the atom. The twelfth column indicates the class of atom in Table 2 and the class of molecule in Table 3.

When these coordinates are used, an inhibitor of L-PGDS can be selected. That is, the present invention is related to the use of the coordinates described in Table 2 or 3 for the selection of lipocalin-type prostaglandin D synthase inhibitor.

The present invention relates to a method for selecting an inhibitor of lipocalin-type prostaglandin D synthase, which comprising:
(a) providing the three dimensional coordinates of Table 2 or 3 representing the three dimensional structure of lipocalin-type prostaglandin D synthase:
(b) providing three dimensional structures of candidate compounds; and
(c) selecting the candidate compound which fits to the substrate-binding site of lipocalin-type prostaglandin D synthase.

The term "substrate-binding site of L-PGDS is an interior space of hollow structure determined by amino acids residues at sites 39, 43-48, 54, 65-67, 77-83, 90-96, 103-107, 116-120, 129-133, 143-149 and 180 in amino acid sequence of L-PGDS of SEQ ID NO:1. These regions include 1 to 5 amino acids before and after the amino acid residues.

The term "fit" means that the whole or the part of candidate compound can stably bind to the whole or the part of the above substrate-binding site in figure and energy. As the result, the binding of the substratre to L-PGDS is inhibited.

In a preferred embodiment, the inhibitor as selected above is contacted with L-PGDS in the presence of prostaglandin H₂ to measure L-PGDS enzyme activity to confirm the inhibiting effect of the inhibitor selected.

According to the method of the present invention, a novel inhibitor of L-PGDS was found. That is, the present invention relates to an inhibitor of lipocalin-type prostaglandin D synthase, 4-dibenzo(a,d)cyclohepten-5-ylidene-1-(4-(2H-tetrazole-5-yl)butyl)piperidine (referred to as "AT-56" hereinafter).

### BRIEF DESCRIPTION OF THE DRAWINGS

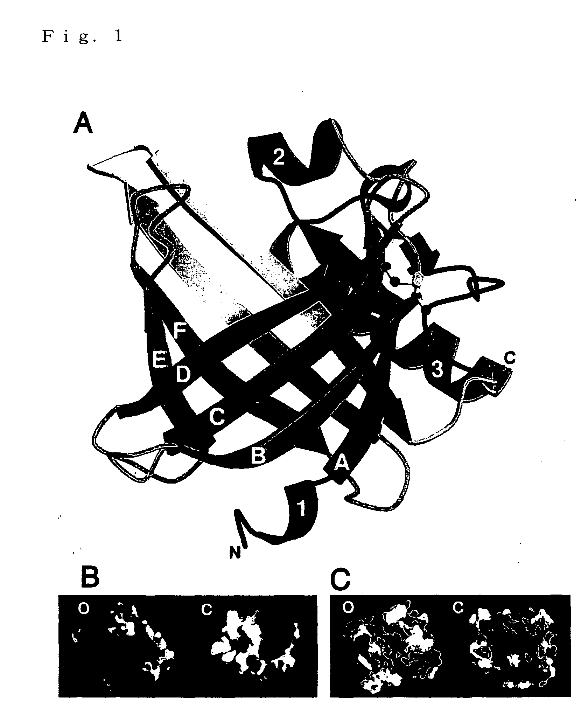

Figure 1 shows the whole structure of L-PGDS.
   (A) is ribbon diagram of P2₁2₁2₁ crystal of native-type L-PGDS which are viewed from right angle to barrel. The upper is open terminus of the barrel and the lower is closed terminus. Strands and helixes are represented as A-H and 1-3, respectively.
   (B) is a drawing showing the electrostatic feature of L-PGDS viewed from open (referred to as "o") and closed (referred to as "c") terminus. Black is electrostaticly positive and white, negative.
   (C) is a drawing showing conserved surface of L-PGDS. The degree of amino acid sequence conservation of known L-PGDS is depicted on the L-PGDS molecule surface. Dark shows higher conservation.

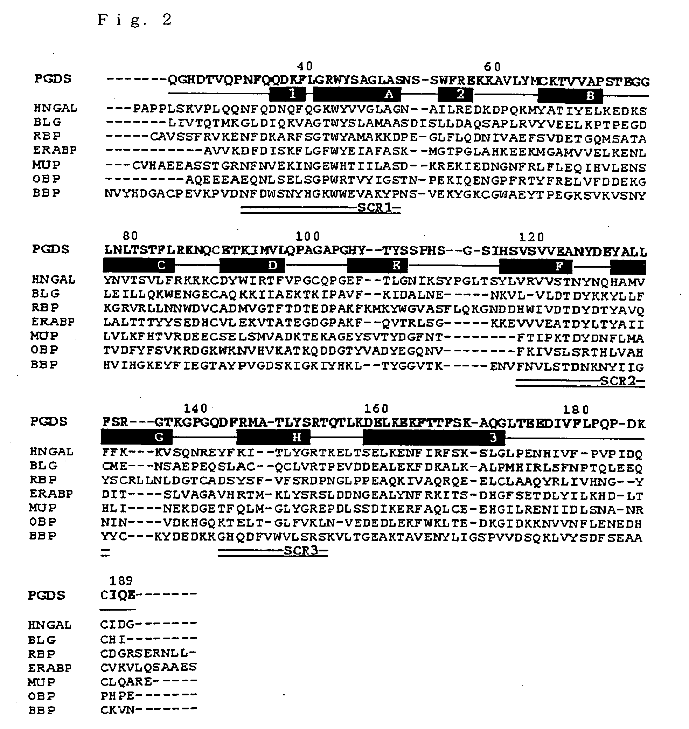

Figure 2 is a sequence alignment in consideration of mouse L-PGDS and other lipocalin structure. SCR region in which high amino acid conservation is observed in all sequences constitute the closed terminus of the barrel structure, and presumed to be important for L-PGDS activity. L-PGDS has a signal sequence consisting of about 20 residues at N-terminus region.
   PGDS: mouse L-PGDS, HNGAL: human neutrophils gelatinase related lipocalin, BLG: human β-lactoglobulin, RBP: human retinol binding protein, ERABP: rat epididymis retinoic acid binding protein, MUP: mouse main urine protein, OBP: ox perfume substance binding protein, BBP: butterfly biline binding protein.

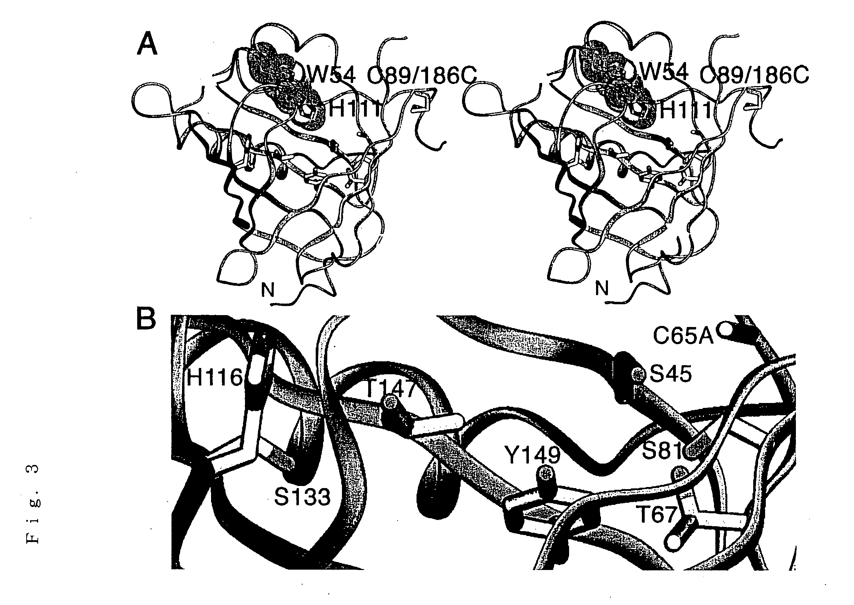

Figure 3 shows characteristic features of L-PGDS structure.
   (A) is a stereo diagram of the whole skeleton structure of L-PGDS of Se-Met type C222₁ crystal having noteworthy feature. Eight residues in hydrophilic belt inside envelop are shown. The structure in which one S-S bridge of Cys89/186 and two aromatic residues Trp⁵⁴ and His ¹¹¹ are contacted is found in the upper end of the barrel structure. Gln⁵¹ at upper left and Gln⁷⁸ at lower light are two presumed glycosylated sites.
   (B) is a closed-up of hydrophilic belt. There are eight polar residues, Cys⁶⁵Ala, Ser⁴⁵, Thr⁶⁷, Ser⁸¹, Tyr¹⁴⁹, Thr¹⁴⁷, Ser¹³³, and His¹¹⁶, which form presumed binding-site of the substrate-binding site of PGH₂.
Figure 4(A) shows a stereo diagram showing closed and open manner of envelop entrance. His¹¹¹ of EF loop can closes (H111c) or open (H111o) the entry of the substrate-binding site by the aromatic interaction of Trp54 of Ω loop.
   (B) is a stereo diagram of the bottom of substrate-binding site around Phe³⁹ and Trp⁴³. Two rotational isomer are shown in P2₁2₁2₁ (F39o) and C222₁ (F39o) crystal structure.
Figure 5 is a stereo diagram showing suggested PGH₂ bonding manner of L-PGDS. The PGDS binding and the presumed residue thereof involved in catalyst are shown with PGH₂ bonded.
Figure 6 is a schematic view of reaction mechanism. Hydrogen bond net work including Cys⁶⁵ promotes the formation of thiolate anion at physiological pH by sifting thiol and thiolate. After the binding of PGH₂ (1), thiolate of Cys⁶⁵ attacks as base endoepoxyoxygen attaching to C11 atom (2) to form S-O adduct as an intermediate(3). The unstable S-O bond is cleaved by synergistic rearrangement (3) to form C11 carbonyl of the product PGD₂(4).
Figure 7 is a graph showing binding and dissociating rate in the binding of PGD₂, PGE₂ and PGF_{2α} to L-PGDS. The binding of PGD₂, PGE₂ and PGF_{2α} to L-PGDS were measured by surface plasmon resonance method. The concentration of PGD₂ ranges 0.0 to 7.5 *µ* M and the concentration of PGE₂ and PGF_{2α} are 10 *µ*M.
Figure 8 is a graph showing the result of measurement of PGDS activity of various protein mutants at pH 8.0, 9.0 and 10.0.

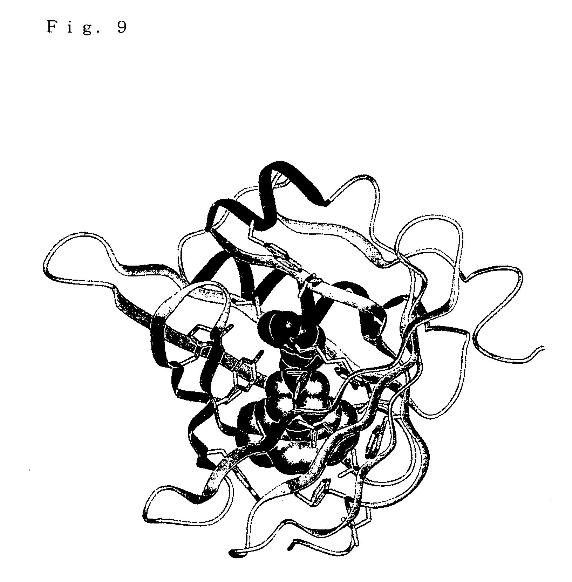

Figure 9 is a ribbon diagram showing L-PGDS to which AT-56 binds.
Figure 10 is a graph showing the inhibition effect of AT-56 on L-PGDS enzyme activity.
Figure 11 is a graph showing the inhibition effect of AT-56 on PGD₂ production.

### Best Mode for Carrying Out the Invention

### (I)Three Dimensional Structure, Substrate-Binding Site and Reaction Mechanism of L-PGDS

### (i) Whole Three Dimensional Structure of L-PGDS

The crystal structure of L-PGDS shows typical folding structure of lipocaline, and comprises anti-parallel *β* barrel of eight strands, three α helix regions and c-terminus strand (Figure 1A). The barrel has a size of 40x30x35 Å and the strands are cross-linked via conserved disulfide linkage of Cys^{89/186} at the outside of cup-formed portion. The outer surface of the protein comprises charged or polar amino acid residues. The molecular surface of the presumed entry for ligand is electrostaticlly positive and can draw the enzyme substrate PGH₂ and the product PGD which are negatively charged.

The amino acid sequence of L-PGDS is subjected to alinnment with amino acid sequence of various known lipocalins in consideration of the three dimensional structure (Figure 2). L-PGDS is classified as a karnel type lipocalin and has three structurally conserved region (SCR) 1, 2, and 3. These SCRs constitute closed end of the barrel of L-PGDS molecule and consists of N-terminus helix 1, strand A, strands F and G, and strand H. The helix 1 is positioned near the closed end of the barrel and is considered to stabilize the barrel structure by hydrophobic interaction. SCR is conserved even on the L-PGDS molecule surface (Figure 1C). EF loop and Ω loop corresponding to loop AB containing short helix 2 construct the closed cover of the barrel. Two glycosylated sites are positioned at Asn⁵¹ of large cover of Ω loop of L-PGDS and at Asn⁷⁸ of strand C (Arrow in Figure 3A)

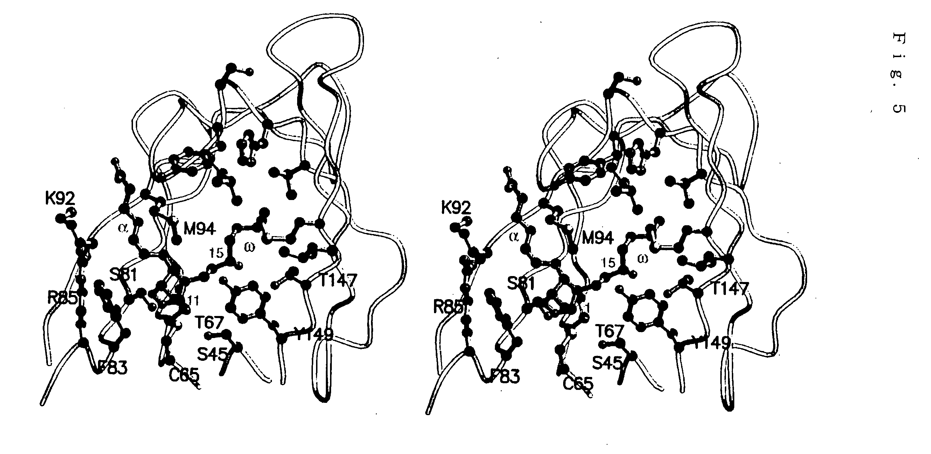

In the inside of L-PGDS controlled by hydrophobic side chain there is a remarkable hydrophilic belt consisting of eight polar residues of Cys⁶⁵Ala, Ser⁸¹, Thr⁶⁷, Ser⁴⁵, Tyr¹⁴⁹ Thr¹⁴⁷, Ser¹³³, and His¹¹⁶ from the right to the left in Figure 5 and Figure 3B. The residues in the polar belt are involved not only in the binding of the substrate PGH₂ but also in the release of the product PGD₂. As shown by site-directed mutagenesis hereinafter, the release of the product PGD₂ is promoted by decreasing the cost for hydration and dehydration of the polar hydroxyl group of ω chain.

The presumed catalyst residue Cys⁶⁵, which is replaced by alanine in the crystal structure, is positioned at the N-terminus of β strand B and faced to the inside of the barrel of open edge of the protein (Figures 5 and 3B) . The Cys⁶⁵Ala residue is surrounded by the hydroxyl side chain cluster of Ser⁴⁵, Thr⁶⁷ , and Ser⁸¹ within a distance of hydrogen bonding (Figures 3B). These residues form a hydrogen bond net work in the hydrophobic barrel together with Tyr¹⁴⁹, Thr¹⁴⁷ and Ser¹³³.

### (ii)Open-Close Conformation Isomer of L-PGDS

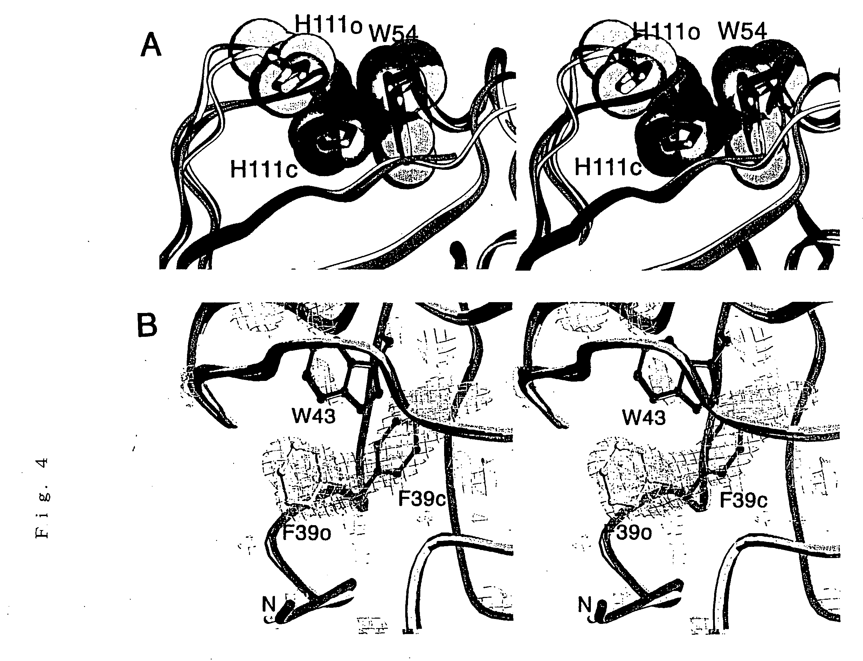

We determined two types of crystal structure having open and closed types at the entry of the substrate-binding site of L-PGDS. In Se-Met type L-PGDS crystal structure having space group C222₁, the active site is separated from the outside of the protein by a closed aromatic bridge between Trp⁵⁴ of Ω loop and His¹¹¹ of EF loop. In native-type L-PGDS crystal having different space group P2₁2₁2₁, the entry of the substrate-binding site is opened due to different conformation of EF loop having His¹¹¹ (Figure 4A) . Trp⁵⁴ and ¹⁰⁹SPHXGS residues of mobile EF loop are conserved in all identified amino acid sequence of L-PGDS including Xenopus homolog. One of the two molecules of nature type-L-PGDS contained by asymmetric unit of P2₁2₁2₁ crystal is low in electron density corresponding to Pro¹¹⁰, His¹¹¹. Atom model is not applied to electro density for the two residues. It is considered that open-closed conformation isomer of flexible loop play an important role in the binding of substrate and non-substrate ligand to the substrate-binding site of L-PGDS.

### (iii) Lipophilic Retinoic Acid Binding of L-PGDS as Lipocalin

L-PGDS was crystallized in the presence of retinoic acid as an essential crystallizing adjunct. L-PGDS binds to retinoic acid and retinal like many other lipocalins. Phe³⁹ at the bottom of the pocket shows different rotational isomer in different crystal forms P2₁2₁2₁ and C222₁ (Figure 4B). Native type P2₁2₁2₁ crystal has bulky and wide cavity with high residual electron density near Phe³⁹. On the other hand, in Se-Met C222₁ crystal, corresponding residual electron density is continuous with that of Phe³⁹ side chain and a narrower cavity is formed than that of the native type. It is concluded that the residual electron density in the neighborhood of indole ring of Trp⁴³ near Phe³⁹ side chain in the native crystal is contributed to boundd retinoic acid molecule. These retinoids non-competitively inhibit L-PGDS activity. The binding of retinoids requires the re-organization of hydrophobic residue cluster of L-PGDS including Trp43 which interact with retinoids

### (iv) Binding Mode of Substrate PGH₂

When minimum energy PGH₂ model is used, the substrate PGH₂ is positioned along hydrophilic belt of substrate-binding site of L-PGDS (Figure 5). In the suggested binding model, ω chain having 15-hydroxyl group of PGH₂ is inserted into the substrate-binding site, and oxygen atom bound to C11 in endoperoxide bound to cyclopentane ring reaches thiol group of Cys⁶⁵ residue of the catalyst. The negative charge of carboxyl group at α chain terminus of PGH₂ is counterbalanced by the positive charge of Lys⁹² and Arg⁸⁵ side chains, which form electrostaticlly positive cover of cup-form portion in a complex model (Figure 5). The hydrophilic belt of the substrate-binding site can promote insertion or release of ω chain of PGH₂ or PGD₂ having polar hydroxyl group since hydrated water of 15 hydroxyl of ω chain in solvent can be exchanged to these side chain without enthalpical dehydration cost. The polar surface of endoperoxide of PGH₂ is faced on hydroxyl cluster including Cys⁶⁵ and Ser⁴⁵, Thr⁶⁷ and Ser⁸¹. The hydrophobic portion of cyclopentane structure is enwrapped by hydrophobic chain including Phe⁸³ and Met⁹⁴. The closed conformation of the flexible EF loop existing at the entry of the substrate-binding site provides catalytic space for PGDS reaction and can help to specifically produce PGD₂ by separating catalytic portion from base in solvent. Furthermore, there is space enough to accommodate bulky product PGD₂ having 9-hydroxyl-11-keto-cyclopentan formed in docking study.

### (v) Reaction Mechanism of L-PGDS

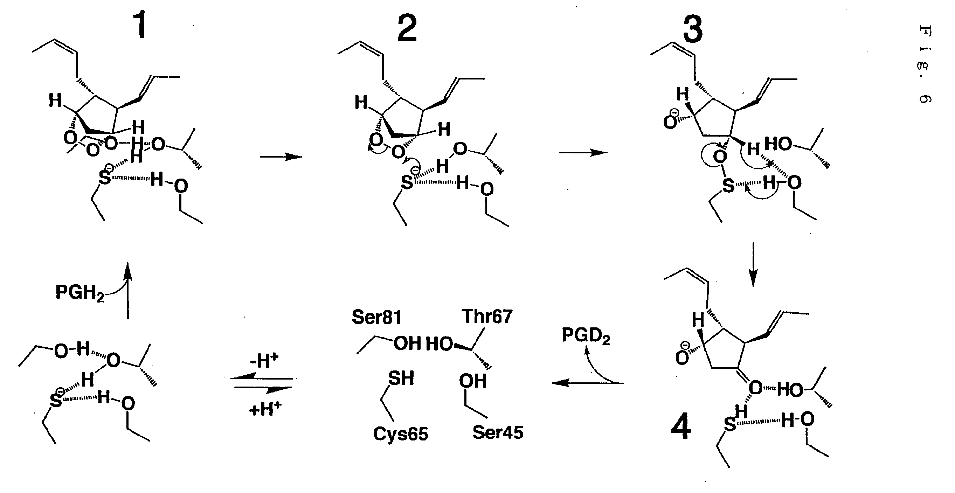

Reaction mechanism of PGDS activity catalyzed by L-PGDS was presumed and shown in Figure 6. The catalytic residue of L-PGDS is Cys⁶⁵ and the thiol group thereof is stabilized as thiolate ion as reaction species by synergistic hydrogen bond network with Ser⁴⁵, Thr⁶⁷ and Ser⁸¹. Binding mode of PGH₂ to L-PGDS in the suggested reaction mechanism provides appropriate configuration to direct endoperoxide oxygen of C11 of PGH₂ to sulfur atom of Cys⁶⁵ in PGDS reaction. In the model, the hydrogen-bond network of Cys⁶⁵ with Ser⁴⁵, Thr⁶⁷ and Ser⁸¹ decreases pKa of Cys⁶⁵ thiol group and stabilize thiolate anion as reaction species of PGDS catalyst at physiological pH. Suggested reaction mechanism is as follows (Figures 6):

Thiolate anion of Cys⁶⁵ attacks endoperoxide oxygen of C11 of PGH₂ as a base (Step 2 in Figure 6) to form S-O adduct presumed as an intermediate (Step 3). Hydroxyl group of Ser⁴⁵ attacks unstable S-O bond to rearrange proton of C11 hydrogen atom to convert into carbonyl group in a synergistic manner (Steps 3 and 4). After the product PGD₂ is released, thiol proton of Cys⁶⁵ is dissociated to regenerate thiolate anion as reaction species

### (vi) Mutant Analysis of L-PGDS

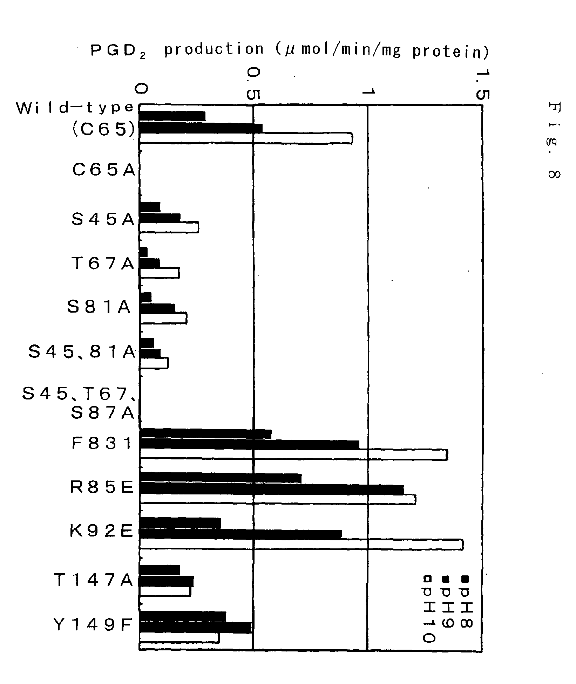

In order to confirm the suggested substrate-binding and reaction mechanism, site-directed mutagenesis of 9 amino acid residue in the interior of the substrate-binding pocket was performed. Since the preparation of recombinant protein having the same PGDS activity as purified wild-type enzyme is easy, Cys^{89,186}Ala construct was used for this purpose. Various mutants were expressed, purified to homogeneity and L-PGDS activities were compared at pH 8, 9 and 10 (Figure 8) .

It was confirmed that Cys⁶⁵ is the catalyst residue of L-PGDS since Cys⁶⁵Ala mutant completely loses enzyme activity. When Ser⁴⁵, Thr⁶⁷ and Ser⁸¹ of hydroxyl cluster surrounding Cys⁶⁵ in the catalyst pocket are replaced with Ala, the PGDS activity decreases to 30 to 15 % without significant reduction of Km for PGH₂ (Figure 8 and Table 1). Accordingly, kcat/Km was 0.36*µ*M⁻¹min⁻¹ for Ser⁴⁵Ala and 0.50 *µ*M⁻¹min⁻¹ for Ser⁸¹Ala, and thus decreased to one fifth in relative to wild type enzyme. L-PGDS activity is decreased to less than 10 % of wild type enzyme in Ser^{45,81} Ala double mutant, and almost disappear in Ser⁴⁵, Thr⁶⁷ and Ser⁸¹ triple mutant. These results correspond to the idea that thiolate anion of Cys⁶⁵ is stabilized by the hydroxyl cluster of Ser⁴⁵, Thr⁶⁷ and Ser⁸¹ to act as a reaction species in the enzyme reaction.

Notably, three mutants Phe⁸³Ile, Arg⁸⁵Glu and Lys⁹²Glu have kcat/Km of 4.47, 4.86 and 5.71 *µ* M⁻¹min⁻¹, respectively. These values were twice to that of wild type enzyme having kcat/Km of 2.38 *µ* M⁻¹min⁻¹ (Figure 8 and Table 1) . On the other hand, Km values of these mutants (11 to 15 *µ* M at pH 8) are comparable to wild type enzyme (13 *µ* M) except for Arg⁸⁵Glu mutant ( 7 *µ* M). These results suggest that the rate-determining step of L-PGDS is not the catalytic process of isomerization, but the product release process. Positive charges of Arg⁸⁵ and Lys⁹² giving positive electrostatic features on ligand entry surface of L-PGDS generate high affinity to negative charge of α chain of PGH₂ and PGD₂ (Figures 1B) and can assist to maintain the product on substrate-binding site. Aromatic ring of Phe⁸⁵ can assist to accommodare the product in a broad cavity around catalytic Cys⁶⁵ residue as PGH₂ binding model shown in figure 5. The activity of Thr¹⁴⁷Ala and Tyr¹⁴⁹Phe mutants decreases to one half in relative to the wild type enzyme. Accordingly, these polar side chains of Thr¹⁴⁷ and Tyr¹⁴⁹ are important for turnover of the catalyst.

**Table 1**

| Kinetic Parameters of L-PGDS Mutants for PGDS Activity | | |
|---|---|---|
| | Km (µM) | Kcat /Km (*µ*M⁻¹Min⁻¹) |
| Wild Type(C65) | 13 | 2.38 |
| S45A | 11 | 0.36 |
| S81A | 12 | 0.50 |
| F83I | 15 | 4.47 |
| R85E | 7 | 4.86 |
| K92E | 14 | 5.71 |
| T147A | 19 | 0.95 |
| Y149F | 1 | 0.82 |
| *kinetic parameter were measured at pH=8.0 | | |

### (vii) High Affinity Binding of Product PGD₂ to L-PGDS

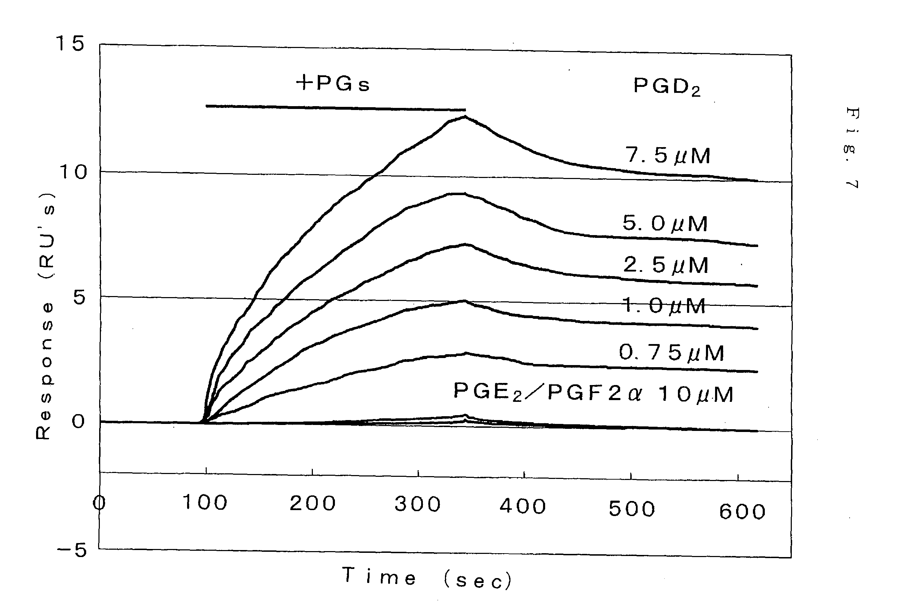

L-PGDS binds the product PGD₂ in high affinity (Kd=89.4 ± 3.4nM, kₒₙ=1.24x10³ ± 37M⁻¹sec⁻¹, k_{off}=1.11x10⁴ ± 7.0x10⁻⁶sec⁻¹), Neither PGE₂ nor PGF_{2α} is bound to L-PGDS as measured by surface plasmon resonance analysis (Figures 7) (Jonson et al., Biotechniques 1991 Nov 11 (5):620-627 ; Beuckmann et al., Biochemistry, 38:8006-8013 (1996)). L-PGDS can act as a PGD₂ transporter protein. Binding and dissociating test show that the bound PGD₂ is slowly released from hydrophobic pocket of L-PGDS. On the other hand, H-PGDS does not show high affinity to PGD₂. These results suggest that L-PGDS has two functions. That is, L-PGDS act as a PGD₂ synthesis enzyme coupled with COX in cells and thereafter as an extracellular transporter of PGD₂.

### (II) Selection of L-PGDS Inhibitor Using Structure Coordinate

It is possible to select compounds which can inhibit L-PGDS using the three dimensional structure coordinates as shown in Table 2 or 3.

The present invention relates to a method for selecting an inhibitor of lipocalin-type prostaglandin D synthase, which comprising
(a) providing the three dimensional structure coordinates in Table 2 or 3 representing the three dimensional structure of lipocalin-type prostaglandin D synthase:
(b) providing three dimensional structures of a candidate compounds; and
(c) selecting a candidate compound which fits to the substrate-binding site of lipocalin-type prostaglandin D synthase.

As described above, the amino acid residues constituting the substrate-binding site of L-PGDS are those at sites 39, 43-48, 54, 65-67, 77-83, 90-96, 103-107, 116-120, 129-133, 143-149 and 180 in L-PGDS amino acid sequence of SEQ ID NO:1.

Based on the three-dimensional structure information, each candidate compound is evaluated for binding fitness to the substrate-binding site of L-PGDS. In the evaluation, candidate compound and the structure exhibiting stable binding manner in energy and steric structure to the active site of L-PGDS are ranked among candidate compounds having various structures. This evaluation can be automatically performed by many kinds of virtual screening program. Among the ranked candidate compounds, a lead compound is selected which readily bind to the substrate-binding site of L-PGDS numerically and visually. The compound thus obtained is used as basic skeleton, and the derivative capable of binding more stably to the substrate binding site of L-PGDS can be designed and synthesized to develop new inhibitors of the enzyme.

It is preferred to design inhibitor using computer. For example, OCTANE, a workstation supplied by Silicon Graphics, Inc., is suitable as a computer used for designing inhibitors. However, the computer is not limited to this one, and any computer may be used so long as it is tuned to run an appropriate program. Likewise, there is no particular limitation on the computer storage medium. For example, Insight II, a computer program commercially available from Accelrys, Inc. may be used as a program for designing. In particular, a program Ludi or DOCK, a module of Insight II specially prepared for such purposes, may be used alone or in combination to facilitate identification, searching, evaluation, or designing.

In designing of inhibitor, there are conceptually two steps. The first step is to find a compound which serves as a starting point for drug design, known for those skilled in the art as a lead compound. The next step is optimization of the lead compound wherein compounds having better properties as medicines, for example, having better activity, having better pharmacokinetics, or having less toxicities and side effects are sought starting from the lead compound.

The step in which a lead compound is found using the structure coordinates of the L-PGDS complex provided by the present invention is achieved, for example, using a database in a computer into which structures of plural compounds have been entered, by a method in which interactions between three-dimensional structures of a compound in the database and L-PGDS are sorted out in a visual manner one after another, or by a method in which amplitudes of binding energy are calculated one after another using a computer and compounds which stably bind to PGDS are found from the database. Although it is preferred that the database of compound's structures contains determined three-dimensional structure coordinates entered therein, for low molecular weight compounds, it does not have to be a database of three-dimensional structure coordinates, because such low molecular weight compounds may change their conformations relatively freely, and also because three-dimensional structure coordinates for each conformation can be derived by calculations in a relatively short time. In the latter cases, information for chemical covalent bonds of low molecular weight compounds are entered into the database.

Specifically, in the visual method, L-PGDS is firstly displayed on a computer screen according to the structure coordinates of the present invention. In this step, although a three-dimensional representation may be made on the computer screen using, for example, Crystal Eye as described above, visual examinations can also be achieved without using such a three-dimensional representation.

Chemical interactions to be considered include electrostatic interaction, hydrophobic interaction, hydrogen bonding, van der Waals interaction, and the like. Thus, the structure should be comprehensively examined whether it is favorable for interactions, for example, so that functional groups which tend to bear negative charge such as carboxyl group, nitro group, and halogens interact with amino acid residues in L-PGDS having positive charge such as lysine, arginine, and histidine, so that functional groups which tend to bear positive charge such as amino, imino, and guanidyl groups interact with amino acid residues in PGDS having negative charge such as glutamic acid and aspartic acid, so that hydrophobic functional groups such as aliphatic groups and aromatic groups interact with hydrophobic amino acid residues such as alanine, leucine, isoleucine, valine, proline, phenylalanine, tryptophan and methionine, so that functional groups involved in hydrogen bonding such as hydroxyl and amide groups can form hydrogen bonds with backbone or side chain portions of L-PGDS, so that binding between the compound and L-PGDS causes no steric hindrance, and so that empty spaces are filled to minimize such empty spaces and maximize van der Waals interaction. Thus, electrostatic interaction, hydrophobic interaction, van der Waals interaction, hydrogen bonding, and other factors are visually and comprehensively considered to finally determine whether or not the compound is suitable as a lead compound.

In the method by energy evaluation with a computer, the energy of binding between a compound and L-PGDS is determined by molecular force field calculations. Such calculations are applied to each compound in the database to find a certain compound which may serve as a lead compound capable of stable binding. As a molecular force field used in the calculations, for example, CVFF, AMBER force field optimized for proteins, which is contained in DISCOVER module of Insight II program may be used. In addition, some computer programs like Ludi in Insight II can automatically output candidates for lead compound when three-dimensional structure coordinates of interacting amino acid residues in a protein molecule are given, and such programs may also be applied to the method of present invention.

Furthermore, the visual examinations and the examination considering energy are not strictly sorted out from each other, and both techniques may be used in combination as appropriate.

The next step, in which optimization of the lead compound is conducted using the structure coordinates of the PGDS complex is used for the purpose of, where a lead compound which binds to L-PGDS has already been found by the above method or separately found in an experimental manner, optimizing the lead compound to obtain a better compound, for example, a compound having higher biological activities as an inhibitor or a compound having a structure favorable for oral administration as a medicine. It becomes possible only after a precise picture of chemical bonding between the lead compound and L-PGDS has been elucidated to directly find a site which is not optimal for interactions between the lead compound and L-PGDS and to design a new compound having an optimal functional group at that site, thereby enabling to design a more optimized compound.

For visual examinations with a computer, a model of the complex between the lead compound and L-PGDS is firstly displayed on a computer screen by entering the three-dimensional structure coordinates of the lead compound and the structure coordinates of L-PGDS provided by the present invention into a computer on which a computer program expressing three-dimensional coordinates of molecules runs or into a storage medium of the computer. In this step, although a three-dimensional representation may be made on the computer screen using, for example, Crystal Eye as described above, visual examinations can also be achieved without using such a three-dimensional representation. It is a logical designing of a compound to modify the lead compound so as to yield a compound more favorably interacting with L-PGDS or a compound having better pharmacokinetics while retaining the interactions.

Chemical interactions to be considered are the same as those in the step to find a lead compound, and a new compound having better properties as an inhibitor is finally designed starting from the lead compound.

In the method by energy evaluation with a computer, the energy of binding between a new compound designed from the lead compound and L-PGDS is determined by molecular force field calculations to judge the validity of the design. In addition, it is also possible to use a method in which other molecules such as solvent molecules are additionally included in the model and the free energy is determined using molecular dynamics to derive a compound capable of stable binding. As a molecular force field used in the calculations, for example, CVFF, AMBER force field optimized for proteins, which is contained in DISCOVER module of Insight II program may be used.

Furthermore, the visual examinations and the method by energy evaluations may be used in combination as appropriate. In addition to the visual examination and energy examination with computer, complex crystal of the lead compound or compound designed from the lead compound and L-PGDS is analyzed with X-ray crystal analysis. This gives important information for improved evaluation for the fitness between L-PGDA substrate-binding site and the compound. Complex crystal is prepared by crystallizing L-PGDS in the solution in which the compound and L-PGDS coexist. Alternatively, the crystal of L-PGDS produced in the solution not containing the compound is immersed in the solution which contains the compound and in which L-PGDS crystal can stably exist. X-ray crystal analysis of the complex crystal can be performed by the method used in the determination of the structure coordinates of native-type Cys⁶⁵Ala L-PGDS. In this case, the analysis can be made using L-PGDS struture coordinates described in Table 2 or 3. If necessary, the method in the determination of the structure coordinates of Se-Met-type L-PGDS may be used.

After a candidate compound of L-PGDS inhibitor is selected, it is preferably contacted with the enzyme in the presence of the substrate (prostaglandin H₂) to confirm an ability of the compound to inhibit the enzyme (see, for example, Shimizu, t., Yamamoto, S., and Hayaishi O. (1979), Purification and Properties of prostaglandin D synthase from rat brain, J. Biol. Chem., 254:5222-5228). The enzyme activity can be measured, for example, as follows:

The substrate [1-¹⁴C]prostaglandin (PG)H₂ is synthesized by reacting [1⁻¹⁴C]arachidonic acid with cyclooxygenase. Since PGH₂ is easily decomposed in an aqueous solution (half life: about 5 minutes), it is evaporated to dryness and stored at low temperature(-80°C). The enzyme reaction is performed by injecting 1 *µ*L of PGH₂ solution(acetone or non-volatile diethylenediglycohol solution) with a microsyringe to 49 *µ* L of 0.1 M phosphate buffer (pH8.0) containing 1 mM dithiothreitol (DTT) and the enzyme. After the reaction at 25 °C for 30 to 60 seconds, the reaction is quenched by the addition of 300 µL of ice-cold mixed solution of ether/methanol/0.1 M citric acid (30:4:1 v/v/v) followed by extraction the substrate and reaction product under acidic condition into ether layer. Subsequently, anhydrous sodium sulfate was added to dehydrate it. An aliquot of organic layer (about 50 *µ* L) is coated on silica gel thin layer in a low temperature room (4°C) and subjected to silica gel thin layer chromatography in a freezer (-20 °C) (developing solvent: ether/methanol/acetic acid (90:2:1 v/v/v) . After the development, radioactivity of PGD₂ fraction and fraction other than it are measured to calculate an enzyme activity based on the conversion ratio to PGD₂.

After the enzymatic reaction is performed using commercially available non-labeled PGH₂, PGH₂ is decomposed to 12(S)-hydroxy-8,10-trans-5-cis-heptadecatrienoic acid with FeCl₂ treatment followed by reverse phase HPLC using 11- β- PGE₂ as internal standard to quantify PGD₂. Commercially available ELISA may be used to quantify PGD₂.

As described above, the inhibitor can used as pain killer, sleep controlling agent, anti-arteriosclerosis agent, anti-megalocardia agent, antiallergic agent, anti-neurodegenrative agent, and the like

### Examples

### Example 1

### Production of L-PGDS

### Production of native-type L-PGDS

L-PGDS gene (EMBL/GenBank/DDBJ™ Accession No.D83329) from Glu24 to C terminus was obtained from a mouse brain library by PCR amplification and inserted into expression vector pGEX-2T (Amasham Pharmacia Biotech inc.). Escherichia coli DH5 α (Toyobo) was then transformed with the expression vector. pGEX-2T is an expression vector in which a gene of interest is inserted downstream of glutathione (GSH) transferase (GST) gene to express the protein of interest as GST fused protein. In order to prepare Cys65Ala, a pair of synthesized oligomer containing mismatch codon was prepared to replace Cys65 by Ala using the resulting expression vector as a matrix:

5′-GCTGTATTGTATATGgcaAAGACAGTGGTA-3′

5'-TACCACTGTCTTtgcCATATACAATACAGC-3'

In addition, Cys65Ala expression vector was prepared using the Quick change site-directed mutagenesis kit (Stratgene, Heiderberg, Germany). This was bound to glutathione (GSH) transferase (GST) gene (EMBL/GenBank/DDBJ™ Accession No.U58012) and Escherichia coli DH5 α (Toyobo) was transformed with the expression vector.

L-PGDS prepared by Escherichia coli DH5 α transformed with the expression vector was GST fused protein. The transformant is cultured in LB medium at 37°C. When OD₆₀₀ₙₘ reached 0.5-0.6, IPTG is added to 0.6 to 1 mM and L-PGDS was produced at 37°C for 6 hours. Then the transformant was isolated from the medium. The obtained transformant was homogenized by sonication and the resultant supernatant was collected by centrifugation to purify the fused enzyme using GSH-Sepharose 4B column chromatography. The fused enzyme was incubated with thrombin to separate L-PGDS and GST, before subjecting to Superdex 75 column chromatography in 5mM Tris/HCl (pH 8.0) followed by Mono-S chromatography in 10 mM sodium citrate to further purify the enzyme.

The site-specific mutagenesis was performed using a Quick change site-directed mutagenesis kit (Stratgene, Heiderberg, Germany). The DNA sequence was confirmed by the determination of cycle sequencing by a SequiTherm cycle sequencing kit (Epicentre Technologies, Madison, WI) followed by LI-COR model 4000L automatic DNA sequencer (LI-COR Inc., Lincoln, NE)

### Production of Se-Met-type L-PGDS

E. coli B834(DE3) (Novagene, WI, USA) was transformed by PGRX-2T vector obtained as described above, and cultured in the following amino acid-rich medium containing selenomethionine (g/L): alanine 1.5g, arginine HCl 1.75g; aspartic acid 1.2g; cystein 0.1g; glutamic acid 2g, glutamin 1 g; glysine 1.626g; histidine 0.175g; isoleucine 0.7g; leucine 0.7g; lysine HCl 1.26g; phenylalanine 0.4g, proline 0.3g; serine 6.25g, threonine 0.7g; tyrosine 0.5g, valine 0.7g; adenine 1g; guanosine 1.33g; thymine 0.33g; uracyl 1g; sodium acetate 1.5g; succinic acid 3g, ammonium chloride 1.5g, sodium hydroxide 0.85g; K₂HPO₄ 10.5g; Mg SO₄ 0.25g; FeSO4 (II) 0.0042; glucose 20g, selenomethionine 0.075g and Kao Michayluk Basal vitamin solution (Sigma-Aldrich). Before the cultivation in the medium above, the transformant was cultured in 1mL of LB medium at 37°C. One mL of the cultured liquid was mixed with 500 mL of the medium above, and further cultured at 37°C overnight. 50 mL of the 500 mL cultured liquid was added to 350 mL of the above fresh medium, and cultured at 37 °C . When OD₆₀₀ₙₘ is 0.5-0.6, IPTG was added to 0.6-1 mM and cultivation was continued at 37°C for 12 hours to produce L-PGDS.

Se-Met-type L-PGDS was purified in a similar manner as in native-type L-PGDS.

### Example 2

### Crystallization of L-PGDS

The purified enzyme was dialyzed against 5 mM Tris/HCl (pH 8, crystallizing stock solution) and then concentrated to 10 mg/ml through ultra filtration using YM-3 membrane (Millipore, Badford, MA). Crystallization was effected with hanging drop vapor diffusion method at a constant temperature of 22.5°C.

The native-type Cys⁶⁵Ala L-PGDS was crystallized by mixing 2 *µ*l of the 10 mg/mL enzyme solution with an equal volume of a reservoir solution containing 2 M sodium malonate, 0.1M Tris/HCl (pH8) and 10% (v/v) 1,4-dioxane. Rod form crystal having the maximum size of 0.1 x 0.1 x 0.4 mm was obtained within 3 weeks.

Se-Met-type L-PGDS Cys⁶⁵Ala L-PGDS was also crystallized by the hanging drop vapor diffusion methods. The liquid drop consisted of 2 *µ* l of the 10 mg/mL enzyme solution containing 10*µ*M all-trans retinoic acid and equal volume of mother liquid containing 1.25 M sodium citrate, 10% dioxane, and 2% Triton X-405 in 0.1M Tris/HCl (pH9.5).

Crystallographic parameters were determined using rotating anode-type X-ray generator and Rigaku RAxIS-IV imaging plate system (wave length 1.0000Å)

The native-type L-PGDS has orthorhombic system space group P2₁2₁2₁ and the size of unit cell is a=46.2 Å, b=66.8 Å, and c=105.3 Å. There are two molecules of L-PGDS in crystallographic asymmetrical unit.

The Se-Met-type L-PGDS has orthorhombic system space group C222₁ and the size of unit cell is a=45.7Å, b=66.8Å, and c=104.5 Å. There is one molecule of L-PGDS in crystallographic asymmetrical unit.

### Example 3

### Data Collection and Structure Determination

Diffraction data for Se-Met type and native-type Cys⁶⁵ Ala L-PGDS crystals were collected on Spring-8 Riken beam line I (BL45XU) protein crystallography station at a temperature of 100K. The diffraction data were treated using DENZO and SCALEPACK (Otwinowski et al., Methods Enzymol., 276:307-326 (1997)). CCP4 Sweet (Collaborative Computational Project, Number 4, 1994) was used for successive crystallographic calculation.

Se-Met type Cys⁶⁵Ala L-PGDS crystal belonging to space group C222₁ was analyzed with multiwaves anomalous dispersion method. The three sites of selenium were found from difference Patterson map. Phase refinement and electron density modification were carried out using SHARP (de La Fortelle et al., Methods Enzymol., 276:472-494(1997)) and SOLOMON (Abrahams et al., Acta Crystallogr., D32:32-42 (1996)). Program O (Jones et al., Acta Crystallogr., A47:110-119(1991)) and XtalView (McRee, Practical protein crystallography, Academic Press, 1993) were used for model construction. Crystallographic refinement was carried out by the cycle in which refinement calculation in consideration of molecule dynamic using CNS (Brunger et al., Acta Crystallogr., D54:905-921 (1996))) and manual model rectification were alternately effected. Flexible loop region of β barrel portion exhibited weak and unclear electron density. The present model does not include Asn88 of CD loop and 9 N-terminus residues. Crystallographic R and R_{free} for Se-Met enzyme was 0.23 and 0.28 at resolving power of 2.5Å, respectively.

The native-type crystal having P2₁2₁2₁ was solved by molecular replacement followed by crystallographic refinement as above, and then refined by full matrix least squares refinement and manual method. Since CD loop region of one molecule of the two molecules in asymmetrical unit has low electron density, it was not determined. Crystallographic R and R_{free} for native enzyme was 0.24 and 0.28 at resolving power of 2.1 Å, respectively. The native-type structure of P2₁2₁2₁ has the quite similar structure as Se-Met type protein structure of C222₁ except for open EF loop exhibiting deviation of more than 4Å and Phe³⁹ (whole atoms r.m.s.d=0.26). There are no residues in the region wherein Ramachandran plot of neither structure coordinates are allowed.

The structure coordinates of native-type L-PGDS are shown in Table 2 and those of Se-Met type L-PGDS in Table 3.

### Example 4

### Binding of PGD₂ to L-PGDS

The binding of PGD₂, PGE₂ or PGF_{2α} to L-PGDS was analyzed with BiaCore 2000 system (BiaCore, Uppsala, Sweden) using the surface plasmon resonance method. L-PGDS mutant Cys^{89,186}Ala was immobilized on CM5 sensor chip (BIAcore AB) coated with carboxymethyldextran. Binding assay was carried out at a constant flow rate of 30*µ* l/min. in the concentration range of 0.1 to 10 *µ* M in both the binding phase and the dissociation phase. After each assay, the sensor chip surface was regenerated with 15 *µ* l of 1.5 M urea. Rate constant values were calculated using analysis soft wear BIA Evaluation 3.0 soft wear after subtracting control surface (bovine serum albumin) using Langmuir 1: 1 binding model by four independent experiments. The results are shown in Figure 7.

### Example 5

### Search of L-PGDS Inhibitor using Three-Dimensional Structure of L-PGDS

Three-dimensional structure of native-type Cys⁶⁵Ala L-PGDS was displayed on a computer screen and three dimensional structures of various compounds were tried to fit to the structure of L-PGDS. As a result, it was found that 4-dibenzo (a,d) cyclohepten-5-ylidene-1-(4-(2H-tetrazole-5-yl)butyl)piperidine (referred to as "AT-56" hereinafter) having the formula: 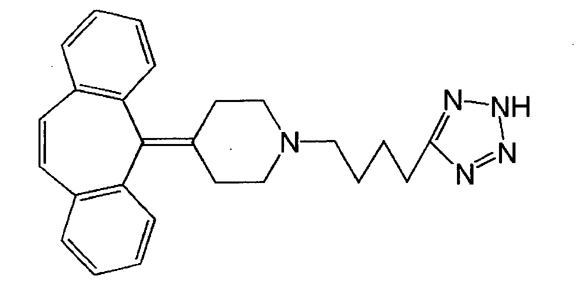 can fit to the three dimensional structure of L-PGDS. AT-56 is in the pocket formed by the amino acid residues of the sites 39, 40, 43, 45, 48, 54, 67, 69, 77, 79, 92, 94, 96, 105, 107, 109, 116, 118, 120, 129, 131, 133, 145, 147, and 149. Among them, the amino acid residues at the sites 45, 92, 147 and 149 is the substrate-binding sites, and therefore AT-56 is a possible inhibitor of L-PGDS.

### Inhibition effect of AT-56 on L-PGDS Enzyme Activity

The inhibition effect of AT-56 on L-PGDS enzyme was examined using human recombinant enzyme and the inhibition effect of AT-56 on H-LGDS enzyme was also examined for comparison.

In the measurement of L-PGDS inhibition activity, [1-¹⁴C] PGH₂ (5 *µ* M) as substrate is reacted in the presence of human recombinant L-PGDS, 0.1M Tris-HCl (pH 8.0) and 1mM DTT (dithiothreitol) at 25°C for 1 minute.

In the measurement of hematopoietic PGD Synthase (H-PGDS), [1-¹⁴C] PGH₂ (40*µ*M) as substrate is reacted in the presence of 0.1 M Tris-HCl (pH 8.0) and 0.1 mM GSH (reduced glutathione) at 25°C for 30 seconds.

AT-56 was pretreated for 5 minutes before the addition of the substrate and then the substrate was added. After the completion of the reaction, the reaction liquid was subjected to thin layer chromatography to isolate and quantify PGD₂ to calculate enzyme activity. AT-56 was prepared according to the method described in Japanese Patent Kokai No. 70112/1995, pages 3 and 4.

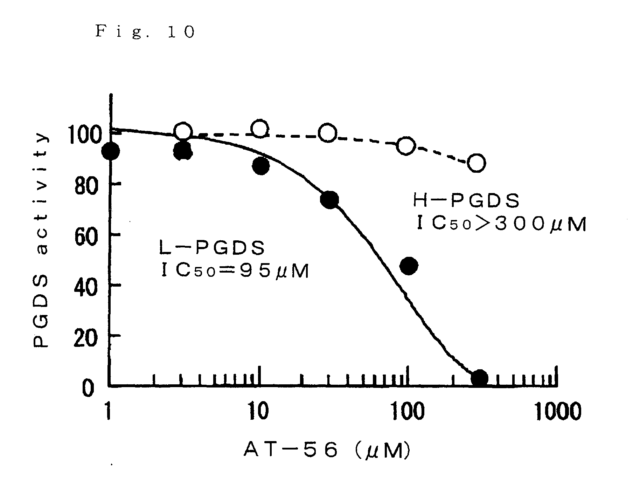

The result is shown in Figure 10. AT-56 dose-dependently inhibits the isomerization reaction from PGH₂ to PGD₂ and 50% inhibition concentration (IC₅₀) is 95 *µ* M. On the other hand, AT-56 (3 to 300 *µ* M) does not inhibit H-PGDS. It is thus confirmed that At-56 specifically inhibits L-PGDS.

### Inhibition Effect of AT-56 on PGD₂ Production from Cell Expressing L-PGDS

The inhibition effect of AT-56 on PGD₂ production by stimulation of calcium ionophore (A23187; 5-(methylamino)-2[[(2R,3R,6S,8S,9r,11R)-3,9,11-trimethl-8-[(1S)-1-methyl-2-oxo-2-(1H-pyrol-2-yl)ethyl]-1,7-dioxaspiro[5.5]undeca-2-yl]methyl-4-benzoxazole carbonic acid) from human cerebellum medulloblast (TE671) expressing L-PGDS was examined. TE-671 is seeded on microplate at the density of 1×10⁷. The cells were stimulated by A231875 (5 *µ* M) to cause PGD₂ production. AT-56 or vehicle was added to the cell culture medium 15 minutes before the A23187 stimulation. The culture medium was recovered 15 minutes after A23187 stimulation to quantify the PGD₂ concentration in the culture medium by enzyme immunoassay (EIA).

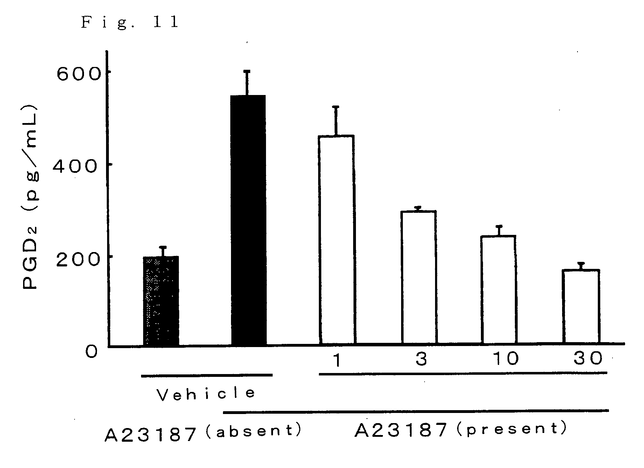

The results are shown in Figure 11. TE-671 cells increase the production of PGD₂ by the stimulation of A23187 as compared with that without stimulation. The production of PGD2 by A23187 stimulation was dose-dependently inhibited by AT-56. The result also confirms that AT-56 is an inhibitor for L-PGDS.

**Table 2**

| Three Dimensional Coordinates of Native-type L-PGDS | | | | | | | | |
|---|---|---|---|---|---|---|---|---|
| ATOM 1 | N | GLN A 35 | 7.532 | 25.687 | -6.080 | 1.00 | 33.40 | N |
| ATOM 2 | CA | GLN A 35 | 7.880 | 26.161 | -4.715 | 1.00 | 32.79 | C |
| ATOM 3 | CB | GLN A 35 | 6.613 | 26.584 | -3.976 | 1.00 | 34.50 | C |
| ATOM 4 | CG | GLN A 35 | 5.450 | 25.616 | -4.062 | 1.00 | 39.74 | C |
| ATOM 5 | CD | GLN A 35 | 4.209 | 26.171 | -3.369 | 1.00 | 49.53 | C |
| ATOM 6 | OE1 | GLN A 35 | 3.153 | 25.528 | -3.327 | 1.00 | 50.48 | O |
| ATOM 7 | NE2 | GLN A 35 | 4.337 | 27.377 | -2.817 | 1.00 | 49.93 | N |
| ATOM 8 | C | GLN A 35 | 8.666 | 25.154 | -3.873 | 1.00 | 30.34 | C |
| ATOM 9 | O | GLN A 35 | 9.147 | 25.489 | -2.794 | 1.00 | 29.61 | O |
| ATOM 10 | N | GLN A 36 | 8.811 | 23.929 | -4.367 | 1.00 | 27.61 | N |
| ATOM 11 | CA | GLN A 36 | 9.561 | 22.906 | -3.636 | 1.00 | 25.74 | C |
| ATOM 12 | CB | GLN A 36 | 9.751 | 21.657 | -4.503 | 1.00 | 24.32 | C |
| ATOM 13 | CG | GLN A 36 | 10.312 | 20.473 | -3.731 | 1.00 | 24.51 | C |
| ATOM 14 | CD | GLN A 36 | 10.259 | 19.181 | -4.516 | 1.00 | 21.94 | C |
| ATOM 15 | OE1 | GLN A 36 | 11.020 | 18.974 | -5.459 | 1.00 | 30.10 | O |
| ATOM 16 | NE2 | GLN A 36 | 9.350 | 18.307 | -4.134 | 1.00 | 26.53 | N |
| ATOM 17 | C | GLN A 36 | 10.930 | 23.421 | -3.170 | 1.00 | 23.41 | C |
| ATOM 18 | O | GLN A 36 | 11.341 | 23.145 | -2.047 | 1.00 | 20.27 | O |
| ATOM 19 | N | ASP A 37 | 11.625 | 24.162 | -4.036 | 1.00 | 24.65 | N |
| ATOM 20 | CA | ASP A 37 | 12.942 | 24.731 | -3.709 | 1.00 | 24.97 | C |
| ATOM 21 | CB | ASP A 37 | 13.470 | 25.596 | -4.859 | 1.00 | 28.96 | C |
| ATOM 22 | CG | ASP A 37 | 14.187 | 24.793 | -5.919 | 1.00 | 34.20 | C |
| ATOM 23 | OD1 | ASP A 37 | 14.733 | 25.418 | -6.856 | 1.00 | 41.87 | O |
| ATOM 24 | OD2 | ASP A 37 | 14.208 | 23.548 | -5.820 | 1.00 | 45.04 | O |
| ATOM 25 | C | ASP A 37 | 12.915 | 25.597 | -2.457 | 1.00 | 23.64 | C |
| ATOM 26 | O | ASP A 37 | 13.938 | 25.778 | -1.795 | 1.00 | 23.30 | O |
| ATOM 27 | N | LYS A 38 | 11.745 | 26.137 | -2.139 | 1.00 | 22.75 | N |
| ATOM 28 | CA | LYS A 38 | 11.595 | 26.996 | -0.974 | 1.00 | 23.00 | C |
| ATOM 29 | CB | LYS A 38 | 10.238 | 27.717 | -1.013 | 1.00 | 23.35 | C |
| ATOM 30 | CG | LYS A 38 | 10.024 | 28.669 | -2.185 | 1.00 | 23.60 | C |
| ATOM 31 | CD | LYS A 38 | 10.993 | 29.825 | -2.126 | 1.00 | 24.32 | C |
| ATOM 32 | CE | LYS A 38 | 10.606 | 30.923 | -3.107 | 1.00 | 27.38 | C |
| ATOM 33 | NZ | LYS A 38 | 9.282 | 31.485 | -2.771 | 1.00 | 26.05 | N |
| ATOM 34 | C | LYS A 38 | 11.704 | 26.225 | 0.341 | 1.00 | 22.03 | C |
| ATOM 35 | O | LYS A 38 | 11.980 | 26.815 | 1.385 | 1.00 | 21.91 | O |
| ATOM 36 | N | PHE A 39 | 11.501 | 24.912 | 0.284 | 1.00 | 20.33 | N |
| ATOM 37 | CA | PHE A 39 | 11.535 | 24.079 | 1.480 | 1.00 | 17.56 | C |
| ATOM 38 | CB | PHE A 39 | 10.391 | 23.056 | 1.442 | 1.00 | 19.18 | C |
| ATOM 39 | CG | PHE A 39 | 9.012 | 23.676 | 1.421 | 1.00 | 21.95 | C |
| ATOM 40 | CD1 | PHE A 39 | 8.514 | 24.254 | 0.263 | 1.00 | 16.05 | C |
| ATOM 41 | CE1 | PHE A 39 | 7.233 | 24.811 | 0.231 | 1.00 | 25.13 | C |
| ATOM 42 | CZ | PHE A 39 | 6.442 | 24.794 | 1.372 | 1.00 | 26.46 | C |
| ATOM 43 | CE2 | PHE A 39 | 6.934 | 24.219 | 2.544 | 1.00 | 28.59 | C |
| ATOM 44 | CD2 | PHE A 39 | 8.213 | 23.664 | 2.561 | 1.00 | 25.15 | C |
| ATOM 45 | C | PHE A 39 | 12.860 | 23.359 | 1.692 | 1.00 | 18.10 | C |
| ATOM 46 | O | PHE A 39 | 12.999 | 22.536 | 2.598 | 1.00 | 15.86 | O |
| ATOM 47 | N | LEU A 40 | 13.840 | 23.662 | 0.855 | 1.00 | 17.02 | N |
| ATOM 48 | CA | LEU A 40 | 15.140 | 23.037 | 1.001 | 1.00 | 17.96 | C |
| ATOM 49 | CB | LEU A 40 | 16.034 | 23.360 | -0.198 | 1.00 | 17.02 | C |
| ATOM 50 | CG | LEU A 40 | 15.501 | 22.886 | -1.546 | 1.00 | 20.11 | C |
| ATOM 51 | CD1 | LEU A 40 | 16.493 | 23.229 | -2.659 | 1.00 | 20.64 | C |
| ATOM 52 | CD2 | LEU A 40 | 15.266 | 21.391 | -1.481 | 1.00 | 15.79 | C |
| ATOM 53 | C | LEU A 40 | 15.750 | 23.602 | 2.271 | 1.00 | 17.56 | C |
| ATOM 54 | O | LEU A 40 | 15.202 | 24.528 | 2.874 | 1.00 | 17.30 | O |
| ATOM 55 | N | GLY A 41 | 16.861 | 23.019 | 2.702 | 1.00 | 16.10 | N |
| ATOM 56 | CA | GLY A 41 | 17.521 | 23.531 | 3.888 | 1.00 | 15.92 | C |
| ATOM 57 | C | GLY A 41 | 17.293 | 22.823 | 5.207 | 1.00 | 15.14 | C |
| ATOM 58 | O | GLY A 41 | 16.873 | 21.667 | 5.253 | 1.00 | 17.51 | O |
| ATOM 59 | N | ARG A 42 | 17.564 | 23.550 | 6.289 | 1.00 | 15.43 | N |
| ATOM 60 | CA | ARG A 42 | 17.461 | 23.028 | 7.645 | 1.00 | 17.08 | C |
| ATOM 61 | CB | ARG A 42 | 18.509 | 23.721 | 8.538 | 1.00 | 15.38 | C |
| ATOM 62 | CG | ARG A 42 | 18.574 | 23.214 | 9.995 | 1.00 | 15.81 | C |
| ATOM 63 | CD | ARG A 42 | 19.475 | 24.086 | 10.917 | 1.00 | 17.38 | C |
| ATOM 64 | NE | ARG A 42 | 18.931 | 25.430 | 11.127 | 1.00 | 20.69 | N |
| ATOM 65 | CZ | ARG A 42 | 19.516 | 26.393 | 11.846 | 1.00 | 24.91 | C |
| ATOM 66 | NH1 | ARG A 42 | 20.676 | 26.180 | 12.449 | 1.00 | 18.52 | N |
| ATOM 67 | NH2 | ARG A 42 | 18.946 | 27.591 | 11.948 | 1.00 | 23.29 | N |
| ATOM 68 | C | ARG A 42 | 16.093 | 23.133 | 8.320 | 1.00 | 16.71 | C |
| ATOM 69 | O | ARG A 42 | 15.490 | 24.209 | 8.393 | 1.00 | 17.77 | O |
| ATOM 70 | N | TRP A 43 | 15.627 | 22.001 | 8.836 | 1.00 | 17.28 | N |
| ATOM 71 | CA | TRP A 43 | 14.356 | 21.939 | 9.552 | 1.00 | 18.13 | C |
| ATOM 72 | CB | TRP A 43 | 13.283 | 21.239 | 8.721 | 1.00 | 15.45 | C |
| ATOM 73 | CG | TRP A 43 | 12.856 | 21.946 | 7.485 | 1.00 | 15.71 | C |
| ATOM 74 | CD1 | TRP A 43 | 13.401 | 21.834 | 6.233 | 1.00 | 15.15 | C |
| ATOM 75 | NE1 | TRP A 43 | 12.662 | 22.576 | 5.330 | 1.00 | 15.14 | N |
| ATOM 76 | CE2 | TRP A 43 | 11.631 | 23.187 | 5.996 | 1.00 | 14.06 | C |
| ATOM 77 | CD2 | TRP A 43 | 11.721 | 22.817 | 7.357 | 1.00 | 12.61 | C |
| ATOM 78 | CE3 | TRP A 43 | 10.778 | 23.319 | 8.264 | 1.00 | 14.15 | C |
| ATOM 79 | CZ3 | TRP A 43 | 9.777 | 24.162 | 7.792 | 1.00 | 11.66 | C |
| ATOM 80 | CH2 | TRP A 43 | 9.709 | 24.512 | 6.426 | 1.00 | 15.44 | C |
| ATOM 81 | CZ2 | TRP A 43 | 10.626 | 24.034 | 5.520 | 1.00 | 15.17 | C |
| ATOM 82 | C | TRP A 43 | 14.565 | 21.126 | 10.821 | 1.00 | 18.08 | C |
| ATOM 83 | O | TRP A 43 | 15.587 | 20.460 | 10.981 | 1.00 | 18.87 | O |
| ATOM 84 | N | TYR A 44 | 13.583 | 21.173 | 11.710 | 1.00 | 19.02 | N |
| ATOM 85 | CA | TYR A 44 | 13.617 | 20.413 | 12.952 | 1.00 | 19.64 | C |
| ATOM 86 | CB | TYR A 44 | 13.822 | 21.335 | 14.154 | 1.00 | 19.30 | C |
| ATOM 87 | CG | TYR A 44 | 15.113 | 22.102 | 14.146 | 1.00 | 20.96 | C |
| ATOM 88 | CD1 | TYR A 44 | 16.306 | 21.494 | 14.524 | 1.00 | 21.90 | C |
| ATOM 89 | CE1 | TYR A 44 | 17.506 | 22.194 | 14.501 | 1.00 | 26.37 | C |
| ATOM 90 | CZ | TYR A 44 | 17.512 | 23.521 | 14.094 | 1.00 | 28.61 | C |
| ATOM 91 | OH | TYR A 44 | 18.690 | 24.228 | 14.082 | 1.00 | 31.66 | O |
| ATOM 92 | CE2 | TYR A 44 | 16.338 | 24.145 | 13.710 | 1.00 | 26.67 | C |
| ATOM 93 | CD2 | TYR A 44 | 15.146 | 23.434 | 13.740 | 1.00 | 21.12 | C |
| ATOM 94 | C | TYR A 44 | 12.270 | 19.717 | 13.120 | 1.00 | 18.71 | C |
| ATOM 95 | O | TYR A 44 | 11.221 | 20.356 | 12.993 | 1.00 | 18.76 | O |
| ATOM 96 | N | SER A 45 | 12.300 | 18.413 | 13.388 | 1.00 | 20.71 | N |
| ATOM 97 | CA | SER A 45 | 11.072 | 17.658 | 13.625 | 1.00 | 20.41 | C |
| ATOM 98 | CB | SER A 45 | 11.349 | 16.153 | 13.541 | 1.00 | 21.74 | C |
| ATOM 99 | OG | SER A 45 | 12.516 | 15.802 | 14.276 | 1.00 | 19.43 | O |
| ATOM 100 | C | SER A 45 | 10.671 | 18.051 | 15.045 | 1.00 | 21.87 | C |
| ATOM 101 | O | SER A 45 | 11.377 | 17.735 | 16.005 | 1.00 | 23.08 | O |
| ATOM 102 | N | ALA A 46 | 9.553 | 18.764 | 15.171 | 1.00 | 21.80 | N |
| ATOM 103 | CA | ALA A 46 | 9.073 | 19.239 | 16.464 | 1.00 | 20.98 | C |
| ATOM 104 | CB | ALA A 46 | 8.868 | 20.756 | 16.402 | 1.00 | 20.73 | C |
| ATOM 105 | C | ALA A 46 | 7.791 | 18.570 | 16.945 | 1.00 | 21.49 | C |
| ATOM 106 | O | ALA A 46 | 7.464 | 18.635 | 18.127 | 1.00 | 22.81 | O |
| ATOM 107 | N | GLY A 47 | 7.053 | 17.947 | 16.034 | 1.00 | 21.44 | N |
| ATOM 108 | CA | GLY A 47 | 5.811 | 17.293 | 16.420 | 1.00 | 21.24 | C |
| ATOM 109 | C | GLY A 47 | 5.614 | 15.989 | 15.682 | 1.00 | 20.50 | C |
| ATOM 110 | O | GLY A 47 | 5.926 | 15.895 | 14.495 | 1.00 | 21.41 | O |
| ATOM 111 | N | LEU A 48 | 5.106 | 14.976 | 16.376 | 1.00 | 22.43 | N |
| ATOM 112 | CA | LEU A 48 | 4.879 | 13.674 | 15.755 | 1.00 | 24.18 | C |
| ATOM 113 | CB | LEU A 48 | 6.119 | 12.796 | 15.952 | 1.00 | 24.03 | C |
| ATOM 114 | CG | LEU A 48 | 6.216 | 11.439 | 15.258 | 1.00 | 25.64 | C |
| ATOM 115 | CD1 | LEU A 48 | 6.256 | 11.620 | 13.745 | 1.00 | 23.98 | C |
| ATOM 116 | CD2 | LEU A 48 | 7.490 | 10.732 | 15.731 | 1.00 | 24.77 | C |
| ATOM 117 | C | LEU A 48 | 3.646 | 12.997 | 16.353 | 1.00 | 25.73 | C |
| ATOM 118 | O | LEU A 48 | 3.460 | 12.988 | 17.568 | 1.00 | 28.24 | O |
| ATOM 119 | N | ALA A 49 | 2.800 | 12.438 | 15.492 | 1.00 | 26.62 | N |
| ATOM 120 | CA | ALA A 49 | 1.590 | 11.755 | 15.934 | 1.00 | 27.70 | C |
| ATOM 121 | CB | ALA A 49 | 0.408 | 12.706 | 15.893 | 1.00 | 26.11 | C |
| ATOM 122 | C | ALA A 49 | 1.337 | 10.556 | 15.029 | 1.00 | 29.92 | C |
| ATOM 123 | O | ALA A 49 | 1.610 | 10.612 | 13.829 | 1.00 | 30.06 | O |
| ATOM 124 | N | SER A 50 | 0.825 | 9.470 | 15.604 | 1.00 | 33.10 | N |
| ATOM 125 | CA | SER A 50 | 0.556 | 8.266 | 14.823 | 1.00 | 35.56 | C |
| ATOM 126 | CB | SER A 50 | 1.874 | 7.657 | 14.346 | 1.00 | 34.98 | C |
| ATOM 127 | OG | SER A 50 | 1.648 | 6.580 | 13.458 | 1.00 | 37.15 | O |
| ATOM 128 | C | SER A 50 | -0.233 | 7.214 | 15.600 | 1.00 | 37.86 | C |
| ATOM 129 | O | SER A 50 | -0.427 | 7.333 | 16.813 | 1.00 | 39.05 | O |
| ATOM 130 | N | ASN A 51 | -0.693 | 6.185 | 14.888 | 1.00 | 39.58 | N |
| ATOM 131 | CA | ASN A 51 | -1.447 | 5.091 | 15.509 | 1.00 | 41.56 | C |
| ATOM 132 | CB | ASN A 51 | -2.817 | 4.940 | 14.841 | 1.00 | 41.04 | C |
| ATOM 133 | CG | ASN A 51 | -2.718 | 4.653 | 13.354 | 1.00 | 42.05 | C |
| ATOM 134 | OD1 | ASN A 51 | -3.726 | 4.392 | 12.698 | 1.00 | 45.32 | O |
| ATOM 135 | ND2 | ASN A 51 | -1.511 | 4.706 | 12.816 | 1.00 | 39.99 | N |
| ATOM 136 | C | ASN A 51 | -0.658 | 3.790 | 15.361 | 1.00 | 43.28 | C |
| ATOM 137 | O | ASN A 51 | -1.097 | 2.723 | 15.788 | 1.00 | 43.84 | O |
| ATOM 138 | N | SER A 52 | 0.516 | 3.901 | 14.751 | 1.00 | 45.47 | N |
| ATOM 139 | CA | SER A 52 | 1.387 | 2.760 | 14.514 | 1.00 | 47.77 | C |
| ATOM 140 | CB | SER A 52 | 2.663 | 3.228 | 13.815 | 1.00 | 48.05 | C |
| ATOM 141 | OG | SER A 52 | 3.599 | 2.173 | 13.701 | 1.00 | 51.48 | O |
| ATOM 142 | C | SER A 52 | 1.755 | 2.027 | 15.796 | 1.00 | 48.61 | C |
| ATOM 143 | O | SER A 52 | 1.395 | 2.444 | 16.895 | 1.00 | 49.34 | O |
| ATOM 144 | N | SER A 53 | 2.475 | 0.923 | 15.636 | 1.00 | 49.72 | N |
| ATOM 145 | CA | SER A 53 | 2.933 | 0.118 | 16.762 | 1.00 | 50.37 | C |
| ATOM 146 | CB | SER A 53 | 3.129 | -1.340 | 16.333 | 1.00 | 50.55 | C |
| ATOM 147 | OG | SER A 53 | 1.912 | -1.919 | 15.891 | 1.00 | 52.17 | O |
| ATOM 148 | C | SER A 53 | 4.270 | 0.704 | 17.190 | 1.00 | 50.30 | C |
| ATOM 149 | O | SER A 53 | 4.579 | 0.805 | 18.379 | 1.00 | 50.13 | O |
| ATOM 150 | N | TRP A 54 | 5.056 | 1.082 | 16.188 | 1.00 | 50.56 | N |
| ATOM 151 | CA | TRP A 54 | 6.372 | 1.678 | 16.380 | 1.00 | 50.79 | C |
| ATOM 152 | CB | TRP A 54 | 6.930 | 2.116 | 15.022 | 1.00 | 51.39 | C |
| ATOM 153 | CG | TRP A 54 | 8.264 | 2.783 | 15.090 | 1.00 | 54.05 | C |
| ATOM 154 | CD1 | TRP A 54 | 9.472 | 2.183 | 15.300 | 1.00 | 55.89 | C |
| ATOM 155 | NE1 | TRP A 54 | 10.473 | 3.124 | 15.315 | 1.00 | 56.28 | N |
| ATOM 156 | CE2 | TRP A 54 | 9.922 | 4.362 | 15.114 | 1.00 | 57.18 | C |
| ATOM 157 | CD2 | TRP A 54 | 8.529 | 4.186 | 14.966 | 1.00 | 56.11 | C |
| ATOM 158 | CE3 | TRP A 54 | 7.724 | 5.313 | 14.746 | 1.00 | 57.68 | C |
| ATOM 159 | CZ3 | TRP A 54 | 8.330 | 6.564 | 14.681 | 1.00 | 56.58 | C |
| ATOM 160 | CH2 | TRP A 54 | 9.721 | 6.706 | 14.833 | 1.00 | 57.14 | C |
| ATOM 161 | CZ2 | TRP A 54 | 10.531 | 5.621 | 15.049 | 1.00 | 57.71 | C |
| ATOM 162 | C | TRP A 54 | 6.261 | 2.889 | 17.301 | 1.00 | 49.63 | C |
| ATOM 163 | O | TRP A 54 | 6.870 | 2.935 | 18.374 | 1.00 | 48.99 | O |
| ATOM 164 | N | PHE A 55 | 5.470 | 3.865 | 16.868 | 1.00 | 48.67 | N |
| ATOM 165 | CA | PHE A 55 | 5.268 | 5.091 | 17.625 | 1.00 | 47.55 | C |
| ATOM 166 | CB | PHE A 55 | 4.228 | 5.972 | 16.928 | 1.00 | 47.41 | C |
| ATOM 167 | CG | PHE A 55 | 4.014 | 7.298 | 17.599 | 1.00 | 46.06 | C |
| ATOM 168 | CD1 | PHE A 55 | 5.020 | 8.258 | 17.606 | 1.00 | 47.31 | C |
| ATOM 169 | CE1 | PHE A 55 | 4.833 | 9.479 | 18.241 | 1.00 | 45.75 | C |
| ATOM 170 | CZ | PHE A 55 | 3.633 | 9.750 | 18.875 | 1.00 | 45.84 | C |
| ATOM 171 | CE2 | PHE A 55 | 2.621 | 8.798 | 18.874 | 1.00 | 44.73 | C |
| ATOM 172 | CD2 | PHE A 55 | 2.816 | 7.582 | 18.238 | 1.00 | 44.94 | C |
| ATOM 173 | C | PHE A 55 | 4.822 | 4.805 | 19.054 | 1.00 | 47.07 | C |
| ATOM 174 | O | PHE A 55 | 5.415 | 5.305 | 20.011 | 1.00 | 45.46 | O |
| ATOM 175 | N | ARG A 56 | 3.775 | 3.997 | 19.193 | 1.00 | 47.71 | N |
| ATOM 176 | CA | ARG A 56 | 3.243 | 3.654 | 20.509 | 1.00 | 48.60 | C |
| ATOM 177 | CB | ARG A 56 | 2.030 | 2.732 | 20.354 | 1.00 | 49.22 | C |
| ATOM 178 | CG | ARG A 56 | 0.822 | 3.456 | 19.777 | 1.00 | 51.68 | C |
| ATOM 179 | CD | ARG A 56 | -0.320 | 2.516 | 19.428 | 1.00 | 56.98 | C |
| ATOM 180 | NE | ARG A 56 | -1.471 | 3.257 | 18.913 | 1.00 | 59.80 | N |
| ATOM 181 | CZ | ARG A 56 | -2.578 | 2.693 | 18.444 | 1.00 | 61.91 | C |
| ATOM 182 | NH1 | ARG A 56 | -2.691 | 1.373 | 18.421 | 1.00 | 62.67 | N |
| ATOM 183 | NH2 | ARG A 56 | -3.575 | 3.449 | 18.000 | 1.00 | 62.84 | N |
| ATOM 184 | C | ARG A 56 | 4.279 | 3.019 | 21.426 | 1.00 | 48.38 | C |
| ATOM 185 | O | ARG A 56 | 4.270 | 3.256 | 22.634 | 1.00 | 48.01 | O |
| ATOM 186 | N | GLU A 57 | 5.183 | 2.230 | 20.854 | 1.00 | 47.94 | N |
| ATOM 187 | CA | GLU A 57 | 6.209 | 1.573 | 21.652 | 1.00 | 48.73 | C |
| ATOM 188 | CB | GLU A 57 | 6.275 | 0.082 | 21.306 | 1.00 | 49.15 | C |
| ATOM 189 | CG | GLU A 57 | 4.958 | -0.663 | 21.500 | 1.00 | 50.89 | C |
| ATOM 190 | CD | GLU A 57 | 5.110 | -2.170 | 21.373 | 1.00 | 53.24 | C |
| ATOM 191 | OE1 | GLU A 57 | 5.686 | -2.632 | 20.363 | 1.00 | 53.06 | O |
| ATOM 192 | OE2 | GLU A 57 | 4.649 | -2.893 | 22.285 | 1.00 | 56.31 | O |
| ATOM 193 | C | GLU A 57 | 7.602 | 2.188 | 21.514 | 1.00 | 48.51 | C |
| ATOM 194 | O | GLU A 57 | 8.602 | 1.489 | 21.668 | 1.00 | 48.84 | O |
| ATOM 195 | N | LYS A 58 | 7.668 | 3.487 | 21.234 | 1.00 | 47.64 | N |
| ATOM 196 | CA | LYS A 58 | 8.950 | 4.177 | 21.096 | 1.00 | 46.97 | C |
| ATOM 197 | CB | LYS A 58 | 9.579 | 3.882 | 19.729 | 1.00 | 48.03 | C |
| ATOM 198 | CG | LYS A 58 | 10.147 | 2.475 | 19.597 | 1.00 | 51.08 | C |
| ATOM 199 | CD | LYS A 58 | 10.780 | 2.245 | 18.239 | 1.00 | 56.50 | C |
| ATOM 200 | CE | LYS A 58 | 11.185 | 0.787 | 18.060 | 1.00 | 58.17 | C |
| ATOM 201 | NZ | LYS A 58 | 11.672 | 0.506 | 16.677 | 1.00 | 59.90 | N |
| ATOM 202 | C | LYS A 58 | 8.826 | 5.683 | 21.277 | 1.00 | 45.19 | C |
| ATOM 203 | O | LYS A 58 | 9.833 | 6.388 | 21.334 | 1.00 | 44.57 | O |
| ATOM 204 | N | LYS A 59 | 7.592 | 6.169 | 21.371 | 1.00 | 43.70 | N |
| ATOM 205 | CA | LYS A 59 | 7.326 | 7.596 | 21.532 | 1.00 | 42.75 | C |
| ATOM 206 | CB | LYS A 59 | 5.827 | 7.826 | 21.698 | 1.00 | 43.46 | C |
| ATOM 207 | CG | LYS A 59 | 5.236 | 7.188 | 22.944 | 1.00 | 45.39 | C |
| ATOM 208 | CD | LYS A 59 | 3.713 | 7.187 | 22.902 | 1.00 | 48.36 | C |
| ATOM 209 | CE | LYS A 59 | 3.153 | 8.591 | 22.714 | 1.00 | 50.84 | C |
| ATOM 210 | NZ | LYS A 59 | 3.580 | 9.517 | 23.798 | 1.00 | 50.65 | N |
| ATOM 211 | C | LYS A 59 | 8.069 | 8.211 | 22.714 | 1.00 | 41.89 | C |
| ATOM 212 | O | LYS A 59 | 8.541 | 9.345 | 22.641 | 1.00 | 41.05 | O |
| ATOM 213 | N | ALA A 60 | 8.176 | 7.457 | 23.802 | 1.00 | 40.68 | N |
| ATOM 214 | CA | ALA A 60 | 8.858 | 7.936 | 24.996 | 1.00 | 39.93 | C |
| ATOM 215 | CB | ALA A 60 | 8.704 | 6.923 | 26.124 | 1.00 | 39.51 | C |
| ATOM 216 | C | ALA A 60 | 10.338 | 8.235 | 24.768 | 1.00 | 39.23 | C |
| ATOM 217 | O | ALA A 60 | 10.897 | 9.104 | 25.436 | 1.00 | 39.44 | O |
| ATOM 218 | N | VAL A 61 | 10.970 | 7.525 | 23.833 | 1.00 | 37.14 | N |
| ATOM 219 | CA | VAL A 61 | 12.395 | 7.726 | 23.560 | 1.00 | 35.90 | C |
| ATOM 220 | CB | VAL A 61 | 13.141 | 6.380 | 23.339 | 1.00 | 36.11 | C |
| ATOM 221 | CG1 | VAL A 61 | 12.952 | 5.463 | 24.537 | 1.00 | 34.24 | C |
| ATOM 222 | CG2 | VAL A 61 | 12.655 | 5.721 | 22.063 | 1.00 | 35.38 | C |
| ATOM 223 | C | VAL A 61 | 12.698 | 8.602 | 22.351 | 1.00 | 35.45 | C |
| ATOM 224 | O | VAL A 61 | 13.856 | 8.716 | 21.940 | 1.00 | 35.12 | O |
| ATOM 225 | N | LEU A 62 | 11.670 | 9.219 | 21.778 | 1.00 | 33.84 | N |
| ATOM 226 | CA | LEU A 62 | 11.873 | 10.074 | 20.614 | 1.00 | 32.75 | C |
| ATOM 227 | CB | LEU A 62 | 10.621 | 10.062 | 19.734 | 1.00 | 33.05 | C |
| ATOM 228 | CG | LEU A 62 | 10.265 | 8.671 | 19.212 | 1.00 | 35.96 | C |
| ATOM 229 | CD1 | LEU A 62 | 9.046 | 8.756 | 18.313 | 1.00 | 34.96 | C |
| ATOM 230 | CD2 | LEU A 62 | 11.457 | 8.086 | 18.460 | 1.00 | 39.51 | C |
| ATOM 231 | C | LEU A 62 | 12.242 | 11.512 | 20.970 | 1.00 | 30.66 | C |
| ATOM 232 | O | LEU A 62 | 11.663 | 12.113 | 21.872 | 1.00 | 30.48 | O |
| ATOM 233 | N | TYR A 63 | 13.226 | 12.053 | 20.261 | 1.00 | 29.47 | N |
| ATOM 234 | CA | TYR A 63 | 13.658 | 13.425 | 20.484 | 1.00 | 27.67 | C |
| ATOM 235 | CB | TYR A 63 | 15.103 | 13.481 | 21.003 | 1.00 | 29.10 | C |
| ATOM 236 | CG | TYR A 63 | 15.316 | 12.881 | 22.378 | 1.00 | 31.25 | C |
| ATOM 237 | CD1 | TYR A 63 | 15.441 | 11.503 | 22.549 | 1.00 | 33.47 | C |
| ATOM 238 | CE1 | TYR A 63 | 15.642 | 10.948 | 23.810 | 1.00 | 39.12 | C |
| ATOM 239 | CZ | TYR A 63 | 15.716 | 11.776 | 24.922 | 1.00 | 38.97 | C |
| ATOM 240 | OH | TYR A 63 | 15.905 | 11.223 | 26.175 | 1.00 | 44.99 | O |
| ATOM 241 | CE2 | TYR A 63 | 15.593 | 13.152 | 24.776 | 1.00 | 37.23 | C |
| ATOM 242 | CD2 | TYR A 63 | 15.394 | 13.694 | 23.508 | 1.00 | 32.70 | C |
| ATOM 243 | C | TYR A 63 | 13.582 | 14.202 | 19.181 | 1.00 | 25.92 | C |
| ATOM 244 | O | TYR A 63 | 13.420 | 13.628 | 18.107 | 1.00 | 26.31 | O |
| ATOM 245 | N | MET A 64 | 13.705 | 15.517 | 19.289 | 1.00 | 24.98 | N |
| ATOM 246 | CA | MET A 64 | 13.685 | 16.381 | 18.127 | 1.00 | 22.50 | C |
| ATOM 247 | CB | MET A 64 | 13.755 | 17.838 | 18.549 | 1.00 | 21.53 | C |
| ATOM 248 | CG | MET A 64 | 14.062 | 18.792 | 17.408 | 1.00 | 23.46 | C |
| ATOM 249 | SD | MET A 64 | 14.026 | 20.469 | 17.999 | 1.00 | 24.75 | S |
| ATOM 250 | CE | MET A 64 | 12.269 | 20.682 | 18.254 | 1.00 | 26.17 | C |
| ATOM 251 | C | MET A 64 | 14.898 | 16.059 | 17.299 | 1.00 | 21.60 | C |
| ATOM 252 | O | MET A 64 | 15.984 | 15.876 | 17.840 | 1.00 | 20.32 | O |
| ATOM 253 | N | ALA A 65 | 14.714 | 16.000 | 15.984 | 1.00 | 21.77 | N |
| ATOM 254 | CA | ALA A 65 | 15.801 | 15.702 | 15.071 | 1.00 | 21.68 | C |
| ATOM 255 | CB | ALA A 65 | 15.484 | 14.450 | 14.268 | 1.00 | 22.60 | C |
| ATOM 256 | C | ALA A 65 | 16.048 | 16.866 | 14.130 | 1.00 | 21.03 | C |
| ATOM 257 | O | ALA A 65 | 15.154 | 17.670 | 13.870 | 1.00 | 20.19 | O |
| ATOM 258 | N | LYS A 66 | 17.274 | 16.959 | 13.633 | 1.00 | 22.99 | N |
| ATOM 259 | CA | LYS A 66 | 17.626 | 18.010 | 12.693 | 1.00 | 22.22 | C |
| ATOM 260 | CB | LYS A 66 | 19.024 | 18.564 | 12.967 | 1.00 | 23.06 | C |
| ATOM 261 | CG | LYS A 66 | 19.499 | 19.477 | 11.847 | 1.00 | 24.65 | C |
| ATOM 262 | CD | LYS A 66 | 21.009 | 19.641 | 11.800 | 1.00 | 30.24 | C |
| ATOM 263 | CE | LYS A 66 | 21.462 | 20.820 | 12.622 | 1.00 | 35.63 | C |
| ATOM 264 | NZ | LYS A 66 | 22.848 | 21.237 | 12.231 | 1.00 | 43.47 | N |
| ATOM 265 | C | LYS A 66 | 17.608 | 17.356 | 11.326 | 1.00 | 23.44 | C |
| ATOM 266 | O | LYS A 66 | 18.312 | 16.375 | 11.091 | 1.00 | 24.56 | O |
| ATOM 267 | N | THR A 67 | 16.802 | 17.898 | 10.427 | 1.00 | 20.70 | N |
| ATOM 268 | CA | THR A 67 | 16.690 | 17.344 | 9.085 | 1.00 | 20.77 | C |
| ATOM 269 | CB | THR A 67 | 15.235 | 16.874 | 8.816 | 1.00 | 19.10 | C |
| ATOM 270 | OG1 | THR A 67 | 14.823 | 16.005 | 9.868 | 1.00 | 22.20 | O |
| ATOM 271 | CG2 | THR A 67 | 15.137 | 16.133 | 7.516 | 1.00 | 22.53 | C |
| ATOM 272 | C | THR A 67 | 17.085 | 18.378 | 8.030 | 1.00 | 19.13 | C |
| ATOM 273 | O | THR A 67 | 16.536 | 19.483 | 7.992 | 1.00 | 19.67 | O |
| ATOM 274 | N | VAL A 68 | 18.047 | 18.016 | 7.189 | 1.00 | 17.58 | N |
| ATOM 275 | CA | VAL A 68 | 18.494 | 18.900 | 6.120 | 1.00 | 18.47 | C |
| ATOM 276 | CB | VAL A 68 | 20.042 | 18.944 | 6.026 | 1.00 | 16.97 | C |
| ATOM 277 | CG1 | VAL A 68 | 20.480 | 19.900 | 4.914 | 1.00 | 19.31 | C |
| ATOM 278 | CG2 | VAL A 68 | 20.622 | 19.408 | 7.349 | 1.00 | 17.36 | C |
| ATOM 279 | C | VAL A 68 | 17.891 | 18.378 | 4.824 | 1.00 | 16.74 | C |
| ATOM 280 | O | VAL A 68 | 18.012 | 17.198 | 4.496 | 1.00 | 17.78 | O |
| ATOM 281 | N | VAL A 69 | 17.235 | 19.271 | 4.095 | 1.00 | 15.74 | N |
| ATOM 282 | CA | VAL A 69 | 16.565 | 18.904 | 2.860 | 1.00 | 14.97 | C |
| ATOM 283 | CB | VAL A 69 | 15.113 | 19.412 | 2.894 | 1.00 | 16.13 | C |
| ATOM 284 | CG1 | VAL A 69 | 14.370 | 18.984 | 1.643 | 1.00 | 18.02 | C |
| ATOM 285 | CG2 | VAL A 69 | 14.417 | 18.867 | 4.145 | 1.00 | 15.17 | C |
| ATOM 286 | C | VAL A 69 | 17.250 | 19.405 | 1.595 | 1.00 | 14.61 | C |
| ATOM 287 | O | VAL A 69 | 17.645 | 20.560 | 1.504 | 1.00 | 14.02 | O |
| ATOM 288 | N | ALA A 70 | 17.387 | 18.519 | 0.616 | 1.00 | 15.92 | N |
| ATOM 289 | CA | ALA A 70 | 18.015 | 18.856 | -0.661 | 1.00 | 16.00 | C |
| ATOM 290 | CB | ALA A 70 | 19.513 | 18.524 | -0.618 | 1.00 | 17.37 | C |
| ATOM 291 | C | ALA A 70 | 17.316 | 18.054 | -1.749 | 1.00 | 15.57 | C |
| ATOM 292 | O | ALA A 70 | 16.585 | 17.110 | -1.452 | 1.00 | 17.54 | O |
| ATOM 293 | N | PRO A 71 | 17.535 | 18.411 | -3.023 | 1.00 | 15.95 | N |
| ATOM 294 | CA | PRO A 71 | 16.886 | 17.686 | -4.123 | 1.00 | 16.43 | C |
| ATOM 295 | CB | PRO A 71 | 17.345 | 18.453 | -5.369 | 1.00 | 16.22 | C |
| ATOM 296 | CG | PRO A 71 | 17.689 | 19.839 | -4.838 | 1.00 | 17.77 | C |
| ATOM 297 | CD | PRO A 71 | 18.362 | 19.521 | -3.536 | 1.00 | 15.68 | C |
| ATOM 298 | C | PRO A 71 | 17.257 | 16.216 | -4.204 | 1.00 | 16.59 | C |
| ATOM 299 | O | PRO A 71 | 18.367 | 15.841 | -3.853 | 1.00 | 16.86 | O |
| ATOM 300 | N | SER A 72 | 16.320 | 15.391 | -4.665 | 1.00 | 18.06 | N |
| ATOM 301 | CA | SER A 72 | 16.567 | 13.972 | -4.830 | 1.00 | 18.01 | C |
| ATOM 302 | CB | SER A 72 | 15.367 | 13.145 | -4.367 | 1.00 | 19.58 | C |
| ATOM 303 | OG | SER A 72 | 14.336 | 13.166 | -5.335 | 1.00 | 21.44 | O |
| ATOM 304 | C | SER A 72 | 16.805 | 13.759 | -6.322 | 1.00 | 18.75 | C |
| ATOM 305 | O | SER A 72 | 16.501 | 14.637 | -7.133 | 1.00 | 16.43 | O |
| ATOM 306 | N | THR A 73 | 17.347 | 12.597 | -6.679 | 1.00 | 18.47 | N |
| ATOM 307 | CA | THR A 73 | 17.654 | 12.272 | -8.075 | 1.00 | 20.83 | C |
| ATOM 308 | CB | THR A 73 | 18.125 | 10.806 | -8.218 | 1.00 | 20.62 | C |
| ATOM 309 | OG1 | THR A 73 | 19.179 | 10.534 | -7.285 | 1.00 | 16.43 | O |
| ATOM 310 | CG2 | THR A 73 | 18.617 | 10.550 | -9.632 | 1.00 | 22.06 | C |
| ATOM 311 | C | THR A 73 | 16.495 | 12.451 | -9.053 | 1.00 | 24.09 | C |
| ATOM 312 | O | THR A 73 | 16.659 | 13.062 | -10.117 | 1.00 | 25.21 | O |
| ATOM 313 | N | GLU A 74 | 15.334 | 11.908 | -8.687 | 1.00 | 25.04 | N |
| ATOM 314 | CA | GLU A 74 | 14.138 | 11.929 | -9.533 | 1.00 | 25.92 | C |
| ATOM 315 | CB | GLU A 74 | 13.236 | 10.750 | -9.159 | 1.00 | 26.06 | C |
| ATOM 316 | CG | GLU A 74 | 12.406 | 10.178 | -10.286 | 1.00 | 34.32 | C |
| ATOM 317 | CD | GLU A 74 | 13.256 | 9.541 | -11.370 | 1.00 | 38.16 | C |
| ATOM 318 | OE1 | GLU A 74 | 14.199 | 8.801 | -11.024 | 1.00 | 41.27 | O |
| ATOM 319 | OE2 | GLU A 74 | 12.975 | 9.771 | -12.568 | 1.00 | 40.53 | O |
| ATOM 320 | C | GLU A 74 | 13.333 | 13.217 | -9.440 | 1.00 | 26.08 | C |
| ATOM 321 | O | GLU A 74 | 12.262 | 13.321 | -10.025 | 1.00 | 24.26 | O |
| ATOM 322 | N | GLY A 75 | 13.840 | 14.194 | -8.701 | 1.00 | 26.42 | N |
| ATOM 323 | CA | GLY A 75 | 13.117 | 15.445 | -8.570 | 1.00 | 22.94 | C |
| ATOM 324 | C | GLY A 75 | 12.382 | 15.554 | -7.247 | 1.00 | 23.36 | C |
| ATOM 325 | O | GLY A 75 | 11.727 | 16.566 | -6.979 | 1.00 | 23.95 | O |
| ATOM 326 | N | GLY A 76 | 12.478 | 14.509 | -6.427 | 1.00 | 21.05 | N |
| ATOM 327 | CA | GLY A 76 | 11.831 | 14.516 | -5.123 | 1.00 | 19.01 | C |
| ATOM 328 | C | GLY A 76 | 12.760 | 15.147 | -4.095 | 1.00 | 18.58 | C |
| ATOM 329 | O | GLY A 76 | 13.564 | 16.004 | -4.450 | 1.00 | 18.03 | O |
| ATOM 330 | N | LEU A 77 | 12.672 | 14.723 | -2.834 | 1.00 | 17.32 | N |
| ATOM 331 | CA | LEU A 77 | 13.518 | 15.288 | -1.789 | 1.00 | 16.39 | C |
| ATOM 332 | CB | LEU A 77 | 12.663 | 16.094 | -0.804 | 1.00 | 17.26 | C |
| ATOM 333 | CG | LEU A 77 | 11.836 | 17.252 | -1.368 | 1.00 | 18.75 | C |
| ATOM 334 | CD1 | LEU A 77 | 10.833 | 17.685 | -0.329 | 1.00 | 21.30 | C |
| ATOM 335 | CD2 | LEU A 77 | 12.741 | 18.423 | -1.794 | 1.00 | 17.34 | C |
| ATOM 336 | C | LEU A 77 | 14.354 | 14.293 | -0.987 | 1.00 | 17.25 | C |
| ATOM 337 | O | LEU A 77 | 13.886 | 13.223 | -0.602 | 1.00 | 17.86 | O |
| ATOM 338 | N | ASN A 78 | 15.603 | 14.676 | -0.741 | 1.00 | 16.40 | N |
| ATOM 339 | CA | ASN A 78 | 16.521 | 13.884 | 0.062 | 1.00 | 16.85 | C |
| ATOM 340 | CB | ASN A 78 | 17.966 | 14.049 | -0.421 | 1.00 | 17.82 | C |
| ATOM 341 | CG | ASN A 78 | 18.362 | 13.029 | -1.456 | 1.00 | 16.18 | C |
| ATOM 342 | OD1 | ASN A 78 | 17.533 | 12.293 | -1.972 | 1.00 | 14.36 | O |
| ATOM 343 | ND2 | ASN A 78 | 19.653 | 12.982 | -1.767 | 1.00 | 16.85 | N |
| ATOM 344 | C | ASN A 78 | 16.401 | 14.508 | 1.444 | 1.00 | 18.05 | C |
| ATOM 345 | O | ASN A 78 | 16.472 | 15.730 | 1.573 | 1.00 | 17.02 | O |
| ATOM 346 | N | LEU A 79 | 16.202 | 13.682 | 2.463 | 1.00 | 17.20 | N |
| ATOM 347 | CA | LEU A 79 | 16.096 | 14.161 | 3.829 | 1.00 | 17.68 | C |
| ATOM 348 | CB | LEU A 79 | 14.717 | 13.826 | 4.427 | 1.00 | 19.58 | C |
| ATOM 349 | CG | LEU A 79 | 13.572 | 14.852 | 4.291 | 1.00 | 19.99 | C |
| ATOM 350 | CD1 | LEU A 79 | 13.158 | 15.002 | 2.851 | 1.00 | 17.35 | C |
| ATOM 351 | CD2 | LEU A 79 | 12.386 | 14.413 | 5.126 | 1.00 | 21.80 | C |
| ATOM 352 | C | LEU A 79 | 17.196 | 13.480 | 4.642 | 1.00 | 18.93 | C |
| ATOM 353 | O | LEU A 79 | 17.177 | 12.264 | 4.833 | 1.00 | 17.64 | O |
| ATOM 354 | N | THR A 80 | 18.167 | 14.271 | 5.096 | 1.00 | 18.20 | N |
| ATOM 355 | CA | THR A 80 | 19.265 | 13.749 | 5.896 | 1.00 | 18.43 | C |
| ATOM 356 | CB | THR A 80 | 20.616 | 14.317 | 5.406 | 1.00 | 17.16 | C |
| ATOM 357 | OG1 | THR A 80 | 20.805 | 13.949 | 4.029 | 1.00 | 17.30 | O |
| ATOM 358 | CG2 | THR A 80 | 21.772 | 13.765 | 6.241 | 1.00 | 17.81 | C |
| ATOM 359 | C | THR A 80 | 18.987 | 14.174 | 7.327 | 1.00 | 19.48 | C |
| ATOM 360 | O | THR A 80 | 19.005 | 15.360 | 7.649 | 1.00 | 19.03 | O |
| ATOM 361 | N | SER A 81 | 18.698 | 13.201 | 8.181 | 1.00 | 19.91 | N |
| ATOM 362 | CA | SER A 81 | 18.383 | 13.489 | 9.569 | 1.00 | 22.53 | C |
| ATOM 363 | CB | SER A 81 | 17.027 | 12.867 | 9.945 | 1.00 | 23.42 | C |
| ATOM 364 | OG | SER A 81 | 15.958 | 13.406 | 9.166 | 1.00 | 25.72 | O |
| ATOM 365 | C | SER A 81 | 19.443 | 13.015 | 10.554 | 1.00 | 24.28 | C |
| ATOM 366 | O | SER A 81 | 20.011 | 11.920 | 10.425 | 1.00 | 25.45 | O |
| ATOM 367 | N | THR A 82 | 19.703 | 13.870 | 11.532 | 1.00 | 25.36 | N |
| ATOM 368 | CA | THR A 82 | 20.655 | 13.602 | 12.596 | 1.00 | 27.24 | C |
| ATOM 369 | CB | THR A 82 | 21.618 | 14.791 | 12.787 | 1.00 | 26.98 | C |
| ATOM 370 | OG1 | THR A 82 | 22.329 | 15.034 | 11.566 | 1.00 | 30.38 | O |
| ATOM 371 | CG2 | THR A 82 | 22.616 | 14.501 | 13.905 | 1.00 | 27.31 | C |
| ATOM 372 | C | THR A 82 | 19.779 | 13.460 | 13.840 | 1.00 | 28.34 | C |
| ATOM 373 | O | THR A 82 | 19.031 | 14.381 | 14.176 | 1.00 | 28.53 | O |
| ATOM 374 | N | PHE A 83 | 19.866 | 12.317 | 14.519 | 1.00 | 29.40 | N |
| ATOM 375 | CA | PHE A 83 | 19.043 | 12.076 | 15.700 | 1.00 | 31.45 | C |
| ATOM 376 | CB | PHE A 83 | 17.748 | 11.382 | 15.279 | 1.00 | 32.44 | C |
| ATOM 377 | CG | PHE A 83 | 17.968 | 10.118 | 14.493 | 1.00 | 33.67 | C |
| ATOM 378 | CD1 | PHE A 83 | 18.310 | 10.171 | 13.146 | 1.00 | 32.21 | C |
| ATOM 379 | CE1 | PHE A 83 | 18.532 | 9.005 | 12.418 | 1.00 | 35.94 | C |
| ATOM 380 | CZ | PHE A 83 | 18.415 | 7.766 | 13.037 | 1.00 | 35.35 | C |
| ATOM 381 | CE2 | PHE A 83 | 18.075 | 7.699 | 14.382 | 1.00 | 37.16 | C |
| ATOM 382 | CD2 | PHE A 83 | 17.853 | 8.873 | 15.104 | 1.00 | 33.77 | C |
| ATOM 383 | C | PHE A 83 | 19.715 | 11.249 | 16.797 | 1.00 | 33.26 | C |
| ATOM 384 | O | PHE A 83 | 20.721 | 10.571 | 16.569 | 1.00 | 33.27 | O |
| ATOM 385 | N | LEU A 84 | 19.137 | 11.315 | 17.991 | 1.00 | 35.25 | N |
| ATOM 386 | CA | LEU A 84 | 19.633 | 10.578 | 19.148 | 1.00 | 37.42 | C |
| ATOM 387 | CB | LEU A 84 | 19.424 | 11.413 | 20.415 | 1.00 | 37.09 | C |
| ATOM 388 | CG | LEU A 84 | 19.882 | 10.858 | 21.769 | 1.00 | 39.37 | C |
| ATOM 389 | CD1 | LEU A 84 | 21.401 | 10.658 | 21.774 | 1.00 | 37.77 | C |
| ATOM 390 | CD2 | LEU A 84 | 19.467 | 11.824 | 22.870 | 1.00 | 39.73 | C |
| ATOM 391 | C | LEU A 84 | 18.836 | 9.272 | 19.226 | 1.00 | 39.36 | C |
| ATOM 392 | O | LEU A 84 | 17.620 | 9.292 | 19.415 | 1.00 | 38.00 | O |
| ATOM 393 | N | ARG A 85 | 19.524 | 8.145 | 19.062 | 1.00 | 42.28 | N |
| ATOM 394 | CA | ARG A 85 | 18.887 | 6.828 | 19.092 | 1.00 | 46.17 | C |
| ATOM 395 | CB | ARG A 85 | 19.617 | 5.895 | 18.124 | 1.00 | 47.06 | C |
| ATOM 396 | CG | ARG A 85 | 19.280 | 4.412 | 18.240 | 1.00 | 50.03 | C |
| ATOM 397 | CD | ARG A 85 | 17.975 | 4.035 | 17.555 | 1.00 | 54.72 | C |
| ATOM 398 | NE | ARG A 85 | 18.016 | 2.648 | 17.088 | 1.00 | 59.25 | N |
| ATOM 399 | CZ | ARG A 85 | 16.987 | 1.994 | 16.553 | 1.00 | 60.78 | C |
| ATOM 400 | NH1 | ARG A 85 | 15.812 | 2.596 | 16.410 | 1.00 | 62.71 | N |
| ATOM 401 | NH2 | ARG A 85 | 17.135 | 0.736 | 16.154 | 1.00 | 59.48 | N |
| ATOM 402 | C | ARG A 85 | 18.894 | 6.224 | 20.493 | 1.00 | 48.27 | C |
| ATOM 403 | O | ARG A 85 | 17.842 | 5.982 | 21.096 | 1.00 | 49.91 | O |
| ATOM 404 | N | LYS A 86 | 20.094 | 5.966 | 20.999 | 1.00 | 49.22 | N |
| ATOM 405 | CA | LYS A 86 | 20.266 | 5.397 | 22.329 | 1.00 | 50.08 | C |
| ATOM 406 | CB | LYS A 86 | 20.385 | 3.873 | 22.242 | 1.00 | 50.26 | C |
| ATOM 407 | CG | LYS A 86 | 19.188 | 3.195 | 21.587 | 1.00 | 52.36 | C |
| ATOM 408 | CD | LYS A 86 | 19.420 | 1.702 | 21.391 | 1.00 | 54.20 | C |
| ATOM 409 | CE | LYS A 86 | 18.224 | 1.049 | 20.709 | 1.00 | 56.17 | C |
| ATOM 410 | NZ | LYS A 86 | 18.420 | -0.410 | 20.487 | 1.00 | 57.21 | N |
| ATOM 411 | C | LYS A 86 | 21.550 | 5.991 | 22.882 | 1.00 | 50.00 | C |
| ATOM 412 | O | LYS A 86 | 22.583 | 5.323 | 22.939 | 1.00 | 49.77 | O |
| ATOM 413 | N | ASN A 87 | 21.478 | 7.260 | 23.273 | 1.00 | 50.54 | N |
| ATOM 414 | CA | ASN A 87 | 22.632 | 7.974 | 23.800 | 1.00 | 50.72 | C |
| ATOM 415 | CB | ASN A 87 | 23.159 | 7.284 | 25.063 | 1.00 | 51.47 | C |
| ATOM 416 | CG | ASN A 87 | 22.168 | 7.337 | 26.219 | 1.00 | 52.54 | C |
| ATOM 417 | OD1 | ASN A 87 | 21.041 | 6.841 | 26.118 | 1.00 | 53.17 | O |
| ATOM 418 | ND2 | ASN A 87 | 22.586 | 7.940 | 27.325 | 1.00 | 53.77 | N |
| ATOM 419 | C | ASN A 87 | 23.722 | 8.028 | 22.735 | 1.00 | 50.39 | C |
| ATOM 420 | O | ASN A 87 | 24.910 | 8.064 | 23.047 | 1.00 | 50.96 | O |
| ATOM 421 | N | GLN A 88 | 23.303 | 8.032 | 21.474 | 1.00 | 49.81 | N |
| ATOM 422 | CA | GLN A 88 | 24.235 | 8.088 | 20.355 | 1.00 | 49.12 | C |
| ATOM 423 | CB | GLN A 88 | 24.552 | 6.676 | 19.859 | 1.00 | 49.66 | C |
| ATOM 424 | CG | GLN A 88 | 25.641 | 6.620 | 18.804 | 1.00 | 52.17 | C |
| ATOM 425 | CD | GLN A 88 | 25.868 | 5.217 | 18.273 | 1.00 | 54.04 | C |
| ATOM 426 | OE1 | GLN A 88 | 25.080 | 4.704 | 17.470 | 1.00 | 55.11 | O |
| ATOM 427 | NE2 | GLN A 88 | 26.943 | 4.582 | 18.729 | 1.00 | 53.14 | N |
| ATOM 428 | C | GLN A 88 | 23.640 | 8.901 | 19.212 | 1.00 | 47.52 | C |
| ATOM 429 | O | GLN A 88 | 22.445 | 8.819 | 18.935 | 1.00 | 46.71 | O |
| ATOM 430 | N | CYS A 89 | 24.482 | 9.690 | 18.554 | 1.00 | 46.02 | N |
| ATOM 431 | CA | CYS A 89 | 24.038 | 10.504 | 17.435 | 1.00 | 44.64 | C |
| ATOM 432 | CB | CYS A 89 | 24.929 | 11.736 | 17.271 | 1.00 | 45.12 | C |
| ATOM 433 | SG | CYS A 89 | 24.884 | 12.871 | 18.685 | 1.00 | 48.12 | S |
| ATOM 434 | C | CYS A 89 | 24.084 | 9.684 | 16.158 | 1.00 | 42.99 | C |
| ATOM 435 | O | CYS A 89 | 25.144 | 9.207 | 15.750 | 1.00 | 42.55 | O |
| ATOM 436 | N | GLU A 90 | 22.928 | 9.515 | 15.531 | 1.00 | 39.97 | N |
| ATOM 437 | CA | GLU A 90 | 22.856 | 8.763 | 14.295 | 1.00 | 38.16 | C |
| ATOM 438 | CB | GLU A 90 | 21.899 | 7.582 | 14.450 | 1.00 | 38.06 | C |
| ATOM 439 | CG | GLU A 90 | 22.573 | 6.322 | 14.964 | 1.00 | 42.17 | C |
| ATOM 440 | CD | GLU A 90 | 21.585 | 5.227 | 15.312 | 1.00 | 46.43 | C |
| ATOM 441 | OE1 | GLU A 90 | 20.657 | 4.981 | 14.511 | 1.00 | 46.83 | O |
| ATOM 442 | OE2 | GLU A 90 | 21.745 | 4.604 | 16.389 | 1.00 | 50.51 | O |
| ATOM 443 | C | GLU A 90 | 22.410 | 9.657 | 13.157 | 1.00 | 36.50 | C |
| ATOM 444 | O | GLU A 90 | 21.854 | 10.729 | 13.372 | 1.00 | 35.05 | O |
| ATOM 445 | N | THR A 91 | 22.678 | 9.214 | 11.940 | 1.00 | 34.79 | N |
| ATOM 446 | CA | THR A 91 | 22.295 | 9.957 | 10.761 | 1.00 | 34.31 | C |
| ATOM 447 | CB | THR A 91 | 23.500 | 10.641 | 10.100 | 1.00 | 34.34 | C |
| ATOM 448 | OG1 | THR A 91 | 23.799 | 11.852 | 10.805 | 1.00 | 30.72 | O |
| ATOM 449 | CG2 | THR A 91 | 23.202 | 10.958 | 8.642 | 1.00 | 33.63 | C |
| ATOM 450 | C | THR A 91 | 21.668 | 8.991 | 9.787 | 1.00 | 34.16 | C |
| ATOM 451 | O | THR A 91 | 22.191 | 7.907 | 9.545 | 1.00 | 35.05 | O |
| ATOM 452 | N | LYS A 92 | 20.530 | 9.393 | 9.240 | 1.00 | 33.69 | N |
| ATOM 453 | CA | LYS A 92 | 19.810 | 8.571 | 8.289 | 1.00 | 33.19 | C |
| ATOM 454 | CB | LYS A 92 | 18.573 | 7.967 | 8.963 | 1.00 | 34.05 | C |
| ATOM 455 | CG | LYS A 92 | 17.636 | 7.235 | 8.023 | 1.00 | 38.78 | C |
| ATOM 456 | CD | LYS A 92 | 16.512 | 6.537 | 8.781 | 1.00 | 46.23 | C |
| ATOM 457 | CE | LYS A 92 | 15.775 | 5.552 | 7.877 | 1.00 | 48.21 | C |
| ATOM 458 | NZ | LYS A 92 | 14.772 | 4.739 | 8.630 | 1.00 | 51.87 | N |
| ATOM 459 | C | LYS A 92 | 19.395 | 9.419 | 7.094 | 1.00 | 31.39 | C |
| ATOM 460 | O | LYS A 92 | 19.155 | 10.619 | 7.224 | 1.00 | 30.85 | O |
| ATOM 461 | N | ILE A 93 | 19.323 | 8.786 | 5.930 | 1.00 | 29.87 | N |
| ATOM 462 | CA | ILE A 93 | 18.924 | 9.466 | 4.714 | 1.00 | 30.20 | C |
| ATOM 463 | CB | ILE A 93 | 20.005 | 9.352 | 3.620 | 1.00 | 29.62 | C |
| ATOM 464 | CG1 | ILE A 93 | 21.270 | 10.100 | 4.057 | 1.00 | 29.97 | C |
| ATOM 465 | CD1 | ILE A 93 | 22.423 | 9.998 | 3.066 | 1.00 | 34.73 | C |
| ATOM 466 | CG2 | ILE A 93 | 19.470 | 9.910 | 2.304 | 1.00 | 32.79 | C |
| ATOM 467 | C | ILE A 93 | 17.635 | 8.853 | 4.190 | 1.00 | 30.28 | C |
| ATOM 468 | O | ILE A 93 | 17.567 | 7.647 | 3.943 | 1.00 | 31.07 | O |
| ATOM 469 | N | MET A 94 | 16.609 | 9.686 | 4.049 | 1.00 | 28.71 | N |
| ATOM 470 | CA | MET A 94 | 15.320 | 9.242 | 3.531 | 1.00 | 28.72 | C |
| ATOM 471 | CB | MET A 94 | 14.180 | 9.605 | 4.482 | 1.00 | 30.09 | C |
| ATOM 472 | CG | MET A 94 | 14.264 | 8.983 | 5.854 | 1.00 | 34.64 | C |
| ATOM 473 | SD | MET A 94 | 12.774 | 9.336 | 6.815 | 1.00 | 47.69 | S |
| ATOM 474 | CE | MET A 94 | 13.206 | 10.852 | 7.645 | 1.00 | 42.95 | C |
| ATOM 475 | C | MET A 94 | 15.077 | 9.934 | 2.198 | 1.00 | 25.85 | C |
| ATOM 476 | O | MET A 94 | 15.382 | 11.111 | 2.028 | 1.00 | 25.26 | O |
| ATOM 477 | N | VAL A 95 | 14.523 | 9.192 | 1.254 | 1.00 | 24.35 | N |
| ATOM 478 | CA | VAL A 95 | 14.234 | 9.727 | -0.067 | 1.00 | 22.51 | C |
| ATOM 479 | CB | VAL A 95 | 14.830 | 8.842 | -1.183 | 1.00 | 22.28 | C |
| ATOM 480 | CG1 | VAL A 95 | 14.286 | 9.285 | -2.523 | 1.00 | 22.94 | C |
| ATOM 481 | CG2 | VAL A 95 | 16.355 | 8.923 | -1.171 | 1.00 | 24.47 | C |
| ATOM 482 | C | VAL A 95 | 12.737 | 9.812 | -0.301 | 1.00 | 21.24 | C |
| ATOM 483 | O | VAL A 95 | 12.052 | 8.791 | -0.331 | 1.00 | 21.21 | O |
| ATOM 484 | N | LEU A 96 | 12.232 | 11.033 | -0.436 | 1.00 | 19.49 | N |
| ATOM 485 | CA | LEU A 96 | 10.825 | 11.245 | -0.729 | 1.00 | 21.14 | C |
| ATOM 486 | CB | LEU A 96 | 10.307 | 12.537 | -0.088 | 1.00 | 19.27 | C |
| ATOM 487 | CG | LEU A 96 | 10.224 | 12.600 | 1.444 | 1.00 | 25.73 | C |
| ATOM 488 | CD1 | LEU A 96 | 9.644 | 13.948 | 1.877 | 1.00 | 26.81 | C |
| ATOM 489 | CD2 | LEU A 96 | 9.366 | 11.463 | 1.966 | 1.00 | 24.23 | C |
| ATOM 490 | C | LEU A 96 | 10.728 | 11.344 | -2.252 | 1.00 | 21.23 | C |
| ATOM 491 | O | LEU A 96 | 11.129 | 12.355 | -2.849 | 1.00 | 21.09 | O |
| ATOM 492 | N | GLN A 97 | 10.214 | 10.282 | -2.873 | 1.00 | 20.36 | N |
| ATOM 493 | CA | GLN A 97 | 10.063 | 10.223 | -4.321 | 1.00 | 22.02 | C |
| ATOM 494 | CB | GLN A 97 | 9.916 | 8.771 | -4.790 | 1.00 | 23.57 | C |
| ATOM 495 | CG | GLN A 97 | 11.029 | 7.823 | -4.391 | 1.00 | 24.69 | C |
| ATOM 496 | CD | GLN A 97 | 12.313 | 8.111 | -5.120 | 1.00 | 28.37 | C |
| ATOM 497 | OE1 | GLN A 97 | 12.318 | 8.775 | -6.155 | 1.00 | 28.08 | O |
| ATOM 498 | NE2 | GLN A 97 | 13.414 | 7.601 | -4.592 | 1.00 | 31.31 | N |
| ATOM 499 | C | GLN A 97 | 8.821 | 10.986 | -4.758 | 1.00 | 21.48 | C |
| ATOM 500 | O | GLN A 97 | 7.757 | 10.836 | -4.164 | 1.00 | 21.39 | O |
| ATOM 501 | N | PRO A 98 | 8.936 | 11.805 | -5.815 | 1.00 | 22.84 | N |
| ATOM 502 | CA | PRO A 98 | 7.764 | 12.554 | -6.273 | 1.00 | 23.56 | C |
| ATOM 503 | CB | PRO A 98 | 8.269 | 13.252 | -7.540 | 1.00 | 22.35 | C |
| ATOM 504 | CG | PRO A 98 | 9.343 | 12.347 | -8.022 | 1.00 | 24.97 | C |
| ATOM 505 | CD | PRO A 98 | 10.057 | 11.974 | -6.749 | 1.00 | 20.37 | C |
| ATOM 506 | C | PRO A 98 | 6.621 | 11.585 | -6.543 | 1.00 | 25.12 | C |
| ATOM 507 | O | PRO A 98 | 6.847 | 10.473 | -7.029 | 1.00 | 25.87 | O |
| ATOM 508 | N | ALA A 99 | 5.401 | 12.001 | -6.219 | 1.00 | 24.77 | N |
| ATOM 509 | CA | ALA A 99 | 4.232 | 11.151 | -6.410 | 1.00 | 26.15 | C |
| ATOM 510 | CB | ALA A 99 | 3.638 | 10.799 | -5.053 | 1.00 | 24.62 | C |
| ATOM 511 | C | ALA A 99 | 3.149 | 11.739 | -7.323 | 1.00 | 27.20 | C |
| ATOM 512 | O | ALA A 99 | 1.973 | 11.766 | -6.968 | 1.00 | 29.29 | O |
| ATOM 513 | N | GLY A 100 | 3.547 | 12.223 | -8.491 | 1.00 | 29.32 | N |
| ATOM 514 | CA | GLY A 100 | 2.574 | 12.756 | -9.435 | 1.00 | 30.75 | C |
| ATOM 515 | C | GLY A 100 | 2.006 | 14.156 | -9.255 | 1.00 | 30.83 | C |
| ATOM 516 | O | GLY A 100 | 1.253 | 14.621 | -10.118 | 1.00 | 32.00 | O |
| ATOM 517 | N | ALA A 101 | 2.337 | 14.832 | -8.158 | 1.00 | 27.81 | N |
| ATOM 518 | CA | ALA A 101 | 1.832 | 16.185 | -7.931 | 1.00 | 27.20 | C |
| ATOM 519 | CB | ALA A 101 | 0.371 | 16.134 | -7.475 | 1.00 | 27.02 | C |
| ATOM 520 | C | ALA A 101 | 2.675 | 16.937 | -6.904 | 1.00 | 25.78 | C |
| ATOM 521 | O | ALA A 101 | 3.185 | 16.348 | -5.947 | 1.00 | 25.96 | O |
| ATOM 522 | N | PRO A 102 | 2.818 | 18.260 | -7.082 | 1.00 | 26.56 | N |
| ATOM 523 | CA | PRO A 102 | 3.615 | 19.056 | -6.146 | 1.00 | 25.86 | C |
| ATOM 524 | CB | PRO A 102 | 3.415 | 20.487 | -6.650 | 1.00 | 27.20 | C |
| ATOM 525 | CG | PRO A 102 | 3.201 | 20.290 | -8.120 | 1.00 | 29.43 | C |
| ATOM 526 | CD | PRO A 102 | 2.255 | 19.118 | -8.144 | 1.00 | 25.60 | C |
| ATOM 527 | C | PRO A 102 | 3.164 | 18.880 | -4.703 | 1.00 | 24.43 | C |
| ATOM 528 | O | PRO A 102 | 1.986 | 19.050 | -4.386 | 1.00 | 25.38 | O |
| ATOM 529 | N | GLY A 103 | 4.102 | 18.526 | -3.831 | 1.00 | 22.57 | N |
| ATOM 530 | CA | GLY A 103 | 3.764 | 18.351 | -2.429 | 1.00 | 21.83 | C |
| ATOM 531 | C | GLY A 103 | 3.324 | 16.944 | -2.063 | 1.00 | 19.05 | C |
| ATOM 532 | O | GLY A 103 | 2.941 | 16.685 | -0.920 | 1.00 | 20.94 | O |
| ATOM 533 | N | HIS A 104 | 3.393 | 16.034 | -3.028 | 1.00 | 18.00 | N |
| ATOM 534 | CA | HIS A 104 | 3.016 | 14.644 | -2.811 | 1.00 | 18.06 | C |
| ATOM 535 | CB | HIS A 104 | 1.845 | 14.277 | -3.719 | 1.00 | 17.03 | C |
| ATOM 536 | CG | HIS A 104 | 0.598 | 15.044 | -3.417 | 1.00 | 21.85 | C |
| ATOM 537 | ND1 | HIS A 104 | -0.398 | 14.555 | -2.599 | 1.00 | 19.71 | N |
| ATOM 538 | CE1 | HIS A 104 | -1.329 | 15.478 | -2.450 | 1.00 | 22.98 | C |
| ATOM 539 | NE2 | HIS A 104 | -0.977 | 16.546 | -3.144 | 1.00 | 16.26 | N |
| ATOM 540 | CD2 | HIS A 104 | 0.221 | 16.299 | -3.761 | 1.00 | 18.82 | C |
| ATOM 541 | C | HIS A 104 | 4.205 | 13.741 | -3.094 | 1.00 | 16.96 | C |
| ATOM 542 | O | HIS A 104 | 4.812 | 13.815 | -4.154 | 1.00 | 15.44 | O |
| ATOM 543 | N | TYR A 105 | 4.521 | 12.873 | -2.143 | 1.00 | 18.87 | N |
| ATOM 544 | CA | TYR A 105 | 5.662 | 11.982 | -2.284 | 1.00 | 18.50 | C |
| ATOM 545 | CB | TYR A 105 | 6.836 | 12.516 | -1.456 | 1.00 | 20.43 | C |
| ATOM 546 | CG | TYR A 105 | 7.115 | 13.996 | -1.628 | 1.00 | 20.21 | C |
| ATOM 547 | CD1 | TYR A 105 | 7.957 | 14.453 | -2.645 | 1.00 | 18.18 | C |
| ATOM 548 | CE1 | TYR A 105 | 8.200 | 15.810 | -2.821 | 1.00 | 20.31 | C |
| ATOM 549 | CZ | TYR A 105 | 7.599 | 16.732 | -1.975 | 1.00 | 20.71 | C |
| ATOM 550 | OH | TYR A 105 | 7.845 | 18.072 | -2.157 | 1.00 | 25.46 | O |
| ATOM 551 | CE2 | TYR A 105 | 6.756 | 16.310 | -0.952 | 1.00 | 18.08 | C |
| ATOM 552 | CD2 | TYR A 105 | 6.521 | 14.941 | -0.784 | 1.00 | 19.32 | C |
| ATOM 553 | C | TYR A 105 | 5.349 | 10.579 | -1.785 | 1.00 | 20.66 | C |
| ATOM 554 | O | TYR A 105 | 4.355 | 10.350 | -1.101 | 1.00 | 21.05 | O |
| ATOM 555 | N | THR A 106 | 6.220 | 9.643 | -2.136 | 1.00 | 21.73 | N |
| ATOM 556 | CA | THR A 106 | 6.110 | 8.274 | -1.666 | 1.00 | 25.27 | C |
| ATOM 557 | CB | THR A 106 | 5.940 | 7.271 | -2.825 | 1.00 | 24.65 | C |
| ATOM 558 | OG1 | THR A 106 | 7.009 | 7.427 | -3.768 | 1.00 | 27.25 | O |
| ATOM 559 | CG2 | THR A 106 | 4.603 | 7.503 | -3.531 | 1.00 | 26.01 | C |
| ATOM 560 | C | THR A 106 | 7.425 | 8.017 | -0.932 | 1.00 | 26.95 | C |
| ATOM 561 | O | THR A 106 | 8.470 | 8.556 | -1.304 | 1.00 | 25.88 | O |
| ATOM 562 | N | TYR A 107 | 7.375 | 7.209 | 0.118 | 1.00 | 30.17 | N |
| ATOM 563 | CA | TYR A 107 | 8.570 | 6.916 | 0.896 | 1.00 | 33.45 | C |
| ATOM 564 | CB | TYR A 107 | 8.648 | 7.896 | 2.077 | 1.00 | 35.24 | C |
| ATOM 565 | CG | TYR A 107 | 8.918 | 7.296 | 3.437 | 1.00 | 39.36 | C |
| ATOM 566 | CD1 | TYR A 107 | 10.146 | 6.707 | 3.736 | 1.00 | 43.99 | C |
| ATOM 567 | CE1 | TYR A 107 | 10.401 | 6.182 | 4.999 | 1.00 | 47.50 | C |
| ATOM 568 | CZ | TYR A 107 | 9.415 | 6.243 | 5.974 | 1.00 | 47.29 | C |
| ATOM 569 | OH | TYR A 107 | 9.645 | 5.718 | 7.230 | 1.00 | 48.91 | O |
| ATOM 570 | CE2 | TYR A 107 | 8.190 | 6.823 | 5.693 | 1.00 | 44.53 | C |
| ATOM 571 | CD2 | TYR A 107 | 7.949 | 7.342 | 4.434 | 1.00 | 43.15 | C |
| ATOM 572 | C | TYR A 107 | 8.642 | 5.478 | 1.379 | 1.00 | 35.14 | C |
| ATOM 573 | O | TYR A 107 | 7.682 | 4.943 | 1.924 | 1.00 | 35.69 | O |
| ATOM 574 | N | SER A 108 | 9.800 | 4.864 | 1.172 | 1.00 | 37.81 | N |
| ATOM 575 | CA | SER A 108 | 10.041 | 3.490 | 1.592 | 1.00 | 41.97 | C |
| ATOM 576 | CB | SER A 108 | 11.115 | 2.855 | 0.711 | 1.00 | 41.43 | C |
| ATOM 577 | OG | SER A 108 | 11.572 | 1.645 | 1.282 | 1.00 | 44.65 | O |
| ATOM 578 | C | SER A 108 | 10.500 | 3.455 | 3.047 | 1.00 | 43.76 | C |
| ATOM 579 | O | SER A 108 | 11.583 | 3.940 | 3.372 | 1.00 | 45.52 | O |
| ATOM 580 | N | SER A 109 | 9.684 | 2.868 | 3.918 | 1.00 | 46.53 | N |
| ATOM 581 | CA | SER A 109 | 10.017 | 2.794 | 5.337 | 1.00 | 47.99 | C |
| ATOM 582 | CB | SER A 109 | 8.740 | 2.876 | 6.173 | 1.00 | 48.18 | C |
| ATOM 583 | OG | SER A 109 | 9.052 | 2.943 | 7.552 | 1.00 | 48.31 | O |
| ATOM 584 | C | SER A 109 | 10.799 | 1.536 | 5.720 | 1.00 | 49.24 | C |
| ATOM 585 | O | SER A 109 | 10.870 | 0.568 | 4.957 | 1.00 | 51.51 | O |
| ATOM 586 | N | SER A 112 | 9.886 | -0.863 | 8.643 | 1.00 | 59.10 | N |
| ATOM 587 | CA | SER A 112 | 8.546 | -1.374 | 8.383 | 1.00 | 58.71 | C |
| ATOM 588 | CB | SER A 112 | 7.531 | -0.229 | 8.378 | 1.00 | 59.10 | C |
| ATOM 589 | OG | SER A 112 | 6.241 | -0.693 | 8.014 | 1.00 | 60.32 | O |
| ATOM 590 | C | SER A 112 | 8.486 | -2.096 | 7.046 | 1.00 | 57.85 | C |
| ATOM 591 | O | SER A 112 | 7.722 | -3.046 | 6.879 | 1.00 | 58.40 | O |
| ATOM 592 | N | GLY A 113 | 9.295 | -1.639 | 6.096 | 1.00 | 56.85 | N |
| ATOM 593 | CA | GLY A 113 | 9.301 | -2.245 | 4.777 | 1.00 | 55.00 | C |
| ATOM 594 | C | GLY A 113 | 8.170 | -1.691 | 3.930 | 1.00 | 52.98 | C |
| ATOM 595 | O | GLY A 113 | 8.254 | -1.656 | 2.703 | 1.00 | 53.44 | O |
| ATOM 596 | N | SER A 114 | 7.106 | -1.257 | 4.595 | 1.00 | 50.93 | N |
| ATOM 597 | CA | SER A 114 | 5.940 | -0.691 | 3.929 | 1.00 | 49.06 | C |
| ATOM 598 | CB | SER A 114 | 4.834 | -0.441 | 4.955 | 1.00 | 49.17 | C |
| ATOM 599 | OG | SER A 114 | 5.283 | 0.435 | 5.980 | 1.00 | 50.92 | O |
| ATOM 600 | C | SER A 114 | 6.268 | 0.620 | 3.222 | 1.00 | 46.96 | C |
| ATOM 601 | O | SER A 114 | 7.323 | 1.211 | 3.443 | 1.00 | 47.26 | O |
| ATOM 602 | N | ILE A 115 | 5.355 | 1.062 | 2.364 | 1.00 | 44.72 | N |
| ATOM 603 | CA | ILE A 115 | 5.508 | 2.315 | 1.636 | 1.00 | 42.22 | C |
| ATOM 604 | CB | ILE A 115 | 5.204 | 2.141 | 0.129 | 1.00 | 43.03 | C |
| ATOM 605 | CG1 | ILE A 115 | 6.307 | 1.316 | -0.535 | 1.00 | 44.28 | C |
| ATOM 606 | CD1 | ILE A 115 | 7.674 | 1.972 | -0.491 | 1.00 | 47.16 | C |
| ATOM 607 | CG2 | ILE A 115 | 5.102 | 3.502 | -0.552 | 1.00 | 43.16 | C |
| ATOM 608 | C | ILE A 115 | 4.518 | 3.314 | 2.223 | 1.00 | 40.03 | C |
| ATOM 609 | O | ILE A 115 | 3.449 | 2.932 | 2.705 | 1.00 | 39.69 | O |
| ATOM 610 | N | HIS A 116 | 4.877 | 4.591 | 2.187 | 1.00 | 36.34 | N |
| ATOM 611 | CA | HIS A 116 | 4.011 | 5.630 | 2.718 | 1.00 | 34.27 | C |
| ATOM 612 | CB | HIS A 116 | 4.662 | 6.284 | 3.937 | 1.00 | 35.45 | C |
| ATOM 613 | CG | HIS A 116 | 4.902 | 5.340 | 5.072 | 1.00 | 38.20 | C |
| ATOM 614 | ND1 | HIS A 116 | 5.654 | 5.683 | 6.176 | 1.00 | 41.13 | N |
| ATOM 615 | CE1 | HIS A 116 | 5.698 | 4.657 | 7.008 | 1.00 | 45.36 | C |
| ATOM 616 | NE2 | HIS A 116 | 5.001 | 3.664 | 6.485 | 1.00 | 40.45 | N |
| ATOM 617 | CD2 | HIS A 116 | 4.492 | 4.066 | 5.273 | 1.00 | 40.93 | C |
| ATOM 618 | C | HIS A 116 | 3.732 | 6.690 | 1.666 | 1.00 | 31.61 | C |
| ATOM 619 | O | HIS A 116 | 4.542 | 6.914 | 0.768 | 1.00 | 30.20 | O |
| ATOM 620 | N | SER A 117 | 2.572 | 7.325 | 1.781 | 1.00 | 28.75 | N |
| ATOM 621 | CA | SER A 117 | 2.176 | 8.387 | 0.872 | 1.00 | 28.44 | C |
| ATOM 622 | CB | SER A 117 | 0.785 | 8.119 | 0.302 | 1.00 | 26.28 | C |
| ATOM 623 | OG | SER A 117 | 0.829 | 7.005 | -0.566 | 1.00 | 32.61 | O |
| ATOM 624 | C | SER A 117 | 2.179 | 9.667 | 1.692 | 1.00 | 26.02 | C |
| ATOM 625 | O | SER A 117 | 1.323 | 9.860 | 2.556 | 1.00 | 26.95 | O |
| ATOM 626 | N | VAL A 118 | 3.148 | 10.534 | 1.419 | 1.00 | 24.58 | N |
| ATOM 627 | CA | VAL A 118 | 3.299 | 11.785 | 2.161 | 1.00 | 22.06 | C |
| ATOM 628 | CB | VAL A 118 | 4.796 | 12.044 | 2.497 | 1.00 | 23.75 | C |
| ATOM 629 | CG1 | VAL A 118 | 4.960 | 13.338 | 3.304 | 1.00 | 23.80 | C |
| ATOM 630 | CG2 | VAL A 118 | 5.349 | 10.867 | 3.279 | 1.00 | 25.01 | C |
| ATOM 631 | C | VAL A 118 | 2.760 | 12.991 | 1.413 | 1.00 | 21.13 | C |
| ATOM 632 | O | VAL A 118 | 3.000 | 13.152 | 0.224 | 1.00 | 21.25 | O |
| ATOM 633 | N | SER A 119 | 2.033 | 13.840 | 2.124 | 1.00 | 19.39 | N |
| ATOM 634 | CA | SER A 119 | 1.495 | 15.046 | 1.522 | 1.00 | 19.65 | C |
| ATOM 635 | CB | SER A 119 | -0.001 | 14.893 | 1.247 | 1.00 | 18.42 | C |
| ATOM 636 | OG | SER A 119 | -0.709 | 14.799 | 2.467 | 1.00 | 16.99 | O |
| ATOM 637 | C | SER A 119 | 1.720 | 16.222 | 2.471 | 1.00 | 20.44 | C |
| ATOM 638 | O | SER A 119 | 1.734 | 16.060 | 3.694 | 1.00 | 20.31 | O |
| ATOM 639 | N | VAL A 120 | 1.927 | 17.398 | 1.894 | 1.00 | 20.60 | N |
| ATOM 640 | CA | VAL A 120 | 2.111 | 18.605 | 2.680 | 1.00 | 20.92 | C |
| ATOM 641 | CB | VAL A 120 | 2.911 | 19.665 | 1.909 | 1.00 | 21.47 | C |
| ATOM 642 | CG1 | VAL A 120 | 3.008 | 20.934 | 2.747 | 1.00 | 21.53 | C |
| ATOM 643 | CG2 | VAL A 120 | 4.304 | 19.123 | 1.559 | 1.00 | 21.20 | C |
| ATOM 644 | C | VAL A 120 | 0.713 | 19.159 | 2.942 | 1.00 | 19.42 | C |
| ATOM 645 | O | VAL A 120 | 0.072 | 19.668 | 2.036 | 1.00 | 17.04 | O |
| ATOM 646 | N | VAL A 121 | 0.238 | 19.040 | 4.174 | 1.00 | 18.30 | N |
| ATOM 647 | CA | VAL A 121 | -1.090 | 19.542 | 4.519 | 1.00 | 17.84 | C |
| ATOM 648 | CB | VAL A 121 | -1.487 | 19.110 | 5.942 | 1.00 | 17.08 | C |
| ATOM 649 | CG1 | VAL A 121 | -2.886 | 19.650 | 6.297 | 1.00 | 15.35 | C |
| ATOM 650 | CG2 | VAL A 121 | -1.429 | 17.607 | 6.050 | 1.00 | 18.80 | C |
| ATOM 651 | C | VAL A 121 | -1.105 | 21.069 | 4.444 | 1.00 | 17.06 | C |
| ATOM 652 | O | VAL A 121 | -1.947 | 21.667 | 3.783 | 1.00 | 16.89 | O |
| ATOM 653 | N | GLU A 122 | -0.157 | 21.687 | 5.129 | 1.00 | 17.44 | N |
| ATOM 654 | CA | GLU A 122 | -0.048 | 23.139 | 5.152 | 1.00 | 19.43 | C |
| ATOM 655 | CB | GLU A 122 | -0.945 | 23.704 | 6.254 | 1.00 | 17.85 | C |
| ATOM 656 | CG | GLU A 122 | -0.988 | 25.207 | 6.322 | 1.00 | 21.33 | C |
| ATOM 657 | CD | GLU A 122 | -1.991 | 25.698 | 7.356 | 1.00 | 24.57 | C |
| ATOM 658 | OE1 | GLU A 122 | -1.598 | 26.454 | 8.275 | 1.00 | 28.32 | O |
| ATOM 659 | OE2 | GLU A 122 | -3.172 | 25.317 | 7.254 | 1.00 | 20.65 | O |
| ATOM 660 | C | GLU A 122 | 1.405 | 23.465 | 5.460 | 1.00 | 18.95 | C |
| ATOM 661 | O | GLU A 122 | 2.083 | 22.676 | 6.114 | 1.00 | 16.79 | O |
| ATOM 662 | N | ALA A 123 | 1.879 | 24.624 | 5.008 | 1.00 | 21.01 | N |
| ATOM 663 | CA | ALA A 123 | 3.257 | 25.003 | 5.280 | 1.00 | 20.93 | C |
| ATOM 664 | CB | ALA A 123 | 4.202 | 24.042 | 4.559 | 1.00 | 20.51 | C |
| ATOM 665 | C | ALA A 123 | 3.608 | 26.444 | 4.913 | 1.00 | 21.90 | C |
| ATOM 666 | O | ALA A 123 | 3.009 | 27.036 | 4.021 | 1.00 | 23.30 | O |
| ATOM 667 | N | ASN A 124 | 4.584 | 26.989 | 5.638 | 1.00 | 21.77 | N |
| ATOM 668 | CA | ASN A 124 | 5.126 | 28.333 | 5.433 | 1.00 | 20.98 | C |
| ATOM 669 | CB | ASN A 124 | 4.780 | 29.253 | 6.614 | 1.00 | 20.38 | C |
| ATOM 670 | CG | ASN A 124 | 5.392 | 30.644 | 6.477 | 1.00 | 22.16 | C |
| ATOM 671 | OD1 | ASN A 124 | 6.495 | 30.809 | 5.951 | 1.00 | 22.44 | O |
| ATOM 672 | ND2 | ASN A 124 | 4.683 | 31.650 | 6.971 | 1.00 | 26.85 | N |
| ATOM 673 | C | ASN A 124 | 6.621 | 28.048 | 5.422 | 1.00 | 20.13 | C |
| ATOM 674 | O | ASN A 124 | 7.211 | 27.813 | 6.466 | 1.00 | 19.09 | O |
| ATOM 675 | N | TYR A 125 | 7.230 | 28.070 | 4.244 | 1.00 | 20.81 | N |
| ATOM 676 | CA | TYR A 125 | 8.641 | 27.753 | 4.109 | 1.00 | 22.71 | C |
| ATOM 677 | CB | TYR A 125 | 9.067 | 27.919 | 2.648 | 1.00 | 21.66 | C |
| ATOM 678 | CG | TYR A 125 | 9.073 | 29.334 | 2.135 | 1.00 | 29.19 | C |
| ATOM 679 | CD1 | TYR A 125 | 10.112 | 30.200 | 2.459 | 1.00 | 31.37 | C |
| ATOM 680 | CE1 | TYR A 125 | 10.161 | 31.485 | 1.956 | 1.00 | 36.25 | C |
| ATOM 681 | CZ | TYR A 125 | 9.160 | 31.924 | 1.115 | 1.00 | 36.44 | C |
| ATOM 682 | OH | TYR A 125 | 9.251 | 33.193 | 0.597 | 1.00 | 39.33 | O |
| ATOM 683 | CE2 | TYR A 125 | 8.104 | 31.089 | 0.777 | 1.00 | 34.28 | C |
| ATOM 684 | CD2 | TYR A 125 | 8.067 | 29.798 | 1.290 | 1.00 | 30.61 | C |
| ATOM 685 | C | TYR A 125 | 9.622 | 28.453 | 5.042 | 1.00 | 21.07 | C |
| ATOM 686 | O | TYR A 125 | 10.748 | 27.986 | 5.203 | 1.00 | 22.41 | O |
| ATOM 687 | N | ASP A 126 | 9.211 | 29.549 | 5.673 | 1.00 | 21.85 | N |
| ATOM 688 | CA | ASP A 126 | 10.098 | 30.251 | 6.603 | 1.00 | 22.62 | C |
| ATOM 689 | CB | ASP A 126 | 9.947 | 31.767 | 6.497 | 1.00 | 23.92 | C |
| ATOM 690 | CG | ASP A 126 | 10.622 | 32.320 | 5.297 | 1.00 | 24.44 | C |
| ATOM 691 | OD1 | ASP A 126 | 11.604 | 31.692 | 4.863 | 1.00 | 29.05 | O |
| ATOM 692 | OD2 | ASP A 126 | 10.179 | 33.376 | 4.803 | 1.00 | 24.13 | O |
| ATOM 693 | C | ASP A 126 | 9.802 | 29.884 | 8.025 | 1.00 | 22.45 | C |
| ATOM 694 | O | ASP A 126 | 10.487 | 30.334 | 8.946 | 1.00 | 22.45 | O |
| ATOM 695 | N | GLU A 127 | 8.767 | 29.081 | 8.217 | 1.00 | 22.34 | N |
| ATOM 696 | CA | GLU A 127 | 8.389 | 28.714 | 9.565 | 1.00 | 23.65 | C |
| ATOM 697 | CB | GLU A 127 | 7.182 | 29.547 | 10.007 | 1.00 | 23.39 | C |
| ATOM 698 | CG | GLU A 127 | 7.483 | 30.999 | 10.315 | 1.00 | 31.89 | C |
| ATOM 699 | CD | GLU A 127 | 6.262 | 31.728 | 10.861 | 1.00 | 39.28 | C |
| ATOM 700 | OE1 | GLU A 127 | 5.611 | 31.186 | 11.783 | 1.00 | 42.40 | O |
| ATOM 701 | OE2 | GLU A 127 | 5.951 | 32.840 | 10.378 | 1.00 | 43.22 | O |
| ATOM 702 | C | GLU A 127 | 8.087 | 27.257 | 9.836 | 1.00 | 21.93 | C |
| ATOM 703 | O | GLU A 127 | 8.627 | 26.694 | 10.778 | 1.00 | 22.92 | O |
| ATOM 704 | N | TYR A 128 | 7.240 | 26.637 | 9.019 | 1.00 | 20.93 | N |
| ATOM 705 | CA | TYR A 128 | 6.854 | 25.263 | 9.306 | 1.00 | 19.08 | C |
| ATOM 706 | CB | TYR A 128 | 5.827 | 25.276 | 10.441 | 1.00 | 19.89 | C |
| ATOM 707 | CG | TYR A 128 | 4.472 | 25.797 | 9.992 | 1.00 | 20.24 | C |
| ATOM 708 | CD1 | TYR A 128 | 3.520 | 24.934 | 9.447 | 1.00 | 20.50 | C |
| ATOM 709 | CE1 | TYR A 128 | 2.296 | 25.402 | 8.981 | 1.00 | 21.38 | C |
| ATOM 710 | CZ | TYR A 128 | 2.008 | 26.754 | 9.056 | 1.00 | 26.85 | C |
| ATOM 711 | OH | TYR A 128 | 0.792 | 27.207 | 8.594 | 1.00 | 28.12 | O |
| ATOM 712 | CE2 | TYR A 128 | 2.934 | 27.644 | 9.593 | 1.00 | 26.44 | C |
| ATOM 713 | CD2 | TYR A 128 | 4.163 | 27.157 | 10.062 | 1.00 | 22.61 | C |
| ATOM 714 | C | TYR A 128 | 6.267 | 24.492 | 8.140 | 1.00 | 19.35 | C |
| ATOM 715 | O | TYR A 128 | 5.911 | 25.054 | 7.110 | 1.00 | 19.41 | O |
| ATOM 716 | N | ALA A 129 | 6.153 | 23.183 | 8.335 | 1.00 | 19.30 | N |
| ATOM 717 | CA | ALA A 129 | 5.600 | 22.290 | 7.337 | 1.00 | 18.44 | C |
| ATOM 718 | CB | ALA A 129 | 6.715 | 21.673 | 6.493 | 1.00 | 19.76 | C |
| ATOM 719 | C | ALA A 129 | 4.858 | 21.203 | 8.079 | 1.00 | 19.35 | C |
| ATOM 720 | O | ALA A 129 | 5.440 | 20.519 | 8.923 | 1.00 | 19.02 | O |
| ATOM 721 | N | LEU A 130 | 3.576 | 21.052 | 7.760 | 1.00 | 17.69 | N |
| ATOM 722 | CA | LEU A 130 | 2.720 | 20.050 | 8.384 | 1.00 | 17.03 | C |
| ATOM 723 | CB | LEU A 130 | 1.342 | 20.655 | 8.678 | 1.00 | 16.93 | C |
| ATOM 724 | CG | LEU A 130 | 0.480 | 20.216 | 9.874 | 1.00 | 25.96 | C |
| ATOM 725 | CD1 | LEU A 130 | -0.956 | 20.686 | 9.609 | 1.00 | 21.15 | C |
| ATOM 726 | CD2 | LEU A 130 | 0.516 | 18.687 | 10.081 | 1.00 | 26.45 | C |
| ATOM 727 | C | LEU A 130 | 2.582 | 18.932 | 7.354 | 1.00 | 17.75 | C |
| ATOM 728 | O | LEU A 130 | 2.005 | 19.140 | 6.284 | 1.00 | 16.09 | O |
| ATOM 729 | N | LEU A 131 | 3.128 | 17.754 | 7.657 | 1.00 | 17.47 | N |
| ATOM 730 | CA | LEU A 131 | 3.040 | 16.640 | 6.727 | 1.00 | 16.94 | C |
| ATOM 731 | CB | LEU A 131 | 4.413 | 16.030 | 6.464 | 1.00 | 18.54 | C |
| ATOM 732 | CG | LEU A 131 | 5.595 | 16.886 | 5.995 | 1.00 | 21.25 | C |
| ATOM 733 | CD1 | LEU A 131 | 6.622 | 15.972 | 5.343 | 1.00 | 19.28 | C |
| ATOM 734 | CD2 | LEU A 131 | 5.153 | 17.941 | 5.021 | 1.00 | 20.56 | C |
| ATOM 735 | C | LEU A 131 | 2.122 | 15.535 | 7.224 | 1.00 | 18.91 | C |
| ATOM 736 | O | LEU A 131 | 2.019 | 15.274 | 8.429 | 1.00 | 18.27 | O |
| ATOM 737 | N | PHE A 132 | 1.444 | 14.892 | 6.286 | 1.00 | 18.52 | N |
| ATOM 738 | CA | PHE A 132 | 0.569 | 13.784 | 6.628 | 1.00 | 20.01 | C |
| ATOM 739 | CB | PHE A 132 | -0.876 | 14.046 | 6.214 | 1.00 | 20.97 | C |
| ATOM 740 | CG | PHE A 132 | -1.786 | 12.870 | 6.461 | 1.00 | 25.43 | C |
| ATOM 741 | CD1 | PHE A 132 | -2.106 | 12.480 | 7.763 | 1.00 | 29.99 | C |
| ATOM 742 | CE1 | PHE A 132 | -2.904 | 11.359 | 8.003 | 1.00 | 30.06 | C |
| ATOM 743 | CZ | PHE A 132 | -3.391 | 10.616 | 6.933 | 1.00 | 29.29 | C |
| ATOM 744 | CE2 | PHE A 132 | -3.083 | 10.993 | 5.629 | 1.00 | 30.24 | C |
| ATOM 745 | CD2 | PHE A 132 | -2.282 | 12.119 | 5.397 | 1.00 | 27.73 | C |
| ATOM 746 | C | PHE A 132 | 1.086 | 12.574 | 5.880 | 1.00 | 20.33 | C |
| ATOM 747 | O | PHE A 132 | 1.369 | 12.650 | 4.694 | 1.00 | 19.14 | O |
| ATOM 748 | N | SER A 133 | 1.213 | 11.460 | 6.582 | 1.00 | 21.25 | N |
| ATOM 749 | CA | SER A 133 | 1.698 | 10.238 | 5.973 | 1.00 | 24.44 | C |
| ATOM 750 | CB | SER A 133 | 3.077 | 9.916 | 6.536 | 1.00 | 22.99 | C |
| ATOM 751 | OG | SER A 133 | 3.655 | 8.799 | 5.894 | 1.00 | 35.35 | O |
| ATOM 752 | C | SER A 133 | 0.724 | 9.092 | 6.247 | 1.00 | 25.26 | C |
| ATOM 753 | O | SER A 133 | 0.270 | 8.895 | 7.375 | 1.00 | 24.85 | O |
| ATOM 754 | N | ARG A 134 | 0.381 | 8.354 | 5.204 | 1.00 | 28.87 | N |
| ATOM 755 | CA | ARG A 134 | -0.525 | 7.231 | 5.352 | 1.00 | 32.47 | C |
| ATOM 756 | CB | ARG A 134 | -1.888 | 7.530 | 4.701 | 1.00 | 32.31 | C |
| ATOM 757 | CG | ARG A 134 | -1.834 | 8.152 | 3.316 | 1.00 | 37.53 | C |
| ATOM 758 | CD | ARG A 134 | -3.169 | 8.816 | 2.978 | 1.00 | 43.53 | C |
| ATOM 759 | NE | ARG A 134 | -3.169 | 9.553 | 1.712 | 1.00 | 44.76 | N |
| ATOM 760 | CZ | ARG A 134 | -3.163 | 8.983 | 0.511 | 1.00 | 44.99 | C |
| ATOM 761 | NH1 | ARG A 134 | -3.147 | 7.661 | 0.405 | 1.00 | 46.67 | N |
| ATOM 762 | NH2 | ARG A 134 | -3.215 | 9.732 | -0.587 | 1.00 | 42.69 | N |
| ATOM 763 | C | ARG A 134 | 0.105 | 5.994 | 4.746 | 1.00 | 35.05 | C |
| ATOM 764 | O | ARG A 134 | 0.695 | 6.038 | 3.661 | 1.00 | 32.09 | O |
| ATOM 765 | N | GLY A 135 | 0.003 | 4.897 | 5.484 | 1.00 | 38.13 | N |
| ATOM 766 | CA | GLY A 135 | 0.556 | 3.640 | 5.033 | 1.00 | 43.65 | C |
| ATOM 767 | C | GLY A 135 | -0.446 | 2.537 | 5.290 | 1.00 | 48.01 | C |
| ATOM 768 | O | GLY A 135 | -1.472 | 2.760 | 5.937 | 1.00 | 47.49 | O |
| ATOM 769 | N | THR A 136 | -0.162 | 1.344 | 4.783 | 1.00 | 51.73 | N |
| ATOM 770 | CA | THR A 136 | -1.070 | 0.219 | 4.969 | 1.00 | 55.59 | C |
| ATOM 771 | CB | THR A 136 | -2.315 | 0.358 | 4.067 | 1.00 | 55.84 | C |
| ATOM 772 | OG1 | THR A 136 | -2.924 | 1.634 | 4.284 | 1.00 | 58.78 | O |
| ATOM 773 | CG2 | THR A 136 | -3.331 | -0.725 | 4.386 | 1.00 | 56.76 | C |
| ATOM 774 | C | THR A 136 | -0.382 | -1.094 | 4.636 | 1.00 | 56.89 | C |
| ATOM 775 | O | THR A 136 | -0.138 | -1.392 | 3.470 | 1.00 | 57.01 | O |
| ATOM 776 | N | LYS A 137 | -0.075 | -1.875 | 5.667 | 1.00 | 59.28 | N |
| ATOM 777 | CA | LYS A 137 | 0.580 | -3.162 | 5.483 | 1.00 | 61.56 | C |
| ATOM 778 | CB | LYS A 137 | 0.971 | -3.763 | 6.834 | 1.00 | 61.96 | C |
| ATOM 779 | CG | LYS A 137 | 1.531 | -2.777 | 7.849 | 1.00 | 63.75 | C |
| ATOM 780 | CD | LYS A 137 | 1.803 | -3.478 | 9.176 | 1.00 | 66.26 | C |
| ATOM 781 | CE | LYS A 137 | 2.248 | -2.499 | 10.252 | 1.00 | 66.38 | C |
| ATOM 782 | NZ | LYS A 137 | 2.545 | -3.196 | 11.536 | 1.00 | 66.02 | N |
| ATOM 783 | C | LYS A 137 | -0.411 | -4.096 | 4.800 | 1.00 | 62.36 | C |
| ATOM 784 | O | LYS A 137 | -0.041 | -5.159 | 4.300 | 1.00 | 63.04 | O |
| ATOM 785 | N | GLY A 138 | -1.678 | -3.691 | 4.791 | 1.00 | 63.05 | N |
| ATOM 786 | CA | GLY A 138 | -2.714 | -4.501 | 4.176 | 1.00 | 63.35 | C |
| ATOM 787 | C | GLY A 138 | -4.104 | -4.069 | 4.610 | 1.00 | 63.61 | C |
| ATOM 788 | O | GLY A 138 | -4.231 | -3.171 | 5.435 | 1.00 | 64.06 | O |
| ATOM 789 | N | PRO A 139 | -5.168 | -4.690 | 4.079 | 1.00 | 63.70 | N |
| ATOM 790 | CA | PRO A 139 | -6.555 | -4.348 | 4.430 | 1.00 | 63.74 | C |
| ATOM 791 | CB | PRO A 139 | -7.369 | -5.364 | 3.639 | 1.00 | 63.65 | C |
| ATOM 792 | CG | PRO A 139 | -6.503 | -5.603 | 2.422 | 1.00 | 64.02 | C |
| ATOM 793 | CD | PRO A 139 | -5.125 | -5.708 | 3.016 | 1.00 | 63.81 | C |
| ATOM 794 | C | PRO A 139 | -6.833 | -4.419 | 5.935 | 1.00 | 63.55 | C |
| ATOM 795 | O | PRO A 139 | -7.057 | -5.490 | 6.494 | 1.00 | 63.71 | O |
| ATOM 796 | N | GLY A 140 | -6.822 | -3.261 | 6.584 | 1.00 | 63.03 | N |
| ATOM 797 | CA | GLY A 140 | -7.059 | -3.199 | 8.018 | 1.00 | 62.01 | C |
| ATOM 798 | C | GLY A 140 | -5.787 | -2.914 | 8.800 | 1.00 | 60.77 | C |
| ATOM 799 | O | GLY A 140 | -5.827 | -2.660 | 10.004 | 1.00 | 60.90 | O |
| ATOM 800 | N | GLN A 141 | -4.653 | -2.947 | 8.112 | 1.00 | 59.05 | N |
| ATOM 801 | CA | GLN A 141 | -3.373 | -2.694 | 8.752 | 1.00 | 57.46 | C |
| ATOM 802 | CB | GLN A 141 | -2.398 | -3.809 | 8.370 | 1.00 | 57.71 | C |
| ATOM 803 | CG | GLN A 141 | -3.090 | -5.178 | 8.473 | 1.00 | 58.74 | C |
| ATOM 804 | CD | GLN A 141 | -2.189 | -6.355 | 8.151 | 1.00 | 59.31 | C |
| ATOM 805 | OE1 | GLN A 141 | -1.552 | -6.402 | 7.093 | 1.00 | 58.75 | O |
| ATOM 806 | NE2 | GLN A 141 | -2.141 | -7.323 | 9.063 | 1.00 | 59.08 | N |
| ATOM 807 | C | GLN A 141 | -2.882 | -1.323 | 8.304 | 1.00 | 55.66 | C |
| ATOM 808 | O | GLN A 141 | -1.699 | -1.125 | 8.029 | 1.00 | 55.85 | O |
| ATOM 809 | N | ASN A 142 | -3.825 | -0.383 | 8.247 | 1.00 | 53.33 | N |
| ATOM 810 | CA | ASN A 142 | -3.570 | 0.998 | 7.836 | 1.00 | 51.55 | C |
| ATOM 811 | CB | ASN A 142 | -4.857 | 1.649 | 7.316 | 1.00 | 51.60 | C |
| ATOM 812 | CG | ASN A 142 | -5.936 | 0.642 | 6.973 | 1.00 | 53.89 | C |
| ATOM 813 | OD1 | ASN A 142 | -7.123 | 0.905 | 7.172 | 1.00 | 53.86 | O |
| ATOM 814 | ND2 | ASN A 142 | -5.534 | -0.511 | 6.442 | 1.00 | 58.03 | N |
| ATOM 815 | C | ASN A 142 | -3.069 | 1.843 | 9.005 | 1.00 | 49.32 | C |
| ATOM 816 | O | ASN A 142 | -3.655 | 1.828 | 10.081 | 1.00 | 49.92 | O |
| ATOM 817 | N | PHE A 143 | -1.995 | 2.593 | 8.787 | 1.00 | 46.30 | N |
| ATOM 818 | CA | PHE A 143 | -1.459 | 3.458 | 9.829 | 1.00 | 43.59 | C |
| ATOM 819 | CB | PHE A 143 | -0.089 | 2.953 | 10.285 | 1.00 | 44.54 | C |
| ATOM 820 | CG | PHE A 143 | 1.056 | 3.775 | 9.777 | 1.00 | 48.28 | C |
| ATOM 821 | CD1 | PHE A 143 | 1.621 | 4.764 | 10.573 | 1.00 | 51.58 | C |
| ATOM 822 | CE1 | PHE A 143 | 2.650 | 5.570 | 10.093 | 1.00 | 54.13 | C |
| ATOM 823 | CZ | PHE A 143 | 3.121 | 5.388 | 8.807 | 1.00 | 52.35 | C |
| ATOM 824 | CE2 | PHE A 143 | 2.566 | 4.400 | 8.001 | 1.00 | 53.51 | C |
| ATOM 825 | CD2 | PHE A 143 | 1.538 | 3.597 | 8.490 | 1.00 | 50.84 | C |
| ATOM 826 | C | PHE A 143 | -1.350 | 4.886 | 9.292 | 1.00 | 40.43 | C |
| ATOM 827 | O | PHE A 143 | -1.134 | 5.094 | 8.103 | 1.00 | 37.35 | O |
| ATOM 828 | N | ARG A 144 | -1.509 | 5.864 | 10.176 | 1.00 | 37.08 | N |
| ATOM 829 | CA | ARG A 144 | -1.441 | 7.267 | 9.796 | 1.00 | 34.89 | C |
| ATOM 830 | CB | ARG A 144 | -2.833 | 7.897 | 9.918 | 1.00 | 35.73 | C |
| ATOM 831 | CG | ARG A 144 | -3.816 | 7.364 | 8.872 | 1.00 | 39.13 | C |
| ATOM 832 | CD | ARG A 144 | -5.240 | 7.894 | 9.029 | 1.00 | 46.28 | C |
| ATOM 833 | NE | ARG A 144 | -6.006 | 7.160 | 10.035 | 1.00 | 49.14 | N |
| ATOM 834 | CZ | ARG A 144 | -5.866 | 7.314 | 11.348 | 1.00 | 52.48 | C |
| ATOM 835 | NH1 | ARG A 144 | -4.988 | 8.182 | 11.829 | 1.00 | 54.40 | N |
| ATOM 836 | NH2 | ARG A 144 | -6.608 | 6.599 | 12.183 | 1.00 | 54.82 | N |
| ATOM 837 | C | ARG A 144 | -0.435 | 8.017 | 10.666 | 1.00 | 32.66 | C |
| ATOM 838 | O | ARG A 144 | -0.278 | 7.712 | 11.846 | 1.00 | 30.70 | O |
| ATOM 839 | N | MET A 145 | 0.259 | 8.988 | 10.084 | 1.00 | 28.76 | N |
| ATOM 840 | CA | MET A 145 | 1.232 | 9.749 | 10.850 | 1.00 | 27.41 | C |
| ATOM 841 | CB | MET A 145 | 2.612 | 9.099 | 10.747 | 1.00 | 27.81 | C |
| ATOM 842 | CG | MET A 145 | 3.715 | 9.914 | 11.408 | 1.00 | 30.09 | C |
| ATOM 843 | SD | MET A 145 | 5.311 | 9.070 | 11.470 | 1.00 | 35.29 | S |
| ATOM 844 | CE | MET A 145 | 5.842 | 9.159 | 9.789 | 1.00 | 30.22 | C |
| ATOM 845 | C | MET A 145 | 1.336 | 11.214 | 10.452 | 1.00 | 26.47 | C |
| ATOM 846 | O | MET A 145 | 1.565 | 11.543 | 9.285 | 1.00 | 27.37 | O |
| ATOM 847 | N | ALA A 146 | 1.178 | 12.086 | 11.442 | 1.00 | 24.27 | N |
| ATOM 848 | CA | ALA A 146 | 1.267 | 13.527 | 11.242 | 1.00 | 22.72 | C |
| ATOM 849 | CB | ALA A 146 | 0.161 | 14.223 | 12.004 | 1.00 | 23.19 | C |
| ATOM 850 | C | ALA A 146 | 2.633 | 13.998 | 11.743 | 1.00 | 21.85 | C |
| ATOM 851 | O | ALA A 146 | 3.047 | 13.670 | 12.860 | 1.00 | 20.76 | O |
| ATOM 852 | N | THR A 147 | 3.332 | 14.765 | 10.914 | 1.00 | 21.71 | N |
| ATOM 853 | CA | THR A 147 | 4.652 | 15.261 | 11.281 | 1.00 | 20.82 | C |
| ATOM 854 | CB | THR A 147 | 5.747 | 14.641 | 10.388 | 1.00 | 22.25 | C |
| ATOM 855 | OG1 | THR A 147 | 5.541 | 13.224 | 10.287 | 1.00 | 23.42 | O |
| ATOM 856 | CG2 | THR A 147 | 7.125 | 14.910 | 10.977 | 1.00 | 22.09 | C |
| ATOM 857 | C | THR A 147 | 4.738 | 16.776 | 11.143 | 1.00 | 20.34 | C |
| ATOM 858 | O | THR A 147 | 4.339 | 17.342 | 10.125 | 1.00 | 19.27 | O |
| ATOM 859 | N | LEU A 148 | 5.269 | 17.430 | 12.170 | 1.00 | 19.28 | N |
| ATOM 860 | CA | LEU A 148 | 5.409 | 18.878 | 12.137 | 1.00 | 19.58 | C |
| ATOM 861 | CB | LEU A 148 | 4.675 | 19.516 | 13.317 | 1.00 | 21.33 | C |
| ATOM 862 | CG | LEU A 148 | 4.786 | 21.045 | 13.445 | 1.00 | 20.43 | C |
| ATOM 863 | CD1 | LEU A 148 | 4.224 | 21.747 | 12.219 | 1.00 | 22.47 | C |
| ATOM 864 | CD2 | LEU A 148 | 4.026 | 21.472 | 14.671 | 1.00 | 24.32 | C |
| ATOM 865 | C | LEU A 148 | 6.877 | 19.289 | 12.155 | 1.00 | 19.02 | C |
| ATOM 866 | O | LEU A 148 | 7.605 | 19.046 | 13.127 | 1.00 | 18.11 | O |
| ATOM 867 | N | TYR A 149 | 7.308 | 19.887 | 11.051 | 1.00 | 19.53 | N |
| ATOM 868 | CA | TYR A 149 | 8.671 | 20.378 | 10.923 | 1.00 | 19.40 | C |
| ATOM 869 | CB | TYR A 149 | 9.206 | 20.132 | 9.518 | 1.00 | 19.80 | C |
| ATOM 870 | CG | TYR A 149 | 9.662 | 18.712 | 9.281 | 1.00 | 22.86 | C |
| ATOM 871 | CD1 | TYR A 149 | 10.829 | 18.219 | 9.873 | 1.00 | 24.22 | C |
| ATOM 872 | CE1 | TYR A 149 | 11.236 | 16.889 | 9.672 | 1.00 | 25.00 | C |
| ATOM 873 | CZ | TYR A 149 | 10.464 | 16.058 | 8.877 | 1.00 | 26.70 | C |
| ATOM 874 | OH | TYR A 149 | 10.819 | 14.742 | 8.693 | 1.00 | 36.47 | O |
| ATOM 875 | CE2 | TYR A 149 | 9.305 | 16.531 | 8.280 | 1.00 | 27.17 | C |
| ATOM 876 | CD2 | TYR A 149 | 8.910 | 17.844 | 8.482 | 1.00 | 25.06 | C |
| ATOM 877 | C | TYR A 149 | 8.658 | 21.872 | 11.208 | 1.00 | 19.85 | C |
| ATOM 878 | O | TYR A 149 | 7.672 | 22.562 | 10.938 | 1.00 | 18.56 | O |
| ATOM 879 | N | SER A 150 | 9.764 | 22.363 | 11.750 | 1.00 | 19.94 | N |
| ATOM 880 | CA | SER A 150 | 9.903 | 23.764 | 12.102 | 1.00 | 20.50 | C |
| ATOM 881 | CB | SER A 150 | 9.704 | 23.915 | 13.615 | 1.00 | 21.21 | C |
| ATOM 882 | OG | SER A 150 | 9.899 | 25.250 | 14.047 | 1.00 | 22.73 | O |
| ATOM 883 | C | SER A 150 | 11.282 | 24.302 | 11.701 | 1.00 | 20.46 | C |
| ATOM 884 | O | SER A 150 | 12.292 | 23.593 | 11.778 | 1.00 | 18.66 | O |
| ATOM 885 | N | ARG A 151 | 11.319 | 25.556 | 11.261 | 1.00 | 21.36 | N |
| ATOM 886 | CA | ARG A 151 | 12.587 | 26.164 | 10.878 | 1.00 | 22.36 | C |
| ATOM 887 | CB | ARG A 151 | 12.355 | 27.467 | 10.102 | 1.00 | 21.14 | C |
| ATOM 888 | CG | ARG A 151 | 11.888 | 27.250 | 8.673 | 1.00 | 22.84 | C |
| ATOM 889 | CD | ARG A 151 | 12.914 | 26.442 | 7.873 | 1.00 | 19.77 | C |
| ATOM 890 | NE | ARG A 151 | 12.582 | 26.404 | 6.448 | 1.00 | 24.92 | N |
| ATOM 891 | CZ | ARG A 151 | 13.337 | 25.841 | 5.511 | 1.00 | 20.64 | C |
| ATOM 892 | NH1 | ARG A 151 | 14.479 | 25.258 | 5.836 | 1.00 | 22.01 | N |
| ATOM 893 | NH2 | ARG A 151 | 12.948 | 25.869 | 4.243 | 1.00 | 21.20 | N |
| ATOM 894 | C | ARG A 151 | 13.387 | 26.437 | 12.145 | 1.00 | 23.99 | C |
| ATOM 895 | O | ARG A 151 | 14.615 | 26.506 | 12.115 | 1.00 | 24.51 | O |
| ATOM 896 | N | THR A 152 | 12.676 | 26.578 | 13.258 | 1.00 | 24.32 | N |
| ATOM 897 | CA | THR A 152 | 13.305 | 26.832 | 14.548 | 1.00 | 26.33 | C |
| ATOM 898 | CB | THR A 152 | 12.765 | 28.120 | 15.185 | 1.00 | 27.25 | C |
| ATOM 899 | OG1 | THR A 152 | 11.347 | 27.997 | 15.378 | 1.00 | 25.22 | O |
| ATOM 900 | CG2 | THR A 152 | 13.063 | 29.327 | 14.295 | 1.00 | 25.83 | C |
| ATOM 901 | C | THR A 152 | 13.047 | 25.687 | 15.528 | 1.00 | 27.69 | C |
| ATOM 902 | O | THR A 152 | 12.090 | 24.928 | 15.372 | 1.00 | 26.48 | O |
| ATOM 903 | N | GLN A 153 | 13.906 | 25.576 | 16.536 | 1.00 | 28.98 | N |
| ATOM 904 | CA | GLN A 153 | 13.776 | 24.538 | 17.551 | 1.00 | 30.08 | C |
| ATOM 905 | CB | GLN A 153 | 15.090 | 24.396 | 18.329 | 1.00 | 29.88 | C |
| ATOM 906 | CG | GLN A 153 | 16.182 | 23.642 | 17.582 | 1.00 | 32.02 | C |
| ATOM 907 | CD | GLN A 153 | 17.493 | 23.587 | 18.347 | 1.00 | 35.05 | C |
| ATOM 908 | OE1 | GLN A 153 | 18.400 | 24.373 | 18.099 | 1.00 | 36.34 | O |
| ATOM 909 | NE2 | GLN A 153 | 17.591 | 22.658 | 19.289 | 1.00 | 36.94 | N |
| ATOM 910 | C | GLN A 153 | 12.629 | 24.848 | 18.513 | 1.00 | 30.99 | C |
| ATOM 911 | O | GLN A 153 | 12.158 | 23.972 | 19.236 | 1.00 | 31.14 | O |
| ATOM 912 | N | THR A 154 | 12.187 | 26.100 | 18.517 | 1.00 | 33.09 | N |
| ATOM 913 | CA | THR A 154 | 11.095 | 26.526 | 19.382 | 1.00 | 35.59 | C |
| ATOM 914 | CB | THR A 154 | 11.334 | 27.944 | 19.923 | 1.00 | 35.74 | C |
| ATOM 915 | OG1 | THR A 154 | 12.404 | 27.905 | 20.870 | 1.00 | 40.23 | O |
| ATOM 916 | CG2 | THR A 154 | 10.090 | 28.481 | 20.608 | 1.00 | 36.81 | C |
| ATOM 917 | C | THR A 154 | 9.788 | 26.492 | 18.611 | 1.00 | 36.05 | C |
| ATOM 918 | O | THR A 154 | 9.737 | 26.835 | 17.431 | 1.00 | 36.13 | O |
| ATOM 919 | N | LEU A 155 | 8.724 | 26.083 | 19.288 | 1.00 | 37.13 | N |
| ATOM 920 | CA | LEU A 155 | 7.422 | 25.975 | 18.648 | 1.00 | 38.42 | C |
| ATOM 921 | CB | LEU A 155 | 6.965 | 24.516 | 18.714 | 1.00 | 37.88 | C |
| ATOM 922 | CG | LEU A 155 | 6.062 | 23.916 | 17.638 | 1.00 | 39.67 | C |
| ATOM 923 | CD1 | LEU A 155 | 6.706 | 24.033 | 16.262 | 1.00 | 35.31 | C |
| ATOM 924 | CD2 | LEU A 155 | 5.816 | 22.456 | 17.984 | 1.00 | 38.75 | C |
| ATOM 925 | C | LEU A 155 | 6.405 | 26.878 | 19.337 | 1.00 | 39.23 | C |
| ATOM 926 | O | LEU A 155 | 6.264 | 26.841 | 20.561 | 1.00 | 39.64 | O |
| ATOM 927 | N | LYS A 156 | 5.708 | 27.697 | 18.553 | 1.00 | 39.22 | N |
| ATOM 928 | CA | LYS A 156 | 4.690 | 28.589 | 19.102 | 1.00 | 39.41 | C |
| ATOM 929 | CB | LYS A 156 | 4.362 | 29.716 | 18.120 | 1.00 | 39.99 | C |
| ATOM 930 | CG | LYS A 156 | 5.469 | 30.741 | 17.955 | 1.00 | 41.50 | C |
| ATOM 931 | CD | LYS A 156 | 5.064 | 31.818 | 16.971 | 1.00 | 44.94 | C |
| ATOM 932 | CE | LYS A 156 | 6.165 | 32.854 | 16.791 | 1.00 | 46.38 | C |
| ATOM 933 | NZ | LYS A 156 | 5.773 | 33.857 | 15.764 | 1.00 | 47.77 | N |
| ATOM 934 | C | LYS A 156 | 3.426 | 27.798 | 19.398 | 1.00 | 39.33 | C |
| ATOM 935 | O | LYS A 156 | 3.010 | 26.953 | 18.602 | 1.00 | 37.57 | O |
| ATOM 936 | N | ASP A 157 | 2.815 | 28.082 | 20.544 | 1.00 | 39.71 | N |
| ATOM 937 | CA | ASP A 157 | 1.603 | 27.389 | 20.961 | 1.00 | 39.53 | C |
| ATOM 938 | CB | ASP A 157 | 1.028 | 28.063 | 22.208 | 1.00 | 41.01 | C |
| ATOM 939 | CG | ASP A 157 | 2.033 | 28.131 | 23.343 | 1.00 | 43.20 | C |
| ATOM 940 | OD1 | ASP A 157 | 3.036 | 28.863 | 23.205 | 1.00 | 47.67 | O |
| ATOM 941 | OD2 | ASP A 157 | 1.828 | 27.445 | 24.368 | 1.00 | 46.90 | O |
| ATOM 942 | C | ASP A 157 | 0.538 | 27.310 | 19.868 | 1.00 | 38.74 | C |
| ATOM 943 | O | ASP A 157 | -0.132 | 26.287 | 19.720 | 1.00 | 37.95 | O |
| ATOM 944 | N | GLU A 158 | 0.384 | 28.383 | 19.101 | 1.00 | 37.72 | N |
| ATOM 945 | CA | GLU A 158 | -0.609 | 28.398 | 18.033 | 1.00 | 37.05 | C |
| ATOM 946 | CB | GLU A 158 | -0.614 | 29.752 | 17.321 | 1.00 | 37.76 | C |
| ATOM 947 | CG | GLU A 158 | -0.463 | 30.954 | 18.243 | 1.00 | 41.78 | C |
| ATOM 948 | CD | GLU A 158 | 0.977 | 31.198 | 18.656 | 1.00 | 46.94 | C |
| ATOM 949 | OE1 | GLU A 158 | 1.798 | 31.525 | 17.772 | 1.00 | 50.90 | O |
| ATOM 950 | OE2 | GLU A 158 | 1.292 | 31.063 | 19.859 | 1.00 | 49.28 | O |
| ATOM 951 | C | GLU A 158 | -0.294 | 27.301 | 17.024 | 1.00 | 35.99 | C |
| ATOM 952 | O | GLU A 158 | -1.187 | 26.761 | 16.378 | 1.00 | 34.01 | O |
| ATOM 953 | N | LEU A 159 | 0.990 | 26.981 | 16.895 | 1.00 | 35.06 | N |
| ATOM 954 | CA | LEU A 159 | 1.437 | 25.948 | 15.969 | 1.00 | 32.93 | C |
| ATOM 955 | CB | LEU A 159 | 2.924 | 26.130 | 15.666 | 1.00 | 33.58 | C |
| ATOM 956 | CG | LEU A 159 | 3.403 | 25.650 | 14.295 | 1.00 | 32.35 | C |
| ATOM 957 | CD1 | LEU A 159 | 2.543 | 26.264 | 13.202 | 1.00 | 35.68 | C |
| ATOM 958 | CD2 | LEU A 159 | 4.857 | 26.040 | 14.104 | 1.00 | 33.17 | C |
| ATOM 959 | C | LEU A 159 | 1.182 | 24.588 | 16.600 | 1.00 | 31.25 | C |
| ATOM 960 | O | LEU A 159 | 0.851 | 23.622 | 15.914 | 1.00 | 29.46 | O |
| ATOM 961 | N | LYS A 160 | 1.330 | 24.518 | 17.918 | 1.00 | 29.57 | N |
| ATOM 962 | CA | LYS A 160 | 1.077 | 23.276 | 18.633 | 1.00 | 29.07 | C |
| ATOM 963 | CB | LYS A 160 | 1.448 | 23.410 | 20.114 | 1.00 | 29.49 | C |
| ATOM 964 | CG | LYS A 160 | 2.938 | 23.535 | 20.410 | 1.00 | 31.30 | C |
| ATOM 965 | CD | LYS A 160 | 3.195 | 23.359 | 21.902 | 1.00 | 32.92 | C |
| ATOM 966 | CE | LYS A 160 | 4.671 | 23.470 | 22.231 | 1.00 | 39.23 | C |
| ATOM 967 | NZ | LYS A 160 | 4.974 | 23.172 | 23.664 | 1.00 | 42.27 | N |
| ATOM 968 | C | LYS A 160 | -0.408 | 22.937 | 18.514 | 1.00 | 28.08 | C |
| ATOM 969 | O | LYS A 160 | -0.779 | 21.773 | 18.330 | 1.00 | 27.38 | O |
| ATOM 970 | N | GLU A 161 | -1.254 | 23.959 | 18.605 | 1.00 | 27.07 | N |
| ATOM 971 | CA | GLU A 161 | -2.695 | 23.762 | 18.517 | 1.00 | 27.31 | C |
| ATOM 972 | CB | GLU A 161 | -3.439 | 25.064 | 18.830 | 1.00 | 28.93 | C |
| ATOM 973 | CG | GLU A 161 | -3.159 | 25.662 | 20.201 | 1.00 | 32.16 | C |
| ATOM 974 | CD | GLU A 161 | -4.254 | 26.629 | 20.639 | 1.00 | 38.78 | C |
| ATOM 975 | OE1 | GLU A 161 | -4.944 | 27.191 | 19.758 | 1.00 | 39.33 | O |
| ATOM 976 | OE2 | GLU A 161 | -4.422 | 26.831 | 21.864 | 1.00 | 43.12 | O |
| ATOM 977 | C | GLU A 161 | -3.100 | 23.270 | 17.134 | 1.00 | 26.18 | C |
| ATOM 978 | O | GLU A 161 | -3.950 | 22.383 | 16.992 | 1.00 | 26.11 | O |
| ATOM 979 | N | LYS A 162 | -2.492 | 23.848 | 16.109 | 1.00 | 24.62 | N |
| ATOM 980 | CA | LYS A 162 | -2.794 | 23.440 | 14.742 | 1.00 | 24.49 | C |
| ATOM 981 | CB | LYS A 162 | -1.989 | 24.293 | 13.751 | 1.00 | 24.75 | C |
| ATOM 982 | CG | LYS A 162 | -2.266 | 23.987 | 12.292 | 1.00 | 26.25 | C |
| ATOM 983 | CD | LYS A 162 | -1.240 | 24.660 | 11.369 | 1.00 | 30.87 | C |
| ATOM 984 | CE | LYS A 162 | -1.321 | 26.180 | 11.412 | 1.00 | 29.17 | C |
| ATOM 985 | NZ | LYS A 162 | -2.572 | 26.713 | 10.801 | 1.00 | 30.29 | N |
| ATOM 986 | C | LYS A 162 | -2.454 | 21.957 | 14.564 | 1.00 | 23.72 | C |
| ATOM 987 | O | LYS A 162 | -3.219 | 21.198 | 13.973 | 1.00 | 21.42 | O |
| ATOM 988 | N | PHE A 163 | -1.304 | 21.545 | 15.090 | 1.00 | 23.78 | N |
| ATOM 989 | CA | PHE A 163 | -0.867 | 20.155 | 14.974 | 1.00 | 23.17 | C |
| ATOM 990 | CB | PHE A 163 | 0.539 | 20.007 | 15.560 | 1.00 | 21.98 | C |
| ATOM 991 | CG | PHE A 163 | 1.159 | 18.664 | 15.306 | 1.00 | 21.44 | C |
| ATOM 992 | CD1 | PHE A 163 | 1.376 | 18.219 | 14.008 | 1.00 | 17.30 | C |
| ATOM 993 | CE1 | PHE A 163 | 1.953 | 16.985 | 13.772 | 1.00 | 18.89 | C |
| ATOM 994 | CZ | PHE A 163 | 2.323 | 16.173 | 14.841 | 1.00 | 22.89 | C |
| ATOM 995 | CE2 | PHE A 163 | 2.111 | 16.602 | 16.138 | 1.00 | 23.52 | C |
| ATOM 996 | CD2 | PHE A 163 | 1.531 | 17.844 | 16.367 | 1.00 | 21.36 | C |
| ATOM 997 | C | PHE A 163 | -1.842 | 19.218 | 15.699 | 1.00 | 23.35 | C |
| ATOM 998 | O | PHE A 163 | -2.291 | 18.213 | 15.139 | 1.00 | 24.15 | O |
| ATOM 999 | N | THR A 164 | -2.161 | 19.550 | 16.946 | 1.00 | 26.31 | N |
| ATOM 1000 | CA | THR A 164 | -3.097 | 18.761 | 17.743 | 1.00 | 27.06 | C |
| ATOM 1001 | CB | THR A 164 | -3.287 | 19.389 | 19.139 | 1.00 | 26.63 | C |
| ATOM 1002 | OG1 | THR A 164 | -2.023 | 19.415 | 19.817 | 1.00 | 29.13 | O |
| ATOM 1003 | CG2 | THR A 164 | -4.288 | 18.585 | 19.969 | 1.00 | 28.94 | C |
| ATOM 1004 | C | THR A 164 | -4.447 | 18.681 | 17.034 | 1.00 | 26.30 | C |
| ATOM 1005 | O | THR A 164 | -5.045 | 17.609 | 16.937 | 1.00 | 27.05 | O |
| ATOM 1006 | N | THR A 165 | -4.917 | 19.817 | 16.534 | 1.00 | 26.32 | N |
| ATOM 1007 | CA | THR A 165 | -6.191 | 19.865 | 15.813 | 1.00 | 28.31 | C |
| ATOM 1008 | CB | THR A 165 | -6.511 | 21.294 | 15.320 | 1.00 | 27.79 | C |
| ATOM 1009 | OG1 | THR A 165 | -6.587 | 22.184 | 16.441 | 1.00 | 31.94 | O |
| ATOM 1010 | CG2 | THR A 165 | -7.834 | 21.316 | 14.579 | 1.00 | 31.80 | C |
| ATOM 1011 | C | THR A 165 | -6.178 | 18.947 | 14.585 | 1.00 | 26.45 | C |
| ATOM 1012 | O | THR A 165 | -7.073 | 18.108 | 14.406 | 1.00 | 25.73 | O |
| ATOM 1013 | N | PHE A 166 | -5.172 | 19.110 | 13.731 | 1.00 | 25.04 | N |
| ATOM 1014 | CA | PHE A 166 | -5.087 | 18.278 | 12.540 | 1.00 | 23.74 | C |
| ATOM 1015 | CB | PHE A 166 | -3.878 | 18.635 | 11.684 | 1.00 | 22.45 | C |
| ATOM 1016 | CG | PHE A 166 | -3.663 | 17.672 | 10.542 | 1.00 | 23.00 | C |
| ATOM 1017 | CD1 | PHE A 166 | -4.578 | 17.605 | 9.492 | 1.00 | 17.54 | C |
| ATOM 1018 | CE1 | PHE A 166 | -4.408 | 16.709 | 8.451 | 1.00 | 15.87 | C |
| ATOM 1019 | CZ | PHE A 166 | -3.305 | 15.859 | 8.437 | 1.00 | 15.82 | C |
| ATOM 1020 | CE2 | PHE A 166 | -2.380 | 15.912 | 9.481 | 1.00 | 20.54 | C |
| ATOM 1021 | CD2 | PHE A 166 | -2.564 | 16.818 | 10.524 | 1.00 | 23.28 | C |
| ATOM 1022 | C | PHE A 166 | -4.986 | 16.808 | 12.909 | 1.00 | 23.55 | C |
| ATOM 1023 | O | PHE A 166 | -5.601 | 15.954 | 12.266 | 1.00 | 24.15 | O |
| ATOM 1024 | N | SER A 167 | -4.196 | 16.522 | 13.937 | 1.00 | 24.73 | N |
| ATOM 1025 | CA | SER A 167 | -3.994 | 15.156 | 14.392 | 1.00 | 25.60 | C |
| ATOM 1026 | CB | SER A 167 | -2.978 | 15.132 | 15.541 | 1.00 | 26.04 | C |
| ATOM 1027 | OG | SER A 167 | -1.734 | 15.675 | 15.130 | 1.00 | 23.06 | O |
| ATOM 1028 | C | SER A 167 | -5.299 | 14.516 | 14.853 | 1.00 | 27.30 | C |
| ATOM 1029 | O | SER A 167 | -5.573 | 13.349 | 14.561 | 1.00 | 27.39 | O |
| ATOM 1030 | N | LYS A 168 | -6.105 | 15.279 | 15.580 | 1.00 | 28.09 | N |
| ATOM 1031 | CA | LYS A 168 | -7.364 | 14.753 | 16.070 | 1.00 | 28.24 | C |
| ATOM 1032 | CB | LYS A 168 | -7.915 | 15.668 | 17.167 | 1.00 | 28.85 | C |
| ATOM 1033 | CG | LYS A 168 | -7.015 | 15.656 | 18.404 | 1.00 | 29.37 | C |
| ATOM 1034 | CD | LYS A 168 | -7.628 | 16.376 | 19.581 | 1.00 | 31.71 | C |
| ATOM 1035 | CE | LYS A 168 | -6.778 | 16.162 | 20.821 | 1.00 | 31.05 | C |
| ATOM 1036 | NZ | LYS A 168 | -7.424 | 16.689 | 22.059 | 1.00 | 33.19 | N |
| ATOM 1037 | C | LYS A 168 | -8.346 | 14.587 | 14.924 | 1.00 | 28.80 | C |
| ATOM 1038 | O | LYS A 168 | -9.116 | 13.632 | 14.896 | 1.00 | 28.47 | O |
| ATOM 1039 | N | ALA A 169 | -8.292 | 15.499 | 13.959 | 1.00 | 29.16 | N |
| ATOM 1040 | CA | ALA A 169 | -9.172 | 15.430 | 12.798 | 1.00 | 30.36 | C |
| ATOM 1041 | CB | ALA A 169 | -9.021 | 16.681 | 11.949 | 1.00 | 29.30 | C |
| ATOM 1042 | C | ALA A 169 | -8.819 | 14.194 | 11.978 | 1.00 | 31.34 | C |
| ATOM 1043 | O | ALA A 169 | -9.472 | 13.880 | 10.988 | 1.00 | 32.11 | O |
| ATOM 1044 | N | GLN A 170 | -7.774 | 13.493 | 12.394 | 1.00 | 32.26 | N |
| ATOM 1045 | CA | GLN A 170 | -7.347 | 12.295 | 11.689 | 1.00 | 32.22 | C |
| ATOM 1046 | CB | GLN A 170 | -5.871 | 12.412 | 11.301 | 1.00 | 31.66 | C |
| ATOM 1047 | CG | GLN A 170 | -5.570 | 13.544 | 10.319 | 1.00 | 31.19 | C |
| ATOM 1048 | CD | GLN A 170 | -6.351 | 13.407 | 9.018 | 1.00 | 30.95 | C |
| ATOM 1049 | OE1 | GLN A 170 | -6.326 | 12.355 | 8.383 | 1.00 | 26.84 | O |
| ATOM 1050 | NE2 | GLN A 170 | -7.042 | 14.470 | 8.618 | 1.00 | 28.03 | N |
| ATOM 1051 | C | GLN A 170 | -7.570 | 11.048 | 12.539 | 1.00 | 33.04 | C |
| ATOM 1052 | O | GLN A 170 | -7.102 | 9.961 | 12.203 | 1.00 | 33.24 | O |
| ATOM 1053 | N | GLY A 171 | -8.291 | 11.211 | 13.641 | 1.00 | 34.12 | N |
| ATOM 1054 | CA | GLY A 171 | -8.565 | 10.082 | 14.509 | 1.00 | 36.19 | C |
| ATOM 1055 | C | GLY A 171 | -7.421 | 9.742 | 15.445 | 1.00 | 37.27 | C |
| ATOM 1056 | O | GLY A 171 | -7.243 | 8.584 | 15.823 | 1.00 | 37.31 | O |
| ATOM 1057 | N | LEU A 172 | -6.633 | 10.747 | 15.812 | 1.00 | 38.22 | N |
| ATOM 1058 | CA | LEU A 172 | -5.518 | 10.537 | 16.721 | 1.00 | 38.52 | C |
| ATOM 1059 | CB | LEU A 172 | -4.226 | 11.119 | 16.135 | 1.00 | 37.99 | C |
| ATOM 1060 | CG | LEU A 172 | -3.719 | 10.490 | 14.825 | 1.00 | 38.07 | C |
| ATOM 1061 | CD1 | LEU A 172 | -2.419 | 11.158 | 14.387 | 1.00 | 34.97 | C |
| ATOM 1062 | CD2 | LEU A 172 | -3.496 | 8.998 | 15.018 | 1.00 | 38.27 | C |
| ATOM 1063 | C | LEU A 172 | -5.843 | 11.204 | 18.049 | 1.00 | 39.21 | C |
| ATOM 1064 | O | LEU A 172 | -6.293 | 12.348 | 18.090 | 1.00 | 40.20 | O |
| ATOM 1065 | N | THR A 173 | -5.627 | 10.480 | 19.138 | 1.00 | 39.94 | N |
| ATOM 1066 | CA | THR A 173 | -5.893 | 11.011 | 20.467 | 1.00 | 40.68 | C |
| ATOM 1067 | CB | THR A 173 | -6.269 | 9.884 | 21.436 | 1.00 | 40.28 | C |
| ATOM 1068 | OG1 | THR A 173 | -5.329 | 8.813 | 21.305 | 1.00 | 41.86 | O |
| ATOM 1069 | CG2 | THR A 173 | -7.666 | 9.370 | 21.133 | 1.00 | 42.41 | C |
| ATOM 1070 | C | THR A 173 | -4.667 | 11.738 | 21.003 | 1.00 | 40.29 | C |
| ATOM 1071 | O | THR A 173 | -3.575 | 11.623 | 20.446 | 1.00 | 39.42 | O |
| ATOM 1072 | N | GLU A 174 | -4.853 | 12.487 | 22.085 | 1.00 | 41.07 | N |
| ATOM 1073 | CA | GLU A 174 | -3.757 | 13.227 | 22.690 | 1.00 | 41.95 | C |
| ATOM 1074 | CB | GLU A 174 | -4.264 | 14.047 | 23.877 | 1.00 | 42.62 | C |
| ATOM 1075 | CG | GLU A 174 | -5.227 | 15.138 | 23.465 | 1.00 | 46.48 | C |
| ATOM 1076 | CD | GLU A 174 | -5.351 | 16.234 | 24.497 | 1.00 | 52.49 | C |
| ATOM 1077 | OE1 | GLU A 174 | -5.735 | 15.926 | 25.647 | 1.00 | 55.08 | O |
| ATOM 1078 | OE2 | GLU A 174 | -5.063 | 17.404 | 24.154 | 1.00 | 53.65 | O |
| ATOM 1079 | C | GLU A 174 | -2.621 | 12.311 | 23.128 | 1.00 | 41.47 | C |
| ATOM 1080 | O | GLU A 174 | -1.451 | 12.688 | 23.063 | 1.00 | 41.74 | O |
| ATOM 1081 | N | GLU A 175 | -2.964 | 11.107 | 23.569 | 1.00 | 40.71 | N |
| ATOM 1082 | CA | GLU A 175 | -1.950 | 10.149 | 23.985 | 1.00 | 41.13 | C |
| ATOM 1083 | CB | GLU A 175 | -2.612 | 8.908 | 24.580 | 1.00 | 41.37 | C |
| ATOM 1084 | CG | GLU A 175 | -3.614 | 8.278 | 23.631 | 1.00 | 47.30 | C |
| ATOM 1085 | CD | GLU A 175 | -4.310 | 7.065 | 24.212 | 1.00 | 54.26 | C |
| ATOM 1086 | OE1 | GLU A 175 | -4.885 | 7.182 | 25.316 | 1.00 | 55.90 | O |
| ATOM 1087 | OE2 | GLU A 175 | -4.287 | 5.998 | 23.556 | 1.00 | 56.76 | O |
| ATOM 1088 | C | GLU A 175 | -1.127 | 9.760 | 22.759 | 1.00 | 39.75 | C |
| ATOM 1089 | O | GLU A 175 | -0.008 | 9.256 | 22.878 | 1.00 | 40.30 | O |
| ATOM 1090 | N | ASP A 176 | -1.688 | 9.995 | 21.578 | 1.00 | 37.64 | N |
| ATOM 1091 | CA | ASP A 176 | -1.001 | 9.678 | 20.328 | 1.00 | 36.84 | C |
| ATOM 1092 | CB | ASP A 176 | -1.994 | 9.192 | 19.265 | 1.00 | 37.43 | C |
| ATOM 1093 | CG | ASP A 176 | -2.510 | 7.795 | 19.532 | 1.00 | 39.43 | C |
| ATOM 1094 | OD1 | ASP A 176 | -1.680 | 6.870 | 19.681 | 1.00 | 42.70 | O |
| ATOM 1095 | OD2 | ASP A 176 | -3.746 | 7.621 | 19.577 | 1.00 | 40.96 | O |
| ATOM 1096 | C | ASP A 176 | -0.247 | 10.884 | 19.767 | 1.00 | 34.37 | C |
| ATOM 1097 | O | ASP A 176 | 0.384 | 10.787 | 18.723 | 1.00 | 33.91 | O |
| ATOM 1098 | N | ILE A 177 | -0.314 | 12.010 | 20.466 | 1.00 | 32.64 | N |
| ATOM 1099 | CA | ILE A 177 | 0.340 | 13.237 | 20.020 | 1.00 | 30.44 | C |
| ATOM 1100 | CB | ILE A 177 | -0.682 | 14.392 | 19.970 | 1.00 | 30.45 | C |
| ATOM 1101 | CG1 | ILE A 177 | -1.812 | 14.034 | 18.994 | 1.00 | 29.49 | C |
| ATOM 1102 | CD1 | ILE A 177 | -3.029 | 14.940 | 19.082 | 1.00 | 31.49 | C |
| ATOM 1103 | CG2 | ILE A 177 | 0.008 | 15.688 | 19.568 | 1.00 | 29.62 | C |
| ATOM 1104 | C | ILE A 177 | 1.499 | 13.633 | 20.933 | 1.00 | 29.89 | C |
| ATOM 1105 | O | ILE A 177 | 1.359 | 13.647 | 22.154 | 1.00 | 28.34 | O |
| ATOM 1106 | N | VAL A 178 | 2.645 | 13.960 | 20.342 | 1.00 | 28.91 | N |
| ATOM 1107 | CA | VAL A 178 | 3.803 | 14.337 | 21.142 | 1.00 | 28.66 | C |
| ATOM 1108 | CB | VAL A 178 | 4.711 | 13.111 | 21.412 | 1.00 | 28.61 | C |
| ATOM 1109 | CG1 | VAL A 178 | 5.249 | 12.566 | 20.101 | 1.00 | 30.80 | C |
| ATOM 1110 | CG2 | VAL A 178 | 5.862 | 13.501 | 22.331 | 1.00 | 30.83 | C |
| ATOM 1111 | C | VAL A 178 | 4.663 | 15.430 | 20.522 | 1.00 | 27.43 | C |
| ATOM 1112 | O | VAL A 178 | 4.968 | 15.402 | 19.332 | 1.00 | 26.89 | O |
| ATOM 1113 | N | PHE A 179 | 5.043 | 16.402 | 21.339 | 1.00 | 27.15 | N |
| ATOM 1114 | CA | PHE A 179 | 5.905 | 17.478 | 20.881 | 1.00 | 27.17 | C |
| ATOM 1115 | CB | PHE A 179 | 5.451 | 18.813 | 21.477 | 1.00 | 26.48 | C |
| ATOM 1116 | CG | PHE A 179 | 4.115 | 19.257 | 20.961 | 1.00 | 27.02 | C |
| ATOM 1117 | CD1 | PHE A 179 | 3.979 | 19.704 | 19.650 | 1.00 | 26.14 | C |
| ATOM 1118 | CE1 | PHE A 179 | 2.724 | 20.041 | 19.136 | 1.00 | 28.69 | C |
| ATOM 1119 | CZ | PHE A 179 | 1.593 | 19.930 | 19.939 | 1.00 | 27.93 | C |
| ATOM 1120 | CE2 | PHE A 179 | 1.717 | 19.488 | 21.250 | 1.00 | 27.21 | C |
| ATOM 1121 | CD2 | PHE A 179 | 2.974 | 19.156 | 21.755 | 1.00 | 30.11 | C |
| ATOM 1122 | C | PHE A 179 | 7.306 | 17.088 | 21.323 | 1.00 | 27.37 | C |
| ATOM 1123 | O | PHE A 179 | 7.644 | 17.138 | 22.509 | 1.00 | 27.77 | O |
| ATOM 1124 | N | LEU A 180 | 8.093 | 16.665 | 20.337 | 1.00 | 27.22 | N |
| ATOM 1125 | CA | LEU A 180 | 9.458 | 16.194 | 20.513 | 1.00 | 26.96 | C |
| ATOM 1126 | CB | LEU A 180 | 10.075 | 15.921 | 19.134 | 1.00 | 26.82 | C |
| ATOM 1127 | CG | LEU A 180 | 9.260 | 14.959 | 18.257 | 1.00 | 26.68 | C |
| ATOM 1128 | CD1 | LEU A 180 | 9.924 | 14.776 | 16.901 | 1.00 | 21.86 | C |
| ATOM 1129 | CD2 | LEU A 180 | 9.123 | 13.616 | 18.966 | 1.00 | 21.60 | C |
| ATOM 1130 | C | LEU A 180 | 10.362 | 17.113 | 21.332 | 1.00 | 28.51 | C |
| ATOM 1131 | O | LEU A 180 | 10.543 | 18.288 | 21.004 | 1.00 | 28.44 | O |
| ATOM 1132 | N | PRO A 181 | 10.943 | 16.579 | 22.421 | 1.00 | 29.38 | N |
| ATOM 1133 | CA | PRO A 181 | 11.826 | 17.378 | 23.271 | 1.00 | 30.92 | C |
| ATOM 1134 | CB | PRO A 181 | 11.957 | 16.524 | 24.528 | 1.00 | 30.61 | C |
| ATOM 1135 | CG | PRO A 181 | 11.939 | 15.148 | 23.982 | 1.00 | 29.83 | C |
| ATOM 1136 | CD | PRO A 181 | 10.827 | 15.204 | 22.940 | 1.00 | 29.63 | C |
| ATOM 1137 | C | PRO A 181 | 13.164 | 17.622 | 22.590 | 1.00 | 31.85 | C |
| ATOM 1138 | O | PRO A 181 | 13.683 | 16.764 | 21.875 | 1.00 | 32.03 | O |
| ATOM 1139 | N | GLN A 182 | 13.700 | 18.814 | 22.812 | 1.00 | 34.15 | N |
| ATOM 1140 | CA | GLN A 182 | 14.972 | 19.230 | 22.248 | 1.00 | 38.42 | C |
| ATOM 1141 | CB | GLN A 182 | 15.160 | 20.727 | 22.513 | 1.00 | 38.11 | C |
| ATOM 1142 | CG | GLN A 182 | 16.349 | 21.358 | 21.838 | 1.00 | 43.04 | C |
| ATOM 1143 | CD | GLN A 182 | 16.516 | 22.813 | 22.231 | 1.00 | 47.27 | C |
| ATOM 1144 | OE1 | GLN A 182 | 15.580 | 23.609 | 22.117 | 1.00 | 48.82 | O |
| ATOM 1145 | NE2 | GLN A 182 | 17.711 | 23.170 | 22.697 | 1.00 | 49.54 | N |
| ATOM 1146 | C | GLN A 182 | 16.112 | 18.428 | 22.879 | 1.00 | 39.16 | C |
| ATOM 1147 | O | GLN A 182 | 16.316 | 18.480 | 24.086 | 1.00 | 38.96 | O |
| ATOM 1148 | N | PRO A 183 | 16.859 | 17.662 | 22.064 | 1.00 | 41.26 | N |
| ATOM 1149 | CA | PRO A 183 | 17.982 | 16.847 | 22.544 | 1.00 | 43.14 | C |
| ATOM 1150 | CB | PRO A 183 | 18.400 | 16.062 | 21.301 | 1.00 | 42.53 | C |
| ATOM 1151 | CG | PRO A 183 | 17.158 | 16.009 | 20.489 | 1.00 | 42.51 | C |
| ATOM 1152 | CD | PRO A 183 | 16.605 | 17.398 | 20.641 | 1.00 | 41.37 | C |
| ATOM 1153 | C | PRO A 183 | 19.108 | 17.739 | 23.043 | 1.00 | 45.17 | C |
| ATOM 1154 | O | PRO A 183 | 19.222 | 18.894 | 22.630 | 1.00 | 45.74 | O |
| ATOM 1155 | N | ASP A 184 | 19.939 | 17.208 | 23.931 | 1.00 | 47.82 | N |
| ATOM 1156 | CA | ASP A 184 | 21.059 | 17.974 | 24.468 | 1.00 | 50.56 | C |
| ATOM 1157 | CB | ASP A 184 | 21.516 | 17.365 | 25.799 | 1.00 | 51.23 | C |
| ATOM 1158 | CG | ASP A 184 | 22.507 | 18.246 | 26.543 | 1.00 | 54.35 | C |
| ATOM 1159 | OD1 | ASP A 184 | 23.479 | 18.717 | 25.914 | 1.00 | 58.16 | O |
| ATOM 1160 | OD2 | ASP A 184 | 22.324 | 18.461 | 27.763 | 1.00 | 57.05 | O |
| ATOM 1161 | C | ASP A 184 | 22.203 | 17.949 | 23.453 | 1.00 | 51.40 | C |
| ATOM 1162 | O | ASP A 184 | 22.988 | 18.890 | 23.366 | 1.00 | 52.48 | O |
| ATOM 1163 | N | LYS A 185 | 22.284 | 16.869 | 22.684 | 1.00 | 52.40 | N |
| ATOM 1164 | CA | LYS A 185 | 23.319 | 16.719 | 21.668 | 1.00 | 53.25 | C |
| ATOM 1165 | CB | LYS A 185 | 24.352 | 15.684 | 22.118 | 1.00 | 53.85 | C |
| ATOM 1166 | CG | LYS A 185 | 23.763 | 14.312 | 22.430 | 1.00 | 54.84 | C |
| ATOM 1167 | CD | LYS A 185 | 24.851 | 13.323 | 22.816 | 1.00 | 57.25 | C |
| ATOM 1168 | CE | LYS A 185 | 24.267 | 11.984 | 23.237 | 1.00 | 59.39 | C |
| ATOM 1169 | NZ | LYS A 185 | 25.336 | 11.017 | 23.617 | 1.00 | 59.87 | N |
| ATOM 1170 | C | LYS A 185 | 22.716 | 16.284 | 20.334 | 1.00 | 53.50 | C |
| ATOM 1171 | O | LYS A 185 | 21.499 | 16.182 | 20.199 | 1.00 | 53.06 | O |
| ATOM 1172 | N | CYS A 186 | 23.585 | 16.034 | 19.357 | 1.00 | 54.14 | N |
| ATOM 1173 | CA | CYS A 186 | 23.183 | 15.596 | 18.021 | 1.00 | 55.37 | C |
| ATOM 1174 | CB | CYS A 186 | 22.282 | 14.353 | 18.107 | 1.00 | 54.41 | C |
| ATOM 1175 | SG | CYS A 186 | 22.903 | 12.974 | 19.126 | 1.00 | 51.22 | S |
| ATOM 1176 | C | CYS A 186 | 22.467 | 16.652 | 17.176 | 1.00 | 56.92 | C |
| ATOM 1177 | O | CYS A 186 | 21.884 | 16.315 | 16.147 | 1.00 | 57.78 | O |
| ATOM 1178 | N | ILE A 187 | 22.513 | 17.919 | 17.580 | 1.00 | 59.30 | N |
| ATOM 1179 | CA | ILE A 187 | 21.817 | 18.953 | 16.812 | 1.00 | 61.99 | C |
| ATOM 1180 | CB | ILE A 187 | 20.357 | 19.109 | 17.314 | 1.00 | 62.28 | C |
| ATOM 1181 | CG1 | ILE A 187 | 19.545 | 17.872 | 16.927 | 1.00 | 61.77 | C |
| ATOM 1182 | CD1 | ILE A 187 | 18.090 | 17.965 | 17.308 | 1.00 | 65.79 | C |
| ATOM 1183 | CG2 | ILE A 187 | 19.710 | 20.354 | 16.726 | 1.00 | 63.84 | C |
| ATOM 1184 | C | ILE A 187 | 22.440 | 20.346 | 16.751 | 1.00 | 63.97 | C |
| ATOM 1185 | O | ILE A 187 | 22.046 | 21.143 | 15.904 | 1.00 | 64.66 | O |
| ATOM 1186 | N | GLN A 188 | 23.412 | 20.642 | 17.609 | 1.00 | 66.08 | N |
| ATOM 1187 | CA | GLN A 188 | 24.019 | 21.978 | 17.637 | 1.00 | 68.34 | C |
| ATOM 1188 | CB | GLN A 188 | 24.150 | 22.572 | 16.226 | 1.00 | 68.29 | C |
| ATOM 1189 | CG | GLN A 188 | 25.566 | 22.753 | 15.711 | 1.00 | 70.61 | C |
| ATOM 1190 | CD | GLN A 188 | 26.306 | 21.441 | 15.570 | 1.00 | 72.82 | C |
| ATOM 1191 | OE1 | GLN A 188 | 25.787 | 20.479 | 14.999 | 1.00 | 73.93 | O |
| ATOM 1192 | NE2 | GLN A 188 | 27.531 | 21.395 | 16.082 | 1.00 | 74.08 | N |
| ATOM 1193 | C | GLN A 188 | 23.042 | 22.830 | 18.444 | 1.00 | 69.30 | C |
| ATOM 1194 | O | GLN A 188 | 23.017 | 24.055 | 18.331 | 1.00 | 69.66 | O |
| ATOM 1195 | N | GLU A 189 | 22.232 | 22.135 | 19.242 | 1.00 | 70.42 | N |
| ATOM 1196 | CA | GLU A 189 | 21.203 | 22.703 | 20.112 | 1.00 | 71.39 | C |
| ATOM 1197 | CB | GLU A 189 | 21.351 | 22.125 | 21.520 | 1.00 | 71.77 | C |
| ATOM 1198 | CG | GLU A 189 | 22.147 | 20.827 | 21.579 | 1.00 | 72.99 | C |
| ATOM 1199 | CD | GLU A 189 | 21.669 | 19.790 | 20.583 | 1.00 | 73.77 | C |
| ATOM 1200 | OE1 | GLU A 189 | 20.472 | 19.433 | 20.610 | 1.00 | 76.28 | O |
| ATOM 1201 | OE2 | GLU A 189 | 22.494 | 19.324 | 19.770 | 1.00 | 73.47 | O |
| ATOM 1202 | C | GLU A 189 | 21.165 | 24.227 | 20.195 | 1.00 | 71.75 | C |
| ATOM 1203 | O | GLU A 189 | 21.377 | 24.768 | 21.303 | 1.00 | 71.94 | O |
| ATOM 1204 | OXT | GLU A 189 | 20.906 | 24.868 | 19.153 | 1.00 | 72.13 | O |
| ATOM 1205 | N | GLN B 35 | 16.520 | 25.594 | 58.782 | 1.00 | 37.06 | N |
| ATOM 1206 | CA | GLN B 35 | 16.131 | 26.126 | 57.446 | 1.00 | 35.29 | C |
| ATOM 1207 | CB | GLN B 35 | 17.377 | 26.612 | 56.704 | 1.00 | 35.86 | C |
| ATOM 1208 | CG | GLN B 35 | 18.466 | 25.564 | 56.576 | 1.00 | 40.43 | C |
| ATOM 1209 | CD | GLN B 35 | 19.622 | 26.021 | 55.700 | 1.00 | 46.53 | C |
| ATOM 1210 | OE1 | GLN B 35 | 20.623 | 25.313 | 55.554 | 1.00 | 51.77 | O |
| ATOM 1211 | NE2 | GLN B 35 | 19.487 | 27.204 | 55.107 | 1.00 | 49.87 | N |
| ATOM 1212 | C | GLN B 35 | 15.384 | 25.108 | 56.577 | 1.00 | 33.30 | C |
| ATOM 1213 | O | GLN B 35 | 15.044 | 25.406 | 55.435 | 1.00 | 32.10 | O |
| ATOM 1214 | N | GLN B 36 | 15.123 | 23.917 | 57.113 | 1.00 | 31.25 | N |
| ATOM 1215 | CA | GLN B 36 | 14.423 | 22.876 | 56.353 | 1.00 | 29.23 | C |
| ATOM 1216 | CB | GLN B 36 | 14.196 | 21.618 | 57.203 | 1.00 | 29.22 | C |
| ATOM 1217 | CG | GLN B 36 | 13.615 | 20.451 | 56.401 | 1.00 | 30.50 | C |
| ATOM 1218 | CD | GLN B 36 | 13.423 | 19.185 | 57.222 | 1.00 | 31.43 | C |
| ATOM 1219 | OE1 | GLN B 36 | 12.508 | 19.086 | 58.053 | 1.00 | 36.00 | O |
| ATOM 1220 | NE2 | GLN B 36 | 14.290 | 18.209 | 56.996 | 1.00 | 27.78 | N |
| ATOM 1221 | C | GLN B 36 | 13.083 | 23.366 | 55.840 | 1.00 | 26.86 | C |
| ATOM 1222 | O | GLN B 36 | 12.708 | 23.095 | 54.699 | 1.00 | 23.30 | O |
| ATOM 1223 | N | ASP B 37 | 12.363 | 24.084 | 56.694 | 1.00 | 27.22 | N |
| ATOM 1224 | CA | ASP B 37 | 11.050 | 24.609 | 56.338 | 1.00 | 27.10 | C |
| ATOM 1225 | CB | ASP B 37 | 10.457 | 25.408 | 57.499 | 1.00 | 30.98 | C |
| ATOM 1226 | CG | ASP B 37 | 9.837 | 24.522 | 58.561 | 1.00 | 35.24 | C |
| ATOM 1227 | OD1 | ASP B 37 | 10.576 | 23.732 | 59.182 | 1.00 | 47.18 | O |
| ATOM 1228 | OD2 | ASP B 37 | 8.609 | 24.616 | 58.778 | 1.00 | 45.07 | O |
| ATOM 1229 | C | ASP B 37 | 11.066 | 25.480 | 55.097 | 1.00 | 25.31 | C |
| ATOM 1230 | O | ASP B 37 | 10.045 | 25.635 | 54.435 | 1.00 | 24.94 | O |
| ATOM 1231 | N | LYS B 38 | 12.223 | 26.054 | 54.786 | 1.00 | 24.03 | N |
| ATOM 1232 | CA | LYS B 38 | 12.345 | 26.911 | 53.617 | 1.00 | 23.34 | C |
| ATOM 1233 | CB | LYS B 38 | 13.643 | 27.722 | 53.691 | 1.00 | 23.59 | C |
| ATOM 1234 | CG | LYS B 38 | 13.641 | 28.770 | 54.800 | 1.00 | 26.63 | C |
| ATOM 1235 | CD | LYS B 38 | 12.474 | 29.733 | 54.619 | 1.00 | 28.10 | C |
| ATOM 1236 | CE | LYS B 38 | 12.507 | 30.861 | 55.636 | 1.00 | 34.26 | C |
| ATOM 1237 | NZ | LYS B 38 | 13.727 | 31.698 | 55.494 | 1.00 | 36.28 | N |
| ATOM 1238 | C | LYS B 38 | 12.297 | 26.139 | 52.304 | 1.00 | 22.17 | C |
| ATOM 1239 | O | LYS B 38 | 12.019 | 26.719 | 51.262 | 1.00 | 23.22 | O |
| ATOM 1240 | N | PHE B 39 | 12.555 | 24.836 | 52.362 | 1.00 | 19.91 | N |
| ATOM 1241 | CA | PHE B 39 | 12.561 | 24.000 | 51.171 | 1.00 | 17.90 | C |
| ATOM 1242 | CB | PHE B 39 | 13.749 | 23.033 | 51.226 | 1.00 | 19.08 | C |
| ATOM 1243 | CG | PHE B 39 | 15.089 | 23.725 | 51.228 | 1.00 | 18.43 | C |
| ATOM 1244 | CD1 | PHE B 39 | 15.607 | 24.265 | 52.398 | 1.00 | 16.91 | C |
| ATOM 1245 | CE1 | PHE B 39 | 16.823 | 24.930 | 52.401 | 1.00 | 19.03 | C |
| ATOM 1246 | CZ | PHE B 39 | 17.546 | 25.067 | 51.211 | 1.00 | 20.75 | C |
| ATOM 1247 | CE2 | PHE B 39 | 17.035 | 24.531 | 50.034 | 1.00 | 21.98 | C |
| ATOM 1248 | CD2 | PHE B 39 | 15.811 | 23.862 | 50.050 | 1.00 | 19.07 | C |
| ATOM 1249 | C | PHE B 39 | 11.270 | 23.224 | 50.967 | 1.00 | 16.73 | C |
| ATOM 1250 | O | PHE B 39 | 11.175 | 22.390 | 50.068 | 1.00 | 16.56 | O |
| ATOM 1251 | N | LEU B 40 | 10.272 | 23.492 | 51.800 | 1.00 | 15.91 | N |
| ATOM 1252 | CA | LEU B 40 | 9.000 | 22.803 | 51.653 | 1.00 | 17.17 | C |
| ATOM 1253 | CB | LEU B 40 | 8.113 | 23.043 | 52.878 | 1.00 | 17.42 | C |
| ATOM 1254 | CG | LEU B 40 | 8.753 | 22.577 | 54.192 | 1.00 | 19.17 | C |
| ATOM 1255 | CD1 | LEU B 40 | 7.779 | 22.756 | 55.337 | 1.00 | 24.49 | C |
| ATOM 1256 | CD2 | LEU B 40 | 9.161 | 21.117 | 54.075 | 1.00 | 20.54 | C |
| ATOM 1257 | C | LEU B 40 | 8.345 | 23.357 | 50.401 | 1.00 | 17.51 | C |
| ATOM 1258 | O | LEU B 40 | 8.832 | 24.325 | 49.816 | 1.00 | 17.99 | O |
| ATOM 1259 | N | GLY B 41 | 7.266 | 22.729 | 49.960 | 1.00 | 17.31 | N |
| ATOM 1260 | CA | GLY B 41 | 6.588 | 23.237 | 48.783 | 1.00 | 17.09 | C |
| ATOM 1261 | C | GLY B 41 | 6.856 | 22.523 | 47.477 | 1.00 | 17.14 | C |
| ATOM 1262 | O | GLY B 41 | 7.352 | 21.394 | 47.444 | 1.00 | 18.05 | O |
| ATOM 1263 | N | ARG B 42 | 6.543 | 23.215 | 46.389 | 1.00 | 16.40 | N |
| ATOM 1264 | CA | ARG B 42 | 6.680 | 22.676 | 45.043 | 1.00 | 18.88 | C |
| ATOM 1265 | CB | ARG B 42 | 5.614 | 23.311 | 44.155 | 1.00 | 17.27 | C |
| ATOM 1266 | CG | ARG B 42 | 5.756 | 22.949 | 42.697 | 1.00 | 25.31 | C |
| ATOM 1267 | CD | ARG B 42 | 5.545 | 24.170 | 41.836 | 1.00 | 28.18 | C |
| ATOM 1268 | NE | ARG B 42 | 4.160 | 24.356 | 41.464 | 1.00 | 27.84 | N |
| ATOM 1269 | CZ | ARG B 42 | 3.700 | 25.456 | 40.879 | 1.00 | 32.90 | C |
| ATOM 1270 | NH1 | ARG B 42 | 4.526 | 26.466 | 40.622 | 1.00 | 29.84 | N |
| ATOM 1271 | NH2 | ARG B 42 | 2.428 | 25.527 | 40.509 | 1.00 | 28.11 | N |
| ATOM 1272 | C | ARG B 42 | 8.043 | 22.809 | 44.355 | 1.00 | 17.36 | C |
| ATOM 1273 | O | ARG B 42 | 8.626 | 23.894 | 44.278 | 1.00 | 16.56 | O |
| ATOM 1274 | N | TRP B 43 | 8.530 | 21.686 | 43.837 | 1.00 | 18.23 | N |
| ATOM 1275 | CA | TRP B 43 | 9.796 | 21.653 | 43.113 | 1.00 | 17.56 | C |
| ATOM 1276 | CB | TRP B 43 | 10.903 | 21.021 | 43.949 | 1.00 | 16.97 | C |
| ATOM 1277 | CG | TRP B 43 | 11.305 | 21.771 | 45.176 | 1.00 | 16.19 | C |
| ATOM 1278 | CD1 | TRP B 43 | 10.757 | 21.668 | 46.424 | 1.00 | 17.38 | C |
| ATOM 1279 | NE1 | TRP B 43 | 11.444 | 22.462 | 47.312 | 1.00 | 14.16 | N |
| ATOM 1280 | CE2 | TRP B 43 | 12.452 | 23.102 | 46.641 | 1.00 | 14.82 | C |
| ATOM 1281 | CD2 | TRP B 43 | 12.393 | 22.692 | 45.290 | 1.00 | 15.35 | C |
| ATOM 1282 | CE3 | TRP B 43 | 13.322 | 23.210 | 44.384 | 1.00 | 13.96 | C |
| ATOM 1283 | CZ3 | TRP B 43 | 14.281 | 24.114 | 44.850 | 1.00 | 18.19 | C |
| ATOM 1284 | CH2 | TRP B 43 | 14.313 | 24.501 | 46.202 | 1.00 | 16.46 | C |
| ATOM 1285 | CZ2 | TRP B 43 | 13.409 | 24.008 | 47.107 | 1.00 | 16.31 | C |
| ATOM 1286 | C | TRP B 43 | 9.621 | 20.813 | 41.863 | 1.00 | 17.89 | C |
| ATOM 1287 | O | TRP B 43 | 8.650 | 20.071 | 41.733 | 1.00 | 17.99 | O |
| ATOM 1288 | N | TYR B 44 | 10.579 | 20.925 | 40.952 | 1.00 | 18.35 | N |
| ATOM 1289 | CA | TYR B 44 | 10.568 | 20.162 | 39.712 | 1.00 | 19.01 | C |
| ATOM 1290 | CB | TYR B 44 | 10.294 | 21.085 | 38.523 | 1.00 | 20.26 | C |
| ATOM 1291 | CG | TYR B 44 | 8.927 | 21.731 | 38.531 | 1.00 | 19.50 | C |
| ATOM 1292 | CD1 | TYR B 44 | 7.799 | 21.020 | 38.120 | 1.00 | 21.39 | C |
| ATOM 1293 | CE1 | TYR B 44 | 6.529 | 21.605 | 38.129 | 1.00 | 21.89 | C |
| ATOM 1294 | CZ | TYR B 44 | 6.387 | 22.916 | 38.557 | 1.00 | 23.64 | C |
| ATOM 1295 | OH | TYR B 44 | 5.135 | 23.485 | 38.590 | 1.00 | 21.63 | O |
| ATOM 1296 | CE2 | TYR B 44 | 7.496 | 23.647 | 38.973 | 1.00 | 22.08 | C |
| ATOM 1297 | CD2 | TYR B 44 | 8.760 | 23.050 | 38.958 | 1.00 | 21.31 | C |
| ATOM 1298 | C | TYR B 44 | 11.937 | 19.506 | 39.528 | 1.00 | 20.14 | C |
| ATOM 1299 | O | TYR B 44 | 12.979 | 20.175 | 39.631 | 1.00 | 19.15 | O |
| ATOM 1300 | N | SER B 45 | 11.940 | 18.197 | 39.273 | 1.00 | 21.35 | N |
| ATOM 1301 | CA | SER B 45 | 13.192 | 17.487 | 39.032 | 1.00 | 23.58 | C |
| ATOM 1302 | CB | SER B 45 | 12.987 | 15.970 | 39.141 | 1.00 | 25.18 | C |
| ATOM 1303 | OG | SER B 45 | 11.926 | 15.531 | 38.307 | 1.00 | 26.39 | O |
| ATOM 1304 | C | SER B 45 | 13.585 | 17.878 | 37.612 | 1.00 | 24.18 | C |
| ATOM 1305 | O | SER B 45 | 12.903 | 17.521 | 36.656 | 1.00 | 25.49 | O |
| ATOM 1306 | N | ALA B 46 | 14.680 | 18.627 | 37.484 | 1.00 | 24.22 | N |
| ATOM 1307 | CA | ALA B 46 | 15.136 | 19.102 | 36.186 | 1.00 | 21.23 | C |
| ATOM 1308 | CB | ALA B 46 | 15.312 | 20.621 | 36.231 | 1.00 | 20.41 | C |
| ATOM 1309 | C | ALA B 46 | 16.423 | 18.458 | 35.693 | 1.00 | 21.73 | C |
| ATOM 1310 | O | ALA B 46 | 16.724 | 18.505 | 34.503 | 1.00 | 21.13 | O |
| ATOM 1311 | N | GLY B 47 | 17.195 | 17.878 | 36.605 | 1.00 | 22.14 | N |
| ATOM 1312 | CA | GLY B 47 | 18.452 | 17.261 | 36.217 | 1.00 | 22.69 | C |
| ATOM 1313 | C | GLY B 47 | 18.706 | 15.987 | 36.987 | 1.00 | 22.41 | C |
| ATOM 1314 | O | GLY B 47 | 18.440 | 15.922 | 38.186 | 1.00 | 21.93 | O |
| ATOM 1315 | N | LEU B 48 | 19.219 | 14.973 | 36.296 | 1.00 | 24.68 | N |
| ATOM 1316 | CA | LEU B 48 | 19.496 | 13.682 | 36.919 | 1.00 | 26.44 | C |
| ATOM 1317 | CB | LEU B 48 | 18.288 | 12.762 | 36.740 | 1.00 | 25.78 | C |
| ATOM 1318 | CG | LEU B 48 | 18.301 | 11.366 | 37.357 | 1.00 | 27.38 | C |
| ATOM 1319 | CD1 | LEU B 48 | 18.364 | 11.455 | 38.880 | 1.00 | 31.44 | C |
| ATOM 1320 | CD2 | LEU B 48 | 17.031 | 10.633 | 36.942 | 1.00 | 29.28 | C |
| ATOM 1321 | C | LEU B 48 | 20.744 | 13.035 | 36.315 | 1.00 | 28.13 | C |
| ATOM 1322 | O | LEU B 48 | 20.924 | 13.018 | 35.096 | 1.00 | 28.67 | O |
| ATOM 1323 | N | ALA B 49 | 21.608 | 12.513 | 37.177 | 1.00 | 29.37 | N |
| ATOM 1324 | CA | ALA B 49 | 22.836 | 11.860 | 36.736 | 1.00 | 30.98 | C |
| ATOM 1325 | CB | ALA B 49 | 23.987 | 12.849 | 36.763 | 1.00 | 29.77 | C |
| ATOM 1326 | C | ALA B 49 | 23.134 | 10.674 | 37.650 | 1.00 | 32.18 | C |
| ATOM 1327 | O | ALA B 49 | 22.876 | 10.731 | 38.849 | 1.00 | 32.22 | O |
| ATOM 1328 | N | SER B 50 | 23.671 | 9.598 | 37.084 | 1.00 | 34.77 | N |
| ATOM 1329 | CA | SER B 50 | 23.980 | 8.408 | 37.872 | 1.00 | 36.25 | C |
| ATOM 1330 | CB | SER B 50 | 22.684 | 7.746 | 38.339 | 1.00 | 35.93 | C |
| ATOM 1331 | OG | SER B 50 | 22.950 | 6.569 | 39.084 | 1.00 | 36.08 | O |
| ATOM 1332 | C | SER B 50 | 24.815 | 7.386 | 37.109 | 1.00 | 37.63 | C |
| ATOM 1333 | O | SER B 50 | 25.029 | 7.518 | 35.903 | 1.00 | 38.29 | O |
| ATOM 1334 | N | ASN B 51 | 25.287 | 6.369 | 37.825 | 1.00 | 38.85 | N |
| ATOM 1335 | CA | ASN B 51 | 26.076 | 5.302 | 37.222 | 1.00 | 40.59 | C |
| ATOM 1336 | CB | ASN B 51 | 27.459 | 5.203 | 37.880 | 1.00 | 40.57 | C |
| ATOM 1337 | CG | ASN B 51 | 27.391 | 4.940 | 39.380 | 1.00 | 39.49 | C |
| ATOM 1338 | OD1 | ASN B 51 | 28.419 | 4.773 | 40.030 | 1.00 | 42.49 | O |
| ATOM 1339 | ND2 | ASN B 51 | 26.185 | 4.903 | 39.931 | 1.00 | 34.80 | N |
| ATOM 1340 | C | ASN B 51 | 25.328 | 3.984 | 37.377 | 1.00 | 42.31 | C |
| ATOM 1341 | O | ASN B 51 | 25.792 | 2.935 | 36.941 | 1.00 | 42.26 | O |
| ATOM 1342 | N | SER B 52 | 24.160 | 4.058 | 38.004 | 1.00 | 44.68 | N |
| ATOM 1343 | CA | SER B 52 | 23.323 | 2.888 | 38.233 | 1.00 | 47.48 | C |
| ATOM 1344 | CB | SER B 52 | 22.033 | 3.306 | 38.944 | 1.00 | 48.00 | C |
| ATOM 1345 | OG | SER B 52 | 21.065 | 2.269 | 38.892 | 1.00 | 52.56 | O |
| ATOM 1346 | C | SER B 52 | 22.964 | 2.146 | 36.952 | 1.00 | 48.12 | C |
| ATOM 1347 | O | SER B 52 | 23.280 | 2.590 | 35.850 | 1.00 | 48.41 | O |
| ATOM 1348 | N | SER B 53 | 22.301 | 1.006 | 37.118 | 1.00 | 49.66 | N |
| ATOM 1349 | CA | SER B 53 | 21.859 | 0.188 | 35.996 | 1.00 | 50.25 | C |
| ATOM 1350 | CB | SER B 53 | 21.713 | -1.273 | 36.437 | 1.00 | 50.27 | C |
| ATOM 1351 | OG | SER B 53 | 22.947 | -1.793 | 36.907 | 1.00 | 51.05 | O |
| ATOM 1352 | C | SER B 53 | 20.507 | 0.745 | 35.567 | 1.00 | 50.62 | C |
| ATOM 1353 | O | SER B 53 | 20.218 | 0.874 | 34.378 | 1.00 | 50.70 | O |
| ATOM 1354 | N | TRP B 54 | 19.693 | 1.077 | 36.564 | 1.00 | 51.37 | N |
| ATOM 1355 | CA | TRP B 54 | 18.364 | 1.638 | 36.358 | 1.00 | 52.70 | C |
| ATOM 1356 | CB | TRP B 54 | 17.776 | 2.047 | 37.709 | 1.00 | 53.23 | C |
| ATOM 1357 | CG | TRP B 54 | 16.450 | 2.737 | 37.630 | 1.00 | 56.64 | C |
| ATOM 1358 | CD1 | TRP B 54 | 15.234 | 2.162 | 37.391 | 1.00 | 59.22 | C |
| ATOM 1359 | NE1 | TRP B 54 | 14.244 | 3.119 | 37.411 | 1.00 | 60.28 | N |
| ATOM 1360 | CE2 | TRP B 54 | 14.813 | 4.340 | 37.661 | 1.00 | 60.04 | C |
| ATOM 1361 | CD2 | TRP B 54 | 16.205 | 4.138 | 37.804 | 1.00 | 58.84 | C |
| ATOM 1362 | CE3 | TRP B 54 | 17.027 | 5.243 | 38.068 | 1.00 | 59.81 | C |
| ATOM 1363 | CZ3 | TRP B 54 | 16.441 | 6.500 | 38.179 | 1.00 | 60.48 | C |
| ATOM 1364 | CH2 | TRP B 54 | 15.052 | 6.669 | 38.032 | 1.00 | 61.73 | C |
| ATOM 1365 | CZ2 | TRP B 54 | 14.224 | 5.606 | 37.774 | 1.00 | 61.41 | C |
| ATOM 1366 | C | TRP B 54 | 18.439 | 2.855 | 35.441 | 1.00 | 52.10 | C |
| ATOM 1367 | O | TRP B 54 | 17.820 | 2.889 | 34.373 | 1.00 | 52.71 | O |
| ATOM 1368 | N | PHE B 55 | 19.205 | 3.851 | 35.869 | 1.00 | 51.33 | N |
| ATOM 1369 | CA | PHE B 55 | 19.376 | 5.082 | 35.109 | 1.00 | 50.85 | C |
| ATOM 1370 | CB | PHE B 55 | 20.395 | 5.984 | 35.812 | 1.00 | 50.43 | C |
| ATOM 1371 | CG | PHE B 55 | 20.594 | 7.314 | 35.146 | 1.00 | 48.28 | C |
| ATOM 1372 | CD1 | PHE B 55 | 19.562 | 8.245 | 35.099 | 1.00 | 46.15 | C |
| ATOM 1373 | CE1 | PHE B 55 | 19.740 | 9.474 | 34.471 | 1.00 | 46.51 | C |
| ATOM 1374 | CZ | PHE B 55 | 20.960 | 9.780 | 33.881 | 1.00 | 46.07 | C |
| ATOM 1375 | CE2 | PHE B 55 | 21.998 | 8.856 | 33.923 | 1.00 | 46.09 | C |
| ATOM 1376 | CD2 | PHE B 55 | 21.810 | 7.632 | 34.553 | 1.00 | 46.98 | C |
| ATOM 1377 | C | PHE B 55 | 19.826 | 4.801 | 33.677 | 1.00 | 51.09 | C |
| ATOM 1378 | O | PHE B 55 | 19.209 | 5.271 | 32.719 | 1.00 | 51.00 | O |
| ATOM 1379 | N | ARG B 56 | 20.903 | 4.034 | 33.541 | 1.00 | 51.57 | N |
| ATOM 1380 | CA | ARG B 56 | 21.445 | 3.677 | 32.229 | 1.00 | 52.15 | C |
| ATOM 1381 | CB | ARG B 56 | 22.640 | 2.726 | 32.393 | 1.00 | 52.46 | C |
| ATOM 1382 | CG | ARG B 56 | 23.954 | 3.386 | 32.783 | 1.00 | 54.53 | C |
| ATOM 1383 | CD | ARG B 56 | 24.907 | 2.357 | 33.397 | 1.00 | 58.48 | C |
| ATOM 1384 | NE | ARG B 56 | 26.299 | 2.806 | 33.475 | 1.00 | 62.11 | N |
| ATOM 1385 | CZ | ARG B 56 | 26.693 | 3.976 | 33.970 | 1.00 | 65.15 | C |
| ATOM 1386 | NH1 | ARG B 56 | 25.802 | 4.841 | 34.437 | 1.00 | 66.66 | N |
| ATOM 1387 | NH2 | ARG B 56 | 27.984 | 4.281 | 34.006 | 1.00 | 65.67 | N |
| ATOM 1388 | C | ARG B 56 | 20.411 | 3.027 | 31.306 | 1.00 | 51.62 | C |
| ATOM 1389 | O | ARG B 56 | 20.424 | 3.258 | 30.099 | 1.00 | 51.26 | O |
| ATOM 1390 | N | GLU B 57 | 19.515 | 2.223 | 31.872 | 1.00 | 51.11 | N |
| ATOM 1391 | CA | GLU B 57 | 18.507 | 1.540 | 31.071 | 1.00 | 51.51 | C |
| ATOM 1392 | CB | GLU B 57 | 18.503 | 0.046 | 31.415 | 1.00 | 51.87 | C |
| ATOM 1393 | CG | GLU B 57 | 19.780 | -0.677 | 31.007 | 1.00 | 53.93 | C |
| ATOM 1394 | CD | GLU B 57 | 19.773 | -2.151 | 31.375 | 1.00 | 56.43 | C |
| ATOM 1395 | OE1 | GLU B 57 | 19.748 | -2.462 | 32.585 | 1.00 | 58.93 | O |
| ATOM 1396 | OE2 | GLU B 57 | 19.794 | -2.996 | 30.452 | 1.00 | 56.91 | O |
| ATOM 1397 | C | GLU B 57 | 17.094 | 2.107 | 31.210 | 1.00 | 50.67 | C |
| ATOM 1398 | O | GLU B 57 | 16.108 | 1.381 | 31.058 | 1.00 | 51.18 | O |
| ATOM 1399 | N | LYS B 58 | 16.991 | 3.402 | 31.491 | 1.00 | 49.06 | N |
| ATOM 1400 | CA | LYS B 58 | 15.683 | 4.039 | 31.646 | 1.00 | 47.54 | C |
| ATOM 1401 | CB | LYS B 58 | 15.107 | 3.722 | 33.032 | 1.00 | 48.39 | C |
| ATOM 1402 | CG | LYS B 58 | 13.686 | 4.226 | 33.258 | 1.00 | 49.62 | C |
| ATOM 1403 | CD | LYS B 58 | 13.212 | 3.952 | 34.682 | 1.00 | 52.77 | C |
| ATOM 1404 | CE | LYS B 58 | 11.825 | 4.545 | 34.941 | 1.00 | 52.82 | C |
| ATOM 1405 | NZ | LYS B 58 | 11.389 | 4.377 | 36.358 | 1.00 | 52.01 | N |
| ATOM 1406 | C | LYS B 58 | 15.764 | 5.549 | 31.457 | 1.00 | 45.21 | C |
| ATOM 1407 | O | LYS B 58 | 14.746 | 6.226 | 31.389 | 1.00 | 44.48 | O |
| ATOM 1408 | N | LYS B 59 | 16.983 | 6.065 | 31.359 | 1.00 | 43.69 | N |
| ATOM 1409 | CA | LYS B 59 | 17.211 | 7.494 | 31.189 | 1.00 | 42.38 | C |
| ATOM 1410 | CB | LYS B 59 | 18.709 | 7.758 | 31.034 | 1.00 | 43.61 | C |
| ATOM 1411 | CG | LYS B 59 | 19.336 | 7.073 | 29.832 | 1.00 | 44.44 | C |
| ATOM 1412 | CD | LYS B 59 | 20.858 | 7.044 | 29.928 | 1.00 | 49.79 | C |
| ATOM 1413 | CE | LYS B 59 | 21.441 | 8.431 | 30.163 | 1.00 | 52.89 | C |
| ATOM 1414 | NZ | LYS B 59 | 21.020 | 9.404 | 29.118 | 1.00 | 56.53 | N |
| ATOM 1415 | C | LYS B 59 | 16.459 | 8.087 | 30.003 | 1.00 | 40.91 | C |
| ATOM 1416 | O | LYS B 59 | 15.966 | 9.213 | 30.071 | 1.00 | 40.09 | O |
| ATOM 1417 | N | ALA B 60 | 16.367 | 7.324 | 28.919 | 1.00 | 39.16 | N |
| ATOM 1418 | CA | ALA B 60 | 15.682 | 7.785 | 27.723 | 1.00 | 36.81 | C |
| ATOM 1419 | CB | ALA B 60 | 15.850 | 6.773 | 26.605 | 1.00 | 37.20 | C |
| ATOM 1420 | C | ALA B 60 | 14.200 | 8.054 | 27.952 | 1.00 | 35.75 | C |
| ATOM 1421 | O | ALA B 60 | 13.627 | 8.923 | 27.300 | 1.00 | 35.18 | O |
| ATOM 1422 | N | VAL B 61 | 13.584 | 7.319 | 28.875 | 1.00 | 33.10 | N |
| ATOM 1423 | CA | VAL B 61 | 12.157 | 7.485 | 29.147 | 1.00 | 32.61 | C |
| ATOM 1424 | CB | VAL B 61 | 11.462 | 6.114 | 29.347 | 1.00 | 32.54 | C |
| ATOM 1425 | CG1 | VAL B 61 | 11.692 | 5.234 | 28.127 | 1.00 | 33.11 | C |
| ATOM 1426 | CG2 | VAL B 61 | 11.981 | 5.434 | 30.612 | 1.00 | 31.73 | C |
| ATOM 1427 | C | VAL B 61 | 11.832 | 8.353 | 30.358 | 1.00 | 31.55 | C |
| ATOM 1428 | O | VAL B 61 | 10.675 | 8.446 | 30.761 | 1.00 | 31.40 | O |
| ATOM 1429 | N | LEU B 62 | 12.844 | 8.991 | 30.935 | 1.00 | 30.56 | N |
| ATOM 1430 | CA | LEU B 62 | 12.621 | 9.839 | 32.103 | 1.00 | 30.36 | C |
| ATOM 1431 | CB | LEU B 62 | 13.877 | 9.865 | 32.975 | 1.00 | 31.00 | C |
| ATOM 1432 | CG | LEU B 62 | 14.292 | 8.491 | 33.516 | 1.00 | 33.97 | C |
| ATOM 1433 | CD1 | LEU B 62 | 15.598 | 8.599 | 34.288 | 1.00 | 33.22 | C |
| ATOM 1434 | CD2 | LEU B 62 | 13.183 | 7.942 | 34.407 | 1.00 | 34.87 | C |
| ATOM 1435 | C | LEU B 62 | 12.212 | 11.265 | 31.740 | 1.00 | 27.77 | C |
| ATOM 1436 | O | LEU B 62 | 12.790 | 11.881 | 30.849 | 1.00 | 27.70 | O |
| ATOM 1437 | N | TYR B 63 | 11.196 | 11.772 | 32.431 | 1.00 | 27.50 | N |
| ATOM 1438 | CA | TYR B 63 | 10.705 | 13.127 | 32.211 | 1.00 | 26.18 | C |
| ATOM 1439 | CB | TYR B 63 | 9.240 | 13.133 | 31.747 | 1.00 | 26.26 | C |
| ATOM 1440 | CG | TYR B 63 | 8.992 | 12.606 | 30.357 | 1.00 | 27.42 | C |
| ATOM 1441 | CD1 | TYR B 63 | 8.814 | 11.243 | 30.127 | 1.00 | 29.98 | C |
| ATOM 1442 | CE1 | TYR B 63 | 8.572 | 10.757 | 28.843 | 1.00 | 33.87 | C |
| ATOM 1443 | CZ | TYR B 63 | 8.514 | 11.640 | 27.775 | 1.00 | 32.87 | C |
| ATOM 1444 | OH | TYR B 63 | 8.286 | 11.168 | 26.500 | 1.00 | 38.94 | O |
| ATOM 1445 | CE2 | TYR B 63 | 8.691 | 13.000 | 27.981 | 1.00 | 32.51 | C |
| ATOM 1446 | CD2 | TYR B 63 | 8.926 | 13.474 | 29.268 | 1.00 | 28.18 | C |
| ATOM 1447 | C | TYR B 63 | 10.783 | 13.909 | 33.513 | 1.00 | 24.64 | C |
| ATOM 1448 | O | TYR B 63 | 10.958 | 13.335 | 34.580 | 1.00 | 26.37 | O |
| ATOM 1449 | N | MET B 64 | 10.654 | 15.224 | 33.406 | 1.00 | 24.11 | N |
| ATOM 1450 | CA | MET B 64 | 10.666 | 16.089 | 34.564 | 1.00 | 21.60 | C |
| ATOM 1451 | CB | MET B 64 | 10.551 | 17.543 | 34.137 | 1.00 | 21.40 | C |
| ATOM 1452 | CG | MET B 64 | 10.203 | 18.491 | 35.274 | 1.00 | 21.55 | C |
| ATOM 1453 | SD | MET B 64 | 10.078 | 20.155 | 34.653 | 1.00 | 23.70 | S |
| ATOM 1454 | CE | MET B 64 | 11.772 | 20.441 | 34.213 | 1.00 | 23.94 | C |
| ATOM 1455 | C | MET B 64 | 9.454 | 15.723 | 35.391 | 1.00 | 20.89 | C |
| ATOM 1456 | O | MET B 64 | 8.387 | 15.462 | 34.841 | 1.00 | 20.65 | O |
| ATOM 1457 | N | ALA B 65 | 9.620 | 15.710 | 36.709 | 1.00 | 21.78 | N |
| ATOM 1458 | CA | ALA B 65 | 8.529 | 15.381 | 37.610 | 1.00 | 22.90 | C |
| ATOM 1459 | CB | ALA B 65 | 8.873 | 14.137 | 38.417 | 1.00 | 23.61 | C |
| ATOM 1460 | C | ALA B 65 | 8.243 | 16.536 | 38.550 | 1.00 | 22.81 | C |
| ATOM 1461 | O | ALA B 65 | 9.114 | 17.356 | 38.829 | 1.00 | 23.08 | O |
| ATOM 1462 | N | LYS B 66 | 7.008 | 16.595 | 39.026 | 1.00 | 24.13 | N |
| ATOM 1463 | CA | LYS B 66 | 6.592 | 17.625 | 39.968 | 1.00 | 24.90 | C |
| ATOM 1464 | CB | LYS B 66 | 5.175 | 18.090 | 39.635 | 1.00 | 24.40 | C |
| ATOM 1465 | CG | LYS B 66 | 4.531 | 19.010 | 40.658 | 1.00 | 25.61 | C |
| ATOM 1466 | CD | LYS B 66 | 3.216 | 19.555 | 40.101 | 1.00 | 24.44 | C |
| ATOM 1467 | CE | LYS B 66 | 2.390 | 20.253 | 41.157 | 1.00 | 29.98 | C |
| ATOM 1468 | NZ | LYS B 66 | 1.154 | 20.853 | 40.569 | 1.00 | 39.00 | N |
| ATOM 1469 | C | LYS B 66 | 6.641 | 16.990 | 41.356 | 1.00 | 24.30 | C |
| ATOM 1470 | O | LYS B 66 | 5.955 | 15.999 | 41.616 | 1.00 | 26.16 | O |
| ATOM 1471 | N | THR B 67 | 7.468 | 17.543 | 42.234 | 1.00 | 21.31 | N |
| ATOM 1472 | CA | THR B 67 | 7.608 | 17.017 | 43.582 | 1.00 | 20.57 | C |
| ATOM 1473 | CB | THR B 67 | 9.066 | 16.619 | 43.871 | 1.00 | 21.25 | C |
| ATOM 1474 | OG1 | THR B 67 | 9.494 | 15.626 | 42.934 | 1.00 | 21.89 | O |
| ATOM 1475 | CG2 | THR B 67 | 9.190 | 16.068 | 45.285 | 1.00 | 25.45 | C |
| ATOM 1476 | C | THR B 67 | 7.186 | 18.043 | 44.639 | 1.00 | 20.93 | C |
| ATOM 1477 | O | THR B 67 | 7.706 | 19.165 | 44.676 | 1.00 | 19.49 | O |
| ATOM 1478 | N | VAL B 68 | 6.244 | 17.655 | 45.494 | 1.00 | 19.26 | N |
| ATOM 1479 | CA | VAL B 68 | 5.784 | 18.536 | 46.556 | 1.00 | 18.83 | C |
| ATOM 1480 | CB | VAL B 68 | 4.248 | 18.560 | 46.641 | 1.00 | 18.95 | C |
| ATOM 1481 | CG1 | VAL B 68 | 3.809 | 19.454 | 47.803 | 1.00 | 21.13 | C |
| ATOM 1482 | CG2 | VAL B 68 | 3.675 | 19.070 | 45.313 | 1.00 | 17.89 | C |
| ATOM 1483 | C | VAL B 68 | 6.382 | 18.032 | 47.848 | 1.00 | 17.05 | C |
| ATOM 1484 | O | VAL B 68 | 6.272 | 16.850 | 48.180 | 1.00 | 18.24 | O |
| ATOM 1485 | N | VAL B 69 | 7.038 | 18.938 | 48.567 | 1.00 | 17.22 | N |
| ATOM 1486 | CA | VAL B 69 | 7.722 | 18.593 | 49.807 | 1.00 | 15.35 | C |
| ATOM 1487 | CB | VAL B 69 | 9.151 | 19.150 | 49.772 | 1.00 | 15.47 | C |
| ATOM 1488 | CG1 | VAL B 69 | 9.898 | 18.773 | 51.041 | 1.00 | 13.26 | C |
| ATOM 1489 | CG2 | VAL B 69 | 9.875 | 18.625 | 48.511 | 1.00 | 15.70 | C |
| ATOM 1490 | C | VAL B 69 | 7.027 | 19.086 | 51.077 | 1.00 | 15.51 | C |
| ATOM 1491 | O | VAL B 69 | 6.649 | 20.256 | 51.182 | 1.00 | 16.17 | O |
| ATOM 1492 | N | ALA B 70 | 6.863 | 18.181 | 52.036 | 1.00 | 16.04 | N |
| ATOM 1493 | CA | ALA B 70 | 6.229 | 18.511 | 53.316 | 1.00 | 16.88 | C |
| ATOM 1494 | CB | ALA B 70 | 4.739 | 18.155 | 53.280 | 1.00 | 17.76 | C |
| ATOM 1495 | C | ALA B 70 | 6.937 | 17.725 | 54.413 | 1.00 | 16.99 | C |
| ATOM 1496 | O | ALA B 70 | 7.651 | 16.766 | 54.126 | 1.00 | 19.87 | O |
| ATOM 1497 | N | PRO B 71 | 6.742 | 18.110 | 55.687 | 1.00 | 18.18 | N |
| ATOM 1498 | CA | PRO B 71 | 7.399 | 17.407 | 56.796 | 1.00 | 17.02 | C |
| ATOM 1499 | CB | PRO B 71 | 6.943 | 18.192 | 58.024 | 1.00 | 18.58 | C |
| ATOM 1500 | CG | PRO B 71 | 6.643 | 19.577 | 57.471 | 1.00 | 19.30 | C |
| ATOM 1501 | CD | PRO B 71 | 5.930 | 19.236 | 56.192 | 1.00 | 16.39 | C |
| ATOM 1502 | C | PRO B 71 | 7.034 | 15.930 | 56.905 | 1.00 | 18.37 | C |
| ATOM 1503 | O | PRO B 71 | 5.907 | 15.537 | 56.594 | 1.00 | 15.78 | O |
| ATOM 1504 | N | SER B 72 | 7.993 | 15.122 | 57.353 | 1.00 | 18.33 | N |
| ATOM 1505 | CA | SER B 72 | 7.769 | 13.699 | 57.529 | 1.00 | 20.27 | C |
| ATOM 1506 | CB | SER B 72 | 8.978 | 12.884 | 57.061 | 1.00 | 21.15 | C |
| ATOM 1507 | OG | SER B 72 | 10.036 | 12.957 | 57.997 | 1.00 | 23.81 | O |
| ATOM 1508 | C | SER B 72 | 7.535 | 13.482 | 59.016 | 1.00 | 21.63 | C |
| ATOM 1509 | O | SER B 72 | 7.788 | 14.377 | 59.825 | 1.00 | 22.26 | O |
| ATOM 1510 | N | THR B 73 | 7.058 | 12.297 | 59.378 | 1.00 | 22.59 | N |
| ATOM 1511 | CA | THR B 73 | 6.757 | 12.006 | 60.779 | 1.00 | 23.74 | C |
| ATOM 1512 | CB | THR B 73 | 6.236 | 10.564 | 60.952 | 1.00 | 22.91 | C |
| ATOM 1513 | OG1 | THR B 73 | 5.172 | 10.318 | 60.025 | 1.00 | 18.94 | O |
| ATOM 1514 | CG2 | THR B 73 | 5.697 | 10.368 | 62.375 | 1.00 | 24.56 | C |
| ATOM 1515 | C | THR B 73 | 7.926 | 12.201 | 61.747 | 1.00 | 24.50 | C |
| ATOM 1516 | O | THR B 73 | 7.758 | 12.806 | 62.800 | 1.00 | 24.98 | O |
| ATOM 1517 | N | GLU B 74 | 9.101 | 11.684 | 61.389 | 1.00 | 25.95 | N |
| ATOM 1518 | CA | GLU B 74 | 10.287 | 11.785 | 62.243 | 1.00 | 26.06 | C |
| ATOM 1519 | CB | GLU B 74 | 11.271 | 10.657 | 61.926 | 1.00 | 26.86 | C |
| ATOM 1520 | CG | GLU B 74 | 10.734 | 9.266 | 62.197 | 1.00 | 29.93 | C |
| ATOM 1521 | CD | GLU B 74 | 10.240 | 9.113 | 63.612 | 1.00 | 34.17 | C |
| ATOM 1522 | OE1 | GLU B 74 | 11.018 | 9.405 | 64.549 | 1.00 | 38.32 | O |
| ATOM 1523 | OE2 | GLU B 74 | 9.075 | 8.701 | 63.790 | 1.00 | 39.68 | O |
| ATOM 1524 | C | GLU B 74 | 11.028 | 13.105 | 62.140 | 1.00 | 26.53 | C |
| ATOM 1525 | O | GLU B 74 | 12.075 | 13.269 | 62.754 | 1.00 | 26.06 | O |
| ATOM 1526 | N | GLY B 75 | 10.495 | 14.042 | 61.365 | 1.00 | 26.90 | N |
| ATOM 1527 | CA | GLY B 75 | 11.163 | 15.321 | 61.217 | 1.00 | 26.25 | C |
| ATOM 1528 | C | GLY B 75 | 11.902 | 15.432 | 59.895 | 1.00 | 26.38 | C |
| ATOM 1529 | O | GLY B 75 | 12.521 | 16.458 | 59.607 | 1.00 | 27.95 | O |
| ATOM 1530 | N | GLY B 76 | 11.854 | 14.367 | 59.097 | 1.00 | 24.68 | N |
| ATOM 1531 | CA | GLY B 76 | 12.498 | 14.376 | 57.796 | 1.00 | 22.08 | C |
| ATOM 1532 | C | GLY B 76 | 11.570 | 14.981 | 56.751 | 1.00 | 21.83 | C |
| ATOM 1533 | O | GLY B 76 | 10.769 | 15.857 | 57.076 | 1.00 | 19.87 | O |
| ATOM 1534 | N | LEU B 77 | 11.655 | 14.508 | 55.505 | 1.00 | 20.87 | N |
| ATOM 1535 | CA | LEU B 77 | 10.820 | 15.038 | 54.433 | 1.00 | 19.22 | C |
| ATOM 1536 | CB | LEU B 77 | 11.679 | 15.795 | 53.418 | 1.00 | 19.77 | C |
| ATOM 1537 | CG | LEU B 77 | 12.495 | 17.003 | 53.873 | 1.00 | 21.45 | C |
| ATOM 1538 | CD1 | LEU B 77 | 13.547 | 17.334 | 52.816 | 1.00 | 23.98 | C |
| ATOM 1539 | CD2 | LEU B 77 | 11.577 | 18.164 | 54.122 | 1.00 | 17.71 | C |
| ATOM 1540 | C | LEU B 77 | 9.981 | 14.022 | 53.657 | 1.00 | 20.65 | C |
| ATOM 1541 | O | LEU B 77 | 10.445 | 12.943 | 53.291 | 1.00 | 20.00 | O |
| ATOM 1542 | N | ASN B 78 | 8.731 | 14.392 | 53.410 | 1.00 | 19.49 | N |
| ATOM 1543 | CA | ASN B 78 | 7.822 | 13.577 | 52.629 | 1.00 | 19.83 | C |
| ATOM 1544 | CB | ASN B 78 | 6.382 | 13.725 | 53.137 | 1.00 | 20.64 | C |
| ATOM 1545 | CG | ASN B 78 | 5.973 | 12.637 | 54.111 | 1.00 | 21.67 | C |
| ATOM 1546 | OD1 | ASN B 78 | 6.799 | 11.869 | 54.604 | 1.00 | 18.96 | O |
| ATOM 1547 | ND2 | ASN B 78 | 4.679 | 12.575 | 54.398 | 1.00 | 16.01 | N |
| ATOM 1548 | C | ASN B 78 | 7.923 | 14.203 | 51.241 | 1.00 | 19.96 | C |
| ATOM 1549 | O | ASN B 78 | 7.819 | 15.424 | 51.106 | 1.00 | 21.03 | O |
| ATOM 1550 | N | LEU B 79 | 8.154 | 13.379 | 50.227 | 1.00 | 19.97 | N |
| ATOM 1551 | CA | LEU B 79 | 8.243 | 13.857 | 48.855 | 1.00 | 20.38 | C |
| ATOM 1552 | CB | LEU B 79 | 9.607 | 13.528 | 48.224 | 1.00 | 21.06 | C |
| ATOM 1553 | CG | LEU B 79 | 10.830 | 14.410 | 48.503 | 1.00 | 20.33 | C |
| ATOM 1554 | CD1 | LEU B 79 | 11.176 | 14.376 | 49.981 | 1.00 | 15.77 | C |
| ATOM 1555 | CD2 | LEU B 79 | 12.006 | 13.919 | 47.655 | 1.00 | 25.25 | C |
| ATOM 1556 | C | LEU B 79 | 7.153 | 13.157 | 48.055 | 1.00 | 21.10 | C |
| ATOM 1557 | O | LEU B 79 | 7.196 | 11.938 | 47.865 | 1.00 | 20.88 | O |
| ATOM 1558 | N | THR B 80 | 6.164 | 13.926 | 47.609 | 1.00 | 20.87 | N |
| ATOM 1559 | CA | THR B 80 | 5.086 | 13.365 | 46.810 | 1.00 | 20.85 | C |
| ATOM 1560 | CB | THR B 80 | 3.702 | 13.899 | 47.273 | 1.00 | 20.49 | C |
| ATOM 1561 | OG1 | THR B 80 | 3.477 | 13.532 | 48.643 | 1.00 | 20.65 | O |
| ATOM 1562 | CG2 | THR B 80 | 2.587 | 13.314 | 46.415 | 1.00 | 19.59 | C |
| ATOM 1563 | C | THR B 80 | 5.363 | 13.783 | 45.370 | 1.00 | 20.80 | C |
| ATOM 1564 | O | THR B 80 | 5.304 | 14.963 | 45.037 | 1.00 | 20.97 | O |
| ATOM 1565 | N | SER B 81 | 5.691 | 12.816 | 44.525 | 1.00 | 21.75 | N |
| ATOM 1566 | CA | SER B 81 | 6.002 | 13.107 | 43.132 | 1.00 | 22.99 | C |
| ATOM 1567 | CB | SER B 81 | 7.352 | 12.494 | 42.752 | 1.00 | 22.82 | C |
| ATOM 1568 | OG | SER B 81 | 8.418 | 13.029 | 43.522 | 1.00 | 27.07 | O |
| ATOM 1569 | C | SER B 81 | 4.951 | 12.612 | 42.145 | 1.00 | 24.46 | C |
| ATOM 1570 | O | SER B 81 | 4.392 | 11.514 | 42.285 | 1.00 | 24.48 | O |
| ATOM 1571 | N | THR B 82 | 4.688 | 13.445 | 41.147 | 1.00 | 25.52 | N |
| ATOM 1572 | CA | THR B 82 | 3.744 | 13.130 | 40.085 | 1.00 | 26.98 | C |
| ATOM 1573 | CB | THR B 82 | 2.716 | 14.253 | 39.910 | 1.00 | 27.26 | C |
| ATOM 1574 | OG1 | THR B 82 | 1.968 | 14.397 | 41.124 | 1.00 | 28.56 | O |
| ATOM 1575 | CG2 | THR B 82 | 1.768 | 13.939 | 38.759 | 1.00 | 26.26 | C |
| ATOM 1576 | C | THR B 82 | 4.618 | 13.023 | 38.841 | 1.00 | 28.72 | C |
| ATOM 1577 | O | THR B 82 | 5.343 | 13.964 | 38.507 | 1.00 | 28.19 | O |
| ATOM 1578 | N | PHE B 83 | 4.560 | 11.877 | 38.166 | 1.00 | 30.09 | N |
| ATOM 1579 | CA | PHE B 83 | 5.385 | 11.659 | 36.989 | 1.00 | 33.13 | C |
| ATOM 1580 | CB | PHE B 83 | 6.702 | 11.011 | 37.408 | 1.00 | 33.32 | C |
| ATOM 1581 | CG | PHE B 83 | 6.532 | 9.719 | 38.164 | 1.00 | 35.08 | C |
| ATOM 1582 | CD1 | PHE B 83 | 6.000 | 9.710 | 39.452 | 1.00 | 35.79 | C |
| ATOM 1583 | CE1 | PHE B 83 | 5.858 | 8.515 | 40.160 | 1.00 | 37.06 | C |
| ATOM 1584 | CZ | PHE B 83 | 6.250 | 7.309 | 39.580 | 1.00 | 35.12 | C |
| ATOM 1585 | CE2 | PHE B 83 | 6.780 | 7.305 | 38.297 | 1.00 | 34.32 | C |
| ATOM 1586 | CD2 | PHE B 83 | 6.919 | 8.508 | 37.595 | 1.00 | 35.78 | C |
| ATOM 1587 | C | PHE B 83 | 4.736 | 10.807 | 35.904 | 1.00 | 35.08 | C |
| ATOM 1588 | O | PHE B 83 | 3.757 | 10.104 | 36.145 | 1.00 | 35.20 | O |
| ATOM 1589 | N | LEU B 84 | 5.300 | 10.888 | 34.703 | 1.00 | 37.29 | N |
| ATOM 1590 | CA | LEU B 84 | 4.827 | 10.126 | 33.552 | 1.00 | 39.17 | C |
| ATOM 1591 | CB | LEU B 84 | 5.018 | 10.947 | 32.273 | 1.00 | 38.63 | C |
| ATOM 1592 | CG | LEU B 84 | 4.592 | 10.337 | 30.934 | 1.00 | 37.74 | C |
| ATOM 1593 | CD1 | LEU B 84 | 3.086 | 10.104 | 30.921 | 1.00 | 36.04 | C |
| ATOM 1594 | CD2 | LEU B 84 | 4.997 | 11.274 | 29.806 | 1.00 | 35.51 | C |
| ATOM 1595 | C | LEU B 84 | 5.663 | 8.847 | 33.485 | 1.00 | 40.90 | C |
| ATOM 1596 | O | LEU B 84 | 6.877 | 8.911 | 33.298 | 1.00 | 40.46 | O |
| ATOM 1597 | N | ARG B 85 | 5.014 | 7.696 | 33.651 | 1.00 | 43.31 | N |
| ATOM 1598 | CA | ARG B 85 | 5.705 | 6.404 | 33.628 | 1.00 | 46.39 | C |
| ATOM 1599 | CB | ARG B 85 | 5.027 | 5.426 | 34.582 | 1.00 | 46.96 | C |
| ATOM 1600 | CG | ARG B 85 | 5.722 | 4.082 | 34.680 | 1.00 | 47.76 | C |
| ATOM 1601 | CD | ARG B 85 | 6.855 | 4.137 | 35.685 | 1.00 | 50.67 | C |
| ATOM 1602 | NE | ARG B 85 | 7.456 | 2.829 | 35.941 | 1.00 | 52.88 | N |
| ATOM 1603 | CZ | ARG B 85 | 6.776 | 1.731 | 36.263 | 1.00 | 55.13 | C |
| ATOM 1604 | NH1 | ARG B 85 | 5.451 | 1.761 | 36.367 | 1.00 | 55.46 | N |
| ATOM 1605 | NH2 | ARG B 85 | 7.425 | 0.598 | 36.499 | 1.00 | 54.74 | N |
| ATOM 1606 | C | ARG B 85 | 5.710 | 5.788 | 32.237 | 1.00 | 48.35 | C |
| ATOM 1607 | O | ARG B 85 | 6.766 | 5.559 | 31.646 | 1.00 | 49.00 | O |
| ATOM 1608 | N | LYS B 86 | 4.514 | 5.500 | 31.735 | 1.00 | 49.68 | N |
| ATOM 1609 | CA | LYS B 86 | 4.346 | 4.913 | 30.414 | 1.00 | 50.77 | C |
| ATOM 1610 | CB | LYS B 86 | 4.293 | 3.390 | 30.521 | 1.00 | 50.85 | C |
| ATOM 1611 | CG | LYS B 86 | 5.499 | 2.811 | 31.242 | 1.00 | 52.18 | C |
| ATOM 1612 | CD | LYS B 86 | 5.446 | 1.302 | 31.337 | 1.00 | 55.10 | C |
| ATOM 1613 | CE | LYS B 86 | 6.667 | 0.775 | 32.067 | 1.00 | 56.19 | C |
| ATOM 1614 | NZ | LYS B 86 | 6.681 | -0.709 | 32.134 | 1.00 | 54.56 | N |
| ATOM 1615 | C | LYS B 86 | 3.038 | 5.457 | 29.872 | 1.00 | 51.30 | C |
| ATOM 1616 | O | LYS B 86 | 2.026 | 4.760 | 29.839 | 1.00 | 51.59 | O |
| ATOM 1617 | N | ASN B 87 | 3.072 | 6.720 | 29.460 | 1.00 | 51.82 | N |
| ATOM 1618 | CA | ASN B 87 | 1.896 | 7.397 | 28.939 | 1.00 | 52.40 | C |
| ATOM 1619 | CB | ASN B 87 | 1.362 | 6.700 | 27.685 | 1.00 | 53.21 | C |
| ATOM 1620 | CG | ASN B 87 | 2.156 | 7.048 | 26.445 | 1.00 | 54.72 | C |
| ATOM 1621 | OD1 | ASN B 87 | 3.274 | 6.565 | 26.247 | 1.00 | 56.95 | O |
| ATOM 1622 | ND2 | ASN B 87 | 1.587 | 7.905 | 25.605 | 1.00 | 55.62 | N |
| ATOM 1623 | C | ASN B 87 | 0.814 | 7.449 | 30.001 | 1.00 | 51.79 | C |
| ATOM 1624 | O | ASN B 87 | -0.375 | 7.490 | 29.688 | 1.00 | 52.58 | O |
| ATOM 1625 | N | GLN B 88 | 1.235 | 7.437 | 31.261 | 1.00 | 51.17 | N |
| ATOM 1626 | CA | GLN B 88 | 0.303 | 7.511 | 32.380 | 1.00 | 50.52 | C |
| ATOM 1627 | CB | GLN B 88 | -0.030 | 6.124 | 32.940 | 1.00 | 50.99 | C |
| ATOM 1628 | CG | GLN B 88 | -1.045 | 6.212 | 34.090 | 1.00 | 52.80 | C |
| ATOM 1629 | CD | GLN B 88 | -1.101 | 4.976 | 34.971 | 1.00 | 55.25 | C |
| ATOM 1630 | OE1 | GLN B 88 | -0.099 | 4.567 | 35.561 | 1.00 | 54.92 | O |
| ATOM 1631 | NE2 | GLN B 88 | -2.285 | 4.382 | 35.076 | 1.00 | 55.03 | N |
| ATOM 1632 | C | GLN B 88 | 0.882 | 8.350 | 33.512 | 1.00 | 49.33 | C |
| ATOM 1633 | O | GLN B 88 | 2.079 | 8.292 | 33.792 | 1.00 | 48.79 | O |
| ATOM 1634 | N | CYS B 89 | 0.025 | 9.129 | 34.161 | 1.00 | 48.09 | N |
| ATOM 1635 | CA | CYS B 89 | 0.451 | 9.956 | 35.278 | 1.00 | 47.25 | C |
| ATOM 1636 | CB | CYS B 89 | -0.463 | 11.173 | 35.441 | 1.00 | 47.96 | C |
| ATOM 1637 | SG | CYS B 89 | -0.440 | 12.321 | 34.033 | 1.00 | 52.30 | S |
| ATOM 1638 | C | CYS B 89 | 0.417 | 9.134 | 36.557 | 1.00 | 45.36 | C |
| ATOM 1639 | O | CYS B 89 | -0.634 | 8.637 | 36.961 | 1.00 | 45.21 | O |
| ATOM 1640 | N | GLU B 90 | 1.573 | 8.986 | 37.187 | 1.00 | 42.70 | N |
| ATOM 1641 | CA | GLU B 90 | 1.662 | 8.234 | 38.423 | 1.00 | 41.56 | C |
| ATOM 1642 | CB | GLU B 90 | 2.663 | 7.085 | 38.283 | 1.00 | 41.43 | C |
| ATOM 1643 | CG | GLU B 90 | 2.074 | 5.833 | 37.660 | 1.00 | 45.26 | C |
| ATOM 1644 | CD | GLU B 90 | 3.097 | 4.728 | 37.493 | 1.00 | 48.56 | C |
| ATOM 1645 | OE1 | GLU B 90 | 3.894 | 4.508 | 38.430 | 1.00 | 49.59 | O |
| ATOM 1646 | OE2 | GLU B 90 | 3.099 | 4.071 | 36.430 | 1.00 | 50.95 | O |
| ATOM 1647 | C | GLU B 90 | 2.075 | 9.146 | 39.564 | 1.00 | 40.06 | C |
| ATOM 1648 | O | GLU B 90 | 2.608 | 10.234 | 39.347 | 1.00 | 39.58 | O |
| ATOM 1649 | N | THR B 91 | 1.809 | 8.697 | 40.781 | 1.00 | 37.76 | N |
| ATOM 1650 | CA | THR B 91 | 2.159 | 9.455 | 41.964 | 1.00 | 37.06 | C |
| ATOM 1651 | CB | THR B 91 | 0.922 | 10.101 | 42.610 | 1.00 | 37.34 | C |
| ATOM 1652 | OG1 | THR B 91 | 0.602 | 11.308 | 41.908 | 1.00 | 37.99 | O |
| ATOM 1653 | CG2 | THR B 91 | 1.180 | 10.428 | 44.069 | 1.00 | 36.87 | C |
| ATOM 1654 | C | THR B 91 | 2.815 | 8.524 | 42.956 | 1.00 | 35.97 | C |
| ATOM 1655 | O | THR B 91 | 2.307 | 7.442 | 43.234 | 1.00 | 36.79 | O |
| ATOM 1656 | N | LYS B 92 | 3.961 | 8.942 | 43.470 | 1.00 | 34.66 | N |
| ATOM 1657 | CA | LYS B 92 | 4.670 | 8.141 | 44.442 | 1.00 | 34.25 | C |
| ATOM 1658 | CB | LYS B 92 | 5.930 | 7.526 | 43.823 | 1.00 | 35.39 | C |
| ATOM 1659 | CG | LYS B 92 | 6.573 | 6.473 | 44.724 | 1.00 | 40.91 | C |
| ATOM 1660 | CD | LYS B 92 | 7.770 | 5.797 | 44.073 | 1.00 | 47.37 | C |
| ATOM 1661 | CE | LYS B 92 | 8.295 | 4.668 | 44.950 | 1.00 | 49.29 | C |
| ATOM 1662 | NZ | LYS B 92 | 9.460 | 3.958 | 44.346 | 1.00 | 51.25 | N |
| ATOM 1663 | C | LYS B 92 | 5.047 | 9.016 | 45.624 | 1.00 | 32.07 | C |
| ATOM 1664 | O | LYS B 92 | 5.201 | 10.229 | 45.492 | 1.00 | 31.59 | O |
| ATOM 1665 | N | ILE B 93 | 5.177 | 8.394 | 46.787 | 1.00 | 30.05 | N |
| ATOM 1666 | CA | ILE B 93 | 5.564 | 9.110 | 47.986 | 1.00 | 29.86 | C |
| ATOM 1667 | CB | ILE B 93 | 4.489 | 9.004 | 49.082 | 1.00 | 28.91 | C |
| ATOM 1668 | CG1 | ILE B 93 | 3.221 | 9.726 | 48.619 | 1.00 | 29.22 | C |
| ATOM 1669 | CD1 | ILE B 93 | 2.075 | 9.651 | 49.590 | 1.00 | 33.59 | C |
| ATOM 1670 | CG2 | ILE B 93 | 5.006 | 9.615 | 50.381 | 1.00 | 31.24 | C |
| ATOM 1671 | C | ILE B 93 | 6.865 | 8.524 | 48.500 | 1.00 | 29.02 | C |
| ATOM 1672 | O | ILE B 93 | 6.973 | 7.314 | 48.697 | 1.00 | 29.59 | O |
| ATOM 1673 | N | MET B 94 | 7.855 | 9.389 | 48.683 | 1.00 | 28.74 | N |
| ATOM 1674 | CA | MET B 94 | 9.157 | 8.982 | 49.192 | 1.00 | 28.69 | C |
| ATOM 1675 | CB | MET B 94 | 10.268 | 9.380 | 48.223 | 1.00 | 30.16 | C |
| ATOM 1676 | CG | MET B 94 | 10.133 | 8.774 | 46.852 | 1.00 | 33.76 | C |
| ATOM 1677 | SD | MET B 94 | 11.342 | 9.469 | 45.713 | 1.00 | 42.95 | S |
| ATOM 1678 | CE | MET B 94 | 12.813 | 8.613 | 46.225 | 1.00 | 39.86 | C |
| ATOM 1679 | C | MET B 94 | 9.386 | 9.693 | 50.519 | 1.00 | 26.85 | C |
| ATOM 1680 | O | MET B 94 | 9.071 | 10.879 | 50.671 | 1.00 | 26.13 | O |
| ATOM 1681 | N | VAL B 95 | 9.938 | 8.962 | 51.477 | 1.00 | 24.54 | N |
| ATOM 1682 | CA | VAL B 95 | 10.222 | 9.525 | 52.781 | 1.00 | 23.92 | C |
| ATOM 1683 | CB | VAL B 95 | 9.664 | 8.653 | 53.915 | 1.00 | 23.21 | C |
| ATOM 1684 | CG1 | VAL B 95 | 10.142 | 9.197 | 55.253 | 1.00 | 23.76 | C |
| ATOM 1685 | CG2 | VAL B 95 | 8.133 | 8.611 | 53.859 | 1.00 | 26.68 | C |
| ATOM 1686 | C | VAL B 95 | 11.725 | 9.645 | 53.001 | 1.00 | 23.60 | C |
| ATOM 1687 | O | VAL B 95 | 12.438 | 8.639 | 53.039 | 1.00 | 24.63 | O |
| ATOM 1688 | N | LEU B 96 | 12.199 | 10.880 | 53.122 | 1.00 | 23.77 | N |
| ATOM 1689 | CA | LEU B 96 | 13.605 | 11.130 | 53.408 | 1.00 | 24.17 | C |
| ATOM 1690 | CB | LEU B 96 | 14.077 | 12.432 | 52.762 | 1.00 | 24.53 | C |
| ATOM 1691 | CG | LEU B 96 | 14.079 | 12.475 | 51.231 | 1.00 | 26.20 | C |
| ATOM 1692 | CD1 | LEU B 96 | 14.585 | 13.838 | 50.746 | 1.00 | 29.41 | C |
| ATOM 1693 | CD2 | LEU B 96 | 14.956 | 11.368 | 50.693 | 1.00 | 26.73 | C |
| ATOM 1694 | C | LEU B 96 | 13.682 | 11.241 | 54.926 | 1.00 | 23.73 | C |
| ATOM 1695 | O | LEU B 96 | 13.213 | 12.221 | 55.516 | 1.00 | 22.46 | O |
| ATOM 1696 | N | GLN B 97 | 14.250 | 10.213 | 55.550 | 1.00 | 25.09 | N |
| ATOM 1697 | CA | GLN B 97 | 14.396 | 10.160 | 56.996 | 1.00 | 26.98 | C |
| ATOM 1698 | CB | GLN B 97 | 14.563 | 8.703 | 57.446 | 1.00 | 28.85 | C |
| ATOM 1699 | CG | GLN B 97 | 13.516 | 8.208 | 58.442 | 1.00 | 37.81 | C |
| ATOM 1700 | CD | GLN B 97 | 12.180 | 7.884 | 57.798 | 1.00 | 46.93 | C |
| ATOM 1701 | OE1 | GLN B 97 | 12.107 | 7.113 | 56.840 | 1.00 | 52.64 | O |
| ATOM 1702 | NE2 | GLN B 97 | 11.116 | 8.464 | 58.329 | 1.00 | 50.81 | N |
| ATOM 1703 | C | GLN B 97 | 15.611 | 10.969 | 57.433 | 1.00 | 26.07 | C |
| ATOM 1704 | O | GLN B 97 | 16.664 | 10.901 | 56.811 | 1.00 | 25.85 | O |
| ATOM 1705 | N | PRO B 98 | 15.489 | 11.725 | 58.533 | 1.00 | 27.95 | N |
| ATOM 1706 | CA | PRO B 98 | 16.634 | 12.522 | 58.991 | 1.00 | 28.20 | C |
| ATOM 1707 | CB | PRO B 98 | 16.143 | 13.128 | 60.310 | 1.00 | 27.26 | C |
| ATOM 1708 | CG | PRO B 98 | 14.649 | 12.996 | 60.249 | 1.00 | 31.44 | C |
| ATOM 1709 | CD | PRO B 98 | 14.448 | 11.672 | 59.567 | 1.00 | 26.47 | C |
| ATOM 1710 | C | PRO B 98 | 17.848 | 11.616 | 59.204 | 1.00 | 28.83 | C |
| ATOM 1711 | O | PRO B 98 | 17.701 | 10.470 | 59.647 | 1.00 | 30.17 | O |
| ATOM 1712 | N | ALA B 99 | 19.041 | 12.124 | 58.902 | 1.00 | 28.42 | N |
| ATOM 1713 | CA | ALA B 99 | 20.254 | 11.331 | 59.076 | 1.00 | 28.17 | C |
| ATOM 1714 | CB | ALA B 99 | 20.883 | 11.034 | 57.709 | 1.00 | 26.53 | C |
| ATOM 1715 | C | ALA B 99 | 21.298 | 11.954 | 60.013 | 1.00 | 28.73 | C |
| ATOM 1716 | O | ALA B 99 | 22.477 | 12.021 | 59.681 | 1.00 | 30.28 | O |
| ATOM 1717 | N | GLY B 100 | 20.860 | 12.424 | 61.174 | 1.00 | 30.15 | N |
| ATOM 1718 | CA | GLY B 100 | 21.788 | 12.988 | 62.143 | 1.00 | 30.98 | C |
| ATOM 1719 | C | GLY B 100 | 22.332 | 14.399 | 61.954 | 1.00 | 32.27 | C |
| ATOM 1720 | O | GLY B 100 | 23.053 | 14.891 | 62.833 | 1.00 | 32.39 | O |
| ATOM 1721 | N | ALA B 101 | 22.005 | 15.050 | 60.836 | 1.00 | 30.10 | N |
| ATOM 1722 | CA | ALA B 101 | 22.483 | 16.407 | 60.575 | 1.00 | 28.93 | C |
| ATOM 1723 | CB | ALA B 101 | 23.926 | 16.364 | 60.098 | 1.00 | 29.35 | C |
| ATOM 1724 | C | ALA B 101 | 21.616 | 17.136 | 59.546 | 1.00 | 27.78 | C |
| ATOM 1725 | O | ALA B 101 | 21.133 | 16.536 | 58.587 | 1.00 | 27.81 | O |
| ATOM 1726 | N | PRO B 102 | 21.428 | 18.455 | 59.724 | 1.00 | 27.63 | N |
| ATOM 1727 | CA | PRO B 102 | 20.606 | 19.232 | 58.789 | 1.00 | 27.52 | C |
| ATOM 1728 | CB | PRO B 102 | 20.770 | 20.668 | 59.286 | 1.00 | 27.31 | C |
| ATOM 1729 | CG | PRO B 102 | 21.021 | 20.482 | 60.767 | 1.00 | 28.49 | C |
| ATOM 1730 | CD | PRO B 102 | 21.980 | 19.317 | 60.785 | 1.00 | 27.05 | C |
| ATOM 1731 | C | PRO B 102 | 21.060 | 19.069 | 57.345 | 1.00 | 26.25 | C |
| ATOM 1732 | O | PRO B 102 | 22.233 | 19.265 | 57.034 | 1.00 | 26.32 | O |
| ATOM 1733 | N | GLY B 103 | 20.125 | 18.706 | 56.471 | 1.00 | 24.95 | N |
| ATOM 1734 | CA | GLY B 103 | 20.448 | 18.533 | 55.066 | 1.00 | 23.66 | C |
| ATOM 1735 | C | GLY B 103 | 20.925 | 17.135 | 54.711 | 1.00 | 20.21 | C |
| ATOM 1736 | O | GLY B 103 | 21.343 | 16.895 | 53.578 | 1.00 | 21.21 | O |
| ATOM 1737 | N | HIS B 104 | 20.857 | 16.218 | 55.672 | 1.00 | 17.17 | N |
| ATOM 1738 | CA | HIS B 104 | 21.280 | 14.844 | 55.451 | 1.00 | 19.03 | C |
| ATOM 1739 | CB | HIS B 104 | 22.481 | 14.536 | 56.334 | 1.00 | 17.66 | C |
| ATOM 1740 | CG | HIS B 104 | 23.672 | 15.379 | 56.015 | 1.00 | 21.52 | C |
| ATOM 1741 | ND1 | HIS B 104 | 24.690 | 14.942 | 55.196 | 1.00 | 17.48 | N |
| ATOM 1742 | CE1 | HIS B 104 | 25.546 | 15.929 | 55.002 | 1.00 | 19.71 | C |
| ATOM 1743 | NE2 | HIS B 104 | 25.123 | 16.987 | 55.671 | 1.00 | 20.79 | N |
| ATOM 1744 | CD2 | HIS B 104 | 23.956 | 16.669 | 56.318 | 1.00 | 19.37 | C |
| ATOM 1745 | C | HIS B 104 | 20.136 | 13.897 | 55.747 | 1.00 | 16.96 | C |
| ATOM 1746 | O | HIS B 104 | 19.540 | 13.953 | 56.810 | 1.00 | 16.73 | O |
| ATOM 1747 | N | TYR B 105 | 19.836 | 13.022 | 54.795 | 1.00 | 19.61 | N |
| ATOM 1748 | CA | TYR B 105 | 18.735 | 12.080 | 54.945 | 1.00 | 21.07 | C |
| ATOM 1749 | CB | TYR B 105 | 17.532 | 12.543 | 54.123 | 1.00 | 21.53 | C |
| ATOM 1750 | CG | TYR B 105 | 17.107 | 13.974 | 54.357 | 1.00 | 21.91 | C |
| ATOM 1751 | CD1 | TYR B 105 | 16.219 | 14.302 | 55.389 | 1.00 | 20.70 | C |
| ATOM 1752 | CE1 | TYR B 105 | 15.843 | 15.621 | 55.616 | 1.00 | 22.27 | C |
| ATOM 1753 | CZ | TYR B 105 | 16.354 | 16.636 | 54.807 | 1.00 | 23.20 | C |
| ATOM 1754 | OH | TYR B 105 | 15.977 | 17.950 | 55.020 | 1.00 | 23.16 | O |
| ATOM 1755 | CE2 | TYR B 105 | 17.237 | 16.335 | 53.776 | 1.00 | 18.32 | C |
| ATOM 1756 | CD2 | TYR B 105 | 17.608 | 15.007 | 53.555 | 1.00 | 21.82 | C |
| ATOM 1757 | C | TYR B 105 | 19.106 | 10.698 | 54.451 | 1.00 | 21.92 | C |
| ATOM 1758 | O | TYR B 105 | 20.104 | 10.514 | 53.763 | 1.00 | 23.47 | O |
| ATOM 1759 | N | THR B 106 | 18.267 | 9.733 | 54.790 | 1.00 | 24.22 | N |
| ATOM 1760 | CA | THR B 106 | 18.438 | 8.364 | 54.333 | 1.00 | 26.64 | C |
| ATOM 1761 | CB | THR B 106 | 18.666 | 7.393 | 55.504 | 1.00 | 25.91 | C |
| ATOM 1762 | OG1 | THR B 106 | 17.607 | 7.541 | 56.463 | 1.00 | 30.22 | O |
| ATOM 1763 | CG2 | THR B 106 | 20.011 | 7.689 | 56.174 | 1.00 | 26.89 | C |
| ATOM 1764 | C | THR B 106 | 17.126 | 8.029 | 53.635 | 1.00 | 27.69 | C |
| ATOM 1765 | O | THR B 106 | 16.056 | 8.487 | 54.057 | 1.00 | 25.39 | O |
| ATOM 1766 | N | TYR B 107 | 17.214 | 7.253 | 52.561 | 1.00 | 29.73 | N |
| ATOM 1767 | CA | TYR B 107 | 16.038 | 6.864 | 51.801 | 1.00 | 33.54 | C |
| ATOM 1768 | CB | TYR B 107 | 15.924 | 7.714 | 50.537 | 1.00 | 33.57 | C |
| ATOM 1769 | CG | TYR B 107 | 15.054 | 7.073 | 49.482 | 1.00 | 37.64 | C |
| ATOM 1770 | CD1 | TYR B 107 | 13.708 | 6.794 | 49.738 | 1.00 | 41.90 | C |
| ATOM 1771 | CE1 | TYR B 107 | 12.909 | 6.159 | 48.790 | 1.00 | 43.55 | C |
| ATOM 1772 | CZ | TYR B 107 | 13.459 | 5.792 | 47.571 | 1.00 | 46.00 | C |
| ATOM 1773 | OH | TYR B 107 | 12.673 | 5.158 | 46.635 | 1.00 | 49.14 | O |
| ATOM 1774 | CE2 | TYR B 107 | 14.796 | 6.060 | 47.292 | 1.00 | 43.74 | C |
| ATOM 1775 | CD2 | TYR B 107 | 15.583 | 6.702 | 48.247 | 1.00 | 38.84 | C |
| ATOM 1776 | C | TYR B 107 | 16.061 | 5.388 | 51.420 | 1.00 | 36.23 | C |
| ATOM 1777 | O | TYR B 107 | 17.018 | 4.899 | 50.818 | 1.00 | 35.45 | O |
| ATOM 1778 | N | SER B 108 | 14.993 | 4.681 | 51.769 | 1.00 | 40.28 | N |
| ATOM 1779 | CA | SER B 108 | 14.877 | 3.259 | 51.470 | 1.00 | 44.66 | C |
| ATOM 1780 | CB | SER B 108 | 13.988 | 2.580 | 52.506 | 1.00 | 44.48 | C |
| ATOM 1781 | OG | SER B 108 | 13.351 | 1.451 | 51.940 | 1.00 | 47.59 | O |
| ATOM 1782 | C | SER B 108 | 14.306 | 2.996 | 50.086 | 1.00 | 47.15 | C |
| ATOM 1783 | O | SER B 108 | 13.296 | 3.581 | 49.703 | 1.00 | 47.89 | O |
| ATOM 1784 | N | SER B 109 | 14.943 | 2.094 | 49.348 | 1.00 | 50.48 | N |
| ATOM 1785 | CA | SER B 109 | 14.487 | 1.751 | 48.007 | 1.00 | 53.87 | C |
| ATOM 1786 | CB | SER B 109 | 15.609 | 1.994 | 46.993 | 1.00 | 54.04 | C |
| ATOM 1787 | OG | SER B 109 | 15.234 | 1.547 | 45.700 | 1.00 | 57.33 | O |
| ATOM 1788 | C | SER B 109 | 14.023 | 0.298 | 47.920 | 1.00 | 55.77 | C |
| ATOM 1789 | O | SER B 109 | 14.760 | -0.567 | 47.445 | 1.00 | 55.70 | O |
| ATOM 1790 | N | PRO B 110 | 12.791 | 0.010 | 48.379 | 1.00 | 57.97 | N |
| ATOM 1791 | CA | PRO B 110 | 12.247 | -1.352 | 48.342 | 1.00 | 59.41 | C |
| ATOM 1792 | CB | PRO B 110 | 10.771 | -1.137 | 48.658 | 1.00 | 59.43 | C |
| ATOM 1793 | CG | PRO B 110 | 10.826 | -0.010 | 49.639 | 1.00 | 59.26 | C |
| ATOM 1794 | CD | PRO B 110 | 11.814 | 0.940 | 48.977 | 1.00 | 58.27 | C |
| ATOM 1795 | C | PRO B 110 | 12.466 | -2.035 | 46.994 | 1.00 | 60.89 | C |
| ATOM 1796 | O | PRO B 110 | 12.780 | -3.225 | 46.941 | 1.00 | 61.42 | O |
| ATOM 1797 | N | HIS B 111 | 12.298 | -1.279 | 45.908 | 1.00 | 62.25 | N |
| ATOM 1798 | CA | HIS B 111 | 12.501 | -1.818 | 44.564 | 1.00 | 63.28 | C |
| ATOM 1799 | CB | HIS B 111 | 12.336 | -0.724 | 43.502 | 1.00 | 64.21 | C |
| ATOM 1800 | CG | HIS B 111 | 10.952 | -0.166 | 43.405 | 1.00 | 67.26 | C |
| ATOM 1801 | ND1 | HIS B 111 | 10.332 | 0.482 | 44.453 | 1.00 | 69.85 | N |
| ATOM 1802 | CE1 | HIS B 111 | 9.130 | 0.877 | 44.074 | 1.00 | 70.46 | C |
| ATOM 1803 | NE2 | HIS B 111 | 8.946 | 0.508 | 42.818 | 1.00 | 70.26 | N |
| ATOM 1804 | CD2 | HIS B 111 | 10.070 | -0.146 | 42.377 | 1.00 | 69.17 | C |
| ATOM 1805 | C | HIS B 111 | 13.918 | -2.370 | 44.469 | 1.00 | 62.59 | C |
| ATOM 1806 | O | HIS B 111 | 14.148 | -3.571 | 44.615 | 1.00 | 63.03 | O |
| ATOM 1807 | N | SER B 112 | 14.862 | -1.466 | 44.221 | 1.00 | 61.45 | N |
| ATOM 1808 | CA | SER B 112 | 16.272 | -1.804 | 44.101 | 1.00 | 60.38 | C |
| ATOM 1809 | CB | SER B 112 | 17.115 | -0.527 | 44.156 | 1.00 | 60.82 | C |
| ATOM 1810 | OG | SER B 112 | 18.482 | -0.823 | 44.399 | 1.00 | 62.59 | O |
| ATOM 1811 | C | SER B 112 | 16.730 | -2.749 | 45.200 | 1.00 | 58.84 | C |
| ATOM 1812 | O | SER B 112 | 17.523 | -3.661 | 44.962 | 1.00 | 58.90 | O |
| ATOM 1813 | N | GLY B 113 | 16.216 | -2.529 | 46.405 | 1.00 | 57.13 | N |
| ATOM 1814 | CA | GLY B 113 | 16.618 | -3.346 | 47.530 | 1.00 | 55.12 | C |
| ATOM 1815 | C | GLY B 113 | 17.859 | -2.704 | 48.119 | 1.00 | 53.27 | C |
| ATOM 1816 | O | GLY B 113 | 18.847 | -3.378 | 48.414 | 1.00 | 52.87 | O |
| ATOM 1817 | N | SER B 114 | 17.811 | -1.384 | 48.274 | 1.00 | 51.27 | N |
| ATOM 1818 | CA | SER B 114 | 18.942 | -0.654 | 48.827 | 1.00 | 49.51 | C |
| ATOM 1819 | CB | SER B 114 | 19.952 | -0.353 | 47.719 | 1.00 | 50.16 | C |
| ATOM 1820 | OG | SER B 114 | 19.337 | 0.344 | 46.644 | 1.00 | 48.59 | O |
| ATOM 1821 | C | SER B 114 | 18.548 | 0.647 | 49.519 | 1.00 | 48.18 | C |
| ATOM 1822 | O | SER B 114 | 17.461 | 1.183 | 49.296 | 1.00 | 48.12 | O |
| ATOM 1823 | N | ILE B 115 | 19.445 | 1.133 | 50.372 | 1.00 | 46.06 | N |
| ATOM 1824 | CA | ILE B 115 | 19.248 | 2.383 | 51.100 | 1.00 | 44.42 | C |
| ATOM 1825 | CB | ILE B 115 | 19.600 | 2.237 | 52.606 | 1.00 | 44.90 | C |
| ATOM 1826 | CG1 | ILE B 115 | 18.543 | 1.394 | 53.322 | 1.00 | 46.80 | C |
| ATOM 1827 | CD1 | ILE B 115 | 17.184 | 2.066 | 53.435 | 1.00 | 45.52 | C |
| ATOM 1828 | CG2 | ILE B 115 | 19.687 | 3.610 | 53.261 | 1.00 | 46.02 | C |
| ATOM 1829 | C | ILE B 115 | 20.189 | 3.412 | 50.480 | 1.00 | 41.86 | C |
| ATOM 1830 | O | ILE B 115 | 21.241 | 3.060 | 49.949 | 1.00 | 41.37 | O |
| ATOM 1831 | N | HIS B 116 | 19.799 | 4.681 | 50.534 | 1.00 | 39.40 | N |
| ATOM 1832 | CA | HIS B 116 | 20.613 | 5.749 | 49.974 | 1.00 | 36.16 | C |
| ATOM 1833 | CB | HIS B 116 | 19.902 | 6.413 | 48.783 | 1.00 | 36.63 | C |
| ATOM 1834 | CG | HIS B 116 | 19.458 | 5.460 | 47.714 | 1.00 | 37.67 | C |
| ATOM 1835 | ND1 | HIS B 116 | 18.918 | 5.892 | 46.519 | 1.00 | 41.47 | N |
| ATOM 1836 | CE1 | HIS B 116 | 18.570 | 4.847 | 45.790 | 1.00 | 40.60 | C |
| ATOM 1837 | NE2 | HIS B 116 | 18.867 | 3.752 | 46.466 | 1.00 | 38.98 | N |
| ATOM 1838 | CD2 | HIS B 116 | 19.426 | 4.109 | 47.671 | 1.00 | 39.98 | C |
| ATOM 1839 | C | HIS B 116 | 20.874 | 6.810 | 51.037 | 1.00 | 33.97 | C |
| ATOM 1840 | O | HIS B 116 | 20.077 | 6.991 | 51.954 | 1.00 | 32.57 | O |
| ATOM 1841 | N | SER B 117 | 22.003 | 7.498 | 50.908 | 1.00 | 31.18 | N |
| ATOM 1842 | CA | SER B 117 | 22.379 | 8.564 | 51.828 | 1.00 | 30.38 | C |
| ATOM 1843 | CB | SER B 117 | 23.787 | 8.339 | 52.382 | 1.00 | 28.97 | C |
| ATOM 1844 | OG | SER B 117 | 23.801 | 7.236 | 53.268 | 1.00 | 35.19 | O |
| ATOM 1845 | C | SER B 117 | 22.336 | 9.840 | 51.009 | 1.00 | 28.86 | C |
| ATOM 1846 | O | SER B 117 | 23.181 | 10.058 | 50.139 | 1.00 | 28.75 | O |
| ATOM 1847 | N | VAL B 118 | 21.344 | 10.677 | 51.293 | 1.00 | 27.21 | N |
| ATOM 1848 | CA | VAL B 118 | 21.139 | 11.920 | 50.555 | 1.00 | 24.78 | C |
| ATOM 1849 | CB | VAL B 118 | 19.634 | 12.130 | 50.259 | 1.00 | 24.46 | C |
| ATOM 1850 | CG1 | VAL B 118 | 19.417 | 13.417 | 49.484 | 1.00 | 25.74 | C |
| ATOM 1851 | CG2 | VAL B 118 | 19.093 | 10.944 | 49.492 | 1.00 | 28.06 | C |
| ATOM 1852 | C | VAL B 118 | 21.644 | 13.155 | 51.281 | 1.00 | 24.11 | C |
| ATOM 1853 | O | VAL B 118 | 21.399 | 13.338 | 52.469 | 1.00 | 23.10 | O |
| ATOM 1854 | N | SER B 119 | 22.346 | 14.011 | 50.551 | 1.00 | 23.66 | N |
| ATOM 1855 | CA | SER B 119 | 22.838 | 15.238 | 51.135 | 1.00 | 23.20 | C |
| ATOM 1856 | CB | SER B 119 | 24.342 | 15.125 | 51.435 | 1.00 | 23.15 | C |
| ATOM 1857 | OG | SER B 119 | 25.095 | 14.833 | 50.271 | 1.00 | 27.22 | O |
| ATOM 1858 | C | SER B 119 | 22.563 | 16.397 | 50.188 | 1.00 | 21.37 | C |
| ATOM 1859 | O | SER B 119 | 22.489 | 16.216 | 48.978 | 1.00 | 20.94 | O |
| ATOM 1860 | N | VAL B 120 | 22.364 | 17.582 | 50.756 | 1.00 | 22.48 | N |
| ATOM 1861 | CA | VAL B 120 | 22.134 | 18.784 | 49.966 | 1.00 | 21.59 | C |
| ATOM 1862 | CB | VAL B 120 | 21.263 | 19.802 | 50.726 | 1.00 | 23.28 | C |
| ATOM 1863 | CG1 | VAL B 120 | 21.150 | 21.090 | 49.926 | 1.00 | 20.40 | C |
| ATOM 1864 | CG2 | VAL B 120 | 19.861 | 19.218 | 50.963 | 1.00 | 23.55 | C |
| ATOM 1865 | C | VAL B 120 | 23.509 | 19.397 | 49.718 | 1.00 | 21.37 | C |
| ATOM 1866 | O | VAL B 120 | 24.131 | 19.938 | 50.628 | 1.00 | 20.79 | O |
| ATOM 1867 | N | VAL B 121 | 23.990 | 19.297 | 48.488 | 1.00 | 20.82 | N |
| ATOM 1868 | CA | VAL B 121 | 25.299 | 19.838 | 48.147 | 1.00 | 18.66 | C |
| ATOM 1869 | CB | VAL B 121 | 25.717 | 19.392 | 46.738 | 1.00 | 19.78 | C |
| ATOM 1870 | CG1 | VAL B 121 | 27.081 | 20.006 | 46.358 | 1.00 | 20.77 | C |
| ATOM 1871 | CG2 | VAL B 121 | 25.759 | 17.882 | 46.681 | 1.00 | 22.61 | C |
| ATOM 1872 | C | VAL B 121 | 25.274 | 21.362 | 48.192 | 1.00 | 18.80 | C |
| ATOM 1873 | O | VAL B 121 | 26.117 | 22.005 | 48.825 | 1.00 | 15.52 | O |
| ATOM 1874 | N | GLU B 122 | 24.285 | 21.931 | 47.524 | 1.00 | 18.78 | N |
| ATOM 1875 | CA | GLU B 122 | 24.139 | 23.376 | 47.447 | 1.00 | 20.84 | C |
| ATOM 1876 | CB | GLU B 122 | 25.021 | 23.914 | 46.321 | 1.00 | 20.82 | C |
| ATOM 1877 | CG | GLU B 122 | 25.154 | 25.410 | 46.270 | 1.00 | 23.89 | C |
| ATOM 1878 | CD | GLU B 122 | 26.095 | 25.847 | 45.169 | 1.00 | 28.52 | C |
| ATOM 1879 | OE1 | GLU B 122 | 25.625 | 26.400 | 44.154 | 1.00 | 31.82 | O |
| ATOM 1880 | OE2 | GLU B 122 | 27.311 | 25.618 | 45.314 | 1.00 | 29.06 | O |
| ATOM 1881 | C | GLU B 122 | 22.684 | 23.665 | 47.132 | 1.00 | 20.92 | C |
| ATOM 1882 | O | GLU B 122 | 22.026 | 22.865 | 46.460 | 1.00 | 20.92 | O |
| ATOM 1883 | N | ALA B 123 | 22.177 | 24.805 | 47.593 | 1.00 | 22.15 | N |
| ATOM 1884 | CA | ALA B 123 | 20.780 | 25.130 | 47.333 | 1.00 | 23.57 | C |
| ATOM 1885 | CB | ALA B 123 | 19.876 | 24.114 | 48.050 | 1.00 | 21.65 | C |
| ATOM 1886 | C | ALA B 123 | 20.361 | 26.545 | 47.714 | 1.00 | 24.94 | C |
| ATOM 1887 | O | ALA B 123 | 20.909 | 27.143 | 48.633 | 1.00 | 27.17 | O |
| ATOM 1888 | N | ASN B 124 | 19.380 | 27.056 | 46.975 | 1.00 | 25.25 | N |
| ATOM 1889 | CA | ASN B 124 | 18.770 | 28.374 | 47.181 | 1.00 | 25.20 | C |
| ATOM 1890 | CB | ASN B 124 | 19.054 | 29.306 | 46.003 | 1.00 | 25.23 | C |
| ATOM 1891 | CG | ASN B 124 | 18.413 | 30.663 | 46.180 | 1.00 | 29.26 | C |
| ATOM 1892 | OD1 | ASN B 124 | 17.257 | 30.770 | 46.594 | 1.00 | 26.62 | O |
| ATOM 1893 | ND2 | ASN B 124 | 19.158 | 31.715 | 45.854 | 1.00 | 32.85 | N |
| ATOM 1894 | C | ASN B 124 | 17.288 | 28.034 | 47.191 | 1.00 | 23.20 | C |
| ATOM 1895 | O | ASN B 124 | 16.723 | 27.733 | 46.149 | 1.00 | 21.33 | O |
| ATOM 1896 | N | TYR B 125 | 16.657 | 28.088 | 48.359 | 1.00 | 23.13 | N |
| ATOM 1897 | CA | TYR B 125 | 15.259 | 27.704 | 48.475 | 1.00 | 24.00 | C |
| ATOM 1898 | CB | TYR B 125 | 14.801 | 27.806 | 49.934 | 1.00 | 25.22 | C |
| ATOM 1899 | CG | TYR B 125 | 15.077 | 29.124 | 50.607 | 1.00 | 28.94 | C |
| ATOM 1900 | CD1 | TYR B 125 | 14.295 | 30.247 | 50.338 | 1.00 | 31.58 | C |
| ATOM 1901 | CE1 | TYR B 125 | 14.559 | 31.469 | 50.950 | 1.00 | 34.01 | C |
| ATOM 1902 | CZ | TYR B 125 | 15.616 | 31.568 | 51.836 | 1.00 | 34.12 | C |
| ATOM 1903 | OH | TYR B 125 | 15.909 | 32.776 | 52.421 | 1.00 | 38.52 | O |
| ATOM 1904 | CE2 | TYR B 125 | 16.404 | 30.462 | 52.123 | 1.00 | 34.39 | C |
| ATOM 1905 | CD2 | TYR B 125 | 16.131 | 29.252 | 51.508 | 1.00 | 30.83 | C |
| ATOM 1906 | C | TYR B 125 | 14.253 | 28.380 | 47.551 | 1.00 | 24.08 | C |
| ATOM 1907 | O | TYR B 125 | 13.123 | 27.909 | 47.424 | 1.00 | 22.03 | O |
| ATOM 1908 | N | ASP B 126 | 14.652 | 29.468 | 46.900 | 1.00 | 23.69 | N |
| ATOM 1909 | CA | ASP B 126 | 13.753 | 30.150 | 45.976 | 1.00 | 24.21 | C |
| ATOM 1910 | CB | ASP B 126 | 13.891 | 31.668 | 46.068 | 1.00 | 25.29 | C |
| ATOM 1911 | CG | ASP B 126 | 13.295 | 32.230 | 47.316 | 1.00 | 26.34 | C |
| ATOM 1912 | OD1 | ASP B 126 | 12.139 | 31.869 | 47.625 | 1.00 | 29.25 | O |
| ATOM 1913 | OD2 | ASP B 126 | 13.985 | 33.038 | 47.974 | 1.00 | 27.99 | O |
| ATOM 1914 | C | ASP B 126 | 14.072 | 29.764 | 44.554 | 1.00 | 25.46 | C |
| ATOM 1915 | O | ASP B 126 | 13.380 | 30.180 | 43.624 | 1.00 | 26.59 | O |
| ATOM 1916 | N | GLU B 127 | 15.124 | 28.983 | 44.365 | 1.00 | 24.21 | N |
| ATOM 1917 | CA | GLU B 127 | 15.502 | 28.628 | 43.011 | 1.00 | 25.61 | C |
| ATOM 1918 | CB | GLU B 127 | 16.654 | 29.531 | 42.536 | 1.00 | 25.52 | C |
| ATOM 1919 | CG | GLU B 127 | 16.297 | 31.019 | 42.467 | 1.00 | 32.98 | C |
| ATOM 1920 | CD | GLU B 127 | 17.396 | 31.871 | 41.838 | 1.00 | 39.11 | C |
| ATOM 1921 | OE1 | GLU B 127 | 17.852 | 31.547 | 40.719 | 1.00 | 41.53 | O |
| ATOM 1922 | OE2 | GLU B 127 | 17.799 | 32.875 | 42.459 | 1.00 | 44.56 | O |
| ATOM 1923 | C | GLU B 127 | 15.870 | 27.184 | 42.745 | 1.00 | 23.89 | C |
| ATOM 1924 | O | GLU B 127 | 15.350 | 26.594 | 41.805 | 1.00 | 23.89 | O |
| ATOM 1925 | N | TYR B 128 | 16.751 | 26.603 | 43.554 | 1.00 | 23.39 | N |
| ATOM 1926 | CA | TYR B 128 | 17.169 | 25.240 | 43.274 | 1.00 | 22.92 | C |
| ATOM 1927 | CB | TYR B 128 | 18.173 | 25.266 | 42.126 | 1.00 | 21.87 | C |
| ATOM 1928 | CG | TYR B 128 | 19.509 | 25.847 | 42.552 | 1.00 | 24.09 | C |
| ATOM 1929 | CD1 | TYR B 128 | 20.464 | 25.047 | 43.182 | 1.00 | 22.01 | C |
| ATOM 1930 | CE1 | TYR B 128 | 21.647 | 25.578 | 43.659 | 1.00 | 24.98 | C |
| ATOM 1931 | CZ | TYR B 128 | 21.900 | 26.927 | 43.511 | 1.00 | 26.66 | C |
| ATOM 1932 | OH | TYR B 128 | 23.079 | 27.431 | 43.998 | 1.00 | 30.76 | O |
| ATOM 1933 | CE2 | TYR B 128 | 20.979 | 27.755 | 42.885 | 1.00 | 29.10 | C |
| ATOM 1934 | CD2 | TYR B 128 | 19.785 | 27.210 | 42.405 | 1.00 | 23.51 | C |
| ATOM 1935 | C | TYR B 128 | 17.790 | 24.506 | 44.452 | 1.00 | 22.50 | C |
| ATOM 1936 | O | TYR B 128 | 18.143 | 25.101 | 45.464 | 1.00 | 23.17 | O |
| ATOM 1937 | N | ALA B 129 | 17.939 | 23.196 | 44.280 | 1.00 | 23.06 | N |
| ATOM 1938 | CA | ALA B 129 | 18.523 | 22.326 | 45.281 | 1.00 | 22.00 | C |
| ATOM 1939 | CB | ALA B 129 | 17.429 | 21.691 | 46.143 | 1.00 | 22.73 | C |
| ATOM 1940 | C | ALA B 129 | 19.300 | 21.251 | 44.546 | 1.00 | 23.97 | C |
| ATOM 1941 | O | ALA B 129 | 18.751 | 20.543 | 43.704 | 1.00 | 23.33 | O |
| ATOM 1942 | N | LEU B 130 | 20.588 | 21.154 | 44.858 | 1.00 | 23.49 | N |
| ATOM 1943 | CA | LEU B 130 | 21.465 | 20.166 | 44.250 | 1.00 | 24.77 | C |
| ATOM 1944 | CB | LEU B 130 | 22.806 | 20.807 | 43.893 | 1.00 | 24.15 | C |
| ATOM 1945 | CG | LEU B 130 | 23.789 | 19.994 | 43.047 | 1.00 | 30.51 | C |
| ATOM 1946 | CD1 | LEU B 130 | 23.151 | 19.653 | 41.706 | 1.00 | 30.79 | C |
| ATOM 1947 | CD2 | LEU B 130 | 25.075 | 20.800 | 42.840 | 1.00 | 28.10 | C |
| ATOM 1948 | C | LEU B 130 | 21.670 | 19.060 | 45.276 | 1.00 | 24.21 | C |
| ATOM 1949 | O | LEU B 130 | 22.287 | 19.285 | 46.322 | 1.00 | 25.91 | O |
| ATOM 1950 | N | LEU B 131 | 21.142 | 17.873 | 44.982 | 1.00 | 24.62 | N |
| ATOM 1951 | CA | LEU B 131 | 21.259 | 16.741 | 45.894 | 1.00 | 24.99 | C |
| ATOM 1952 | CB | LEU B 131 | 19.907 | 16.062 | 46.097 | 1.00 | 23.32 | C |
| ATOM 1953 | CG | LEU B 131 | 18.654 | 16.817 | 46.533 | 1.00 | 27.65 | C |
| ATOM 1954 | CD1 | LEU B 131 | 17.598 | 15.779 | 46.854 | 1.00 | 27.12 | C |
| ATOM 1955 | CD2 | LEU B 131 | 18.909 | 17.681 | 47.747 | 1.00 | 24.33 | C |
| ATOM 1956 | C | LEU B 131 | 22.238 | 15.681 | 45.406 | 1.00 | 26.69 | C |
| ATOM 1957 | O | LEU B 131 | 22.398 | 15.454 | 44.199 | 1.00 | 27.32 | O |
| ATOM 1958 | N | PHE B 132 | 22.894 | 15.035 | 46.362 | 1.00 | 26.02 | N |
| ATOM 1959 | CA | PHE B 132 | 23.820 | 13.969 | 46.052 | 1.00 | 26.25 | C |
| ATOM 1960 | CB | PHE B 132 | 25.237 | 14.305 | 46.493 | 1.00 | 26.48 | C |
| ATOM 1961 | CG | PHE B 132 | 26.221 | 13.193 | 46.239 | 1.00 | 30.21 | C |
| ATOM 1962 | CD1 | PHE B 132 | 26.596 | 12.860 | 44.933 | 1.00 | 33.16 | C |
| ATOM 1963 | CE1 | PHE B 132 | 27.476 | 11.805 | 44.686 | 1.00 | 34.30 | C |
| ATOM 1964 | CZ | PHE B 132 | 27.992 | 11.071 | 45.751 | 1.00 | 34.39 | C |
| ATOM 1965 | CE2 | PHE B 132 | 27.629 | 11.392 | 47.057 | 1.00 | 36.12 | C |
| ATOM 1966 | CD2 | PHE B 132 | 26.746 | 12.452 | 47.296 | 1.00 | 32.16 | C |
| ATOM 1967 | C | PHE B 132 | 23.345 | 12.750 | 46.807 | 1.00 | 26.32 | C |
| ATOM 1968 | O | PHE B 132 | 23.065 | 12.816 | 48.001 | 1.00 | 27.04 | O |
| ATOM 1969 | N | SER B 133 | 23.231 | 11.640 | 46.099 | 1.00 | 27.21 | N |
| ATOM 1970 | CA | SER B 133 | 22.802 | 10.401 | 46.714 | 1.00 | 30.77 | C |
| ATOM 1971 | CB | SER B 133 | 21.440 | 9.993 | 46.170 | 1.00 | 30.65 | C |
| ATOM 1972 | OG | SER B 133 | 21.074 | 8.721 | 46.675 | 1.00 | 36.04 | O |
| ATOM 1973 | C | SER B 133 | 23.820 | 9.294 | 46.446 | 1.00 | 32.06 | C |
| ATOM 1974 | O | SER B 133 | 24.303 | 9.123 | 45.326 | 1.00 | 32.43 | O |
| ATOM 1975 | N | ARG B 134 | 24.169 | 8.559 | 47.487 | 1.00 | 33.25 | N |
| ATOM 1976 | CA | ARG B 134 | 25.104 | 7.465 | 47.334 | 1.00 | 35.64 | C |
| ATOM 1977 | CB | ARG B 134 | 26.446 | 7.788 | 48.007 | 1.00 | 34.49 | C |
| ATOM 1978 | CG | ARG B 134 | 26.345 | 8.228 | 49.450 | 1.00 | 37.30 | C |
| ATOM 1979 | CD | ARG B 134 | 27.377 | 9.305 | 49.764 | 1.00 | 38.87 | C |
| ATOM 1980 | NE | ARG B 134 | 27.331 | 9.725 | 51.162 | 1.00 | 39.78 | N |
| ATOM 1981 | CZ | ARG B 134 | 27.740 | 8.970 | 52.176 | 1.00 | 39.69 | C |
| ATOM 1982 | NH1 | ARG B 134 | 28.229 | 7.757 | 51.945 | 1.00 | 42.68 | N |
| ATOM 1983 | NH2 | ARG B 134 | 27.659 | 9.418 | 53.420 | 1.00 | 36.98 | N |
| ATOM 1984 | C | ARG B 134 | 24.479 | 6.221 | 47.930 | 1.00 | 36.40 | C |
| ATOM 1985 | O | ARG B 134 | 23.870 | 6.268 | 49.002 | 1.00 | 35.15 | O |
| ATOM 1986 | N | GLY B 135 | 24.603 | 5.119 | 47.198 | 1.00 | 38.19 | N |
| ATOM 1987 | CA | GLY B 135 | 24.066 | 3.846 | 47.642 | 1.00 | 41.62 | C |
| ATOM 1988 | C | GLY B 135 | 25.098 | 2.763 | 47.406 | 1.00 | 44.13 | C |
| ATOM 1989 | O | GLY B 135 | 26.115 | 2.998 | 46.753 | 1.00 | 42.97 | O |
| ATOM 1990 | N | THR B 136 | 24.844 | 1.571 | 47.931 | 1.00 | 47.49 | N |
| ATOM 1991 | CA | THR B 136 | 25.777 | 0.471 | 47.764 | 1.00 | 50.61 | C |
| ATOM 1992 | CB | THR B 136 | 27.029 | 0.670 | 48.632 | 1.00 | 51.09 | C |
| ATOM 1993 | OG1 | THR B 136 | 27.838 | -0.514 | 48.580 | 1.00 | 52.66 | O |
| ATOM 1994 | CG2 | THR B 136 | 26.635 | 0.975 | 50.071 | 1.00 | 52.23 | C |
| ATOM 1995 | C | THR B 136 | 25.148 | -0.858 | 48.131 | 1.00 | 52.28 | C |
| ATOM 1996 | O | THR B 136 | 24.947 | -1.158 | 49.306 | 1.00 | 52.60 | O |
| ATOM 1997 | N | LYS B 137 | 24.830 | -1.651 | 47.114 | 1.00 | 53.62 | N |
| ATOM 1998 | CA | LYS B 137 | 24.238 | -2.964 | 47.327 | 1.00 | 54.93 | C |
| ATOM 1999 | CB | LYS B 137 | 23.811 | -3.563 | 45.986 | 1.00 | 55.60 | C |
| ATOM 2000 | CG | LYS B 137 | 22.315 | -3.750 | 45.819 | 1.00 | 56.78 | C |
| ATOM 2001 | CD | LYS B 137 | 21.977 | -4.167 | 44.384 | 1.00 | 58.70 | C |
| ATOM 2002 | CE | LYS B 137 | 22.698 | -5.457 | 43.978 | 1.00 | 58.92 | C |
| ATOM 2003 | NZ | LYS B 137 | 22.598 | -5.762 | 42.517 | 1.00 | 58.82 | N |
| ATOM 2004 | C | LYS B 137 | 25.245 | -3.890 | 48.007 | 1.00 | 55.28 | C |
| ATOM 2005 | O | LYS B 137 | 24.880 | -4.940 | 48.531 | 1.00 | 55.22 | O |
| ATOM 2006 | N | GLY B 138 | 26.515 | -3.490 | 47.978 | 1.00 | 55.65 | N |
| ATOM 2007 | CA | GLY B 138 | 27.573 | -4.277 | 48.584 | 1.00 | 55.72 | C |
| ATOM 2008 | C | GLY B 138 | 28.934 | -3.779 | 48.145 | 1.00 | 55.93 | C |
| ATOM 2009 | O | GLY B 138 | 29.019 | -2.852 | 47.334 | 1.00 | 55.96 | O |
| ATOM 2010 | N | PRO B 139 | 30.021 | -4.374 | 48.662 | 1.00 | 55.62 | N |
| ATOM 2011 | CA | PRO B 139 | 31.381 | -3.969 | 48.309 | 1.00 | 55.42 | C |
| ATOM 2012 | CB | PRO B 139 | 32.250 | -4.922 | 49.125 | 1.00 | 55.83 | C |
| ATOM 2013 | CG | PRO B 139 | 31.413 | -5.209 | 50.321 | 1.00 | 55.60 | C |
| ATOM 2014 | CD | PRO B 139 | 30.048 | -5.415 | 49.701 | 1.00 | 55.87 | C |
| ATOM 2015 | C | PRO B 139 | 31.682 | -4.044 | 46.819 | 1.00 | 54.93 | C |
| ATOM 2016 | O | PRO B 139 | 32.059 | -5.092 | 46.298 | 1.00 | 56.18 | O |
| ATOM 2017 | N | GLY B 140 | 31.505 | -2.920 | 46.134 | 1.00 | 53.90 | N |
| ATOM 2018 | CA | GLY B 140 | 31.783 | -2.864 | 44.705 | 1.00 | 52.20 | C |
| ATOM 2019 | C | GLY B 140 | 30.527 | -2.585 | 43.913 | 1.00 | 51.21 | C |
| ATOM 2020 | O | GLY B 140 | 30.576 | -2.319 | 42.713 | 1.00 | 51.38 | O |
| ATOM 2021 | N | GLN B 141 | 29.396 | -2.665 | 44.603 | 1.00 | 49.60 | N |
| ATOM 2022 | CA | GLN B 141 | 28.103 | -2.413 | 43.994 | 1.00 | 47.90 | C |
| ATOM 2023 | CB | GLN B 141 | 27.085 | -3.474 | 44.431 | 1.00 | 47.88 | C |
| ATOM 2024 | CG | GLN B 141 | 27.328 | -4.884 | 43.913 | 1.00 | 46.95 | C |
| ATOM 2025 | CD | GLN B 141 | 26.237 | -5.858 | 44.349 | 1.00 | 45.80 | C |
| ATOM 2026 | OE1 | GLN B 141 | 26.126 | -6.201 | 45.522 | 1.00 | 46.77 | O |
| ATOM 2027 | NE2 | GLN B 141 | 25.422 | -6.296 | 43.401 | 1.00 | 44.27 | N |
| ATOM 2028 | C | GLN B 141 | 27.622 | -1.030 | 44.429 | 1.00 | 46.82 | C |
| ATOM 2029 | O | GLN B 141 | 26.440 | -0.827 | 44.695 | 1.00 | 45.66 | O |
| ATOM 2030 | N | ASN B 142 | 28.557 | -0.086 | 44.493 | 1.00 | 45.91 | N |
| ATOM 2031 | CA | ASN B 142 | 28.275 | 1.284 | 44.906 | 1.00 | 44.46 | C |
| ATOM 2032 | CB | ASN B 142 | 29.542 | 1.943 | 45.455 | 1.00 | 44.95 | C |
| ATOM 2033 | CG | ASN B 142 | 30.593 | 0.932 | 45.861 | 1.00 | 46.96 | C |
| ATOM 2034 | OD1 | ASN B 142 | 30.373 | 0.115 | 46.759 | 1.00 | 51.35 | O |
| ATOM 2035 | ND2 | ASN B 142 | 31.745 | 0.978 | 45.197 | 1.00 | 47.04 | N |
| ATOM 2036 | C | ASN B 142 | 27.778 | 2.110 | 43.740 | 1.00 | 43.18 | C |
| ATOM 2037 | O | ASN B 142 | 28.372 | 2.092 | 42.669 | 1.00 | 42.88 | O |
| ATOM 2038 | N | PHE B 143 | 26.689 | 2.837 | 43.943 | 1.00 | 40.49 | N |
| ATOM 2039 | CA | PHE B 143 | 26.172 | 3.688 | 42.885 | 1.00 | 39.21 | C |
| ATOM 2040 | CB | PHE B 143 | 24.855 | 3.120 | 42.335 | 1.00 | 39.22 | C |
| ATOM 2041 | CG | PHE B 143 | 23.646 | 3.464 | 43.147 | 1.00 | 41.52 | C |
| ATOM 2042 | CD1 | PHE B 143 | 23.104 | 4.743 | 43.097 | 1.00 | 43.86 | C |
| ATOM 2043 | CE1 | PHE B 143 | 21.971 | 5.063 | 43.823 | 1.00 | 46.05 | C |
| ATOM 2044 | CZ | PHE B 143 | 21.363 | 4.097 | 44.612 | 1.00 | 47.94 | C |
| ATOM 2045 | CE2 | PHE B 143 | 21.893 | 2.813 | 44.673 | 1.00 | 44.88 | C |
| ATOM 2046 | CD2 | PHE B 143 | 23.029 | 2.503 | 43.940 | 1.00 | 43.37 | C |
| ATOM 2047 | C | PHE B 143 | 25.991 | 5.110 | 43.420 | 1.00 | 37.46 | C |
| ATOM 2048 | O | PHE B 143 | 25.731 | 5.308 | 44.607 | 1.00 | 35.21 | O |
| ATOM 2049 | N | ARG B 144 | 26.143 | 6.090 | 42.537 | 1.00 | 35.85 | N |
| ATOM 2050 | CA | ARG B 144 | 26.013 | 7.494 | 42.902 | 1.00 | 35.66 | C |
| ATOM 2051 | CB | ARG B 144 | 27.356 | 8.201 | 42.737 | 1.00 | 37.36 | C |
| ATOM 2052 | CG | ARG B 144 | 28.516 | 7.552 | 43.461 | 1.00 | 40.81 | C |
| ATOM 2053 | CD | ARG B 144 | 29.802 | 8.273 | 43.113 | 1.00 | 47.72 | C |
| ATOM 2054 | NE | ARG B 144 | 30.956 | 7.704 | 43.796 | 1.00 | 52.59 | N |
| ATOM 2055 | CZ | ARG B 144 | 32.207 | 8.103 | 43.596 | 1.00 | 56.44 | C |
| ATOM 2056 | NH1 | ARG B 144 | 32.461 | 9.076 | 42.732 | 1.00 | 59.03 | N |
| ATOM 2057 | NH2 | ARG B 144 | 33.202 | 7.525 | 44.254 | 1.00 | 58.04 | N |
| ATOM 2058 | C | ARG B 144 | 24.985 | 8.207 | 42.025 | 1.00 | 34.31 | C |
| ATOM 2059 | O | ARG B 144 | 24.860 | 7.913 | 40.835 | 1.00 | 32.59 | O |
| ATOM 2060 | N | MET B 145 | 24.260 | 9.157 | 42.605 | 1.00 | 32.17 | N |
| ATOM 2061 | CA | MET B 145 | 23.270 | 9.900 | 41.842 | 1.00 | 31.38 | C |
| ATOM 2062 | CB | MET B 145 | 21.907 | 9.224 | 41.957 | 1.00 | 32.20 | C |
| ATOM 2063 | CG | MET B 145 | 20.805 | 10.000 | 41.282 | 1.00 | 36.58 | C |
| ATOM 2064 | SD | MET B 145 | 19.260 | 9.102 | 41.257 | 1.00 | 41.13 | S |
| ATOM 2065 | CE | MET B 145 | 18.612 | 9.475 | 42.864 | 1.00 | 39.42 | C |
| ATOM 2066 | C | MET B 145 | 23.146 | 11.371 | 42.239 | 1.00 | 30.58 | C |
| ATOM 2067 | O | MET B 145 | 22.959 | 11.707 | 43.413 | 1.00 | 29.84 | O |
| ATOM 2068 | N | ALA B 146 | 23.261 | 12.241 | 41.241 | 1.00 | 28.92 | N |
| ATOM 2069 | CA | ALA B 146 | 23.136 | 13.681 | 41.429 | 1.00 | 26.73 | C |
| ATOM 2070 | CB | ALA B 146 | 24.231 | 14.409 | 40.666 | 1.00 | 26.72 | C |
| ATOM 2071 | C | ALA B 146 | 21.765 | 14.101 | 40.904 | 1.00 | 26.22 | C |
| ATOM 2072 | O | ALA B 146 | 21.378 | 13.743 | 39.792 | 1.00 | 25.15 | O |
| ATOM 2073 | N | THR B 147 | 21.034 | 14.858 | 41.712 | 1.00 | 26.04 | N |
| ATOM 2074 | CA | THR B 147 | 19.707 | 15.320 | 41.335 | 1.00 | 24.43 | C |
| ATOM 2075 | CB | THR B 147 | 18.635 | 14.690 | 42.225 | 1.00 | 24.48 | C |
| ATOM 2076 | OG1 | THR B 147 | 18.895 | 13.287 | 42.346 | 1.00 | 28.38 | O |
| ATOM 2077 | CG2 | THR B 147 | 17.255 | 14.891 | 41.617 | 1.00 | 24.67 | C |
| ATOM 2078 | C | THR B 147 | 19.606 | 16.837 | 41.464 | 1.00 | 23.63 | C |
| ATOM 2079 | O | THR B 147 | 20.044 | 17.422 | 42.462 | 1.00 | 22.67 | O |
| ATOM 2080 | N | LEU B 148 | 19.033 | 17.472 | 40.448 | 1.00 | 22.08 | N |
| ATOM 2081 | CA | LEU B 148 | 18.861 | 18.919 | 40.465 | 1.00 | 21.25 | C |
| ATOM 2082 | CB | LEU B 148 | 19.545 | 19.556 | 39.255 | 1.00 | 22.23 | C |
| ATOM 2083 | CG | LEU B 148 | 19.335 | 21.066 | 39.029 | 1.00 | 23.35 | C |
| ATOM 2084 | CD1 | LEU B 148 | 19.992 | 21.874 | 40.123 | 1.00 | 24.17 | C |
| ATOM 2085 | CD2 | LEU B 148 | 19.920 | 21.453 | 37.696 | 1.00 | 25.57 | C |
| ATOM 2086 | C | LEU B 148 | 17.383 | 19.275 | 40.467 | 1.00 | 20.22 | C |
| ATOM 2087 | O | LEU B 148 | 16.664 | 18.997 | 39.510 | 1.00 | 19.83 | O |
| ATOM 2088 | N | TYR B 149 | 16.937 | 19.865 | 41.567 | 1.00 | 20.43 | N |
| ATOM 2089 | CA | TYR B 149 | 15.552 | 20.297 | 41.715 | 1.00 | 21.42 | C |
| ATOM 2090 | CB | TYR B 149 | 15.058 | 20.016 | 43.132 | 1.00 | 22.01 | C |
| ATOM 2091 | CG | TYR B 149 | 14.633 | 18.591 | 43.344 | 1.00 | 23.22 | C |
| ATOM 2092 | CD1 | TYR B 149 | 13.435 | 18.123 | 42.820 | 1.00 | 27.87 | C |
| ATOM 2093 | CE1 | TYR B 149 | 13.022 | 16.814 | 43.019 | 1.00 | 29.19 | C |
| ATOM 2094 | CZ | TYR B 149 | 13.815 | 15.955 | 43.746 | 1.00 | 30.82 | C |
| ATOM 2095 | OH | TYR B 149 | 13.394 | 14.659 | 43.937 | 1.00 | 37.77 | O |
| ATOM 2096 | CE2 | TYR B 149 | 15.020 | 16.390 | 44.277 | 1.00 | 30.92 | C |
| ATOM 2097 | CD2 | TYR B 149 | 15.423 | 17.705 | 44.075 | 1.00 | 27.50 | C |
| ATOM 2098 | C | TYR B 149 | 15.482 | 21.789 | 41.434 | 1.00 | 21.29 | C |
| ATOM 2099 | O | TYR B 149 | 16.435 | 22.530 | 41.707 | 1.00 | 20.13 | O |
| ATOM 2100 | N | SER B 150 | 14.350 | 22.223 | 40.895 | 1.00 | 21.81 | N |
| ATOM 2101 | CA | SER B 150 | 14.144 | 23.623 | 40.557 | 1.00 | 22.08 | C |
| ATOM 2102 | CB | SER B 150 | 14.355 | 23.807 | 39.050 | 1.00 | 23.22 | C |
| ATOM 2103 | OG | SER B 150 | 14.148 | 25.147 | 38.655 | 1.00 | 20.11 | O |
| ATOM 2104 | C | SER B 150 | 12.745 | 24.116 | 40.946 | 1.00 | 22.46 | C |
| ATOM 2105 | O | SER B 150 | 11.767 | 23.378 | 40.830 | 1.00 | 20.73 | O |
| ATOM 2106 | N | ARG B 151 | 12.659 | 25.360 | 41.411 | 1.00 | 22.10 | N |
| ATOM 2107 | CA | ARG B 151 | 11.373 | 25.934 | 41.777 | 1.00 | 22.76 | C |
| ATOM 2108 | CB | ARG B 151 | 11.561 | 27.252 | 42.541 | 1.00 | 20.89 | C |
| ATOM 2109 | CG | ARG B 151 | 12.009 | 27.087 | 43.994 | 1.00 | 19.59 | C |
| ATOM 2110 | CD | ARG B 151 | 10.946 | 26.397 | 44.861 | 1.00 | 14.05 | C |
| ATOM 2111 | NE | ARG B 151 | 11.344 | 26.371 | 46.270 | 1.00 | 16.55 | N |
| ATOM 2112 | CZ | ARG B 151 | 10.649 | 25.800 | 47.250 | 1.00 | 17.56 | C |
| ATOM 2113 | NH1 | ARG B 151 | 9.501 | 25.182 | 46.998 | 1.00 | 18.38 | N |
| ATOM 2114 | NH2 | ARG B 151 | 11.096 | 25.867 | 48.496 | 1.00 | 16.44 | N |
| ATOM 2115 | C | ARG B 151 | 10.573 | 26.184 | 40.497 | 1.00 | 25.21 | C |
| ATOM 2116 | O | ARG B 151 | 9.351 | 26.262 | 40.524 | 1.00 | 25.14 | O |
| ATOM 2117 | N | THR B 152 | 11.275 | 26.294 | 39.373 | 1.00 | 26.93 | N |
| ATOM 2118 | CA | THR B 152 | 10.635 | 26.539 | 38.086 | 1.00 | 29.69 | C |
| ATOM 2119 | CB | THR B 152 | 11.167 | 27.824 | 37.451 | 1.00 | 29.18 | C |
| ATOM 2120 | OG1 | THR B 152 | 12.569 | 27.667 | 37.190 | 1.00 | 32.70 | O |
| ATOM 2121 | CG2 | THR B 152 | 10.956 | 29.007 | 38.377 | 1.00 | 30.82 | C |
| ATOM 2122 | C | THR B 152 | 10.922 | 25.403 | 37.116 | 1.00 | 31.23 | C |
| ATOM 2123 | O | THR B 152 | 11.878 | 24.645 | 37.293 | 1.00 | 31.69 | O |
| ATOM 2124 | N | GLN B 153 | 10.101 | 25.300 | 36.080 | 1.00 | 32.65 | N |
| ATOM 2125 | CA | GLN B 153 | 10.283 | 24.264 | 35.077 | 1.00 | 35.10 | C |
| ATOM 2126 | CB | GLN B 153 | 8.985 | 24.043 | 34.322 | 1.00 | 35.06 | C |
| ATOM 2127 | CG | GLN B 153 | 7.878 | 23.553 | 35.214 | 1.00 | 36.53 | C |
| ATOM 2128 | CD | GLN B 153 | 6.621 | 23.266 | 34.447 | 1.00 | 38.81 | C |
| ATOM 2129 | OE1 | GLN B 153 | 6.607 | 22.421 | 33.560 | 1.00 | 42.69 | O |
| ATOM 2130 | NE2 | GLN B 153 | 5.554 | 23.970 | 34.780 | 1.00 | 37.04 | N |
| ATOM 2131 | C | GLN B 153 | 11.394 | 24.614 | 34.100 | 1.00 | 36.36 | C |
| ATOM 2132 | O | GLN B 153 | 11.876 | 23.755 | 33.368 | 1.00 | 37.01 | O |
| ATOM 2133 | N | THR B 154 | 11.802 | 25.877 | 34.100 | 1.00 | 38.47 | N |
| ATOM 2134 | CA | THR B 154 | 12.862 | 26.341 | 33.217 | 1.00 | 40.05 | C |
| ATOM 2135 | CB | THR B 154 | 12.584 | 27.772 | 32.722 | 1.00 | 40.61 | C |
| ATOM 2136 | OG1 | THR B 154 | 11.270 | 27.831 | 32.153 | 1.00 | 42.12 | O |
| ATOM 2137 | CG2 | THR B 154 | 13.605 | 28.183 | 31.668 | 1.00 | 40.94 | C |
| ATOM 2138 | C | THR B 154 | 14.169 | 26.333 | 33.992 | 1.00 | 40.87 | C |
| ATOM 2139 | O | THR B 154 | 14.198 | 26.665 | 35.180 | 1.00 | 42.00 | O |
| ATOM 2140 | N | LEU B 155 | 15.249 | 25.956 | 33.319 | 1.00 | 40.55 | N |
| ATOM 2141 | CA | LEU B 155 | 16.554 | 25.888 | 33.961 | 1.00 | 41.78 | C |
| ATOM 2142 | CB | LEU B 155 | 17.058 | 24.441 | 33.923 | 1.00 | 41.27 | C |
| ATOM 2143 | CG | LEU B 155 | 17.825 | 23.880 | 35.120 | 1.00 | 41.78 | C |
| ATOM 2144 | CD1 | LEU B 155 | 16.977 | 23.974 | 36.390 | 1.00 | 36.65 | C |
| ATOM 2145 | CD2 | LEU B 155 | 18.186 | 22.432 | 34.828 | 1.00 | 40.61 | C |
| ATOM 2146 | C | LEU B 155 | 17.556 | 26.810 | 33.266 | 1.00 | 42.44 | C |
| ATOM 2147 | O | LEU B 155 | 17.723 | 26.751 | 32.045 | 1.00 | 42.40 | O |
| ATOM 2148 | N | LYS B 156 | 18.214 | 27.667 | 34.041 | 1.00 | 42.99 | N |
| ATOM 2149 | CA | LYS B 156 | 19.206 | 28.580 | 33.485 | 1.00 | 43.59 | C |
| ATOM 2150 | CB | LYS B 156 | 19.458 | 29.743 | 34.449 | 1.00 | 43.63 | C |
| ATOM 2151 | CG | LYS B 156 | 18.268 | 30.680 | 34.561 | 1.00 | 45.43 | C |
| ATOM 2152 | CD | LYS B 156 | 18.510 | 31.823 | 35.528 | 1.00 | 45.63 | C |
| ATOM 2153 | CE | LYS B 156 | 17.297 | 32.749 | 35.567 | 1.00 | 47.26 | C |
| ATOM 2154 | NZ | LYS B 156 | 17.407 | 33.814 | 36.609 | 1.00 | 47.89 | N |
| ATOM 2155 | C | LYS B 156 | 20.509 | 27.836 | 33.203 | 1.00 | 43.26 | C |
| ATOM 2156 | O | LYS B 156 | 20.967 | 27.032 | 34.012 | 1.00 | 41.69 | O |
| ATOM 2157 | N | ASP B 157 | 21.098 | 28.110 | 32.045 | 1.00 | 44.05 | N |
| ATOM 2158 | CA | ASP B 157 | 22.340 | 27.463 | 31.637 | 1.00 | 44.05 | C |
| ATOM 2159 | CB | ASP B 157 | 22.901 | 28.163 | 30.402 | 1.00 | 45.56 | C |
| ATOM 2160 | CG | ASP B 157 | 22.056 | 27.924 | 29.172 | 1.00 | 46.47 | C |
| ATOM 2161 | OD1 | ASP B 157 | 20.830 | 28.140 | 29.243 | 1.00 | 49.10 | O |
| ATOM 2162 | OD2 | ASP B 157 | 22.619 | 27.521 | 28.134 | 1.00 | 50.45 | O |
| ATOM 2163 | C | ASP B 157 | 23.403 | 27.410 | 32.732 | 1.00 | 43.87 | C |
| ATOM 2164 | O | ASP B 157 | 24.088 | 26.396 | 32.891 | 1.00 | 43.50 | O |
| ATOM 2165 | N | GLU B 158 | 23.548 | 28.492 | 33.487 | 1.00 | 43.29 | N |
| ATOM 2166 | CA | GLU B 158 | 24.536 | 28.512 | 34.555 | 1.00 | 42.24 | C |
| ATOM 2167 | CB | GLU B 158 | 24.580 | 29.895 | 35.211 | 1.00 | 43.13 | C |
| ATOM 2168 | CG | GLU B 158 | 23.234 | 30.592 | 35.298 | 1.00 | 45.98 | C |
| ATOM 2169 | CD | GLU B 158 | 23.329 | 32.071 | 34.947 | 1.00 | 48.36 | C |
| ATOM 2170 | OE1 | GLU B 158 | 24.183 | 32.773 | 35.533 | 1.00 | 48.34 | O |
| ATOM 2171 | OE2 | GLU B 158 | 22.545 | 32.529 | 34.085 | 1.00 | 49.65 | O |
| ATOM 2172 | C | GLU B 158 | 24.222 | 27.432 | 35.581 | 1.00 | 40.64 | C |
| ATOM 2173 | O | GLU B 158 | 25.115 | 26.915 | 36.250 | 1.00 | 39.52 | O |
| ATOM 2174 | N | LEU B 159 | 22.945 | 27.080 | 35.681 | 1.00 | 39.39 | N |
| ATOM 2175 | CA | LEU B 159 | 22.494 | 26.051 | 36.613 | 1.00 | 37.85 | C |
| ATOM 2176 | CB | LEU B 159 | 20.995 | 26.202 | 36.847 | 1.00 | 38.48 | C |
| ATOM 2177 | CG | LEU B 159 | 20.420 | 25.783 | 38.196 | 1.00 | 38.76 | C |
| ATOM 2178 | CD1 | LEU B 159 | 21.193 | 26.457 | 39.314 | 1.00 | 39.56 | C |
| ATOM 2179 | CD2 | LEU B 159 | 18.948 | 26.167 | 38.248 | 1.00 | 38.13 | C |
| ATOM 2180 | C | LEU B 159 | 22.806 | 24.687 | 36.005 | 1.00 | 37.07 | C |
| ATOM 2181 | O | LEU B 159 | 23.174 | 23.742 | 36.703 | 1.00 | 36.52 | O |
| ATOM 2182 | N | LYS B 160 | 22.661 | 24.591 | 34.690 | 1.00 | 36.16 | N |
| ATOM 2183 | CA | LYS B 160 | 22.968 | 23.358 | 33.992 | 1.00 | 35.31 | C |
| ATOM 2184 | CB | LYS B 160 | 22.590 | 23.471 | 32.517 | 1.00 | 36.22 | C |
| ATOM 2185 | CG | LYS B 160 | 21.096 | 23.433 | 32.231 | 1.00 | 37.33 | C |
| ATOM 2186 | CD | LYS B 160 | 20.848 | 23.347 | 30.727 | 1.00 | 38.65 | C |
| ATOM 2187 | CE | LYS B 160 | 19.369 | 23.283 | 30.398 | 1.00 | 40.87 | C |
| ATOM 2188 | NZ | LYS B 160 | 19.138 | 23.283 | 28.921 | 1.00 | 44.32 | N |
| ATOM 2189 | C | LYS B 160 | 24.465 | 23.068 | 34.113 | 1.00 | 34.93 | C |
| ATOM 2190 | O | LYS B 160 | 24.875 | 21.920 | 34.311 | 1.00 | 34.72 | O |
| ATOM 2191 | N | GLU B 161 | 25.278 | 24.115 | 33.999 | 1.00 | 33.85 | N |
| ATOM 2192 | CA | GLU B 161 | 26.730 | 23.981 | 34.089 | 1.00 | 33.69 | C |
| ATOM 2193 | CB | GLU B 161 | 27.388 | 25.316 | 33.729 | 1.00 | 34.67 | C |
| ATOM 2194 | CG | GLU B 161 | 27.208 | 25.686 | 32.260 | 1.00 | 36.90 | C |
| ATOM 2195 | CD | GLU B 161 | 27.516 | 27.143 | 31.964 | 1.00 | 44.37 | C |
| ATOM 2196 | OE1 | GLU B 161 | 28.518 | 27.668 | 32.504 | 1.00 | 44.62 | O |
| ATOM 2197 | OE2 | GLU B 161 | 26.759 | 27.761 | 31.179 | 1.00 | 45.41 | O |
| ATOM 2198 | C | GLU B 161 | 27.163 | 23.522 | 35.478 | 1.00 | 32.63 | C |
| ATOM 2199 | O | GLU B 161 | 28.034 | 22.658 | 35.623 | 1.00 | 32.33 | O |
| ATOM 2200 | N | LYS B 162 | 26.542 | 24.088 | 36.503 | 1.00 | 32.15 | N |
| ATOM 2201 | CA | LYS B 162 | 26.868 | 23.700 | 37.862 | 1.00 | 31.19 | C |
| ATOM 2202 | CB | LYS B 162 | 26.037 | 24.515 | 38.858 | 1.00 | 32.11 | C |
| ATOM 2203 | CG | LYS B 162 | 26.316 | 24.191 | 40.317 | 1.00 | 30.24 | C |
| ATOM 2204 | CD | LYS B 162 | 25.256 | 24.803 | 41.226 | 1.00 | 29.84 | C |
| ATOM 2205 | CE | LYS B 162 | 25.296 | 26.322 | 41.201 | 1.00 | 28.84 | C |
| ATOM 2206 | NZ | LYS B 162 | 26.579 | 26.847 | 41.743 | 1.00 | 28.90 | N |
| ATOM 2207 | C | LYS B 162 | 26.595 | 22.209 | 38.044 | 1.00 | 29.86 | C |
| ATOM 2208 | O | LYS B 162 | 27.412 | 21.490 | 38.612 | 1.00 | 30.46 | O |
| ATOM 2209 | N | PHE B 163 | 25.450 | 21.751 | 37.546 | 1.00 | 29.69 | N |
| ATOM 2210 | CA | PHE B 163 | 25.054 | 20.346 | 37.655 | 1.00 | 29.00 | C |
| ATOM 2211 | CB | PHE B 163 | 23.648 | 20.157 | 37.071 | 1.00 | 28.39 | C |
| ATOM 2212 | CG | PHE B 163 | 23.074 | 18.795 | 37.301 | 1.00 | 26.32 | C |
| ATOM 2213 | CD1 | PHE B 163 | 22.834 | 18.337 | 38.587 | 1.00 | 22.99 | C |
| ATOM 2214 | CE1 | PHE B 163 | 22.304 | 17.077 | 38.802 | 1.00 | 22.53 | C |
| ATOM 2215 | CZ | PHE B 163 | 22.007 | 16.256 | 37.723 | 1.00 | 21.90 | C |
| ATOM 2216 | CE2 | PHE B 163 | 22.239 | 16.698 | 36.440 | 1.00 | 25.70 | C |
| ATOM 2217 | CD2 | PHE B 163 | 22.771 | 17.963 | 36.231 | 1.00 | 26.75 | C |
| ATOM 2218 | C | PHE B 163 | 26.038 | 19.432 | 36.933 | 1.00 | 29.29 | C |
| ATOM 2219 | O | PHE B 163 | 26.504 | 18.434 | 37.487 | 1.00 | 30.36 | O |
| ATOM 2220 | N | THR B 164 | 26.348 | 19.772 | 35.688 | 1.00 | 30.76 | N |
| ATOM 2221 | CA | THR B 164 | 27.291 | 18.981 | 34.904 | 1.00 | 31.26 | C |
| ATOM 2222 | CB | THR B 164 | 27.446 | 19.556 | 33.478 | 1.00 | 30.79 | C |
| ATOM 2223 | OG1 | THR B 164 | 26.173 | 19.540 | 32.817 | 1.00 | 32.00 | O |
| ATOM 2224 | CG2 | THR B 164 | 28.432 | 18.716 | 32.664 | 1.00 | 33.63 | C |
| ATOM 2225 | C | THR B 164 | 28.657 | 18.958 | 35.595 | 1.00 | 30.45 | C |
| ATOM 2226 | O | THR B 164 | 29.313 | 17.915 | 35.660 | 1.00 | 31.24 | O |
| ATOM 2227 | N | THR B 165 | 29.079 | 20.107 | 36.114 | 1.00 | 30.54 | N |
| ATOM 2228 | CA | THR B 165 | 30.359 | 20.202 | 36.811 | 1.00 | 31.33 | C |
| ATOM 2229 | CB | THR B 165 | 30.640 | 21.648 | 37.243 | 1.00 | 31.71 | C |
| ATOM 2230 | OG1 | THR B 165 | 30.720 | 22.475 | 36.078 | 1.00 | 32.17 | O |
| ATOM 2231 | CG2 | THR B 165 | 31.945 | 21.738 | 38.013 | 1.00 | 31.92 | C |
| ATOM 2232 | C | THR B 165 | 30.385 | 19.305 | 38.050 | 1.00 | 31.31 | C |
| ATOM 2233 | O | THR B 165 | 31.304 | 18.499 | 38.228 | 1.00 | 30.04 | O |
| ATOM 2234 | N | PHE B 166 | 29.378 | 19.451 | 38.908 | 1.00 | 31.86 | N |
| ATOM 2235 | CA | PHE B 166 | 29.300 | 18.635 | 40.111 | 1.00 | 32.35 | C |
| ATOM 2236 | CB | PHE B 166 | 28.069 | 19.007 | 40.939 | 1.00 | 31.30 | C |
| ATOM 2237 | CG | PHE B 166 | 27.861 | 18.121 | 42.138 | 1.00 | 34.42 | C |
| ATOM 2238 | CD1 | PHE B 166 | 28.755 | 18.149 | 43.206 | 1.00 | 32.31 | C |
| ATOM 2239 | CE1 | PHE B 166 | 28.580 | 17.302 | 44.303 | 1.00 | 28.62 | C |
| ATOM 2240 | CZ | PHE B 166 | 27.505 | 16.420 | 44.337 | 1.00 | 29.27 | C |
| ATOM 2241 | CE2 | PHE B 166 | 26.610 | 16.386 | 43.281 | 1.00 | 34.35 | C |
| ATOM 2242 | CD2 | PHE B 166 | 26.790 | 17.234 | 42.187 | 1.00 | 33.74 | C |
| ATOM 2243 | C | PHE B 166 | 29.224 | 17.153 | 39.739 | 1.00 | 32.79 | C |
| ATOM 2244 | O | PHE B 166 | 29.836 | 16.304 | 40.398 | 1.00 | 34.11 | O |
| ATOM 2245 | N | SER B 167 | 28.471 | 16.847 | 38.687 | 1.00 | 33.75 | N |
| ATOM 2246 | CA | SER B 167 | 28.311 | 15.464 | 38.245 | 1.00 | 33.72 | C |
| ATOM 2247 | CB | SER B 167 | 27.298 | 15.385 | 37.102 | 1.00 | 32.77 | C |
| ATOM 2248 | OG | SER B 167 | 26.006 | 15.736 | 37.551 | 1.00 | 32.40 | O |
| ATOM 2249 | C | SER B 167 | 29.629 | 14.854 | 37.791 | 1.00 | 34.67 | C |
| ATOM 2250 | O | SER B 167 | 29.936 | 13.701 | 38.102 | 1.00 | 34.94 | O |
| ATOM 2251 | N | LYS B 168 | 30.404 | 15.630 | 37.048 | 1.00 | 35.59 | N |
| ATOM 2252 | CA | LYS B 168 | 31.679 | 15.145 | 36.555 | 1.00 | 36.93 | C |
| ATOM 2253 | CB | LYS B 168 | 32.221 | 16.099 | 35.488 | 1.00 | 37.02 | C |
| ATOM 2254 | CG | LYS B 168 | 31.407 | 16.084 | 34.194 | 1.00 | 38.92 | C |
| ATOM 2255 | CD | LYS B 168 | 31.949 | 17.072 | 33.171 | 1.00 | 39.84 | C |
| ATOM 2256 | CE | LYS B 168 | 31.218 | 16.942 | 31.845 | 1.00 | 42.88 | C |
| ATOM 2257 | NZ | LYS B 168 | 31.693 | 17.934 | 30.832 | 1.00 | 42.91 | N |
| ATOM 2258 | C | LYS B 168 | 32.658 | 15.002 | 37.713 | 1.00 | 37.30 | C |
| ATOM 2259 | O | LYS B 168 | 33.441 | 14.053 | 37.758 | 1.00 | 36.54 | O |
| ATOM 2260 | N | ALA B 169 | 32.589 | 15.934 | 38.661 | 1.00 | 38.00 | N |
| ATOM 2261 | CA | ALA B 169 | 33.460 | 15.906 | 39.828 | 1.00 | 38.86 | C |
| ATOM 2262 | CB | ALA B 169 | 33.263 | 17.165 | 40.655 | 1.00 | 37.89 | C |
| ATOM 2263 | C | ALA B 169 | 33.171 | 14.670 | 40.671 | 1.00 | 39.70 | C |
| ATOM 2264 | O | ALA B 169 | 33.854 | 14.408 | 41.660 | 1.00 | 41.32 | O |
| ATOM 2265 | N | GLN B 170 | 32.157 | 13.910 | 40.275 | 1.00 | 40.38 | N |
| ATOM 2266 | CA | GLN B 170 | 31.785 | 12.696 | 40.992 | 1.00 | 39.99 | C |
| ATOM 2267 | CB | GLN B 170 | 30.317 | 12.763 | 41.415 | 1.00 | 39.42 | C |
| ATOM 2268 | CG | GLN B 170 | 30.049 | 13.757 | 42.528 | 1.00 | 36.89 | C |
| ATOM 2269 | CD | GLN B 170 | 30.870 | 13.469 | 43.770 | 1.00 | 37.17 | C |
| ATOM 2270 | OE1 | GLN B 170 | 30.931 | 12.327 | 44.235 | 1.00 | 36.89 | O |
| ATOM 2271 | NE2 | GLN B 170 | 31.502 | 14.501 | 44.317 | 1.00 | 33.10 | N |
| ATOM 2272 | C | GLN B 170 | 32.019 | 11.456 | 40.137 | 1.00 | 40.67 | C |
| ATOM 2273 | O | GLN B 170 | 31.554 | 10.361 | 40.464 | 1.00 | 40.53 | O |
| ATOM 2274 | N | GLY B 171 | 32.739 | 11.637 | 39.034 | 1.00 | 42.18 | N |
| ATOM 2275 | CA | GLY B 171 | 33.031 | 10.519 | 38.158 | 1.00 | 42.99 | C |
| ATOM 2276 | C | GLY B 171 | 31.891 | 10.138 | 37.234 | 1.00 | 44.08 | C |
| ATOM 2277 | O | GLY B 171 | 31.738 | 8.970 | 36.870 | 1.00 | 44.03 | O |
| ATOM 2278 | N | LEU B 172 | 31.077 | 11.118 | 36.861 | 1.00 | 44.44 | N |
| ATOM 2279 | CA | LEU B 172 | 29.966 | 10.863 | 35.957 | 1.00 | 44.81 | C |
| ATOM 2280 | CB | LEU B 172 | 28.664 | 11.432 | 36.524 | 1.00 | 45.23 | C |
| ATOM 2281 | CG | LEU B 172 | 28.158 | 10.879 | 37.858 | 1.00 | 44.93 | C |
| ATOM 2282 | CD1 | LEU B 172 | 26.829 | 11.536 | 38.188 | 1.00 | 45.43 | C |
| ATOM 2283 | CD2 | LEU B 172 | 27.993 | 9.368 | 37.783 | 1.00 | 45.17 | C |
| ATOM 2284 | C | LEU B 172 | 30.281 | 11.524 | 34.624 | 1.00 | 45.45 | C |
| ATOM 2285 | O | LEU B 172 | 30.717 | 12.675 | 34.576 | 1.00 | 45.61 | O |
| ATOM 2286 | N | THR B 173 | 30.073 | 10.788 | 33.541 | 1.00 | 45.63 | N |
| ATOM 2287 | CA | THR B 173 | 30.338 | 11.309 | 32.209 | 1.00 | 46.03 | C |
| ATOM 2288 | CB | THR B 173 | 30.765 | 10.183 | 31.267 | 1.00 | 45.95 | C |
| ATOM 2289 | OG1 | THR B 173 | 29.743 | 9.183 | 31.228 | 1.00 | 47.13 | O |
| ATOM 2290 | CG2 | THR B 173 | 32.054 | 9.545 | 31.761 | 1.00 | 46.88 | C |
| ATOM 2291 | C | THR B 173 | 29.089 | 11.981 | 31.651 | 1.00 | 45.65 | C |
| ATOM 2292 | O | THR B 173 | 27.991 | 11.801 | 32.179 | 1.00 | 45.14 | O |
| ATOM 2293 | N | GLU B 174 | 29.260 | 12.751 | 30.581 | 1.00 | 45.76 | N |
| ATOM 2294 | CA | GLU B 174 | 28.140 | 13.448 | 29.969 | 1.00 | 45.79 | C |
| ATOM 2295 | CB | GLU B 174 | 28.615 | 14.278 | 28.777 | 1.00 | 46.84 | C |
| ATOM 2296 | CG | GLU B 174 | 29.518 | 15.428 | 29.181 | 1.00 | 49.10 | C |
| ATOM 2297 | CD | GLU B 174 | 29.375 | 16.630 | 28.273 | 1.00 | 51.93 | C |
| ATOM 2298 | OE1 | GLU B 174 | 29.639 | 16.496 | 27.058 | 1.00 | 53.25 | O |
| ATOM 2299 | OE2 | GLU B 174 | 28.993 | 17.709 | 28.775 | 1.00 | 50.59 | O |
| ATOM 2300 | C | GLU B 174 | 27.032 | 12.501 | 29.541 | 1.00 | 44.90 | C |
| ATOM 2301 | O | GLU B 174 | 25.850 | 12.846 | 29.615 | 1.00 | 45.22 | O |
| ATOM 2302 | N | GLU B 175 | 27.401 | 11.304 | 29.100 | 1.00 | 43.45 | N |
| ATOM 2303 | CA | GLU B 175 | 26.389 | 10.341 | 28.693 | 1.00 | 42.73 | C |
| ATOM 2304 | CB | GLU B 175 | 27.028 | 9.121 | 28.017 | 1.00 | 43.30 | C |
| ATOM 2305 | CG | GLU B 175 | 28.093 | 8.403 | 28.815 | 1.00 | 47.17 | C |
| ATOM 2306 | CD | GLU B 175 | 27.817 | 6.913 | 28.917 | 1.00 | 52.72 | C |
| ATOM 2307 | OE1 | GLU B 175 | 27.495 | 6.295 | 27.877 | 1.00 | 54.49 | O |
| ATOM 2308 | OE2 | GLU B 175 | 27.922 | 6.359 | 30.034 | 1.00 | 54.58 | O |
| ATOM 2309 | C | GLU B 175 | 25.582 | 9.918 | 29.916 | 1.00 | 40.71 | C |
| ATOM 2310 | O | GLU B 175 | 24.478 | 9.390 | 29.793 | 1.00 | 40.87 | O |
| ATOM 2311 | N | ASP B 176 | 26.136 | 10.166 | 31.099 | 1.00 | 39.09 | N |
| ATOM 2312 | CA | ASP B 176 | 25.460 | 9.826 | 32.345 | 1.00 | 38.13 | C |
| ATOM 2313 | CB | ASP B 176 | 26.465 | 9.332 | 33.391 | 1.00 | 38.20 | C |
| ATOM 2314 | CG | ASP B 176 | 26.930 | 7.910 | 33.132 | 1.00 | 38.78 | C |
| ATOM 2315 | OD1 | ASP B 176 | 26.083 | 7.055 | 32.782 | 1.00 | 36.70 | O |
| ATOM 2316 | OD2 | ASP B 176 | 28.139 | 7.644 | 33.296 | 1.00 | 40.22 | O |
| ATOM 2317 | C | ASP B 176 | 24.693 | 11.019 | 32.912 | 1.00 | 36.59 | C |
| ATOM 2318 | O | ASP B 176 | 24.104 | 10.930 | 33.982 | 1.00 | 36.42 | O |
| ATOM 2319 | N | ILE B 177 | 24.698 | 12.130 | 32.191 | 1.00 | 35.68 | N |
| ATOM 2320 | CA | ILE B 177 | 24.017 | 13.332 | 32.650 | 1.00 | 34.90 | C |
| ATOM 2321 | CB | ILE B 177 | 25.009 | 14.507 | 32.722 | 1.00 | 35.58 | C |
| ATOM 2322 | CG1 | ILE B 177 | 26.172 | 14.132 | 33.649 | 1.00 | 33.32 | C |
| ATOM 2323 | CD1 | ILE B 177 | 27.339 | 15.103 | 33.605 | 1.00 | 34.46 | C |
| ATOM 2324 | CG2 | ILE B 177 | 24.301 | 15.762 | 33.207 | 1.00 | 34.43 | C |
| ATOM 2325 | C | ILE B 177 | 22.855 | 13.697 | 31.729 | 1.00 | 34.02 | C |
| ATOM 2326 | O | ILE B 177 | 23.002 | 13.704 | 30.509 | 1.00 | 33.69 | O |
| ATOM 2327 | N | VAL B 178 | 21.701 | 13.996 | 32.316 | 1.00 | 32.85 | N |
| ATOM 2328 | CA | VAL B 178 | 20.532 | 14.345 | 31.517 | 1.00 | 31.32 | C |
| ATOM 2329 | CB | VAL B 178 | 19.650 | 13.107 | 31.252 | 1.00 | 31.12 | C |
| ATOM 2330 | CG1 | VAL B 178 | 19.180 | 12.513 | 32.563 | 1.00 | 31.16 | C |
| ATOM 2331 | CG2 | VAL B 178 | 18.445 | 13.500 | 30.400 | 1.00 | 33.20 | C |
| ATOM 2332 | C | VAL B 178 | 19.637 | 15.412 | 32.122 | 1.00 | 29.38 | C |
| ATOM 2333 | O | VAL B 178 | 19.302 | 15.355 | 33.295 | 1.00 | 30.40 | O |
| ATOM 2334 | N | PHE B 179 | 19.248 | 16.387 | 31.308 | 1.00 | 28.78 | N |
| ATOM 2335 | CA | PHE B 179 | 18.341 | 17.438 | 31.759 | 1.00 | 28.53 | C |
| ATOM 2336 | CB | PHE B 179 | 18.737 | 18.786 | 31.143 | 1.00 | 27.73 | C |
| ATOM 2337 | CG | PHE B 179 | 20.042 | 19.315 | 31.684 | 1.00 | 29.80 | C |
| ATOM 2338 | CD1 | PHE B 179 | 20.116 | 19.813 | 32.983 | 1.00 | 30.84 | C |
| ATOM 2339 | CE1 | PHE B 179 | 21.336 | 20.190 | 33.544 | 1.00 | 32.23 | C |
| ATOM 2340 | CZ | PHE B 179 | 22.502 | 20.072 | 32.797 | 1.00 | 34.74 | C |
| ATOM 2341 | CE2 | PHE B 179 | 22.443 | 19.582 | 31.492 | 1.00 | 33.64 | C |
| ATOM 2342 | CD2 | PHE B 179 | 21.215 | 19.209 | 30.942 | 1.00 | 32.32 | C |
| ATOM 2343 | C | PRE B 179 | 16.956 | 16.974 | 31.331 | 1.00 | 29.31 | C |
| ATOM 2344 | O | PHE B 179 | 16.622 | 16.949 | 30.143 | 1.00 | 29.18 | O |
| ATOM 2345 | N | LEU B 180 | 16.178 | 16.565 | 32.330 | 1.00 | 30.16 | N |
| ATOM 2346 | CA | LEU B 180 | 14.836 | 16.031 | 32.150 | 1.00 | 29.93 | C |
| ATOM 2347 | CB | LEU B 180 | 14.245 | 15.708 | 33.524 | 1.00 | 30.00 | C |
| ATOM 2348 | CG | LEU B 180 | 15.155 | 14.806 | 34.375 | 1.00 | 30.73 | C |
| ATOM 2349 | CD1 | LEU B 180 | 14.558 | 14.593 | 35.762 | 1.00 | 26.95 | C |
| ATOM 2350 | CD2 | LEU B 180 | 15.352 | 13.468 | 33.672 | 1.00 | 26.54 | C |
| ATOM 2351 | C | LEU B 180 | 13.887 | 16.912 | 31.351 | 1.00 | 30.61 | C |
| ATOM 2352 | O | LEU B 180 | 13.620 | 18.057 | 31.720 | 1.00 | 30.66 | O |
| ATOM 2353 | N | PRO B 181 | 13.362 | 16.382 | 30.232 | 1.00 | 32.29 | N |
| ATOM 2354 | CA | PRO B 181 | 12.437 | 17.139 | 29.388 | 1.00 | 33.25 | C |
| ATOM 2355 | CB | PRO B 181 | 12.331 | 16.276 | 28.134 | 1.00 | 33.09 | C |
| ATOM 2356 | CG | PRO B 181 | 12.459 | 14.902 | 28.676 | 1.00 | 33.42 | C |
| ATOM 2357 | CD | PRO B 181 | 13.589 | 15.036 | 29.674 | 1.00 | 30.91 | C |
| ATOM 2358 | C | PRO B 181 | 11.091 | 17.341 | 30.070 | 1.00 | 34.85 | C |
| ATOM 2359 | O | PRO B 181 | 10.603 | 16.463 | 30.773 | 1.00 | 33.36 | O |
| ATOM 2360 | N | GLN B 182 | 10.509 | 18.517 | 29.853 | 1.00 | 37.61 | N |
| ATOM 2361 | CA | GLN B 182 | 9.220 | 18.878 | 30.421 | 1.00 | 41.17 | C |
| ATOM 2362 | CB | GIN B 182 | 8.963 | 20.365 | 30.174 | 1.00 | 41.43 | C |
| ATOM 2363 | CG | GLN B 182 | 7.645 | 20.876 | 30.706 | 1.00 | 43.12 | C |
| ATOM 2364 | CD | GLN B 182 | 7.403 | 22.321 | 30.326 | 1.00 | 46.68 | C |
| ATOM 2365 | OE1 | GLN B 182 | 8.151 | 23.214 | 30.729 | 1.00 | 45.85 | O |
| ATOM 2366 | NE2 | GLN B 182 | 6.360 | 22.559 | 29.536 | 1.00 | 46.64 | N |
| ATOM 2367 | C | GLN B 182 | 8.095 | 18.043 | 29.805 | 1.00 | 42.85 | C |
| ATOM 2368 | O | GLN B 182 | 7.862 | 18.090 | 28.602 | 1.00 | 43.90 | O |
| ATOM 2369 | N | PRO B 183 | 7.390 | 17.257 | 30.629 | 1.00 | 44.63 | N |
| ATOM 2370 | CA | PRO B 183 | 6.288 | 16.416 | 30.150 | 1.00 | 46.17 | C |
| ATOM 2371 | CB | PRO B 183 | 5.904 | 15.610 | 31.390 | 1.00 | 45.60 | C |
| ATOM 2372 | CG | PRO B 183 | 7.167 | 15.564 | 32.173 | 1.00 | 45.82 | C |
| ATOM 2373 | CD | PRO B 183 | 7.687 | 16.964 | 32.038 | 1.00 | 44.40 | C |
| ATOM 2374 | C | PRO B 183 | 5.130 | 17.272 | 29.657 | 1.00 | 47.47 | C |
| ATOM 2375 | O | PRO B 183 | 4.973 | 18.413 | 30.091 | 1.00 | 47.66 | O |
| ATOM 2376 | N | ASP B 184 | 4.319 | 16.726 | 28.757 | 1.00 | 49.52 | N |
| ATOM 2377 | CA | ASP B 184 | 3.181 | 17.470 | 28.239 | 1.00 | 52.24 | C |
| ATOM 2378 | CB | ASP B 184 | 2.737 | 16.898 | 26.891 | 1.00 | 52.89 | C |
| ATOM 2379 | CG | ASP B 184 | 2.513 | 17.982 | 25.849 | 1.00 | 55.95 | C |
| ATOM 2380 | OD1 | ASP B 184 | 3.480 | 18.715 | 25.534 | 1.00 | 57.87 | O |
| ATOM 2381 | OD2 | ASP B 184 | 1.373 | 18.107 | 25.346 | 1.00 | 61.13 | O |
| ATOM 2382 | C | ASP B 184 | 2.034 | 17.410 | 29.248 | 1.00 | 53.04 | C |
| ATOM 2383 | O | ASP B 184 | 1.228 | 18.332 | 29.343 | 1.00 | 53.26 | O |
| ATOM 2384 | N | LYS B 185 | 1.975 | 16.321 | 30.007 | 1.00 | 54.24 | N |
| ATOM 2385 | CA | LYS B 185 | 0.945 | 16.148 | 31.024 | 1.00 | 55.51 | C |
| ATOM 2386 | CB | LYS B 185 | -0.080 | 15.100 | 30.578 | 1.00 | 55.50 | C |
| ATOM 2387 | CG | LYS B 185 | 0.500 | 13.738 | 30.233 | 1.00 | 57.16 | C |
| ATOM 2388 | CD | LYS B 185 | -0.576 | 12.827 | 29.658 | 1.00 | 59.05 | C |
| ATOM 2389 | CE | LYS B 185 | 0.029 | 11.582 | 29.027 | 1.00 | 60.15 | C |
| ATOM 2390 | NZ | LYS B 185 | -0.982 | 10.804 | 28.253 | 1.00 | 62.13 | N |
| ATOM 2391 | C | LYS B 185 | 1.569 | 15.728 | 32.351 | 1.00 | 55.95 | C |
| ATOM 2392 | O | LYS B 185 | 2.792 | 15.651 | 32.472 | 1.00 | 55.12 | O |
| ATOM 2393 | N | CYS B 186 | 0.716 | 15.473 | 33.340 | 1.00 | 57.05 | N |
| ATOM 2394 | CA | CYS B 186 | 1.139 | 15.050 | 34.675 | 1.00 | 58.53 | C |
| ATOM 2395 | CB | CYS B 186 | 2.096 | 13.857 | 34.595 | 1.00 | 57.56 | C |
| ATOM 2396 | SG | CYS B 186 | 1.530 | 12.467 | 33.576 | 1.00 | 54.12 | S |
| ATOM 2397 | C | CYS B 186 | 1.815 | 16.131 | 35.513 | 1.00 | 60.82 | C |
| ATOM 2398 | O | CYS B 186 | 2.384 | 15.823 | 36.559 | 1.00 | 61.78 | O |
| ATOM 2399 | N | ILE B 187 | 1.763 | 17.387 | 35.080 | 1.00 | 63.56 | N |
| ATOM 2400 | CA | ILE B 187 | 2.415 | 18.454 | 35.839 | 1.00 | 66.53 | C |
| ATOM 2401 | CB | ILE B 187 | 3.874 | 18.652 | 35.363 | 1.00 | 66.78 | C |
| ATOM 2402 | CG1 | ILE B 187 | 4.702 | 17.408 | 35.696 | 1.00 | 66.73 | C |
| ATOM 2403 | CD1 | ILE B 187 | 6.159 | 17.512 | 35.298 | 1.00 | 69.46 | C |
| ATOM 2404 | CG2 | ILE B 187 | 4.487 | 19.879 | 36.026 | 1.00 | 67.81 | C |
| ATOM 2405 | C | ILE B 187 | 1.711 | 19.806 | 35.806 | 1.00 | 68.53 | C |
| ATOM 2406 | O | ILE B 187 | 1.952 | 20.651 | 36.669 | 1.00 | 69.20 | O |
| ATOM 2407 | N | GLN B 188 | 0.855 | 20.003 | 34.805 | 1.00 | 71.16 | N |
| ATOM 2408 | CA | GLN B 188 | 0.093 | 21.243 | 34.635 | 1.00 | 73.58 | C |
| ATOM 2409 | CB | GLN B 188 | -0.529 | 21.695 | 35.961 | 1.00 | 73.62 | C |
| ATOM 2410 | CG | GLN B 188 | -1.130 | 20.595 | 36.807 | 1.00 | 75.18 | C |
| ATOM 2411 | CD | GLN B 188 | -1.452 | 21.083 | 38.206 | 1.00 | 78.27 | C |
| ATOM 2412 | OE1 | GLN B 188 | -0.749 | 21.937 | 38.748 | 1.00 | 79.42 | O |
| ATOM 2413 | NE2 | GLN B 188 | -2.502 | 20.535 | 38.806 | 1.00 | 78.56 | N |
| ATOM 2414 | C | GLN B 188 | 0.950 | 22.385 | 34.095 | 1.00 | 75.04 | C |
| ATOM 2415 | O | GLN B 188 | 0.985 | 23.464 | 34.687 | 1.00 | 75.36 | O |
| ATOM 2416 | N | GLU B 189 | 1.628 | 22.136 | 32.977 | 1.00 | 76.95 | N |
| ATOM 2417 | CA | GLU B 189 | 2.494 | 23.116 | 32.311 | 1.00 | 78.69 | C |
| ATOM 2418 | CB | GLU B 189 | 3.166 | 24.058 | 33.320 | 1.00 | 79.07 | C |
| ATOM 2419 | CG | GLU B 189 | 2.460 | 25.402 | 33.534 | 1.00 | 81.42 | C |
| ATOM 2420 | CD | GLU B 189 | 2.511 | 26.310 | 32.312 | 1.00 | 84.40 | C |
| ATOM 2421 | OE1 | GLU B 189 | 1.987 | 25.916 | 31.246 | 1.00 | 85.84 | O |
| ATOM 2422 | OE2 | GLU B 189 | 3.073 | 27.423 | 32.419 | 1.00 | 84.76 | O |
| ATOM 2423 | C | GLU B 189 | 3.572 | 22.399 | 31.504 | 1.00 | 79.10 | C |
| ATOM 2424 | O | GLU B 189 | 4.766 | 22.599 | 31.810 | 1.00 | 79.70 | O |
| ATOM 2425 | OXT | GLU B 189 | 3.209 | 21.644 | 30.576 | 1.00 | 79.48 | O |
| ATOM 2426 | O | HOH W 1 | 4.040 | 12.508 | 8.261 | 1.00 | 26.70 | O |
| ATOM 2427 | O | HOH W 2 | 13.933 | 21.047 | -5.444 | 1.00 | 32.16 | O |
| ATOM 2428 | O | HOH W 3 | 14.595 | 10.712 | -6.463 | 1.00 | 26.68 | O |
| ATOM 2429 | O | HOH W 4 | 19.496 | 15.770 | 2.484 | 1.00 | 31.13 | O |
| ATOM 2430 | O | HOH W 5 | 18.407 | 11.181 | -4.230 | 1.00 | 34.02 | O |
| ATOM 2431 | O | HOH W 6 | 16.852 | 13.205 | 18.050 | 1.00 | 33.11 | O |
| ATOM 2432 | O | HOH W 7 | 10.214 | 21.072 | 58.242 | 1.00 | 37.47 | O |
| ATOM 2433 | O | HOH W 8 | 9.748 | 10.525 | 59.061 | 1.00 | 34.17 | O |
| ATOM 2434 | O | HOH W 9 | 20.458 | 12.571 | 44.330 | 1.00 | 35.78 | O |
| ATOM 2435 | O | HOH W 10 | 8.700 | 11.028 | 45.277 | 1.00 | 51.91 | O |
| ATOM 2436 | O | HOH W 11 | 16.314 | 26.308 | 10.121 | 1.00 | 33.23 | O |
| ATOM 2437 | O | HOH W 12 | 15.210 | 20.001 | 32.653 | 1.00 | 39.92 | O |
| ATOM 2438 | O | HOH W 13 | 3.960 | 5.716 | 47.160 | 1.00 | 45.80 | O |
| ATOM 2439 | O | HOH W 14 | 26.435 | 17.047 | 49.748 | 1.00 | 40.15 | O |
| ATOM 2440 | O | HOH W 15 | -1.112 | 11.755 | 2.219 | 1.00 | 64.01 | O |
| ATOM 2441 | O | HOH W 16 | 10.171 | 28.451 | 50.750 | 1.00 | 43.61 | O |
| ATOM 2442 | O | HOH W 17 | 10.104 | 20.203 | 4.975 | 1.00 | 53.19 | O |
| ATOM 2443 | O | HOH W 18 | 20.745 | 16.542 | -2.769 | 1.00 | 28.77 | O |
| ATOM 2444 | O | HOH W 19 | -5.660 | -7.610 | 5.960 | 1.00 | 45.24 | O |
| ATOM 2445 | O | HOH W 20 | 9.000 | 20.045 | 19.857 | 1.00 | 38.22 | O |
| ATOM 2446 | O | HOH W 21 | 8.480 | 7.462 | 30.871 | 1.00 | 47.46 | O |
| ATOM 2447 | O | HOH W 22 | 27.822 | 17.883 | 54.991 | 1.00 | 28.92 | O |
| ATOM 2448 | O | HOH W 23 | 2.188 | 18.872 | 32.043 | 1.00 | 48.84 | O |
| ATOM 2449 | O | HOH W 24 | 17.633 | 18.838 | 57.504 | 1.00 | 41.89 | O |
| ATOM 2450 | O | HOH W 25 | 21.652 | 25.017 | 51.935 | 1.00 | 57.47 | O |
| ATOM 2451 | O | HOH W 26 | 7.604 | 12.850 | 34.861 | 1.00 | 41.07 | O |
| ATOM 2452 | O | HOH W 27 | 25.207 | 11.648 | 50.638 | 1.00 | 69.60 | O |
| ATOM 2453 | O | HOH W 29 | 16.729 | 13.179 | 27.696 | 1.00 | 47.29 | O |
| ATOM 2454 | O | HOH W 30 | 5.081 | 13.816 | 26.987 | 1.00 | 66.72 | O |
| ATOM 2455 | O | HOH W 31 | 12.960 | 30.863 | 40.957 | 1.00 | 47.76 | O |
| ATOM 2456 | O | HOH W 32 | 15.970 | 11.521 | 7.301 | 1.00 | 49.79 | O |
| ATOM 2457 | O | HOH W 33 | 21.271 | 23.315 | 14.636 | 1.00 | 42.32 | O |
| ATOM 2458 | O | HOH W 34 | 5.009 | 10.689 | 25.767 | 1.00 | 58.86 | O |
| ATOM 2459 | O | HOH W 35 | 1.694 | 10.843 | -1.667 | 1.00 | 38.92 | O |
| ATOM 2460 | O | HOH W 36 | -0.310 | 25.857 | 2.103 | 1.00 | 60.86 | O |
| ATOM 2461 | O | HOH W 37 | -8.796 | 15.352 | 6.606 | 1.00 | 69.93 | O |
| ATOM 2462 | O | HOH W 38 | 20.951 | 15.510 | -0.080 | 1.00 | 28.14 | O |
| ATOM 2463 | O | HOH W 39 | 15.352 | 11.784 | 30.109 | 1.00 | 51.67 | O |
| ATOM 2464 | O | HOH W 40 | 3.355 | 14.719 | 53.024 | 1.00 | 37.70 | O |
| ATOM 2465 | O | HOH W 42 | -2.050 | 9.295 | -4.545 | 1.00 | 57.33 | O |
| ATOM 2466 | O | HOH W 43 | -9.976 | 9.916 | 9.991 | 1.00 | 87.82 | O |
| ATOM 2467 | O | HOH W 44 | 5.572 | 21.524 | -2.004 | 1.00 | 74.94 | O |
| ATOM 2468 | O | HOH W 46 | 9.193 | 11.834 | 22.390 | 1.00 | 50.58 | O |
| ATOM 2469 | O | HOH W 47 | 17.876 | 15.823 | 58.201 | 1.00 | 45.45 | O |
| ATOM 2470 | O | HOH W 48 | 10.186 | 1.800 | 11.898 | 1.00 | 58.91 | O |
| ATOM 2471 | O | HOH W 49 | 9.488 | 10.004 | 34.123 | 1.00 | 48.87 | O |
| ATOM 2472 | O | HOH W 50 | 0.567 | 26.733 | 39.027 | 1.00 | 52.93 | O |
| ATOM 2473 | O | HOH W 51 | 10.518 | 24.208 | -7.159 | 1.00 | 43.91 | O |
| ATOM 2474 | O | HOH W 52 | 11.275 | 31.220 | 11.609 | 1.00 | 72.49 | O |
| ATOM 2475 | O | HOH W 53 | 20.256 | 5.993 | 5.735 | 1.00 | 48.72 | O |
| ATOM 2476 | O | HOH W 54 | -4.488 | 19.914 | 37.431 | 1.00 | 55.84 | O |
| ATOM 2477 | O | HOH W 55 | 9.259 | 18.064 | 3.239 | 1.00 | 58.33 | O |
| ATOM 2478 | O | HOH W 56 | 23.933 | 22.944 | 51.722 | 1.00 | 54.99 | O |
| ATOM 2479 | O | HOH W 57 | 24.841 | 9.119 | -0.096 | 1.00 | 53.03 | O |
| ATOM 2480 | O | HOH W 58 | 4.424 | 16.540 | 23.959 | 1.00 | 45.30 | O |
| ATOM 2481 | O | HOH W 59 | 23.572 | 18.774 | 53.301 | 1.00 | 43.19 | O |
| ATOM 2482 | O | HOH W 60 | 19.627 | 11.112 | 27.097 | 1.00 | 56.82 | O |
| ATOM 2483 | O | HOH W 61 | 7.728 | 25.890 | 42.676 | 1.00 | 40.99 | O |
| ATOM 2484 | O | HOH W 62 | 13.333 | 1.796 | 4.471 | 1.00 | 48.50 | O |
| ATOM 2485 | O | HOH W 63 | 5.991 | 15.776 | -5.712 | 1.00 | 36.49 | O |
| ATOM 2486 | O | HOH W 65 | 6.100 | 10.580 | 56.983 | 1.00 | 44.25 | O |
| ATOM 2487 | O | HOH W 66 | 10.175 | 17.800 | -9.148 | 1.00 | 50.73 | O |
| ATOM 2488 | O | HOH W 67 | 12.429 | 10.671 | 26.357 | 1.00 | 43.62 | O |
| ATOM 2489 | O | HOH W 68 | 14.092 | 19.997 | 47.805 | 1.00 | 56.02 | O |
| ATOM 2490 | O | HOH W 69 | 27.526 | 13.816 | 40.679 | 1.00 | 143.11 | O |
| ATOM 2491 | O | HOH W 70 | 27.171 | 25.976 | 50.883 | 1.00 | 70.04 | O |
| ATOM 2492 | O | HOH W 71 | 25.949 | 13.041 | 12.265 | 1.00 | 61.46 | O |
| ATOM 2493 | O | HOH W 73 | 20.947 | 34.715 | 46.208 | 1.00 | 68.24 | O |
| ATOM 2494 | O | HOH W 74 | 16.555 | 26.377 | 22.214 | 1.00 | 59.16 | O |
| ATOM 2495 | O | HOH W 75 | -3.547 | 26.746 | 2.866 | 1.00 | 71.13 | O |
| ATOM 2496 | O | HOH W 76 | 5.540 | 14.794 | -9.696 | 1.00 | 69.89 | O |
| ATOM 2497 | O | HOH W 77 | 23.279 | -0.635 | 39.750 | 1.00 | 62.15 | O |
| ATOM 2498 | O | HOHW 80 | 19.922 | 16.795 | 28.541 | 1.00 | 44.27 | O |
| ATOM 2499 | O | HOH W 81 | 27.483 | 11.963 | 53.993 | 1.00 | 45.20 | O |
| ATOM 2500 | O | HOH W 82 | 13.906 | 22.578 | 32.044 | 1.00 | 55.11 | O |
| ATOM 2501 | O | HOH W 83 | 22.987 | 24.494 | 12.307 | 1.00 | 58.59 | O |
| ATOM 2502 | O | HOH W 84 | 16.335 | 8.026 | 21.642 | 1.00 | 46.23 | O |
| ATOM 2503 | O | HOH W 85 | 12.684 | 3.455 | 13.075 | 1.00 | 78.38 | O |
| ATOM 2504 | O | HOH W 86 | 20.879 | 16.549 | 9.771 | 1.00 | 42.57 | O |
| ATOM 2505 | O | HOH W 87 | 0.757 | 21.593 | 28.527 | 1.00 | 69.69 | O |
| ATOM 2506 | O | HOH W 88 | 13.879 | 17.013 | 47.543 | 1.00 | 54.48 | O |
| ATOM 2507 | O | HOH W 89 | 15.694 | 18.445 | 49.669 | 1.00 | 54.79 | O |
| ATOM 2508 | O | HOH W 90 | -2.076 | 0.968 | 51.836 | 1.00 | 59.41 | O |
| ATOM 2509 | O | HOH W 91 | 19.272 | 30.879 | 50.117 | 1.00 | 58.03 | O |
| ATOM 2510 | O | HOH W 92 | 17.785 | 26.541 | 5.860 | 1.00 | 58.78 | O |
| ATOM 2511 | O | HOH W 93 | 15.194 | 10.406 | 18.655 | 1.00 | 51.55 | O |
| ATOM 2512 | O | HOH W 94 | 14.191 | 10.352 | 15.784 | 1.00 | 59.53 | O |
| ATOM 2513 | O | HOH W 95 | 14.530 | 8.322 | 12.335 | 1.00 | 57.57 | O |
| ATOM 2514 | O | HOH W 96 | 14.382 | 6.550 | 1.265 | 1.00 | 39.82 | O |
| ATOM 2515 | O | HOH W 97 | 3.491 | 9.628 | -10.219 | 1.00 | 64.18 | O |
| ATOM 2516 | O | HOH W 98 | 7.943 | 11.032 | 6.651 | 1.00 | 61.55 | O |
| ATOM 2517 | O | HOH W 99 | 2.917 | 34.621 | 6.036 | 1.00 | 76.28 | O |
| ATOM 2518 | O | HOH W 100 | 6.976 | 33.080 | 4.434 | 1.00 | 63.23 | O |
| ATOM 2519 | O | HOH W 101 | 4.575 | 31.407 | 2.694 | 1.00 | 62.67 | O |
| ATOM 2520 | O | HOH W 102 | 10.274 | 34.137 | 10.082 | 1.00 | 56.85 | O |
| ATOM 2521 | O | HOH W 103 | 10.163 | 22.575 | 20.912 | 1.00 | 54.58 | O |
| ATOM 2522 | O | HOH W 104 | 8.270 | 24.585 | 21.965 | 1.00 | 47.21 | O |
| ATOM 2523 | O | HOH W 105 | 17.520 | 21.811 | 53.118 | 1.00 | 56.66 | O |
| ATOM 2524 | O | HOH W 106 | 9.883 | 6.038 | 51.329 | 1.00 | 35.50 | O |
| ATOM 2525 | O | HOH W 107 | 14.590 | 8.397 | 41.921 | 1.00 | 81.43 | O |
| ATOM 2526 | O | HOH W 108 | 16.218 | 11.381 | 46.157 | 1.00 | 72.99 | O |
| ATOM 2527 | O | HOH W 109 | 24.706 | 15.452 | 29.112 | 1.00 | 57.26 | O |
| ATOM 2528 | O | HOH W 110 | 18.692 | 22.125 | 55.822 | 1.00 | 70.38 | O |
| ATOM 2529 | O | HOH W 111 | 22.284 | 24.536 | 58.178 | 1.00 | 54.74 | O |
| ATOM 2530 | O | HOH W 112 | 17.887 | 1.620 | 25.979 | 1.00 | 51.74 | O |
| ATOM 2531 | O | HOH W 113 | 25.868 | 4.467 | 51.867 | 1.00 | 74.64 | O |

**Table 3**

| Three Dimensional Coordinates of Se-Met-type L-PGDS | | | | | | | | |
|---|---|---|---|---|---|---|---|---|
| ATOM 1 | CB | PHE A 34 | -8.574 | -24.037 | 55.181 | 1.00 | 80.07 | A |
| ATOM 2 | CG | PHE A 34 | -7.718 | -22.864 | 54.718 | 1.00 | 79.58 | A |
| ATOM 3 | CD1 | PHE A 34 | -7.455 | -22.691 | 53.358 | 1.00 | 78.93 | A |
| ATOM 4 | CD2 | PHE A 34 | -7.119 | -21.988 | 55.626 | 1.00 | 78.38 | A |
| ATOM 5 | CE1 | PHE A 34 | -6.605 | -21.683 | 52.908 | 1.00 | 77.67 | A |
| ATOM 6 | CE2 | PHE A 34 | -6.265 | -20.975 | 55.184 | 1.00 | 78.08 | A |
| ATOM 7 | CZ | PHE A 34 | -6.008 | -20.827 | 53.819 | 1.00 | 78.70 | A |
| ATOM 8 | C | PHE A 34 | -7.194 | -24.354 | 57.289 | 1.00 | 78.35 | A |
| ATOM 9 | O | PHE A 34 | -6.887 | -23.715 | 58.301 | 1.00 | 79.55 | A |
| ATOM 10 | N | PHE A 34 | -9.359 | -23.145 | 57.369 | 1.00 | 80.95 | A |
| ATOM 11 | CA | PHE A 34 | -8.607 | -24.254 | 56.712 | 1.00 | 79.32 | A |
| ATOM 12 | N | GLN A 35 | -6.335 | -25.151 | 56.657 | 1.00 | 75.88 | A |
| ATOM 13 | CA | GLN A 35 | -4.962 | -25.309 | 57.136 | 1.00 | 72.38 | A |
| ATOM 14 | CB | GLN A 35 | -4.643 | -26.790 | 57.280 | 1.00 | 74.51 | A |
| ATOM 15 | CG | GLN A 35 | -5.890 | -27.647 | 57.401 | 1.00 | 75.90 | A |
| ATOM 16 | CD | GLN A 35 | -5.570 | -29.094 | 57.698 | 1.00 | 76.72 | A |
| ATOM 17 | OE1 | GLN A 35 | -5.107 | -29.428 | 58.794 | 1.00 | 78.20 | A |
| ATOM 18 | NE2 | GLN A 35 | -5.808 | -29.965 | 56.722 | 1.00 | 75.23 | A |
| ATOM 19 | C | GLN A 35 | -4.008 | -24.658 | 56.138 | 1.00 | 67.48 | A |
| ATOM 20 | O | GLN A 35 | -3.528 | -25.318 | 55.216 | 1.00 | 65.63 | A |
| ATOM 21 | N | GLN A 36 | -3.727 | -23.369 | 56.335 | 1.00 | 62.20 | A |
| ATOM 22 | CA | GLN A 36 | -2.859 | -22.638 | 55.420 | 1.00 | 55.74 | A |
| ATOM 23 | CB | GLN A 36 | -2.796 | -21.139 | 55.794 | 1.00 | 53.90 | A |
| ATOM 24 | CG | GLN A 36 | -2.278 | -20.778 | 57.183 | 1.00 | 53.70 | A |
| ATOM 25 | CD | GLN A 36 | -1.986 | -19.277 | 57.323 | 1.00 | 51.50 | A |
| ATOM 26 | OE1 | GLN A 36 | -2.866 | -18.437 | 57.155 | 1.00 | 52.05 | A |
| ATOM 27 | NE2 | GLN A 36 | -0.742 | -18.945 | 57.626 | 1.00 | 53.40 | A |
| ATOM 28 | C | GLN A 36 | -1.453 | -23.210 | 55.233 | 1.00 | 52.02 | A |
| ATOM 29 | O | GLN A 36 | -0.789 | -22.879 | 54.252 | 1.00 | 52.24 | A |
| ATOM 30 | N | ASP A 37 | -1.005 | -24.066 | 56.155 | 1.00 | 49.11 | A |
| ATOM 31 | CA | ASP A 37 | 0.317 | -24.702 | 56.046 | 1.00 | 44.61 | A |
| ATOM 32 | CB | ASP A 37 | 0.564 | -25.655 | 57.224 | 1.00 | 49.16 | A |
| ATOM 33 | CG | ASP A 37 | 1.188 | -24.968 | 58.439 | 1.00 | 54.29 | A |
| ATOM 34 | OD1 | ASP A 37 | 2.411 | -24.701 | 58.416 | 1.00 | 56.85 | A |
| ATOM 35 | OD2 | ASP A 37 | 0.461 | -24.703 | 59.425 | 1.00 | 55.81 | A |
| ATOM 36 | C | ASP A 37 | 0.356 | -25.495 | 54.728 | 1.00 | 41.76 | A |
| ATOM 37 | O | ASP A 37 | 1.408 | -25.659 | 54.120 | 1.00 | 37.74 | A |
| ATOM 38 | N | LYS A 38 | -0.808 | -25.976 | 54.297 | 1.00 | 40.27 | A |
| ATOM 39 | CA | LYS A 38 | -0.935 | -26.728 | 53.046 | 1.00 | 39.95 | A |
| ATOM 40 | CB | LYS A 38 | -2.220 | -27.572 | 53.057 | 1.00 | 41.46 | A |
| ATOM 41 | CG | LYS A 38 | -2.305 | -28.608 | 54.160 | 1.00 | 43.07 | A |
| ATOM 42 | CD | LYS A 38 | -1.357 | -29.759 | 53.888 | 1.00 | 49.21 | A |
| ATOM 43 | CE | LYS A 38 | -1.457 | -30.840 | 54.968 | 1.00 | 53.99 | A |
| ATOM 44 | NZ | LYS A 38 | -0.391 | -31.886 | 54.806 | 1.00 | 53.22 | A |
| ATOM 45 | C | LYS A 38 | -0.978 | -25.770 | 51.847 | 1.00 | 39.66 | A |
| ATOM 46 | O | LYS A 38 | -0.533 | -26.115 | 50.757 | 1.00 | 39.39 | A |
| ATOM 47 | N | PHE A 39 | -1.532 | -24.576 | 52.053 | 1.00 | 40.20 | A |
| ATOM 48 | CA | PHE A 39 | -1.628 | -23.556 | 51.001 | 1.00 | 37.86 | A |
| ATOM 49 | CB | PHE A 39 | -2.463 | -22.362 | 51.514 | 1.00 | 44.93 | A |
| ATOM 50 | CG | PHE A 39 | -2.920 | -21.388 | 50.433 | 1.00 | 51.45 | A |
| ATOM 51 | CD1 | PHE A 39 | -2.409 | -21.439 | 49.132 | 1.00 | 55.30 | A |
| ATOM 52 | CD2 | PHE A 39 | -3.866 | -20.403 | 50.731 | 1.00 | 51.56 | A |
| ATOM 53 | CE1 | PHE A 39 | -2.835 | -20.523 | 48.144 | 1.00 | 54.21 | A |
| ATOM 54 | CE2 | PHE A 39 | -4.296 | -19.485 | 49.751 | 1.00 | 50.99 | A |
| ATOM 55 | CZ | PHE A 39 | -3.779 | -19.547 | 48.460 | 1.00 | 51.28 | A |
| ATOM 56 | C | PHE A 39 | -0.204 | -23.098 | 50.639 | 1.00 | 32.24 | A |
| ATOM 57 | O | PHE A 39 | 0.055 | -22.637 | 49.535 | 1.00 | 28.40 | A |
| ATOM 58 | N | LEU A 40 | 0.719 | -23.244 | 51.580 | 1.00 | 29.46 | A |
| ATOM 59 | CA | LEU A 40 | 2.103 | -22.845 | 51.349 | 1.00 | 31.74 | A |
| ATOM 60 | CB | LEU A 40 | 2.952 | -23.163 | 52.579 | 1.00 | 32.26 | A |
| ATOM 61 | CG | LEU A 40 | 2.491 | -22.423 | 53.834 | 1.00 | 33.81 | A |
| ATOM 62 | CD1 | LEU A 40 | 3.404 | -22.745 | 54.998 | 1.00 | 35.43 | A |
| ATOM 63 | CD2 | LEU A 40 | 2.511 | -20.906 | 53.548 | 1.00 | 36.44 | A |
| ATOM 64 | C | LEU A 40 | 2.743 | -23.482 | 50.114 | 1.00 | 32.72 | A |
| ATOM 65 | O | LEU A 40 | 2.308 | -24.546 | 49.630 | 1.00 | 32.75 | A |
| ATOM 66 | N | GLY A 41 | 3.773 | -22.820 | 49.594 | 1.00 | 32.46 | A |
| ATOM 67 | CA | GLY A 41 | 4.447 | -23.356 | 48.432 | 1.00 | 32.86 | A |
| ATOM 68 | C | GLY A 41 | 4.363 | -22.578 | 47.134 | 1.00 | 33.14 | A |
| ATOM 69 | O | GLY A 41 | 4.061 | -21.380 | 47.106 | 1.00 | 33.14 | A |
| ATOM 70 | N | ARG A 42 | 4.628 | -23.281 | 46.040 | 1.00 | 33.66 | A |
| ATOM 71 | CA | ARG A 42 | 4.636 | -22.665 | 44.725 | 1.00 | 35.33 | A |
| ATOM 72 | CB | ARG A 42 | 5.796 | -23.233 | 43.894 | 1.00 | 38.39 | A |
| ATOM 73 | CG | ARG A 42 | 5.705 | -22.932 | 42.393 | 1.00 | 45.57 | A |
| ATOM 74 | CD | ARG A 42 | 5.769 | -24.211 | 41.537 | 1.00 | 52.54 | A |
| ATOM 75 | NE | ARG A 42 | 7.141 | -24.604 | 41.210 | 1.00 | 57.48 | A |
| ATOM 76 | CZ | ARG A 42 | 7.486 | -25.760 | 40.643 | 1.00 | 60.34 | A |
| ATOM 77 | NH1 | ARG A 42 | 6.564 | -26.668 | 40.334 | 1.00 | 61.55 | A |
| ATOM 78 | NH2 | ARG A 42 | 8.761 | -25.998 | 40.369 | 1.00 | 61.04 | A |
| ATOM 79 | C | ARG A 42 | 3.343 | -22.842 | 43.961 | 1.00 | 35.35 | A |
| ATOM 80 | O | ARG A 42 | 2.838 | -23.964 | 43.821 | 1.00 | 35.49 | A |
| ATOM 81 | N | TRP A 43 | 2.806 | -21.734 | 43.466 | 1.00 | 33.00 | A |
| ATOM 82 | CA | TRP A 43 | 1.592 | -21.791 | 42.674 | 1.00 | 33.72 | A |
| ATOM 83 | CB | TRP A 43 | 0.406 | -21.172 | 43.428 | 1.00 | 26.08 | A |
| ATOM 84 | CG | TRP A 43 | -0.007 | -21.884 | 44.680 | 1.00 | 23.97 | A |
| ATOM 85 | CD2 | TRP A 43 | -1.096 | -22.810 | 44.818 | 1.00 | 22.06 | A |
| ATOM 86 | CE2 | TRP A 43 | -1.171 | -23.169 | 46.183 | 1.00 | 18.60 | A |
| ATOM 87 | CE3 | TRP A 43 | -2.015 | -23.371 | 43.917 | 1.00 | 20.78 | A |
| ATOM 88 | CD1 | TRP A 43 | 0.529 | -21.740 | 45.936 | 1.00 | 25.06 | A |
| ATOM 89 | NE1 | TRP A 43 | -0.171 | -22.508 | 46.843 | 1.00 | 18.01 | A |
| ATOM 90 | CZ2 | TRP A 43 | -2.128 | -24.053 | 46.669 | 1.00 | 23.31 | A |
| ATOM 91 | CZ3 | TRP A 43 | -2.964 | -24.249 | 44.400 | 1.00 | 24.79 | A |
| ATOM 92 | CH2 | TRP A 43 | -3.019 | -24.583 | 45.766 | 1.00 | 23.79 | A |
| ATOM 93 | C | TRP A 43 | 1.826 | -21.037 | 41.358 | 1.00 | 35.56 | A |
| ATOM 94 | O | TRP A 43 | 2.929 | -20.550 | 41.095 | 1.00 | 37.26 | A |
| ATOM 95 | N | TYR A 44 | 0.793 | -20.975 | 40.525 | 1.00 | 37.30 | A |
| ATOM 96 | CA | TYR A 44 | 0.859 | -20.248 | 39.263 | 1.00 | 38.32 | A |
| ATOM 97 | CB | TYR A 44 | 1.120 | -21.172 | 38.072 | 1.00 | 39.52 | A |
| ATOM 98 | CG | TYR A 44 | 2.402 | -21.933 | 38.138 | 1.00 | 40.70 | A |
| ATOM 99 | CD1 | TYR A 44 | 2.402 | -23.324 | 38.354 | 1.00 | 38.14 | A |
| ATOM 100 | CE1 | TYR A 44 | 3.587 | -24.029 | 38.430 | 1.00 | 37.42 | A |
| ATOM 101 | CD2 | TYR A 44 | 3.625 | -21.274 | 37.997 | 1.00 | 39.25 | A |
| ATOM 102 | CE2 | TYR A 44 | 4.814 | -21.970 | 38.067 | 1.00 | 41.43 | A |
| ATOM 103 | CZ | TYR A 44 | 4.792 | -23.349 | 38.286 | 1.00 | 41.53 | A |
| ATOM 104 | OH | TYR A 44 | 5.985 | -24.029 | 38.370 | 1.00 | 45.03 | A |
| ATOM 105 | C | TYR A 44 | -0.487 | -19.577 | 39.018 | 1.00 | 40.46 | A |
| ATOM 106 | O | TYR A 44 | -1.544 | -20.232 | 39.158 | 1.00 | 39.17 | A |
| ATOM 107 | N | SER A 45 | -0.433 | -18.290 | 38.657 | 1.00 | 40.82 | A |
| ATOM 108 | CA | SER A 45 | -1.618 | -17.505 | 38.318 | 1.00 | 43.52 | A |
| ATOM 109 | CB | SER A 45 | -1.243 | -16.053 | 37.994 | 1.00 | 44.83 | A |
| ATOM 110 | OG | SER A 45 | -0.342 | -15.512 | 38.936 | 1.00 | 52.14 | A |
| ATOM 111 | C | SER A 45 | -2.092 | -18.152 | 37.021 | 1.00 | 45.66 | A |
| ATOM 112 | O | SER A 45 | -1.315 | -18.257 | 36.073 | 1.00 | 47.25 | A |
| ATOM 113 | N | ALA A 46 | -3.348 | -18.580 | 36.961 | 1.00 | 46.41 | A |
| ATOM 114 | CA | ALA A 46 | -3.850 | -19.219 | 35.748 | 1.00 | 45.31 | A |
| ATOM 115 | CB | ALA A 46 | -4.012 | -20.698 | 35.977 | 1.00 | 46.81 | A |
| ATOM 116 | C | ALA A 46 | -5.166 | -18.623 | 35.296 | 1.00 | 45.28 | A |
| ATOM 117 | O | ALA A 46 | -5.489 | -18.639 | 34.113 | 1.00 | 48.63 | A |
| ATOM 118 | N | GLY A 47 | -5.930 | -18.104 | 36.246 | 1.00 | 45.61 | A |
| ATOM 119 | CA | GLY A 47 | -7.205 | -17.511 | 35.910 | 1.00 | 43.75 | A |
| ATOM 120 | C | GLY A 47 | -7.329 | -16.131 | 36.505 | 1.00 | 43.31 | A |
| ATOM 121 | O | GLY A 47 | -6.677 | -15.803 | 37.485 | 1.00 | 44.49 | A |
| ATOM 122 | N | LEU A 48 | -8.170 | -15.313 | 35.901 | 1.00 | 43.33 | A |
| ATOM 123 | CA | LEU A 48 | -8.396 | -13.968 | 36.384 | 1.00 | 43.41 | A |
| ATOM 124 | CB | LEU A 48 | -7.227 | -13.074 | 36.001 | 1.00 | 40.63 | A |
| ATOM 125 | CG | LEU A 48 | -6.373 | -12.435 | 37.103 | 1.00 | 40.99 | A |
| ATOM 126 | CD1 | LEU A 48 | -5.112 | -11.878 | 36.455 | 1.00 | 38.94 | A |
| ATOM 127 | CD2 | LEU A 48 | -7.127 | -11.320 | 37.839 | 1.00 | 39.06 | A |
| ATOM 128 | C | LEU A 48 | -9.675 | -13.458 | 35.741 | 1.00 | 46.49 | A |
| ATOM 129 | O | LEU A 48 | -9.966 | -13.752 | 34.577 | 1.00 | 47.60 | A |
| ATOM 130 | N | ALA A 49 | -10.442 | -12.706 | 36.515 | 1.00 | 49.82 | A |
| ATOM 131 | CA | ALA A 49 | -11.687 | -12.128 | 36.043 | 1.00 | 54.89 | A |
| ATOM 132 | CB | ALA A 49 | -12.791 | -13.149 | 36.156 | 1.00 | 54.30 | A |
| ATOM 133 | C | ALA A 49 | -11.976 | -10.916 | 36.928 | 1.00 | 58.99 | A |
| ATOM 134 | O | ALA A 49 | -12.011 | -11.043 | 38.155 | 1.00 | 61.81 | A |
| ATOM 135 | N | SER A 50 | -12.182 | -9.745 | 36.326 | 1.00 | 62.36 | A |
| ATOM 136 | CA | SER A 50 | -12.445 | -8.550 | 37.123 | 1.00 | 66.74 | A |
| ATOM 137 | CB | SER A 50 | -11.118 | -7.939 | 37.577 | 1.00 | 65.83 | A |
| ATOM 138 | OG | SER A 50 | -11.334 | -6.795 | 38.386 | 1.00 | 63.09 | A |
| ATOM 139 | C | SER A 50 | -13.284 | -7.461 | 36.456 | 1.00 | 70.85 | A |
| ATOM 140 | O | SER A 50 | -13.550 | -7.511 | 35.252 | 1.00 | 71.11 | A |
| ATOM 141 | N | ASN A 51 | -13.692 | -6.480 | 37.266 | 1.00 | 75.09 | A |
| ATOM 142 | CA | ASN A 51 | -14.481 | -5.331 | 36.819 | 1.00 | 79.23 | A |
| ATOM 143 | CB | ASN A 51 | -15.582 | -4.964 | 37.824 | 1.00 | 81.26 | A |
| ATOM 144 | CG | ASN A 51 | -16.260 | -6.168 | 38.435 | 1.00 | 84.64 | A |
| ATOM 145 | OD1 | ASN A 51 | -16.714 | -7.072 | 37.730 | 1.00 | 87.24 | A |
| ATOM 146 | ND2 | ASN A 51 | -16.352 | -6.177 | 39.762 | 1.00 | 83.95 | A |
| ATOM 147 | C | ASN A 51 | -13.537 | -4.137 | 36.751 | 1.00 | 81.34 | A |
| ATOM 148 | O | ASN A 51 | -13.843 | -3.120 | 36.127 | 1.00 | 82.27 | A |
| ATOM 149 | N | SER A 52 | -12.401 | -4.264 | 37.428 | 1.00 | 83.15 | A |
| ATOM 150 | CA | SER A 52 | -11.403 | -3.205 | 37.484 | 1.00 | 86.71 | A |
| ATOM 151 | CB | SER A 52 | -10.126 | -3.725 | 38.142 | 1.00 | 88.84 | A |
| ATOM 152 | OG | SER A 52 | -10.400 | -4.259 | 39.423 | 1.00 | 94.06 | A |
| ATOM 153 | C | SER A 52 | -11.052 | -2.632 | 36.125 | 1.00 | 88.19 | A |
| ATOM 154 | O | SER A 52 | -10.666 | -3.363 | 35.214 | 1.00 | 88.95 | A |
| ATOM 155 | N | SER A 53 | -11.191 | -1.318 | 35.989 | 1.00 | 90.75 | A |
| ATOM 156 | CA | SER A 53 | -10.846 | -0.656 | 34.740 | 1.00 | 93.53 | A |
| ATOM 157 | CB | SER A 53 | -10.982 | 0.863 | 34.888 | 1.00 | 94.58 | A |
| ATOM 158 | OG | SER A 53 | -10.221 | 1.345 | 35.985 | 1.00 | 94.06 | A |
| ATOM 159 | C | SER A 53 | -9.392 | -1.034 | 34.485 | 1.00 | 94.71 | A |
| ATOM 160 | O | SER A 53 | -8.986 | -1.288 | 33.350 | 1.00 | 94.70 | A |
| ATOM 161 | N | TRP A 54 | -8.626 | -1.077 | 35.573 | 1.00 | 95.46 | A |
| ATOM 162 | CA | TRP A 54 | -7.216 | -1.439 | 35.542 | 1.00 | 96.17 | A |
| ATOM 163 | CB | TRP A 54 | -6.710 | -1.638 | 36.966 | 1.00 | 99.29 | A |
| ATOM 164 | CG | TRP A 54 | -5.362 | -2.259 | 37.036 | 1.00 | 103.51 | A |
| ATOM 165 | CD2 | TRP A 54 | -5.075 | -3.654 | 37.188 | 1.00 | 107.04 | A |
| ATOM 166 | CE2 | TRP A 54 | -3.669 | -3.795 | 37.159 | 1.00 | 108.07 | A |
| ATOM 167 | CE3 | TRP A 54 | -5.870 | -4.801 | 37.344 | 1.00 | 107.61 | A |
| ATOM 168 | CD1 | TRP A 54 | -4.160 | -1.628 | 36.926 | 1.00 | 103.66 | A |
| ATOM 169 | NE1 | TRP A 54 | -3.136 | -2.542 | 36.999 | 1.00 | 106.47 | A |
| ATOM 170 | CZ2 | TRP A 54 | -3.035 | -5.043 | 37.281 | 1.00 | 108.58 | A |
| ATOM 171 | CZ3 | TRP A 54 | -5.241 | -6.043 | 37.465 | 1.00 | 107.90 | A |
| ATOM 172 | CH2 | TRP A 54 | -3.835 | -6.151 | 37.433 | 1.00 | 107.76 | A |
| ATOM 173 | C | TRP A 54 | -7.044 | -2.733 | 34.759 | 1.00 | 95.39 | A |
| ATOM 174 | O | TRP A 54 | -6.128 | -2.870 | 33.946 | 1.00 | 96.21 | A |
| ATOM 175 | N | PHE A 55 | -7.930 | -3.684 | 35.030 | 1.00 | 93.38 | A |
| ATOM 176 | CA | PHE A 55 | -7.917 | -4.975 | 34.359 | 1.00 | 91.11 | A |
| ATOM 177 | CB | PHE A 55 | -8.759 | -5.978 | 35.152 | 1.00 | 87.81 | A |
| ATOM 178 | CG | PHE A 55 | -8.933 | -7.302 | 34.469 | 1.00 | 82.50 | A |
| ATOM 179 | CD1 | PRE A 55 | -7.837 | -8.109 | 34.196 | 1.00 | 79.44 | A |
| ATOM 180 | CD2 | PHE A 55 | -10.201 | -7.745 | 34.102 | 1.00 | 80.69 | A |
| ATOM 181 | CE1 | PHE A 55 | -8.001 | -9.339 | 33.569 | 1.00 | 78.70 | A |
| ATOM 182 | CE2 | PHE A 55 | -10.376 | -8.971 | 33.476 | 1.00 | 78.47 | A |
| ATOM 183 | CZ | PHE A 55 | -9.275 | -9.770 | 33.208 | 1.00 | 78.56 | A |
| ATOM 184 | C | PHE A 55 | -8.487 | -4.799 | 32.954 | 1.00 | 92.16 | A |
| ATOM 185 | O | PHE A 55 | -7.896 | -5.255 | 31.974 | 1.00 | 92.45 | A |
| ATOM 186 | N | ARG A 56 | -9.638 | -4.135 | 32.867 | 1.00 | 92.56 | A |
| ATOM 187 | CA | ARG A 56 | -10.285 | -3.872 | 31.586 | 1.00 | 92.90 | A |
| ATOM 188 | CB | ARG A 56 | -11.320 | -2.755 | 31.739 | 1.00 | 94.04 | A |
| ATOM 189 | CG | ARG A 56 | -12.690 | -3.218 | 32.212 | 1.00 | 96.96 | A |
| ATOM 190 | CD | ARG A 56 | -13.540 | -2.048 | 32.705 | 1.00 | 98.68 | A |
| ATOM 191 | NE | ARG A 56 | -14.968 | -2.368 | 32.803 | 1.00 | 101.63 | A |
| ATOM 192 | CZ | ARG A 56 | -15.476 | -3.458 | 33.381 | 1.00 | 101.88 | A |
| ATOM 193 | NH1 | ARG A 56 | -14.682 | -4.369 | 33.927 | 1.00 | 102.50 | A |
| ATOM 194 | NH2 | ARG A 56 | -16.790 | -3.636 | 33.421 | 1.00 | 100.90 | A |
| ATOM 195 | C | ARG A 56 | -9.227 | -3.455 | 30.572 | 1.00 | 92.68 | A |
| ATOM 196 | O | ARG A 56 | -9.123 | -4.030 | 29.490 | 1.00 | 93.70 | A |
| ATOM 197 | N | GLU A 57 | -8.439 | -2.452 | 30.941 | 1.00 | 91.14 | A |
| ATOM 198 | CA | GLU A 57 | -7.380 | -1.956 | 30.080 | 1.00 | 89.61 | A |
| ATOM 199 | CB | GLU A 57 | -6.835 | -0.644 | 30.631 | 1.00 | 90.93 | A |
| ATOM 200 | CG | GLU A 57 | -7.913 | 0.376 | 30.947 | 1.00 | 94.22 | A |
| ATOM 201 | CD | GLU A 57 | -7.384 | 1.576 | 31.711 | 1.00 | 94.94 | A |
| ATOM 202 | OE1 | GLU A 57 | -8.198 | 2.461 | 32.057 | 1.00 | 94.57 | A |
| ATOM 203 | OE2 | GLU A 57 | -6.160 | 1.635 | 31.965 | 1.00 | 94.94 | A |
| ATOM 204 | C | GLU A 57 | -6.272 | -2.991 | 30.055 | 1.00 | 88.82 | A |
| ATOM 205 | O | GLU A 57 | -6.254 | -3.885 | 29.207 | 1.00 | 89.63 | A |
| ATOM 206 | N | ALA A 58 | -5.358 | -2.871 | 31.011 | 1.00 | 87.18 | A |
| ATOM 207 | CA | ALA A 58 | -4.222 | -3.777 | 31.124 | 1.00 | 86.44 | A |
| ATOM 208 | CB | ALA A 58 | -3.286 | -3.285 | 32.226 | 1.00 | 85.99 | A |
| ATOM 209 | C | ALA A 58 | -4.621 | -5.233 | 31.389 | 1.00 | 84.70 | A |
| ATOM 210 | O | ALA A 58 | -4.558 | -5.702 | 32.525 | 1.00 | 85.72 | A |
| ATOM 211 | N | LYS A 59 | -5.030 | -5.946 | 30.343 | 1.00 | 81.60 | A |
| ATOM 212 | CA | LYS A 59 | -5.408 | -7.347 | 30.486 | 1.00 | 78.55 | A |
| ATOM 213 | CB | LYS A 59 | -6.926 | -7.496 | 30.586 | 1.00 | 77.93 | A |
| ATOM 214 | CG | LYS A 59 | -7.698 | -6.993 | 29.391 | 1.00 | 78.22 | A |
| ATOM 215 | CD | LYS A 59 | -9.189 | -6.930 | 29.701 | 1.00 | 79.39 | A |
| ATOM 216 | CE | LYS A 59 | -9.760 | -8.287 | 30.103 | 1.00 | 79.68 | A |
| ATOM 217 | NZ | LYS A 59 | -9.769 | -9.276 | 28.990 | 1.00 | 78.25 | A |
| ATOM 218 | C | LYS A 59 | -4.880 | -8.157 | 29.317 | 1.00 | 78.12 | A |
| ATOM 219 | O | LYS A 59 | -5.082 | -9.366 | 29.245 | 1.00 | 77.03 | A |
| ATOM 220 | N | ALA A 60 | -4.202 | -7.479 | 28.399 | 1.00 | 78.69 | A |
| ATOM 221 | CA | ALA A 60 | -3.617 | -8.137 | 27.239 | 1.00 | 78.11 | A |
| ATOM 222 | CB | ALA A 60 | -3.852 | -7.306 | 25.987 | 1.00 | 77.68 | A |
| ATOM 223 | C | ALA A 60 | -2.122 | -8.287 | 27.506 | 1.00 | 78.00 | A |
| ATOM 224 | O | ALA A 60 | -1.389 | -8.895 | 26.722 | 1.00 | 79.90 | A |
| ATOM 225 | N | VAL A 61 | -1.683 | -7.729 | 28.629 | 1.00 | 76.44 | A |
| ATOM 226 | CA | VAL A 61 | -0.280 | -7.787 | 29.023 | 1.00 | 74.53 | A |
| ATOM 227 | CB | VAL A 61 | 0.300 | -6.361 | 29.167 | 1.00 | 73.76 | A |
| ATOM 228 | CG1 | VAL A 61 | 0.258 | -5.657 | 27.824 | 1.00 | 74.36 | A |
| ATOM 229 | CG2 | VAL A 61 | -0.497 | -5.570 | 30.199 | 1.00 | 71.91 | A |
| ATOM 230 | C | VAL A 61 | -0.102 | -8.549 | 30.340 | 1.00 | 72.77 | A |
| ATOM 231 | O | VAL A 61 | 0.669 | -8.153 | 31.214 | 1.00 | 72.52 | A |
| ATOM 232 | N | LEU A 62 | -0.829 | -9.649 | 30.476 | 1.00 | 69.41 | A |
| ATOM 233 | CA | LEU A 62 | -0.752 | -10.454 | 31.680 | 1.00 | 64.91 | A |
| ATOM 234 | CB | LEU A 62 | -2.019 | -10.270 | 32.524 | 1.00 | 64.77 | A |
| ATOM 235 | CG | LEU A 62 | -2.228 | -8.917 | 33.220 | 1.00 | 64.55 | A |
| ATOM 236 | CD1 | LEU A 62 | -1.076 | -8.658 | 34.180 | 1.00 | 65.57 | A |
| ATOM 237 | CD2 | LEU A 62 | -2.319 | -7.792 | 32.191 | 1.00 | 65.13 | A |
| ATOM 238 | C | LEU A 62 | -0.555 | -11.925 | 31.354 | 1.00 | 61.50 | A |
| ATOM 239 | O | LEU A 62 | -1.413 | -12.566 | 30.760 | 1.00 | 61.77 | A |
| ATOM 240 | N | TYR A 63 | 0.600 | -12.446 | 31.738 | 1.00 | 57.28 | A |
| ATOM 241 | CA | TYR A 63 | 0.922 | -13.845 | 31.515 | 1.00 | 52.71 | A |
| ATOM 242 | CB | TYR A 63 | 2.378 | -14.011 | 31.031 | 1.00 | 57.16 | A |
| ATOM 243 | CG | TYR A 63 | 2.705 | -13.431 | 29.663 | 1.00 | 61.57 | A |
| ATOM 244 | CD1 | TYR A 63 | 2.613 | -12.054 | 29.423 | 1.00 | 60.81 | A |
| ATOM 245 | CE1 | TYR A 63 | 2.936 | -11.517 | 28.178 | 1.00 | 62.65 | A |
| ATOM 246 | CD2 | TYR A 63 | 3.130 | -14.262 | 28.612 | 1.00 | 61.57 | A |
| ATOM 247 | CE2 | TYR A 63 | 3.456 | -13.734 | 27.361 | 1.00 | 62.16 | A |
| ATOM 248 | CZ | TYR A 63 | 3.357 | -12.358 | 27.152 | 1.00 | 64.02 | A |
| ATOM 249 | OH | TYR A 63 | 3.688 | -11.812 | 25.927 | 1.00 | 66.92 | A |
| ATOM 250 | C | TYR A 63 | 0.782 | -14.579 | 32.845 | 1.00 | 48.05 | A |
| ATOM 251 | O | TYR A 63 | 0.553 | -13.970 | 33.895 | 1.00 | 46.41 | A |
| ATOM 252 | N | MET A 64 | 0.938 | -15.895 | 32.780 | 1.00 | 43.07 | A |
| ATOM 253 | CA | MET A 64 | 0.885 | -16.751 | 33.949 | 1.00 | 37.59 | A |
| ATOM 254 | CB | MET A 64 | 1.044 | -18.218 | 33.515 | 1.00 | 33.62 | A |
| ATOM 255 | CG | MET A 64 | 1.270 | -19.229 | 34.631 | 1.00 | 34.88 | A |
| ATOM 256 | SD | MET A 64 | 1.253 | -20.956 | 34.013 | 1.00 | 32.69 | A |
| ATOM 257 | CE | MET A 64 | -0.441 | -21.029 | 33.468 | 1.00 | 29.03 | A |
| ATOM 258 | C | MET A 64 | 2.062 | -16.304 | 34.802 | 1.00 | 37.67 | A |
| ATOM 259 | O | MET A 64 | 3.102 | -15.923 | 34.273 | 1.00 | 35.70 | A |
| ATOM 260 | N | ALA A 65 | 1.887 | -16.316 | 36.119 | 1.00 | 38.51 | A |
| ATOM 261 | CA | ALA A 65 | 2.959 | -15.928 | 37.023 | 1.00 | 36.56 | A |
| ATOM 262 | CB | ALA A 65 | 2.628 | -14.620 | 37.717 | 1.00 | 32.37 | A |
| ATOM 263 | C | ALA A 65 | 3.163 | -17.012 | 38.052 | 1.00 | 38.63 | A |
| ATOM 264 | O | ALA A 65 | 2.299 | -17.862 | 38.267 | 1.00 | 39.28 | A |
| ATOM 265 | N | LYS A 66 | 4.332 | -16.997 | 38.672 | 1.00 | 41.29 | A |
| ATOM 266 | CA | LYS A 66 | 4.644 | -17.962 | 39.709 | 1.00 | 41.75 | A |
| ATOM 267 | CB | LYS A 66 | 6.048 | -18.539 | 39.543 | 1.00 | 42.34 | A |
| ATOM 268 | CG | LYS A 66 | 6.470 | -19.366 | 40.750 | 1.00 | 45.37 | A |
| ATOM 269 | CD | LYS A 66 | 7.980 | -19.398 | 40.965 | 1.00 | 47.49 | A |
| ATOM 270 | CE | LYS A 66 | 8.674 | -20.357 | 40.013 | 1.00 | 50.27 | A |
| ATOM 271 | NZ | LYS A 66 | 10.093 | -20.595 | 40.427 | 1.00 | 49.08 | A |
| ATOM 272 | C | LYS A 66 | 4.594 | -17.174 | 40.992 | 1.00 | 43.12 | A |
| ATOM 273 | O | LYS A 66 | 5.139 | -16.072 | 41.074 | 1.00 | 42.51 | A |
| ATOM 274 | N | THR A 67 | 3.916 | -17.723 | 41.987 | 1.00 | 44.36 | A |
| ATOM 275 | CA | THR A 67 | 3.828 | -17.060 | 43.275 | 1.00 | 45.19 | A |
| ATOM 276 | CB | THR A 67 | 2.391 | -16.536 | 43.554 | 1.00 | 46.71 | A |
| ATOM 277 | OG1 | THR A 67 | 2.030 | -15.565 | 42.566 | 1.00 | 48.01 | A |
| ATOM 278 | CG2 | THR A 67 | 2.308 | -15.892 | 44.930 | 1.00 | 46.33 | A |
| ATOM 279 | C | THR A 67 | 4.205 | -18.087 | 44.336 | 1.00 | 43.89 | A |
| ATOM 280 | O | THR A 67 | 3.664 | -19.195 | 44.358 | 1.00 | 44.64 | A |
| ATOM 281 | N | VAL A 68 | 5.156 | -17.745 | 45.194 | 1.00 | 40.84 | A |
| ATOM 282 | CA | VAL A 68 | 5.525 | -18.679 | 46.237 | 1.00 | 39.30 | A |
| ATOM 283 | CB | VAL A 68 | 7.063 | -18.803 | 46.403 | 1.00 | 40.93 | A |
| ATOM 284 | CG1 | VAL A 68 | 7.383 | -19.742 | 47.585 | 1.00 | 40.53 | A |
| ATOM 285 | CG2 | VAL A 68 | 7.673 | -19.350 | 45.119 | 1.00 | 36.88 | A |
| ATOM 286 | C | VAL A 68 | 4.882 | -18.161 | 47.505 | 1.00 | 36.98 | A |
| ATOM 287 | O | VAL A 68 | 4.999 | -16.981 | 47.839 | 1.00 | 32.83 | A |
| ATOM 288 | N | VAL A 69 | 4.192 | -19.060 | 48.192 | 1.00 | 38.01 | A |
| ATOM 289 | CA | VAL A 69 | 3.475 | -18.722 | 49.401 | 1.00 | 40.63 | A |
| ATOM 290 | CB | VAL A 69 | 2.051 | -19.349 | 49.374 | 1.00 | 42.62 | A |
| ATOM 291 | CG1 | VAL A 69 | 1.302 | -19.042 | 50.668 | 1.00 | 37.81 | A |
| ATOM 292 | CG2 | VAL A 69 | 1.274 | -18.813 | 48.160 | 1.00 | 40.66 | A |
| ATOM 293 | C | VAL A 69 | 4.191 | -19.161 | 50.662 | 1.00 | 41.24 | A |
| ATOM 294 | O | VAL A 69 | 4.516 | -20.337 | 50.839 | 1.00 | 42.46 | A |
| ATOM 295 | N | ALA A 70 | 4.423 | -18.197 | 51.542 | 1.00 | 40.47 | A |
| ATOM 296 | CA | ALA A 70 | 5.079 | -18.463 | 52.808 | 1.00 | 39.50 | A |
| ATOM 297 | CB | ALA A 70 | 6.533 | -18.046 | 52.737 | 1.00 | 41.04 | A |
| ATOM 298 | C | ALA A 70 | 4.347 | -17.669 | 53.880 | 1.00 | 39.27 | A |
| ATOM 299 | O | ALA A 70 | 3.555 | -16.784 | 53.571 | 1.00 | 37.53 | A |
| ATOM 300 | N | PRO A 71 | 4.578 | -18.001 | 55.156 | 1.00 | 39.87 | A |
| ATOM 301 | CD | PRO A 71 | 5.257 | -19.227 | 55.609 | 1.00 | 40.51 | A |
| ATOM 302 | CA | PRO A 71 | 3.945 | -17.321 | 56.287 | 1.00 | 39.82 | A |
| ATOM 303 | CB | PRO A 71 | 4.468 | -18.109 | 57.482 | 1.00 | 40.61 | A |
| ATOM 304 | CG | PRO A 71 | 4.598 | -19.485 | 56.936 | 1.00 | 38.21 | A |
| ATOM 305 | C | PRO A 71 | 4.322 | -15.847 | 56.371 | 1.00 | 39.47 | A |
| ATOM 306 | O | PRO A 71 | 5.402 | -15.457 | 55.928 | 1.00 | 41.31 | A |
| ATOM 307 | N | SER A 72 | 3.437 | -15.034 | 56.940 | 1.00 | 39.29 | A |
| ATOM 308 | CA | SER A 72 | 3.703 | -13.605 | 57.099 | 1.00 | 39.85 | A |
| ATOM 309 | CB | SER A 72 | 2.528 | -12.765 | 56.610 | 1.00 | 38.06 | A |
| ATOM 310 | OG | SERA 72 | 1.574 | -12.600 | 57.645 | 1.00 | 36.83 | A |
| ATOM 311 | C | SERA 72 | 3.915 | -13.311 | 58.575 | 1.00 | 42.28 | A |
| ATOM 312 | O | SER A 72 | 3.416 | -14.032 | 59.445 | 1.00 | 43.21 | A |
| ATOM 313 | N | THR A 73 | 4.629 | -12.228 | 58.851 | 1.00 | 44.86 | A |
| ATOM 314 | CA | THR A 73 | 4.928 | -11.823 | 60.219 | 1.00 | 44.43 | A |
| ATOM 315 | CB | THR A 73 | 5.477 | -10.373 | 60.271 | 1.00 | 42.84 | A |
| ATOM 316 | OG1 | THR A 73 | 6.640 | -10.257 | 59.439 | 1.00 | 41.61 | A |
| ATOM 317 | CG2 | THR A 73 | 5.863 | -10.014 | 61.687 | 1.00 | 42.56 | A |
| ATOM 318 | C | THR A 73 | 3.748 | -11.903 | 61.179 | 1.00 | 45.50 | A |
| ATOM 319 | O | THR A 73 | 3.906 | -12.321 | 62.330 | 1.00 | 44.76 | A |
| ATOM 320 | N | GLU A 74 | 2.568 | -11.514 | 60.707 | 1.00 | 47.50 | A |
| ATOM 321 | CA | GLU A 74 | 1.379 | -11.506 | 61.553 | 1.00 | 48.36 | A |
| ATOM 322 | CB | GLU A 74 | 0.489 | -10.330 | 61.177 | 1.00 | 50.79 | A |
| ATOM 323 | CG | GLU A 74 | -0.323 | -9.789 | 62.322 | 1.00 | 59.06 | A |
| ATOM 324 | CD | GLU A 74 | 0.517 | -8.961 | 63.274 | 1.00 | 62.89 | A |
| ATOM 325 | OE1 | GLU A 74 | 0.946 | -7.860 | 62.867 | 1.00 | 63.68 | A |
| ATOM 326 | OE2 | GLU A 74 | 0.754 | -9.411 | 64.418 | 1.00 | 65.54 | A |
| ATOM 327 | C | GLU A 74 | 0.547 | -12.775 | 61.510 | 1.00 | 47.32 | A |
| ATOM 328 | O | GLU A 74 | -0.590 | -12.786 | 61.971 | 1.00 | 45.23 | A |
| ATOM 329 | N | GLY A 75 | 1.104 | -13.849 | 60.967 | 1.00 | 48.50 | A |
| ATOM 330 | CA | GLY A 75 | 0.338 | -15.076 | 60.892 | 1.00 | 51.57 | A |
| ATOM 331 | C | GLY A 75 | -0.492 | -15.094 | 59.621 | 1.00 | 52.32 | A |
| ATOM 332 | O | GLY A 75 | -1.429 | -15.884 | 59.487 | 1.00 | 55.22 | A |
| ATOM 333 | N | GLY A 76 | -0.153 | -14.202 | 58.695 | 1.00 | 48.02 | A |
| ATOM 334 | CA | GLY A 76 | -0.851 | -14.148 | 57.429 | 1.00 | 41.91 | A |
| ATOM 335 | C | GLY A 76 | -0.010 | -14.877 | 56.400 | 1.00 | 39.57 | A |
| ATOM 336 | O | GLY A 76 | 0.739 | -15.794 | 56.737 | 1.00 | 37.39 | A |
| ATOM 337 | N | LEU A 77 | -0.117 | -14.464 | 55.144 | 1.00 | 37.70 | A |
| ATOM 338 | CA | LEU A 77 | 0.647 | -15.099 | 54.085 | 1.00 | 35.31 | A |
| ATOM 339 | CB | LEU A 77 | -0.269 | -15.889 | 53.161 | 1.00 | 34.86 | A |
| ATOM 340 | CG | LEU A 77 | -1.165 | -16.945 | 53.809 | 1.00 | 37.16 | A |
| ATOM 341 | CD1 | LEU A 77 | -1.925 | -17.682 | 52.736 | 1.00 | 36.06 | A |
| ATOM 342 | CD2 | LEU A 77 | -0.326 | -17.909 | 54.607 | 1.00 | 41.20 | A |
| ATOM 343 | C | LEU A 77 | 1.425 | -14.120 | 53.245 | 1.00 | 34.86 | A |
| ATOM 344 | O | LEU A 77 | 0.892 | -13.093 | 52.831 | 1.00 | 36.48 | A |
| ATOM 345 | N | ASN A 78 | 2.697 | -14.450 | 53.013 | 1.00 | 35.83 | A |
| ATOM 346 | CA | ASN A 78 | 3.594 | -13.661 | 52.173 | 1.00 | 34.70 | A |
| ATOM 347 | CB | ASN A 78 | 5.051 | -13.833 | 52.588 | 1.00 | 35.21 | A |
| ATOM 348 | CG | ASN A 78 | 5.513 | -12.770 | 53.556 | 1.00 | 35.91 | A |
| ATOM 349 | OD1 | ASN A 78 | 4.744 | -11.876 | 53.925 | 1.00 | 37.78 | A |
| ATOM 350 | ND2 | ASN A 78 | 6.781 | -12.855 | 53.972 | 1.00 | 34.50 | A |
| ATOM 351 | C | ASN A 78 | 3.447 | -14.244 | 50.799 | 1.00 | 36.13 | A |
| ATOM 352 | O | ASN A 78 | 3.528 | -15.464 | 50.636 | 1.00 | 40.71 | A |
| ATOM 353 | N | LEU A 79 | 3.206 | -13.398 | 49.810 | 1.00 | 36.53 | A |
| ATOM 354 | CA | LEU A 79 | 3.088 | -13.890 | 48.448 | 1.00 | 40.14 | A |
| ATOM 355 | CB | LEU A 79 | 1.702 | -13.583 | 47.864 | 1.00 | 42.52 | A |
| ATOM 356 | CG | LEU A 79 | 0.678 | -14.712 | 48.099 | 1.00 | 43.47 | A |
| ATOM 357 | CD1 | LEU A 79 | 0.435 | -14.889 | 49.586 | 1.00 | 42.65 | A |
| ATOM 358 | CD2 | LEU A 79 | -0.635 | -14.403 | 47.401 | 1.00 | 42.52 | A |
| ATOM 359 | C | LEU A 79 | 4.185 | -13.240 | 47.638 | 1.00 | 40.10 | A |
| ATOM 360 | O | LEU A 79 | 4.192 | -12.023 | 47.450 | 1.00 | 42.17 | A |
| ATOM 361 | N | THR A 80 | 5.138 | -14.058 | 47.201 | 1.00 | 40.26 | A |
| ATOM 362 | CA | THR A 80 | 6.270 | -13.587 | 46.409 | 1.00 | 40.18 | A |
| ATOM 363 | CB | THR A 80 | 7.612 | -14.139 | 46.971 | 1.00 | 39.78 | A |
| ATOM 364 | OG1 | THR A 80 | 7.816 | -13.649 | 48.301 | 1.00 | 39.20 | A |
| ATOM 365 | CG2 | THR A 80 | 8.779 | -13.713 | 46.104 | 1.00 | 38.24 | A |
| ATOM 366 | C | THR A 80 | 6.069 | -14.086 | 44.990 | 1.00 | 40.51 | A |
| ATOM 367 | O | THR A 80 | 6.047 | -15.290 | 44.745 | 1.00 | 39.73 | A |
| ATOM 368 | N | SER A 81 | 5.915 | -13.165 | 44.053 | 1.00 | 42.62 | A |
| ATOM 369 | CA | SER A 81 | 5.692 | -13.569 | 42.668 | 1.00 | 47.88 | A |
| ATOM 370 | CB | SER A 81 | 4.310 | -13.107 | 42.205 | 1.00 | 47.74 | A |
| ATOM 371 | OG | SER A 81 | 3.340 | -13.307 | 43.224 | 1.00 | 50.44 | A |
| ATOM 372 | C | SER A 81 | 6.743 | -13.071 | 41.679 | 1.00 | 49.35 | A |
| ATOM 373 | O | SER A 81 | 7.263 | -11.957 | 41.794 | 1.00 | 49.68 | A |
| ATOM 374 | N | THR A 82 | 7.045 | -13.929 | 40.713 | 1.00 | 53.53 | A |
| ATOM 375 | CA | THR A 82 | 8.001 | -13.646 | 39.655 | 1.00 | 57.80 | A |
| ATOM 376 | CB | THR A 82 | 9.063 | -14.761 | 39.564 | 1.00 | 59.61 | A |
| ATOM 377 | OG1 | THR A 82 | 9.951 | -14.666 | 40.685 | 1.00 | 61.12 | A |
| ATOM 378 | CG2 | THR A 82 | 9.854 | -14.653 | 38.268 | 1.00 | 59.28 | A |
| ATOM 379 | C | THR A 82 | 7.192 | -13.603 | 38.357 | 1.00 | 59.75 | A |
| ATOM 380 | O | THR A 82 | 6.699 | -14.632 | 37.884 | 1.00 | 58.51 | A |
| ATOM 381 | N | PHE A 83 | 7.050 | -12.407 | 37.793 | 1.00 | 62.90 | A |
| ATOM 382 | CA | PHE A 83 | 6.276 | -12.235 | 36.571 | 1.00 | 65.89 | A |
| ATOM 383 | CB | PHE A 83 | 4.961 | -11.511 | 36.871 | 1.00 | 61.94 | A |
| ATOM 384 | CG | PHE A 83 | 5.135 | -10.148 | 37.483 | 1.00 | 57.31 | A |
| ATOM 385 | CD1 | PHE A 83 | 4.515 | -9.037 | 36.916 | 1.00 | 57.80 | A |
| ATOM 386 | CD2 | PHE A 83 | 5.869 | -9.979 | 38.656 | 1.00 | 56.46 | A |
| ATOM 387 | CE1 | PHE A 83 | 4.617 | -7.774 | 37.509 | 1.00 | 57.20 | A |
| ATOM 388 | CE2 | PHE A 83 | 5.979 | -8.724 | 39.257 | 1.00 | 57.43 | A |
| ATOM 389 | CZ | PHE A 83 | 5.351 | -7.618 | 38.683 | 1.00 | 58.05 | A |
| ATOM 390 | C | PHE A 83 | 6.990 | -11.496 | 35.452 | 1.00 | 70.72 | A |
| ATOM 391 | O | PHE A 83 | 8.068 | -10.935 | 35.639 | 1.00 | 71.97 | A |
| ATOM 392 | N | LEU A 84 | 6.346 | -11.501 | 34.288 | 1.00 | 75.56 | A |
| ATOM 393 | CA | LEU A 84 | 6.854 | -10.856 | 33.085 | 1.00 | 77.58 | A |
| ATOM 394 | CB | LEU A 84 | 6.588 | -11.760 | 31.879 | 1.00 | 76.28 | A |
| ATOM 395 | CG | LEU A 84 | 7.137 | -11.352 | 30.513 | 1.00 | 75.52 | A |
| ATOM 396 | CD1 | LEU A 84 | 8.635 | -11.047 | 30.622 | 1.00 | 76.74 | A |
| ATOM 397 | CD2 | LEU A 84 | 6.870 | -12.481 | 29.515 | 1.00 | 73.55 | A |
| ATOM 398 | C | LEU A 84 | 6.156 | -9.517 | 32.893 | 1.00 | 79.65 | A |
| ATOM 399 | O | LEU A 84 | 4.934 | -9.463 | 32.757 | 1.00 | 78.63 | A |
| ATOM 400 | N | ARG A 85 | 6.933 | -8.438 | 32.899 | 1.00 | 84.46 | A |
| ATOM 401 | CA | ARG A 85 | 6.378 | -7.100 | 32.714 | 1.00 | 90.39 | A |
| ATOM 402 | CB | ARC A 85 | 6.306 | -6.343 | 34.046 | 1.00 | 92.46 | A |
| ATOM 403 | CG | ARG A 85 | 5.538 | -5.030 | 33.952 | 1.00 | 95.39 | A |
| ATOM 404 | CD | ARG A 85 | 5.562 | -4.245 | 35.251 | 1.00 | 99.40 | A |
| ATOM 405 | NE | ARG A 85 | 4.834 | -2.982 | 35.124 | 1.00 | 103.44 | A |
| ATOM 406 | CZ | ARG A 85 | 4.795 | -2.037 | 36.061 | 1.00 | 105.03 | A |
| ATOM 407 | NH1 | ARG A 85 | 5.445 | -2.204 | 37.207 | 1.00 | 106.01 | A |
| ATOM 408 | NH2 | ARG A 85 | 4.105 | -0.921 | 35.852 | 1.00 | 103.52 | A |
| ATOM 409 | C | ARG A 85 | 7.237 | -6.319 | 31.726 | 1.00 | 92.84 | A |
| ATOM 410 | O | ARG A 85 | 8.449 | -6.178 | 31.912 | 1.00 | 93.32 | A |
| ATOM 411 | N | LYS A 86 | 6.600 | -5.806 | 30.676 | 1.00 | 95.15 | A |
| ATOM 412 | CA | LYS A 86 | 7.311 | -5.059 | 29.653 | 1.00 | 96.71 | A |
| ATOM 413 | CB | LYS A 86 | 7.747 | -3.693 | 30.203 | 1.00 | 97.65 | A |
| ATOM 414 | CG | LYS A 86 | 6.654 | -2.621 | 30.061 | 1.00 | 99.15 | A |
| ATOM 415 | CD | LYS A 86 | 6.720 | -1.537 | 31.131 | 1.00 | 100.81 | A |
| ATOM 416 | CE | LYS A 86 | 6.267 | -2.073 | 32.481 | 1.00 | 101.99 | A |
| ATOM 417 | NZ | LYS A 86 | 6.208 | -1.012 | 33.526 | 1.00 | 102.38 | A |
| ATOM 418 | C | LYS A 86 | 8.497 | -5.898 | 29.208 | 1.00 | 98.14 | A |
| ATOM 419 | O | LYS A 86 | 9.621 | -5.417 | 29.080 | 1.00 | 97.38 | A |
| ATOM 420 | N | ASN A 87 | 8.206 | -7.180 | 28.998 | 1.00 | 101.05 | A |
| ATOM 421 | CA | ASN A 87 | 9.169 | -8.177 | 28.547 | 1.00 | 103.17 | A |
| ATOM 422 | CB | ASN A 87 | 9.542 | -7.902 | 27.089 | 1.00 | 105.05 | A |
| ATOM 423 | CG | ASN A 87 | 8.322 | -7.843 | 26.180 | 1.00 | 107.39 | A |
| ATOM 424 | OD1 | ASN A 87 | 7.484 | -6.946 | 26.299 | 1.00 | 107.32 | A |
| ATOM 425 | ND2 | ASN A 87 | 8.213 | -8.809 | 25.273 | 1.00 | 109.34 | A |
| ATOM 426 | C | ASN A 87 | 10.425 | -8.279 | 29.402 | 1.00 | 103.13 | A |
| ATOM 427 | O | ASN A 87 | 10.526 | -7.532 | 30.397 | 1.00 | 104.41 | A |
| ATOM 428 | OT | ASN A 87 | 11.289 | -9.117 | 29.065 | 1.00 | 101.73 | A |
| ATOM 429 | C | CYS A 89 | 11.135 | -9.613 | 35.440 | 1.00 | 79.14 | A |
| ATOM 430 | O | CYS A 89 | 12.261 | -9.207 | 35.721 | 1.00 | 79.54 | A |
| ATOM 431 | CB | CYS A 89 | 11.946 | -11.311 | 33.779 | 1.00 | 82.95 | A |
| ATOM 432 | SG | CYS A 89 | 11.778 | -12.786 | 34.808 | 1.00 | 94.11 | A |
| ATOM 433 | N | CYS A 89 | 10.776 | -9.300 | 32.990 | 1.00 | 79.52 | A |
| ATOM 434 | CA | CYS A 89 | 10.857 | -10.295 | 34.094 | 1.00 | 80.27 | A |
| ATOM 435 | N | GLU A 90 | 10.100 | -9.516 | 36.273 | 1.00 | 78.18 | A |
| ATOM 436 | CA | GLU A 90 | 10.185 | -8.842 | 37.571 | 1.00 | 74.49 | A |
| ATOM 437 | CB | GLU A 90 | 9.472 | -7.495 | 37.455 | 1.00 | 75.68 | A |
| ATOM 438 | CG | GLU A 90 | 9.398 | -6.692 | 38.725 | 1.00 | 80.75 | A |
| ATOM 439 | CD | GLU A 90 | 8.770 | -5.329 | 38.509 | 1.00 | 83.66 | A |
| ATOM 440 | OE1 | GLU A 90 | 8.511 | -4.631 | 39.519 | 1.00 | 87.73 | A |
| ATOM 441 | OE2 | GLU A 90 | 8.540 | -4.957 | 37.334 | 1.00 | 80.98 | A |
| ATOM 442 | C | GLU A 90 | 9.615 | -9.632 | 38.758 | 1.00 | 70.33 | A |
| ATOM 443 | O | GLU A 90 | 8.910 | -10.621 | 38.581 | 1.00 | 69.86 | A |
| ATOM 444 | N | THR A 91 | 9.928 | -9.188 | 39.971 | 1.00 | 66.65 | A |
| ATOM 445 | CA | THR A 91 | 9.446 | -9.858 | 41.176 | 1.00 | 64.70 | A |
| ATOM 446 | CB | THR A 91 | 10.544 | -10.758 | 41.812 | 1.00 | 64.79 | A |
| ATOM 447 | OG1 | THR A 91 | 10.763 | -11.908 | 40.985 | 1.00 | 66.28 | A |
| ATOM 448 | CG2 | THR A 91 | 10.128 | -11.221 | 43.202 | 1.00 | 62.60 | A |
| ATOM 449 | C | THR A 91 | 8.969 | -8.893 | 42.243 | 1.00 | 62.74 | A |
| ATOM 450 | O | THR A 91 | 9.674 | -7.953 | 42.593 | 1.00 | 62.35 | A |
| ATOM 451 | N | LYS A 92 | 7.775 | -9.148 | 42.767 | 1.00 | 61.97 | A |
| ATOM 452 | CA | LYS A 92 | 7.203 | -8.314 | 43.820 | 1.00 | 61.35 | A |
| ATOM 453 | CB | LYS A 92 | 6.190 | -7.327 | 43.234 | 1.00 | 64.09 | A |
| ATOM 454 | CG | LYS A 92 | 6.827 | -6.002 | 42.810 | 1.00 | 67.74 | A |
| ATOM 455 | CD | LYS A 92 | 7.705 | -5.439 | 43.933 | 1.00 | 69.95 | A |
| ATOM 456 | CE | LYS A 92 | 6.954 | -5.374 | 45.268 | 1.00 | 68.61 | A |
| ATOM 457 | NZ | LYS A 92 | 7.876 | -5.187 | 46.421 | 1.00 | 69.01 | A |
| ATOM 458 | C | LYS A 92 | 6.562 | -9.113 | 44.950 | 1.00 | 59.21 | A |
| ATOM 459 | O | LYS A 92 | 6.189 | -10.274 | 44.764 | 1.00 | 58.49 | A |
| ATOM 460 | N | ILE A 93 | 6.433 | -8.477 | 46.116 | 1.00 | 57.93 | A |
| ATOM 461 | CA | ILE A 93 | 5.878 | -9.133 | 47.300 | 1.00 | 57.73 | A |
| ATOM 462 | CB | ILE A 93 | 6.929 | -9.223 | 48.459 | 1.00 | 57.31 | A |
| ATOM 463 | CG2 | ILE A 93 | 6.352 | -10.027 | 49.624 | 1.00 | 53.34 | A |
| ATOM 464 | CG1 | ILE A 93 | 8.209 | -9.921 | 47.989 | 1.00 | 60.14 | A |
| ATOM 465 | CD1 | ILE A 93 | 8.995 | -9.163 | 46.915 | 1.00 | 61.98 | A |
| ATOM 466 | C | ILE A 93 | 4.638 | -8.477 | 47.891 | 1.00 | 57.41 | A |
| ATOM 467 | O | ILE A 93 | 4.690 | -7.348 | 48.368 | 1.00 | 58.52 | A |
| ATOM 468 | N | MET A 94 | 3.526 | -9.202 | 47.872 | 1.00 | 57.18 | A |
| ATOM 469 | CA | MET A 94 | 2.275 | -8.716 | 48.446 | 1.00 | 56.08 | A |
| ATOM 470 | CB | MET A 94 | 1.094 | -9.102 | 47.552 | 1.00 | 57.29 | A |
| ATOM 471 | CG | MET A 94 | 1.178 | -8.660 | 46.094 | 1.00 | 59.72 | A |
| ATOM 472 | SD | MET A 94 | -0.043 | -9.548 | 45.028 | 1.00 | 66.73 | A |
| ATOM 473 | CE | MET A 94 | -1.634 | -9.093 | 45.788 | 1.00 | 58.01 | A |
| ATOM 474 | C | MET A 94 | 2.141 | -9.434 | 49.799 | 1.00 | 57.08 | A |
| ATOM 475 | O | MET A 94 | 2.504 | -10.613 | 49.914 | 1.00 | 58.30 | A |
| ATOM 476 | N | VAL A 95 | 1.644 | -8.738 | 50.821 | 1.00 | 53.57 | A |
| ATOM 477 | CA | VAL A 95 | 1.460 | -9.360 | 52.130 | 1.00 | 49.11 | A |
| ATOM 478 | CB | VAL A 95 | 2.092 | -8.543 | 53.298 | 1.00 | 49.18 | A |
| ATOM 479 | CG1 | VAL A 95 | 1.530 | -9.046 | 54.624 | 1.00 | 46.36 | A |
| ATOM 480 | CG2 | VAL A 95 | 3.620 | -8.677 | 53.310 | 1.00 | 46.36 | A |
| ATOM 481 | C | VAL A 95 | -0.012 | -9.502 | 52.452 | 1.00 | 48.44 | A |
| ATOM 482 | O | VAL A 95 | -0.700 | -8.509 | 52.651 | 1.00 | 50.11 | A |
| ATOM 483 | N | LEU A 96 | -0.494 | -10.737 | 52.509 | 1.00 | 47.81 | A |
| ATOM 484 | CA | LEU A 96 | -1.889 | -10.985 | 52.852 | 1.00 | 44.86 | A |
| ATOM 485 | CB | LEU A 96 | -2.347 | -12.339 | 52.306 | 1.00 | 44.26 | A |
| ATOM 486 | CG | LEU A 96 | -3.377 | -12.254 | 51.178 | 1.00 | 44.31 | A |
| ATOM 487 | CD1 | LEU A 96 | -3.072 | -11.080 | 50.245 | 1.00 | 45.94 | A |
| ATOM 488 | CD2 | LEU A 96 | -3.370 | -13.565 | 50.421 | 1.00 | 42.82 | A |
| ATOM 489 | C | LEU A 96 | -2.031 | -10.968 | 54.365 | 1.00 | 43.84 | A |
| ATOM 490 | O | LEU A 96 | -1.466 | -11.801 | 55.070 | 1.00 | 42.58 | A |
| ATOM 491 | N | GLN A 97 | -2.784 | -10.006 | 54.867 | 1.00 | 45.66 | A |
| ATOM 492 | CA | GLN A 97 | -2.986 | -9.908 | 56.298 | 1.00 | 47.39 | A |
| ATOM 493 | CB | GLN A 97 | -3.217 | -8.451 | 56.680 | 1.00 | 49.05 | A |
| ATOM 494 | CG | GLN A 97 | -2.753 | -8.111 | 58.069 | 1.00 | 51.88 | A |
| ATOM 495 | CD | GLN A 97 | -1.240 | -8.117 | 58.188 | 1.00 | 52.17 | A |
| ATOM 496 | OE1 | GLN A 97 | -0.538 | -7.474 | 57.405 | 1.00 | 52.83 | A |
| ATOM 497 | NE2 | GLN A 97 | -0.732 | -8.834 | 59.177 | 1.00 | 51.77 | A |
| ATOM 498 | C | GLN A 97 | -4.187 | -10.768 | 56.713 | 1.00 | 48.91 | A |
| ATOM 499 | O | GLN A 97 | -5.225 | -10.791 | 56.042 | 1.00 | 45.86 | A |
| ATOM 500 | N | PRO A 98 | -4.051 | -11.506 | 57.818 | 1.00 | 50.41 | A |
| ATOM 501 | CD | PRO A 98 | -2.897 | -11.599 | 58.733 | 1.00 | 53.20 | A |
| ATOM 502 | CA | PRO A 98 | -5.166 | -12.347 | 58.267 | 1.00 | 52.85 | A |
| ATOM 503 | CB | PRO A 98 | -4.566 | -13.109 | 59.456 | 1.00 | 54.22 | A |
| ATOM 504 | CG | PRO A 98 | -3.519 | -12.157 | 59.986 | 1.00 | 54.74 | A |
| ATOM 505 | C | PRO A 98 | -6.368 | -11.469 | 58.637 | 1.00 | 51.82 | A |
| ATOM 506 | O | PRO A 98 | -6.194 | -10.401 | 59.221 | 1.00 | 52.75 | A |
| ATOM 507 | N | ALA A 99 | -7.578 | -11.909 | 58.291 | 1.00 | 50.67 | A |
| ATOM 508 | CA | ALA A 99 | -8.778 | -11.123 | 58.572 | 1.00 | 49.91 | A |
| ATOM 509 | CB | ALA A 99 | -9.428 | -10.698 | 57.261 | 1.00 | 50.29 | A |
| ATOM 510 | C | ALA A 99 | -9.809 | -11.808 | 59.468 | 1.00 | 51.08 | A |
| ATOM 511 | O | ALA A 99 | -10.991 | -11.910 | 59.125 | 1.00 | 51.56 | A |
| ATOM 512 | N | GLY A 100 | -9.352 | -12.282 | 60.619 | 1.00 | 53.49 | A |
| ATOM 513 | CA | GLY A 100 | -10.245 | -12.920 | 61.569 | 1.00 | 55.78 | A |
| ATOM 514 | C | GLY A 100 | -10.778 | -14.305 | 61.260 | 1.00 | 56.87 | A |
| ATOM 515 | O | GLY A 100 | -11.526 | -14.869 | 62.068 | 1.00 | 58.84 | A |
| ATOM 516 | N | ALA A 101 | -10.418 | -14.863 | 60.111 | 1.00 | 55.01 | A |
| ATOM 517 | CA | ALA A 101 | -10.898 | -16.197 | 59.773 | 1.00 | 53.78 | A |
| ATOM 518 | CB | ALA A 101 | -12.313 | -16.105 | 59.194 | 1.00 | 51.59 | A |
| ATOM 519 | C | ALA A 101 | -9.963 | -16.917 | 58.799 | 1.00 | 52.67 | A |
| ATOM 520 | O | ALA A 101 | -9.417 | -16.302 | 57.882 | 1.00 | 53.80 | A |
| ATOM 521 | N | PRO A 102 | -9.760 | -18.231 | 58.997 | 1.00 | 50.85 | A |
| ATOM 522 | CD | PRO A 102 | -10.339 | -19.068 | 60.065 | 1.00 | 49.23 | A |
| ATOM 523 | CA | PRO A 102 | -8.887 | -19.024 | 58.121 | 1.00 | 49.90 | A |
| ATOM 524 | CB | PRO A 102 | -9.110 | -20.457 | 58.616 | 1.00 | 49.27 | A |
| ATOM 525 | CG | PRO A 102 | -9.415 | -20.273 | 60.066 | 1.00 | 47.89 | A |
| ATOM 526 | C | PRO A 102 | -9.336 | -18.841 | 56.672 | 1.00 | 48.42 | A |
| ATOM 527 | O | PRO A 102 | -10.544 | -18.874 | 56.405 | 1.00 | 49.19 | A |
| ATOM 528 | N | GLY A 103 | -8.383 | -18.649 | 55.752 | 1.00 | 45.93 | A |
| ATOM 529 | CA | GLY A 103 | -8.726 | -18.444 | 54.342 | 1.00 | 45.39 | A |
| ATOM 530 | C | GLY A 103 | -9.323 | -17.059 | 54.045 | 1.00 | 45.32 | A |
| ATOM 531 | O | GLY A 103 | -9.872 | -16.787 | 52.963 | 1.00 | 42.49 | A |
| ATOM 532 | N | HIS A 104 | -9.202 | -16.172 | 55.025 | 1.00 | 43.72 | A |
| ATOM 533 | CA | HIS A 104 | -9.727 | -14.822 | 54.907 | 1.00 | 42.49 | A |
| ATOM 534 | CB | HIS A 104 | -10.847 | -14.611 | 55.926 | 1.00 | 41.21 | A |
| ATOM 535 | CG | HIS A 104 | -12.101 | -15.334 | 55.571 | 1.00 | 40.30 | A |
| ATOM 536 | CD2 | HIS A 104 | -12.547 | -16.564 | 55.914 | 1.00 | 42.49 | A |
| ATOM 537 | ND1 | HIS A 104 | -13.028 | -14.820 | 54.690 | 1.00 | 40.22 | A |
| ATOM 538 | CE1 | HIS A 104 | -13.993 | -15.701 | 54.504 | 1.00 | 41.84 | A |
| ATOM 539 | NE2 | HIS A 104 | -13.724 | -16.770 | 55.235 | 1.00 | 45.92 | A |
| ATOM 540 | C | HIS A 104 | -8.621 | -13.831 | 55.142 | 1.00 | 41.81 | A |
| ATOM 541 | O | HIS A 104 | -8.072 | -13.746 | 56.242 | 1.00 | 41.25 | A |
| ATOM 542 | N | TYR A 105 | -8.285 | -13.083 | 54.107 | 1.00 | 41.35 | A |
| ATOM 543 | CA | TYR A 105 | -7.221 | -12.110 | 54.255 | 1.00 | 43.95 | A |
| ATOM 544 | CB | TYR A 105 | -5.999 | -12.584 | 53.487 | 1.00 | 40.86 | A |
| ATOM 545 | CG | TYR A 105 | -5.594 | -13.968 | 53.900 | 1.00 | 40.55 | A |
| ATOM 546 | CD1 | TYR A 105 | -4.701 | -14.167 | 54.953 | 1.00 | 41.09 | A |
| ATOM 547 | CE1 | TYR A 105 | -4.358 | -15.442 | 55.362 | 1.00 | 41.55 | A |
| ATOM 548 | CD2 | TYR A 105 | -6.138 | -15.089 | 53.265 | 1.00 | 38.66 | A |
| ATOM 549 | CE2 | TYR A 105 | -5.802 | - 16.371 | 53.667 | 1.00 | 38.48 | A |
| ATOM 550 | CZ | TYR A 105 | -4.914 | -16.545 | 54.716 | 1.00 | 40.67 | A |
| ATOM 551 | OH | TYR A 105 | -4.593 | -17.811 | 55.135 | 1.00 | 45.56 | A |
| ATOM 552 | C | TYR A 105 | -7.604 | -10.720 | 53.804 | 1.00 | 47.35 | A |
| ATOM 553 | O | TYR A 105 | -8.684 | -10.487 | 53.249 | 1.00 | 48.78 | A |
| ATOM 554 | N | THR A 106 | -6.684 | -9.802 | 54.048 | 1.00 | 51.30 | A |
| ATOM 555 | CA | THR A 106 | -6.857 | -8.414 | 53.683 | 1.00 | 52.89 | A |
| ATOM 556 | CB | THR A 106 | -7.216 | -7.598 | 54.931 | 1.00 | 48.76 | A |
| ATOM 557 | OG1 | THR A 106 | -8.639 | -7.407 | 54.957 | 1.00 | 46.67 | A |
| ATOM 558 | CG2 | THR A 106 | -6.499 | -6.270 | 54.948 | 1.00 | 47.39 | A |
| ATOM 559 | C | THR A 106 | -5.565 | -7.937 | 53.034 | 1.00 | 57.01 | A |
| ATOM 560 | O | THR A 106 | -4.475 | -8.326 | 53.447 | 1.00 | 57.07 | A |
| ATOM 561 | N | TYR A 107 | -5.695 | -7.106 | 52.007 | 1.00 | 63.36 | A |
| ATOM 562 | CA | TYR A 107 | -4.530 | -6.607 | 51.293 | 1.00 | 72.56 | A |
| ATOM 563 | CB | TYR A 107 | -4.409 | -7.355 | 49.968 | 1.00 | 73.77 | A |
| ATOM 564 | CG | TYR A 107 | -3.209 | -6.940 | 49.175 | 1.00 | 77.55 | A |
| ATOM 565 | CD1 | TYR A 107 | -1.930 | -7.033 | 49.723 | 1.00 | 78.79 | A |
| ATOM 566 | CE1 | TYR A 107 | -0.816 | -6.592 | 49.023 | 1.00 | 81.45 | A |
| ATOM 567 | CD2 | TYR A 107 | -3.348 | -6.400 | 47.897 | 1.00 | 79.21 | A |
| ATOM 568 | CE2 | TYR A 107 | -2.239 | -5.954 | 47.183 | 1.00 | 80.59 | A |
| ATOM 569 | CZ | TYR A 107 | -0.975 | -6.050 | 47.754 | 1.00 | 81.83 | A |
| ATOM 570 | OH | TYR A 107 | 0.128 | -5.593 | 47.070 | 1.00 | 82.92 | A |
| ATOM 571 | C | TYR A 107 | -4.531 | -5.087 | 51.049 | 1.00 | 78.59 | A |
| ATOM 572 | O | TYR A 107 | -5.565 | -4.432 | 51.172 | 1.00 | 80.57 | A |
| ATOM 573 | N | SER A 108 | -3.362 | -4.541 | 50.701 | 1.00 | 84.94 | A |
| ATOM 574 | CA | SER A 108 | -3.186 | -3.102 | 50.445 | 1.00 | 90.11 | A |
| ATOM 575 | CB | SER A 108 | -1.694 | -2.750 | 50.393 | 1.00 | 91.85 | A |
| ATOM 576 | OG | SER A 108 | -1.053 | -3.346 | 49.277 | 1.00 | 92.66 | A |
| ATOM 577 | C | SER A 108 | -3.857 | -2.620 | 49.161 | 1.00 | 94.11 | A |
| ATOM 578 | O | SER A 108 | -4.755 | -3.282 | 48.644 | 1.00 | 95.60 | A |
| ATOM 579 | N | SER A 109 | -3.413 | -1.476 | 48.637 | 1.00 | 98.37 | A |
| ATOM 580 | CA | SER A 109 | -4.018 | -0.920 | 47.424 | 1.00 | 102.82 | A |
| ATOM 581 | CB | SER A 109 | -4.804 | 0.347 | 47.772 | 1.00 | 102.05 | A |
| ATOM 582 | OG | SER A 109 | -3.931 | 1.407 | 48.119 | 1.00 | 101.78 | A |
| ATOM 583 | C | SER A 109 | -3.098 | -0.603 | 46.239 | 1.00 | 106.03 | A |
| ATOM 584 | O | SER A 109 | -3.205 | 0.471 | 45.638 | 1.00 | 107.26 | A |
| ATOM 585 | N | PRO A 110 | -2.184 | -1.523 | 45.882 | 1.00 | 108.55 | A |
| ATOM 586 | CD | PRO A 110 | -1.863 | -2.824 | 46.492 | 1.00 | 109.04 | A |
| ATOM 587 | CA | PRO A 110 | -1.297 | -1.253 | 44.747 | 1.00 | 109.83 | A |
| ATOM 588 | CB | PRO A 110 | -0.192 | -2.281 | 44.930 | 1.00 | 109.77 | A |
| ATOM 589 | CG | PRO A 110 | -0.956 | -3.452 | 45.446 | 1.00 | 109.90 | A |
| ATOM 590 | C | PRO A 110 | -2.086 | -1.467 | 43.452 | 1.00 | 110.93 | A |
| ATOM 591 | O | PRO A 110 | -3.207 | -1.985 | 43.482 | 1.00 | 110.51 | A |
| ATOM 592 | N | HIS A 111 | -1.495 | -1.087 | 42.323 | 1.00 | 111.85 | A |
| ATOM 593 | CA | HIS A 111 | -2.161 | -1.204 | 41.028 | 1.00 | 112.29 | A |
| ATOM 594 | CB | HIS A 111 | -2.707 | -2.620 | 40.798 | 1.00 | 113.17 | A |
| ATOM 595 | CG | HIS A 111 | -1.666 | -3.696 | 40.869 | 1.00 | 115.64 | A |
| ATOM 596 | CD2 | HIS A 111 | -1.106 | -4.458 | 39.901 | 1.00 | 116.28 | A |
| ATOM 597 | ND1 | HIS A 111 | -1.092 | -4.101 | 42.055 | 1.00 | 116.85 | A |
| ATOM 598 | CE1 | HIS A 111 | -0.224 | -5.067 | 41.815 | 1.00 | 117.01 | A |
| ATOM 599 | NE2 | HIS A 111 | -0.213 | -5.303 | 40.514 | 1.00 | 117.62 | A |
| ATOM 600 | C | HIS A 111 | -3.318 | -0.214 | 41.054 | 1.00 | 112.02 | A |
| ATOM 601 | O | HIS A 111 | -3.121 | 0.994 | 40.901 | 1.00 | 111.74 | A |
| ATOM 602 | N | SER A 112 | -4.524 | -0.735 | 41.257 | 1.00 | 112.13 | A |
| ATOM 603 | CA | SER A 112 | -5.720 | 0.096 | 41.332 | 1.00 | 111.51 | A |
| ATOM 604 | CB | SER A 112 | -6.390 | 0.225 | 39.958 | 1.00 | 112.93 | A |
| ATOM 605 | OG | SER A 112 | -7.103 | -0.953 | 39.618 | 1.00 | 112.49 | A |
| ATOM 606 | C | SER A 112 | -6.686 | -0.563 | 42.303 | 1.00 | 109.76 | A |
| ATOM 607 | O | SER A 112 | -7.174 | -1.668 | 42.057 | 1.00 | 109.39 | A |
| ATOM 608 | N | GLY A 113 | -6.957 | 0.108 | 43.414 | 1.00 | 107.11 | A |
| ATOM 609 | CA | GLY A 113 | -7.871 | -0.458 | 44.383 | 1.00 | 103.45 | A |
| ATOM 610 | C | GLY A 113 | -7.781 | 0.218 | 45.731 | 1.00 | 101.21 | A |
| ATOM 611 | O | GLY A 113 | -7.451 | 1.407 | 45.832 | 1.00 | 103.49 | A |
| ATOM 612 | N | SER A 114 | -8.072 | -0.550 | 46.772 | 1.00 | 95.74 | A |
| ATOM 613 | CA | SER A 114 | -8.045 | -0.042 | 48.134 | 1.00 | 89.73 | A |
| ATOM 614 | CB | SER A 114 | -9.398 | 0.595 | 48.466 | 1.00 | 92.13 | A |
| ATOM 615 | OG | SER A 114 | -10.475 | -0.257 | 48.097 | 1.00 | 94.65 | A |
| ATOM 616 | C | SER A 114 | -7.752 | -1.200 | 49.073 | 1.00 | 84.14 | A |
| ATOM 617 | O | SER A 114 | -6.641 | -1.714 | 49.107 | 1.00 | 84.52 | A |
| ATOM 618 | N | ILE A 115 | -8.750 | -1.605 | 49.843 | 1.00 | 77.31 | A |
| ATOM 619 | CA | ILE A 115 | -8.583 | -2.725 | 50.745 | 1.00 | 71.63 | A |
| ATOM 620 | CB | ILE A 115 | -9.162 | -2.436 | 52.135 | 1.00 | 71.29 | A |
| ATOM 621 | CG2 | ILE A 115 | -8.163 | -1.654 | 52.951 | 1.00 | 71.00 | A |
| ATOM 622 | CG1 | ILE A 115 | -10.488 | -1.683 | 52.001 | 1.00 | 73.17 | A |
| ATOM 623 | CD1 | ILE A 115 | -11.223 | -1.500 | 53.317 | 1.00 | 76.01 | A |
| ATOM 624 | C | ILE A 115 | -9.311 | -3.908 | 50.139 | 1.00 | 68.78 | A |
| ATOM 625 | O | ILE A 115 | -10.514 | -3.852 | 49.887 | 1.00 | 70.59 | A |
| ATOM 626 | N | HIS A 116 | -8.569 | -4.973 | 49.879 | 1.00 | 63.17 | A |
| ATOM 627 | CA | HIS A 116 | -9.157 | -6.162 | 49.308 | 1.00 | 56.48 | A |
| ATOM 628 | CB | HIS A 116 | -8.219 | -6.768 | 48.269 | 1.00 | 52.58 | A |
| ATOM 629 | CG | HIS A 116 | -8.046 | -5.922 | 47.048 | 1.00 | 52.19 | A |
| ATOM 630 | CD2 | HIS A 116 | -7.667 | -4.630 | 46.903 | 1.00 | 51.18 | A |
| ATOM 631 | ND1 | HIS A 116 | -8.252 | -6.406 | 45.772 | 1.00 | 53.39 | A |
| ATOM 632 | CE1 | HIS A 116 | -8.006 | -5.450 | 44.894 | 1.00 | 52.34 | A |
| ATOM 633 | NE2 | HIS A 116 | -7.648 | -4.362 | 45.554 | 1.00 | 52.64 | A |
| ATOM 634 | C | HIS A 116 | -9.420 | -7.166 | 50.410 | 1.00 | 54.37 | A |
| ATOM 635 | O | HIS A 116 | -8.579 | -7.379 | 51.273 | 1.00 | 56.26 | A |
| ATOM 636 | N | SER A 117 | -10.607 | -7.754 | 50.401 | 1.00 | 51.47 | A |
| ATOM 637 | CA | SER A 117 | -10.952 | -8.770 | 51.381 | 1.00 | 48.84 | A |
| ATOM 638 | CB | SER A 117 | -12.363 | -8.566 | 51.918 | 1.00 | 50.14 | A |
| ATOM 639 | OG | SER A 117 | -12.451 | -7.332 | 52.601 | 1.00 | 59.59 | A |
| ATOM 640 | C | SER A 117 | -10.882 | -10.032 | 50.563 | 1.00 | 45.66 | A |
| ATOM 641 | O | SER A 117 | -11.744 | -10.289 | 49.721 | 1.00 | 45.39 | A |
| ATOM 642 | N | VAL A 118 | -9.832 | -10.804 | 50.800 | 1.00 | 42.70 | A |
| ATOM 643 | CA | VAL A 118 | -9.604 | -12.030 | 50.055 | 1.00 | 39.83 | A |
| ATOM 644 | CB | VAL A 118 | -8.088 | -12.175 | 49.706 | 1.00 | 39.01 | A |
| ATOM 645 | CG1 | VAL A 118 | -7.839 | -13.416 | 48.830 | 1.00 | 34.52 | A |
| ATOM 646 | CG2 | VAL A 118 | -7.599 | -10.907 | 49.022 | 1.00 | 33.42 | A |
| ATOM 647 | C | VAL A 118 | -10.068 | -13.258 | 50.826 | 1.00 | 38.57 | A |
| ATOM 648 | O | VAL A 118 | -9.781 | -13.414 | 52.030 | 1.00 | 35.32 | A |
| ATOM 649 | N | SER A 119 | -10.789 | -14.124 | 50.122 | 1.00 | 36.68 | A |
| ATOM 650 | CA | SER A 119 | -11.289 | -15.359 | 50.718 | 1.00 | 36.15 | A |
| ATOM 651 | CB | SER A 119 | -12.798 | -15.274 | 51.001 | 1.00 | 33.83 | A |
| ATOM 652 | OG | SER A 119 | -13.533 | -15.202 | 49.792 | 1.00 | 29.76 | A |
| ATOM 653 | C | SER A 119 | -11.038 | -16.501 | 49.762 | 1.00 | 36.50 | A |
| ATOM 654 | O | SER A 119 | -11.099 | -16.336 | 48.533 | 1.00 | 34.43 | A |
| ATOM 655 | N | VAL A 120 | -10.760 | -17.663 | 50.340 | 1.00 | 36.83 | A |
| ATOM 656 | CA | VAL A 120 | -10.513 | -18.864 | 49.567 | 1.00 | 36.43 | A |
| ATOM 657 | CB | VAL A 120 | -9.653 | -19.861 | 50.355 | 1.00 | 33.61 | A |
| ATOM 658 | CG1 | VAL A 120 | -9.535 | -21.166 | 49.584 | 1.00 | 27.88 | A |
| ATOM 659 | CG2 | VAL A 120 | -8.270 | -19.250 | 50.629 | 1.00 | 31.31 | A |
| ATOM 660 | C | VAL A 120 | -11.854 | -19.490 | 49.274 | 1.00 | 39.98 | A |
| ATOM 661 | O | VAL A 120 | -12.484 | -20.053 | 50.170 | 1.00 | 42.93 | A |
| ATOM 662 | N | VAL A 121 | -12.304 | -19.385 | 48.027 | 1.00 | 40.55 | A |
| ATOM 663 | CA | VAL A 121 | -13.593 | -19.959 | 47.654 | 1.00 | 41.92 | A |
| ATOM 664 | CB | VAL A 121 | -13.989 | -19.551 | 46.234 | 1.00 | 38.14 | A |
| ATOM 665 | OG1 | VAL A 121 | -15.379 | -20.125 | 45.891 | 1.00 | 32.71 | A |
| ATOM 666 | CG2 | VAL A 121 | -13.961 | -18.030 | 46.120 | 1.00 | 38.48 | A |
| ATOM 667 | C | VAL A 121 | -13.563 | -21.485 | 47.725 | 1.00 | 44.84 | A |
| ATOM 668 | O | VAL A 121 | -14.395 | -22.103 | 48.386 | 1.00 | 46.44 | A |
| ATOM 669 | N | GLU A 122 | -12.593 | -22.080 | 47.034 | 1.00 | 45.48 | A |
| ATOM 670 | CA | GLU A 122 | -12.429 | -23.527 | 46.993 | 1.00 | 41.76 | A |
| ATOM 671 | CB | GLU A 122 | -13.139 | -24.092 | 45.766 | 1.00 | 39.34 | A |
| ATOM 672 | CG | GLU A 122 | -13.627 | -25.518 | 45.910 | 1.00 | 37.67 | A |
| ATOM 673 | CD | GLU A 122 | -14.569 | -25.899 | 44.789 | 1.00 | 39.19 | A |
| ATOM 674 | OE1 | GLU A 122 | -14.093 | -26.096 | 43.656 | 1.00 | 43.74 | A |
| ATOM 675 | OE2 | GLU A 122 | -15.789 | -25.984 | 45.028 | 1.00 | 39.37 | A |
| ATOM 676 | C | GLU A 122 | -10.937 | -23.749 | 46.863 | 1.00 | 41.01 | A |
| ATOM 677 | O | GLU A 122 | -10.235 | -22.884 | 46.325 | 1.00 | 40.90 | A |
| ATOM 678 | N | ALA A 123 | -10.452 | -24.890 | 47.357 | 1.00 | 39.08 | A |
| ATOM 679 | CA | ALA A 123 | -9.028 | -25.191 | 47.289 | 1.00 | 38.09 | A |
| ATOM 680 | CB | ALA A 123 | -8.300 | -24.367 | 48.308 | 1.00 | 33.08 | A |
| ATOM 681 | C | ALA A 123 | -8.635 | -26.668 | 47.456 | 1.00 | 39.43 | A |
| ATOM 682 | O | ALA A 123 | -9.014 | -27.323 | 48.420 | 1.00 | 39.20 | A |
| ATOM 683 | N | ASN A 124 | -7.864 | -27.170 | 46.495 | 1.00 | 42.59 | A |
| ATOM 684 | CA | ASN A 124 | -7.347 | -28.532 | 46.525 | 1.00 | 47.72 | A |
| ATOM 685 | CB | ASN A 124 | -7.603 | -29.251 | 45.201 | 1.00 | 50.83 | A |
| ATOM 686 | CG | ASN A 124 | -7.084 | -30.673 | 45.210 | 1.00 | 52.71 | A |
| ATOM 687 | OD1 | ASN A 124 | -6.047 | -30.957 | 45.809 | 1.00 | 54.16 | A |
| ATOM 688 | ND2 | ASN A 124 | -7.794 | -31.573 | 44.536 | 1.00 | 54.16 | A |
| ATOM 689 | C | ASN A 124 | -5.857 | -28.278 | 46.683 | 1.00 | 51.11 | A |
| ATOM 690 | O | ASN A 124 | -5.143 | -28.071 | 45.699 | 1.00 | 51.57 | A |
| ATOM 691 | N | TYR A 125 | -5.396 | -28.297 | 47.928 | 1.00 | 54.48 | A |
| ATOM 692 | CA | TYR A 125 | -4.007 | -27.993 | 48.235 | 1.00 | 54.44 | A |
| ATOM 693 | CB | TYR A 125 | -3.734 | -28.230 | 49.727 | 1.00 | 56.30 | A |
| ATOM 694 | CG | TYR A 125 | -3.279 | -29.620 | 50.088 | 1.00 | 63.82 | A |
| ATOM 695 | CD1 | TYR A 125 | -4.195 | -30.627 | 50.397 | 1.00 | 65.44 | A |
| ATOM 696 | CE1 | TYR A 125 | -3.760 | -31.906 | 50.761 | 1.00 | 68.05 | A |
| ATOM 697 | CD2 | TYR A 125 | -1.917 | -29.925 | 50.147 | 1.00 | 67.54 | A |
| ATOM 698 | CE2 | TYR A 125 | -1.472 | -31.193 | 50.504 | 1.00 | 69.05 | A |
| ATOM 699 | CZ | TYR A 125 | -2.393 | -32.179 | 50.810 | 1.00 | 69.43 | A |
| ATOM 700 | OH | TYR A 125 | -1.929 | -33.430 | 51.161 | 1.00 | 70.34 | A |
| ATOM 701 | C | TYR A 125 | -2.930 | -28.650 | 47.365 | 1.00 | 53.21 | A |
| ATOM 702 | O | TYR A 125 | -1.757 | -28.278 | 47.444 | 1.00 | 53.08 | A |
| ATOM 703 | N | ASP A 126 | -3.306 | -29.603 | 46.521 | 1.00 | 51.21 | A |
| ATOM 704 | CA | ASP A 126 | -2.309 | -30.229 | 45.654 | 1.00 | 50.82 | A |
| ATOM 705 | CB | ASP A 126 | -2.291 | -31.746 | 45.821 | 1.00 | 50.35 | A |
| ATOM 706 | CG | ASP A 126 | -1.829 | -32.174 | 47.178 | 1.00 | 51.64 | A |
| ATOM 707 | OD1 | ASP A 126 | -0.687 | -31.842 | 47.557 | 1.00 | 57.64 | A |
| ATOM 708 | OD2 | ASP A 126 | -2.613 | -32.851 | 47.865 | 1.00 | 50.91 | A |
| ATOM 709 | C | ASP A 126 | -2.616 | -29.936 | 44.204 | 1.00 | 49.67 | A |
| ATOM 710 | O | ASP A 126 | -1.801 | -30.214 | 43.321 | 1.00 | 49.83 | A |
| ATOM 711 | N | GLU A 127 | -3.789 | -29.356 | 43.969 | 1.00 | 48.57 | A |
| ATOM 712 | CA | GLU A 127 | -4.249 | -29.088 | 42.613 | 1.00 | 47.68 | A |
| ATOM 713 | CB | GLU A 127 | -5.536 | -29.886 | 42.381 | 1.00 | 49.84 | A |
| ATOM 714 | CG | GLU A 127 | -5.846 | -30.274 | 40.949 | 1.00 | 53.65 | A |
| ATOM 715 | CD | GLU A 127 | -7.135 | -31.087 | 40.846 | 1.00 | 57.80 | A |
| ATOM 716 | OE1 | GLU A 127 | -7.293 | -32.051 | 41.626 | 1.00 | 59.17 | A |
| ATOM 717 | OE2 | GLU A 127 | -7.989 | -30.767 | 39.987 | 1.00 | 60.10 | A |
| ATOM 718 | C | GLU A 127 | -4.481 | -27.619 | 42.260 | 1.00 | 45.31 | A |
| ATOM 719 | O | GLU A 127 | -3.827 | -27.077 | 41.362 | 1.00 | 44.52 | A |
| ATOM 720 | N | TYR A 128 | -5.397 | -26.970 | 42.970 | 1.00 | 41.80 | A |
| ATOM 721 | CA | TYR A 128 | -5.734 | -25.590 | 42.657 | 1.00 | 37.85 | A |
| ATOM 722 | CB | TYR A 128 | -6.814 | -25.556 | 41.583 | 1.00 | 37.33 | A |
| ATOM 723 | CG | TYR A 128 | -8.148 | -26.061 | 42.113 | 1.00 | 37.22 | A |
| ATOM 724 | CD1 | TYR A 128 | -8.439 | -27.433 | 42.131 | 1.00 | 37.72 | A |
| ATOM 725 | CE1 | TYR A 128 | -9.613 | -27.917 | 42.709 | 1.00 | 36.03 | A |
| ATOM 726 | CD2 | TYR A 128 | -9.080 | -25.177 | 42.687 | 1.00 | 36.78 | A |
| ATOM 727 | CE2 | TYR A 128 | -10.260 | -25.650 | 43.271 | 1.00 | 36.78 | A |
| ATOM 728 | CZ | TYR A 128 | -10.517 | -27.027 | 43.278 | 1.00 | 38.06 | A |
| ATOM 729 | OH | TYR A 128 | -11.661 | -27.516 | 43.867 | 1.00 | 38.71 | A |
| ATOM 730 | C | TYR A 128 | -6.283 | -24.823 | 43.842 | 1.00 | 38.21 | A |
| ATOM 731 | O | TYR A 128 | -6.538 | -25.378 | 44.917 | 1.00 | 38.98 | A |
| ATOM 732 | N | ALA A 129 | -6.513 | -23.536 | 43.608 | 1.00 | 36.38 | A |
| ATOM 733 | CA | ALA A 129 | -7.074 | -22.675 | 44.620 | 1.00 | 36.08 | A |
| ATOM 734 | CB | ALA A 129 | -5.979 | -22.118 | 45.531 | 1.00 | 33.11 | A |
| ATOM 735 | C | ALA A 129 | -7.800 | -21.555 | 43.923 | 1.00 | 38.48 | A |
| ATOM 736 | O | ALA A 129 | -7.239 | -20.857 | 43.073 | 1.00 | 41.82 | A |
| ATOM 737 | N | LEU A 130 | -9.068 | -21.402 | 44.269 | 1.00 | 39.88 | A |
| ATOM 738 | CA | LEU A 130 | -9.889 | -20.353 | 43.699 | 1.00 | 39.61 | A |
| ATOM 739 | CB | LEU A 130 | -11.242 | -20.931 | 43.276 | 1.00 | 40.60 | A |
| ATOM 740 | CG | LEU A 130 | -12.118 | -20.126 | 42.305 | 1.00 | 43.04 | A |
| ATOM 741 | CD1 | LEU A 130 | -13.577 | -20.402 | 42.644 | 1.00 | 43.37 | A |
| ATOM 742 | CD2 | LEU A 130 | -11.828 | -18.615 | 42.409 | 1.00 | 42.58 | A |
| ATOM 743 | C | LEU A 130 | -10.080 | -19.287 | 44.788 | 1.00 | 40.62 | A |
| ATOM 744 | O | LEU A 130 | -10.650 | -19.571 | 45.848 | 1.00 | 40.06 | A |
| ATOM 745 | N | LEU A 131 | -9.588 | -18.073 | 44.540 | 1.00 | 42.77 | A |
| ATOM 746 | CA | LEU A 131 | -9.729 | -16.992 | 45.518 | 1.00 | 42.89 | A |
| ATOM 747 | CB | LEU A 131 | -8.394 | -16.328 | 45.830 | 1.00 | 42.47 | A |
| ATOM 748 | CG | LEU A 131 | -7.096 | -17.120 | 45.887 | 1.00 | 46.12 | A |
| ATOM 749 | CD1 | LEU A 131 | -5.972 | -16.158 | 46.240 | 1.00 | 45.03 | A |
| ATOM 750 | CD2 | LEU A 131 | -7.207 | -18.267 | 46.896 | 1.00 | 48.77 | A |
| ATOM 751 | C | LEU A 131 | -10.648 | -15.911 | 44.995 | 1.00 | 43.71 | A |
| ATOM 752 | O | LEU A 131 | -10.784 | -15.720 | 43.777 | 1.00 | 43.25 | A |
| ATOM 753 | N | PHE A 132 | -11.274 | -15.196 | 45.922 | 1.00 | 44.52 | A |
| ATOM 754 | CA | PHE A 132 | -12.156 | -14.096 | 45.560 | 1.00 | 44.69 | A |
| ATOM 755 | CB | PHE A 132 | -13.604 | -14.419 | 45.940 | 1.00 | 47.03 | A |
| ATOM 756 | CG | PHE A 132 | -14.554 | -13.278 | 45.704 | 1.00 | 50.56 | A |
| ATOM 757 | CD1 | PHE A 132 | -14.952 | -12.942 | 44.413 | 1.00 | 50.73 | A |
| ATOM 758 | CD2 | PHE A 132 | -15.030 | -12.518 | 46.775 | 1.00 | 53.02 | A |
| ATOM 759 | CE1 | PHE A 132 | -15.808 | -11.869 | 44.181 | 1.00 | 50.28 | A |
| ATOM 760 | CE2 | PHE A 132 | -15.885 | -11.443 | 46.558 | 1.00 | 53.82 | A |
| ATOM 761 | CZ | PHE A 132 | -16.276 | -11.116 | 45.251 | 1.00 | 51.52 | A |
| ATOM 762 | C | PHE A 132 | -11.685 | -12.823 | 46.277 | 1.00 | 43.70 | A |
| ATOM 763 | O | PHE A 132 | -11.307 | -12.859 | 47.460 | 1.00 | 42.17 | A |
| ATOM 764 | N | SER A 133 | -11.704 | -11.709 | 45.549 | 1.00 | 41.97 | A |
| ATOM 765 | CA | SER A 133 | -11.283 | -10.427 | 46.089 | 1.00 | 43.63 | A |
| ATOM 766 | CB | SER A 133 | -9.939 | -10.019 | 45.500 | 1.00 | 45.00 | A |
| ATOM 767 | OG | SER A 133 | -8.966 | -11.025 | 45.678 | 1.00 | 51.07 | A |
| ATOM 768 | C | SER A 133 | -12.288 | -9.321 | 45.791 | 1.00 | 44.02 | A |
| ATOM 769 | O | SER A 133 | -12.542 | -8.972 | 44.641 | 1.00 | 43.30 | A |
| ATOM 770 | N | ARG A 134 | -12.852 | -8.756 | 46.841 | 1.00 | 46.89 | A |
| ATOM 771 | CA | ARG A 134 | -13.812 | -7.679 | 46.679 | 1.00 | 50.11 | A |
| ATOM 772 | CB | ARG A 134 | -15.161 | -8.079 | 47.299 | 1.00 | 48.79 | A |
| ATOM 773 | CG | ARG A 134 | -15.081 | -8.394 | 48.774 | 1.00 | 44.61 | A |
| ATOM 774 | CD | ARG A 134 | -16.347 | -9.035 | 49.281 | 1.00 | 45.37 | A |
| ATOM 775 | NE | ARG A 134 | -16.258 | -9.372 | 50.700 | 1.00 | 48.80 | A |
| ATOM 776 | CZ | ARG A 134 | -16.123 | -8.480 | 51.682 | 1.00 | 49.92 | A |
| ATOM 777 | NH1 | ARG A 134 | -16.060 | -7.179 | 51.412 | 1.00 | 48.02 | A |
| ATOM 778 | NH2 | ARG A 134 | -16.051 | -8.894 | 52.941 | 1.00 | 48.55 | A |
| ATOM 779 | C | ARG A 134 | -13.229 | -6.450 | 47.380 | 1.00 | 52.29 | A |
| ATOM 780 | O | ARG A 134 | -12.667 | -6.561 | 48.478 | 1.00 | 51.57 | A |
| ATOM 781 | N | GLY A 135 | -13.341 | -5.293 | 46.729 | 1.00 | 51.95 | A |
| ATOM 782 | CA | GLY A 135 | -12.825 | -4.063 | 47.305 | 1.00 | 53.96 | A |
| ATOM 783 | C | GLY A 135 | -13.709 | -2.884 | 46.942 | 1.00 | 57.08 | A |
| ATOM 784 | O | GLY A 135 | -14.756 | -3.065 | 46.324 | 1.00 | 56.19 | A |
| ATOM 785 | N | THR A 136 | -13.296 | -1.678 | 47.325 | 1.00 | 60.63 | A |
| ATOM 786 | CA | THR A 136 | -14.070 | -0.487 | 47.004 | 1.00 | 64.41 | A |
| ATOM 787 | CB | THR A 136 | -15.524 | -0.631 | 47.508 | 1.00 | 64.44 | A |
| ATOM 788 | OG1 | THR A 136 | -16.384 | 0.228 | 46.746 | 1.00 | 67.53 | A |
| ATOM 789 | CG2 | THR A 136 | -15.622 | -0.267 | 48.981 | 1.00 | 59.31 | A |
| ATOM 790 | C | THR A 136 | -13.475 | 0.793 | 47.590 | 1.00 | 66.65 | A |
| ATOM 791 | O | THR A 136 | -13.016 | 0.811 | 48.734 | 1.00 | 66.93 | A |
| ATOM 792 | N | LYS A 137 | -13.469 | 1.864 | 46.800 | 1.00 | 68.98 | A |
| ATOM 793 | CA | LYS A 137 | -12.963 | 3.141 | 47.297 | 1.00 | 71.69 | A |
| ATOM 794 | CB | LYS A 137 | -12.422 | 4.005 | 46.154 | 1.00 | 70.74 | A |
| ATOM 795 | CG | LYS A 137 | -10.967 | 3.727 | 45.804 | 1.00 | 70.35 | A |
| ATOM 796 | CD | LYS A 137 | -10.796 | 2.436 | 45.030 | 1.00 | 70.21 | A |
| ATOM 797 | CE | LYS A 137 | -11.240 | 2.603 | 43.591 | 1.00 | 71.89 | A |
| ATOM 798 | NZ | LYS A 137 | -10.407 | 3.632 | 42.890 | 1.00 | 73.45 | A |
| ATOM 799 | C | LYS A 137 | -14.147 | 3.833 | 47.965 | 1.00 | 73.03 | A |
| ATOM 800 | O | LYS A 137 | -13.991 | 4.803 | 48.723 | 1.00 | 74.00 | A |
| ATOM 801 | N | GLY A 138 | -15.329 | 3.298 | 47.675 | 1.00 | 71.01 | A |
| ATOM 802 | CA | GLY A 138 | -16.562 | 3.826 | 48.217 | 1.00 | 68.95 | A |
| ATOM 803 | C | GLY A 138 | -17.727 | 3.326 | 47.386 | 1.00 | 68.09 | A |
| ATOM 804 | O | GLY A 138 | -17.521 | 2.676 | 46.360 | 1.00 | 64.95 | A |
| ATOM 805 | N | PRO A 139 | -18.968 | 3.639 | 47.788 | 1.00 | 69.29 | A |
| ATOM 806 | CD | PRO A 139 | -19.280 | 4.704 | 48.760 | 1.00 | 69.05 | A |
| ATOM 807 | CA | PRO A 139 | -20.180 | 3.213 | 47.075 | 1.00 | 68.53 | A |
| ATOM 808 | CB | PRO A 139 | -21.285 | 3.989 | 47.787 | 1.00 | 68.70 | A |
| ATOM 809 | CG | PRO A 139 | -20.575 | 5.246 | 48.222 | 1.00 | 69.56 | A |
| ATOM 810 | C | PRO A 139 | -20.127 | 3.519 | 45.584 | 1.00 | 67.82 | A |
| ATOM 811 | O | PRO A 139 | -19.480 | 4.477 | 45.165 | 1.00 | 70.47 | A |
| ATOM 812 | N | GLY A 140 | -20.805 | 2.706 | 44.783 | 1.00 | 67.01 | A |
| ATOM 813 | CA | GLY A 140 | -20.805 | 2.928 | 43.347 | 1.00 | 69.03 | A |
| ATOM 814 | C | GLY A 140 | -19.445 | 2.699 | 42.710 | 1.00 | 70.29 | A |
| ATOM 815 | O | GLY A 140 | -19.263 | 2.910 | 41.506 | 1.00 | 67.84 | A |
| ATOM 816 | N | GLN A 141 | -18.487 | 2.269 | 43.528 | 1.00 | 72.03 | A |
| ATOM 817 | CA | GLN A 141 | -17.129 | 1.996 | 43.069 | 1.00 | 73.21 | A |
| ATOM 818 | CB | GLN A 141 | -16.141 | 2.874 | 43.839 | 1.00 | 70.86 | A |
| ATOM 819 | CG | GLN A 141 | -16.382 | 4.352 | 43.607 | 1.00 | 69.25 | A |
| ATOM 820 | CD | GLN A 141 | -15.226 | 5.209 | 44.056 | 1.00 | 69.17 | A |
| ATOM 821 | OE1 | GLN A 141 | -14.951 | 5.327 | 45.249 | 1.00 | 69.67 | A |
| ATOM 822 | NE2 | GLN A 141 | -14.531 | 5.807 | 43.099 | 1.00 | 69.51 | A |
| ATOM 823 | C | GLN A 141 | -16.793 | 0.512 | 43.252 | 1.00 | 74.14 | A |
| ATOM 824 | O | GLN A 141 | -15.897 | -0.033 | 42.597 | 1.00 | 73.63 | A |
| ATOM 825 | N | ASN A 142 | -17.532 | -0.125 | 44.153 | 1.00 | 74.41 | A |
| ATOM 826 | CA | ASN A 142 | -17.387 | -1.541 | 44.458 | 1.00 | 74.91 | A |
| ATOM 827 | CB | ASN A 142 | -18.714 | -2.043 | 45.033 | 1.00 | 77.40 | A |
| ATOM 828 | CG | ASN A 142 | -18.766 | -3.549 | 45.163 | 1.00 | 81.04 | A |
| ATOM 829 | OD1 | ASN A 142 | -19.839 | -4.152 | 45.072 | 1.00 | 82.62 | A |
| ATOM 830 | ND2 | ASN A 142 | -17.609 | -4.168 | 45.393 | 1.00 | 81.59 | A |
| ATOM 831 | C | ASN A 142 | -16.991 | -2.395 | 43.239 | 1.00 | 74.86 | A |
| ATOM 832 | O | ASN A 142 | -17.623 | -2.322 | 42.182 | 1.00 | 73.84 | A |
| ATOM 833 | N | PHE A 143 | -15.947 | -3.206 | 43.395 | 1.00 | 75.81 | A |
| ATOM 834 | CA | PHE A 143 | -15.496 | -4.084 | 42.317 | 1.00 | 76.18 | A |
| ATOM 835 | CB | PHE A 143 | -14.329 | -3.446 | 41.568 | 1.00 | 76.28 | A |
| ATOM 836 | CG | PHE A 143 | -13.055 | -3.422 | 42.345 | 1.00 | 75.95 | A |
| ATOM 837 | CD1 | PHE A 143 | -12.237 | -4.546 | 42.396 | 1.00 | 74.80 | A |
| ATOM 838 | CD2 | PHE A 143 | -12.669 | -2.274 | 43.033 | 1.00 | 77.92 | A |
| ATOM 839 | CE1 | PHE A 143 | -11.047 | -4.528 | 43.122 | 1.00 | 77.10 | A |
| ATOM 840 | CE2 | PHE A 143 | -11.480 | -2.241 | 43.765 | 1.00 | 78.61 | A |
| ATOM 841 | CZ | PHE A 143 | -10.667 | -3.371 | 43.808 | 1.00 | 79.19 | A |
| ATOM 842 | C | PHE A 143 | -15.092 | -5.476 | 42.815 | 1.00 | 75.21 | A |
| ATOM 843 | O | PHE A 143 | -14.874 | -5.695 | 44.008 | 1.00 | 74.84 | A |
| ATOM 844 | N | ARG A 144 | -14.981 | -6.407 | 41.876 | 1.00 | 74.55 | A |
| ATOM 845 | CA | ARG A 144 | -14.629 | -7.790 | 42.184 | 1.00 | 73.79 | A |
| ATOM 846 | CB | ARG A 144 | -15.827 | -8.702 | 41.880 | 1.00 | 76.82 | A |
| ATOM 847 | CG | ARG A 144 | -17.108 | -8.334 | 42.626 | 1.00 | 82.57 | A |
| ATOM 848 | CD | ARG A 144 | -18.340 | -8.494 | 41.732 | 1.00 | 88.12 | A |
| ATOM 849 | NE | ARG A 144 | -19.588 | -8.230 | 42.457 | 1.00 | 92.89 | A |
| ATOM 850 | CZ | ARG A 144 | -20.775 | -8.046 | 41.877 | 1.00 | 92.42 | A |
| ATOM 851 | NH1 | ARG A 144 | -20.888 | -8.091 | 40.554 | 1.00 | 89.93 | A |
| ATOM 852 | NH2 | ARG A 144 | -21.852 | -7.822 | 42.624 | 1.00 | 90.40 | A |
| ATOM 853 | C | ARG A 144 | -13.411 | -8.289 | 41.399 | 1.00 | 70.45 | A |
| ATOM 854 | O | ARG A 144 | -12.908 | -7.618 | 40.493 | 1.00 | 71.36 | A |
| ATOM 855 | N | MET A 145 | -12.937 | -9.475 | 41.768 | 1.00 | 66.18 | A |
| ATOM 856 | CA | MET A 145 | -11.809 | -10.100 | 41.097 | 1.00 | 60.85 | A |
| ATOM 857 | CB | MET A 145 | -10.519 | -9.310 | 41.315 | 1.00 | 63.62 | A |
| ATOM 858 | CG | MET A 145 | -9.309 | -9.999 | 40.685 | 1.00 | 66.34 | A |
| ATOM 859 | SD | MET A 145 | -7.851 | -8.958 | 40.573 | 1.00 | 70.82 | A |
| ATOM 860 | CE | MET A 145 | -7.924 | -8.129 | 42.194 | 1.00 | 67.76 | A |
| ATOM 861 | C | MET A 145 | -11.607 | -11.529 | 41.564 | 1.00 | 56.57 | A |
| ATOM 862 | O | MET A 145 | -11.229 | -11.780 | 42.706 | 1.00 | 57.01 | A |
| ATOM 863 | N | ALA A 146 | -11.880 | -12.467 | 40.668 | 1.00 | 51.49 | A |
| ATOM 864 | CA | ALA A 146 | -11.706 | -13.876 | 40.968 | 1.00 | 49.13 | A |
| ATOM 865 | CB | ALA A 146 | -12.766 | -14.696 | 40.260 | 1.00 | 42.05 | A |
| ATOM 866 | C | ALA A 146 | -10.321 | -14.252 | 40.462 | 1.00 | 48.78 | A |
| ATOM 867 | O | ALA A 146 | -9.869 | -13.732 | 39.439 | 1.00 | 51.44 | A |
| ATOM 868 | N | THR A 147 | -9.637 | -15.136 | 41.174 | 1.00 | 46.49 | A |
| ATOM 869 | CA | THR A 147 | -8.318 | -15.555 | 40.738 | 1.00 | 44.25 | A |
| ATOM 870 | CB | THR A 147 | -7.225 | -14.925 | 41.581 | 1.00 | 43.85 | A |
| ATOM 871 | OG1 | THR A 147 | -7.387 | -13.507 | 41.573 | 1.00 | 49.82 | A |
| ATOM 872 | CG2 | THR A 147 | -5.864 | -15.269 | 41.020 | 1.00 | 42.42 | A |
| ATOM 873 | C | THR A 147 | -8.201 | -17.061 | 40.838 | 1.00 | 44.03 | A |
| ATOM 874 | O | THR A 147 | -8.708 | -17.668 | 41.777 | 1.00 | 46.81 | A |
| ATOM 875 | N | LEU A 148 | -7.534 | -17.668 | 39.865 | 1.00 | 43.69 | A |
| ATOM 876 | CA | LEU A 148 | -7.374 | -19.111 | 39.880 | 1.00 | 42.50 | A |
| ATOM 877 | CB | LEU A 148 | -8.032 | -19.731 | 38.648 | 1.00 | 40.72 | A |
| ATOM 878 | CG | LEU A 148 | -7.738 | -21.228 | 38.472 | 1.00 | 38.40 | A |
| ATOM 879 | CD1 | LEU A 148 | -8.257 | -22.027 | 39.662 | 1.00 | 33.14 | A |
| ATOM 880 | CD2 | LEU A 148 | -8.383 | -21.700 | 37.174 | 1.00 | 40.34 | A |
| ATOM 881 | C | LEU A 148 | -5.917 | -19.529 | 39.932 | 1.00 | 43.54 | A |
| ATOM 882 | O | LEU A 148 | -5.172 | -19.348 | 38.964 | 1.00 | 41.99 | A |
| ATOM 883 | N | TYR A 149 | -5.505 | -20.089 | 41.063 | 1.00 | 43.29 | A |
| ATOM 884 | CA | TYR A 149 | -4.127 | -20.529 | 41.187 | 1.00 | 44.40 | A |
| ATOM 885 | CB | TYR A 149 | -3.579 | -20.188 | 42.557 | 1.00 | 45.21 | A |
| ATOM 886 | CG | TYR A 149 | -3.203 | -18.731 | 42.702 | 1.00 | 50.01 | A |
| ATOM 887 | CD1 | TYR A 149 | -4.145 | -17.778 | 43.094 | 1.00 | 54.12 | A |
| ATOM 888 | CE1 | TYR A 149 | -3.784 | -16.432 | 43.272 | 1.00 | 58.48 | A |
| ATOM 889 | CD2 | TYR A 149 | -1.890 | -18.312 | 42.482 | 1.00 | 51.47 | A |
| ATOM 890 | CE2 | TYR A 149 | -1.516 | -16.982 | 42.653 | 1.00 | 55.89 | A |
| ATOM 891 | CZ | TYR A 149 | -2.463 | -16.045 | 43.051 | 1.00 | 58.78 | A |
| ATOM 892 | OH | TYR A 149 | -2.078 | -14.739 | 43.238 | 1.00 | 60.02 | A |
| ATOM 893 | C | TYR A 149 | -4.003 | -22.025 | 40.933 | 1.00 | 44.94 | A |
| ATOM 894 | O | TYR A 149 | -4.868 | -22.804 | 41.342 | 1.00 | 44.18 | A |
| ATOM 895 | N | SER A 150 | -2.928 | -22.410 | 40.240 | 1.00 | 44.38 | A |
| ATOM 896 | CA | SER A 150 | -2.668 | -23.808 | 39.914 | 1.00 | 41.89 | A |
| ATOM 897 | CB | SER A 150 | -2.740 | -24.004 | 38.393 | 1.00 | 40.38 | A |
| ATOM 898 | OG | SER A 150 | -2.579 | -25.371 | 38.026 | 1.00 | 40.76 | A |
| ATOM 899 | C | SER A 150 | -1.297 | -24.271 | 40.428 | 1.00 | 40.25 | A |
| ATOM 900 | O | SER A 150 | -0.342 | -23.479 | 40.500 | 1.00 | 39.43 | A |
| ATOM 901 | N | ARG A 151 | -1.202 | -25.547 | 40.803 | 1.00 | 36.60 | A |
| ATOM 902 | CA | ARG A 151 | 0.074 | -26.082 | 41.259 | 1.00 | 34.90 | A |
| ATOM 903 | CB | ARG A 151 | -0.117 | -27.347 | 42.104 | 1.00 | 28.21 | A |
| ATOM 904 | CG | ARG A 151 | -0.656 | -27.122 | 43.514 | 1.00 | 25.36 | A |
| ATOM 905 | CD | ARG A 151 | 0.340 | -26.356 | 44.388 | 1.00 | 23.83 | A |
| ATOM 906 | NE | ARG A 151 | -0.013 | -26.412 | 45.807 | 1.00 | 24.06 | A |
| ATOM 907 | CZ | ARG A 151 | 0.683 | -25.813 | 46.770 | 1.00 | 23.10 | A |
| ATOM 908 | NH1 | ARG A 151 | 1.761 | -25.100 | 46.465 | 1.00 | 21.98 | A |
| ATOM 909 | NH2 | ARG A 151 | 0.328 | -25.958 | 48.046 | 1.00 | 24.51 | A |
| ATOM 910 | C | ARG A 151 | 0.890 | -26.416 | 40.015 | 1.00 | 35.99 | A |
| ATOM 911 | O | ARG A 151 | 2.095 | -26.617 | 40.071 | 1.00 | 38.57 | A |
| ATOM 912 | N | THR A 152 | 0.222 | -26.458 | 38.879 | 1.00 | 38.62 | A |
| ATOM 913 | CA | THR A 152 | 0.893 | -26.782 | 37.631 | 1.00 | 41.72 | A |
| ATOM 914 | CB | THR A 152 | 0.314 | -28.060 | 37.036 | 1.00 | 41.39 | A |
| ATOM 915 | OG1 | THR A 152 | -1.108 | -27.927 | 36.952 | 1.00 | 41.87 | A |
| ATOM 916 | CG2 | THR A 152 | 0.650 | -29.253 | 37.911 | 1.00 | 38.85 | A |
| ATOM 917 | C | THR A 152 | 0.659 | -25.647 | 36.664 | 1.00 | 45.58 | A |
| ATOM 918 | O | THR A 152 | -0.238 | -24.831 | 36.878 | 1.00 | 51.32 | A |
| ATOM 919 | N | GLN A 153 | 1.457 | -25.573 | 35.608 | 1.00 | 46.22 | A |
| ATOM 920 | CA | GLN A 153 | 1.273 | -24.502 | 34.637 | 1.00 | 47.59 | A |
| ATOM 921 | CB | GLN A 153 | 2.577 | -24.241 | 33.902 | 1.00 | 43.91 | A |
| ATOM 922 | CG | GLN A 153 | 3.668 | -23.760 | 34.825 | 1.00 | 45.50 | A |
| ATOM 923 | CD | GLN A 153 | 5.026 | -23.783 | 34.173 | 1.00 | 43.12 | A |
| ATOM 924 | OE1 | GLN A 153 | 5.196 | -23.280 | 33.071 | 1.00 | 44.43 | A |
| ATOM 925 | NE2 | GLN A 153 | 6.007 | -24.362 | 34.854 | 1.00 | 46.67 | A |
| ATOM 926 | C | GLN A 153 | 0.170 | -24.889 | 33.666 | 1.00 | 52.48 | A |
| ATOM 927 | O | GLN A 153 | -0.458 | -24.033 | 33.047 | 1.00 | 54.29 | A |
| ATOM 928 | N | THR A 154 | -0.068 | -26.190 | 33.541 | 1.00 | 57.63 | A |
| ATOM 929 | CA | THR A 154 | -1.118 | -26.686 | 32.667 | 1.00 | 61.55 | A |
| ATOM 930 | CB | THR A 154 | -0.829 | -28.128 | 32.220 | 1.00 | 62.65 | A |
| ATOM 931 | OG1 | THR A 154 | -1.977 | -28.654 | 31.540 | 1.00 | 64.17 | A |
| ATOM 932 | CG2 | THR A 154 | -0.491 | -29.003 | 33.426 | 1.00 | 60.73 | A |
| ATOM 933 | C | THR A 154 | -2.411 | -26.652 | 33.469 | 1.00 | 64.71 | A |
| ATOM 934 | O | THR A 154 | -2.415 | -26.973 | 34.657 | 1.00 | 65.83 | A |
| ATOM 935 | N | LEU A 155 | -3.509 | -26.263 | 32.831 | 1.00 | 67.39 | A |
| ATOM 936 | CA | LEU A 155 | -4.787 | -26.179 | 33.535 | 1.00 | 70.46 | A |
| ATOM 937 | CB | LEU A 155 | -5.283 | -24.728 | 33.518 | 1.00 | 68.52 | A |
| ATOM 938 | CG | LEU A 155 | -6.259 | -24.291 | 34.616 | 1.00 | 65.62 | A |
| ATOM 939 | CD1 | LEU A 155 | -5.572 | -24.339 | 35.968 | 1.00 | 64.23 | A |
| ATOM 940 | CD2 | LEU A 155 | -6.739 | -22.881 | 34.332 | 1.00 | 63.47 | A |
| ATOM 941 | C | LEU A 155 | -5.866 | -27.113 | 32.970 | 1.00 | 72.41 | A |
| ATOM 942 | O | LEU A 155 | -6.189 | -27.069 | 31.782 | 1.00 | 74.08 | A |
| ATOM 943 | N | LYS A 156 | -6.423 | -27.950 | 33.843 | 1.00 | 72.81 | A |
| ATOM 944 | CA | LYS A 156 | -7.457 | -28.917 | 33.482 | 1.00 | 71.61 | A |
| ATOM 945 | CB | LYS A 156 | -7.656 | -29.861 | 34.660 | 1.00 | 72.76 | A |
| ATOM 946 | CG | LYS A 156 | -8.563 | -31.037 | 34.411 | 1.00 | 75.31 | A |
| ATOM 947 | CD | LYS A 156 | -8.518 | -31.997 | 35.609 | 1.00 | 77.01 | A |
| ATOM 948 | CE | LYS A 156 | -7.131 | -32.624 | 35.781 | 1.00 | 77.35 | A |
| ATOM 949 | NZ | LYS A 156 | -7.020 | -33.484 | 36.993 | 1.00 | 77.04 | A |
| ATOM 950 | C | LYS A 156 | -8.771 | -28.219 | 33.125 | 1.00 | 71.35 | A |
| ATOM 951 | O | LYS A 156 | -9.241 | -27.357 | 33.865 | 1.00 | 72.68 | A |
| ATOM 952 | N | ASP A 157 | -9.368 | -28.597 | 31.997 | 1.00 | 70.30 | A |
| ATOM 953 | CA | ASP A 157 | -10.617 | -27.981 | 31.539 | 1.00 | 68.59 | A |
| ATOM 954 | CB | ASP A 157 | -11.173 | -28.711 | 30.308 | 1.00 | 68.09 | A |
| ATOM 955 | CG | ASP A 157 | -10.446 | -28.338 | 29.028 | 1.00 | 68.81 | A |
| ATOM 956 | OD1 | ASP A 157 | -11.012 | -28.564 | 27.936 | 1.00 | 68.86 | A |
| ATOM 957 | OD2 | ASP A 157 | -9.307 | -27.824 | 29.109 | 1.00 | 68.64 | A |
| ATOM 958 | C | ASP A 157 | -11.723 | -27.860 | 32.577 | 1.00 | 68.17 | A |
| ATOM 959 | O | ASP A 157 | -12.291 | -26.779 | 32.751 | 1.00 | 66.02 | A |
| ATOM 960 | N | GLU A 158 | -12.046 | -28.957 | 33.256 | 1.00 | 68.99 | A |
| ATOM 961 | CA | GLU A 158 | -13.105 | -28.904 | 34.261 | 1.00 | 70.32 | A |
| ATOM 962 | CB | GLU A 158 | -13.251 | -30.246 | 34.976 | 1.00 | 72.37 | A |
| ATOM 963 | CG | GLU A 158 | -11.947 | -30.899 | 35.372 | 1.00 | 78.69 | A |
| ATOM 964 | CD | GLU A 158 | -12.033 | -32.419 | 35.311 | 1.00 | 82.57 | A |
| ATOM 965 | OE1 | GLU A 158 | -12.844 | -33.011 | 36.061 | 1.00 | 81.84 | A |
| ATOM 966 | OE2 | GLU A 158 | -11.293 | -33.022 | 34.502 | 1.00 | 84.84 | A |
| ATOM 967 | C | GLU A 158 | -12.796 | -27.807 | 35.254 | 1.00 | 69.10 | A |
| ATOM 968 | O | GLU A 158 | -13.694 | -27.178 | 35.804 | 1.00 | 67.41 | A |
| ATOM 969 | N | LEU A 159 | -11.512 | -27.569 | 35.466 | 1.00 | 70.39 | A |
| ATOM 970 | CA | LEU A 159 | -11.096 | -26.521 | 36.375 | 1.00 | 70.62 | A |
| ATOM 971 | CB | LEU A 159 | -9.608 | -26.667 | 36.705 | 1.00 | 72.90 | A |
| ATOM 972 | CG | LEU A 159 | -9.266 | -27.035 | 38.152 | 1.00 | 71.29 | A |
| ATOM 973 | CD1 | LEU A 159 | -10.266 | -28.043 | 38.713 | 1.00 | 73.85 | A |
| ATOM 974 | CD2 | LEU A 159 | -7.858 | -27.592 | 38.186 | 1.00 | 72.04 | A |
| ATOM 975 | C | LEU A 159 | -11.369 | -25.177 | 35.716 | 1.00 | 68.60 | A |
| ATOM 976 | O | LEU A 159 | -11.785 | -24.224 | 36.375 | 1.00 | 68.52 | A |
| ATOM 977 | N | LYS A 160 | -11.140 | -25.097 | 34.411 | 1.00 | 66.56 | A |
| ATOM 978 | CA | LYS A 160 | -11.395 | -23.849 | 33.714 | 1.00 | 66.04 | A |
| ATOM 979 | CB | LYS A 160 | -10.941 | -23.931 | 32.254 | 1.00 | 66.84 | A |
| ATOM 980 | CG | LYS A 160 | -9.431 | -23.947 | 32.083 | 1.00 | 68.12 | A |
| ATOM 981 | CD | LYS A 160 | -9.007 | -23.675 | 30.648 | 1.00 | 67.76 | A |
| ATOM 982 | CE | LYS A 160 | -9.354 | -24.828 | 29.727 | 1.00 | 68.38 | A |
| ATOM 983 | NZ | LYS A 160 | -8.843 | -24.581 | 28.352 | 1.00 | 69.27 | A |
| ATOM 984 | C | LYS A 160 | -12.882 | -23.572 | 33.775 | 1.00 | 65.61 | A |
| ATOM 985 | O | LYS A 160 | -13.309 | -22.454 | 34.072 | 1.00 | 64.28 | A |
| ATOM 986 | N | GLU A 161 | -13.667 | -24.609 | 33.509 | 1.00 | 66.07 | A |
| ATOM 987 | CA | GLU A 161 | -15.119 | -24.495 | 33.524 | 1.00 | 67.41 | A |
| ATOM 988 | CB | GLU A 161 | -15.732 | -25.852 | 33.162 | 1.00 | 72.11 | A |
| ATOM 989 | CG | GLU A 161 | -17.236 | -25.860 | 32.915 | 1.00 | 78.85 | A |
| ATOM 990 | CD | GLU A 161 | -18.038 | -26.299 | 34.135 | 1.00 | 85.16 | A |
| ATOM 991 | OE1 | GLU A 161 | -17.583 | -27.226 | 34.850 | 1.00 | 86.78 | A |
| ATOM 992 | OE2 | GLU A 161 | -19.130 | -25.729 | 34.366 | 1.00 | 85.92 | A |
| ATOM 993 | C | GLU A 161 | -15.574 | -24.036 | 34.907 | 1.00 | 64.62 | A |
| ATOM 994 | O | GLU A 161 | -16.447 | -23.171 | 35.041 | 1.00 | 62.40 | A |
| ATOM 995 | N | LYS A 162 | -14.959 | -24.607 | 35.936 | 1.00 | 61.68 | A |
| ATOM 996 | CA | LYS A 162 | -15.299 | -24.255 | 37.308 | 1.00 | 58.49 | A |
| ATOM 997 | CB | LYS A 162 | -14.516 | -25.118 | 38.301 | 1.00 | 57.08 | A |
| ATOM 998 | CG | LYS A 162 | -14.909 | -24.877 | 39.748 | 1.00 | 52.74 | A |
| ATOM 999 | CD | LYS A 162 | -13.720 | -25.005 | 40.695 | 1.00 | 52.91 | A |
| ATOM 1000 | CE | LYS A 162 | -13.062 | -26.378 | 40.620 | 1.00 | 54.40 | A |
| ATOM 1001 | NZ | LYS A 162 | -13.972 | -27.464 | 41.079 | 1.00 | 58.06 | A |
| ATOM 1002 | C | LYS A 162 | -15.001 | -22.790 | 37.582 | 1.00 | 56.70 | A |
| ATOM 1003 | O | LYS A 162 | -15.753 | -22.135 | 38.292 | 1.00 | 57.73 | A |
| ATOM 1004 | N | PHE A 163 | -13.898 | -22.285 | 37.028 | 1.00 | 56.10 | A |
| ATOM 1005 | CA | PHE A 163 | -13.505 | -20.885 | 37.218 | 1.00 | 54.44 | A |
| ATOM 1006 | CB | PHE A 163 | -12.088 | -20.638 | 36.667 | 1.00 | 51.03 | A |
| ATOM 1007 | CG | PHE A 163 | -11.620 | -19.204 | 36.781 | 1.00 | 44.03 | A |
| ATOM 1008 | CD1 | PHE A 163 | -11.203 | -18.504 | 35.660 | 1.00 | 43.43 | A |
| ATOM 1009 | CD2 | PHE A 163 | -11.575 | -18.563 | 38.011 | 1.00 | 41.07 | A |
| ATOM 1010 | CE1 | PHE A 163 | -10.743 | -17.177 | 35.765 | 1.00 | 40.22 | A |
| ATOM 1011 | CE2 | PHE A 163 | -11.115 | -17.235 | 38.117 | 1.00 | 38.32 | A |
| ATOM 1012 | CZ | PHE A 163 | -10.702 | -16.552 | 36.991 | 1.00 | 34.24 | A |
| ATOM 1013 | C | PHE A 163 | -14.494 | -19.998 | 36.482 | 1.00 | 56.29 | A |
| ATOM 1014 | O | PHE A 163 | -14.910 | -18.958 | 36.986 | 1.00 | 55.86 | A |
| ATOM 1015 | N | THR A 164 | -14.864 | -20.429 | 35.281 | 1.00 | 57.68 | A |
| ATOM 1016 | CA | THR A 164 | -15.807 | -19.694 | 34.465 | 1.00 | 59.18 | A |
| ATOM 1017 | CB | THR A 164 | -16.103 | -20.449 | 33.167 | 1.00 | 62.54 | A |
| ATOM 1018 | OG1 | THR A 164 | -14.887 | -20.598 | 32.418 | 1.00 | 66.72 | A |
| ATOM 1019 | CG2 | THR A 164 | -17.137 | -19.691 | 32.325 | 1.00 | 64.98 | A |
| ATOM 1020 | C | THR A 164 | -17.102 | -19.525 | 35.228 | 1.00 | 58.45 | A |
| ATOM 1021 | O | THR A 164 | -17.550 | -18.408 | 35.484 | 1.00 | 58.64 | A |
| ATOM 1022 | N | THR A 165 | -17.697 | -20.650 | 35.590 | 1.00 | 57.76 | A |
| ATOM 1023 | CA | THR A 165 | -18.949 | -20.653 | 36.326 | 1.00 | 57.32 | A |
| ATOM 1024 | CB | THR A 165 | -19.300 | -22.068 | 36.809 | 1.00 | 59.13 | A |
| ATOM 1025 | OG1 | THR A 165 | -19.423 | -22.945 | 35.681 | 1.00 | 60.25 | A |
| ATOM 1026 | CG2 | THR A 165 | -20.600 | -22.058 | 37.575 | 1.00 | 59.06 | A |
| ATOM 1027 | C | THR A 165 | -18.882 | -19.735 | 37.533 | 1.00 | 55.51 | A |
| ATOM 1028 | O | THR A 165 | -19.756 | -18.884 | 37.722 | 1.00 | 53.88 | A |
| ATOM 1029 | N | PHE A 166 | -17.852 | -19.899 | 38.357 | 1.00 | 55.03 | A |
| ATOM 1030 | CA | PHE A 166 | -17.748 | -19.052 | 39.529 | 1.00 | 56.67 | A |
| ATOM 1031 | CB | PHE A 166 | -16.520 | -19.373 | 40.372 | 1.00 | 55.47 | A |
| ATOM 1032 | CG | PHE A 166 | -16.287 | -18.365 | 41.467 | 1.00 | 55.56 | A |
| ATOM 1033 | CD1 | PHE A 166 | -17.173 | -18.270 | 42.548 | 1.00 | 55.01 | A |
| ATOM 1034 | CD2 | PHE A 166 | -15.252 | -17.435 | 41.369 | 1.00 | 52.09 | A |
| ATOM 1035 | CE1 | PHE A 166 | -17.036 | -17.250 | 43.522 | 1.00 | 49.17 | A |
| ATOM 1036 | CE2 | PHE A 166 | -15.108 | -16.416 | 42.331 | 1.00 | 50.06 | A |
| ATOM 1037 | CZ | PHE A 166 | -16.007 | -16.326 | 43.408 | 1.00 | 48.33 | A |
| ATOM 1038 | C | PHE A 166 | -17.664 | -17.600 | 39.112 | 1.00 | 58.10 | A |
| ATOM 1039 | O | PHE A 166 | -18.306 | -16.739 | 39.709 | 1.00 | 58.87 | A |
| ATOM 1040 | N | SER A 167 | -16.856 | -17.347 | 38.089 | 1.00 | 59.52 | A |
| ATOM 1041 | CA | SER A 167 | -16.647 | -16.007 | 37.561 | 1.00 | 61.95 | A |
| ATOM 1042 | CB | SER A 167 | -15.751 | -16.070 | 36.320 | 1.00 | 64.01 | A |
| ATOM 1043 | OG | SER A 167 | -14.496 | -16.670 | 36.603 | 1.00 | 68.65 | A |
| ATOM 1044 | C | SER A 167 | -17.969 | -15.342 | 37.195 | 1.00 | 62.37 | A |
| ATOM 1045 | O | SER A 167 | -18.236 | -14.203 | 37.576 | 1.00 | 60.74 | A |
| ATOM 1046 | N | LYS A 168 | -18.794 | -16.059 | 36.445 | 1.00 | 64.25 | A |
| ATOM 1047 | CA | LYS A 168 | -20.079 | -15.519 | 36.032 | 1.00 | 65.94 | A |
| ATOM 1048 | CB | LYS A 168 | -20.693 | -16.374 | 34.918 | 1.00 | 68.53 | A |
| ATOM 1049 | CG | LYS A 168 | -19.931 | -16.315 | 33.592 | 1.00 | 70.35 | A |
| ATOM 1050 | CD | LYS A 168 | -20.558 | -17.226 | 32.537 | 1.00 | 72.07 | A |
| ATOM 1051 | CE | LYS A 168 | -19.737 | -17.228 | 31.250 | 1.00 | 74.35 | A |
| ATOM 1052 | NZ | LYS A 168 | -20.251 | -18.206 | 30.238 | 1.00 | 76.09 | A |
| ATOM 1053 | C | LYS A 168 | -21.016 | -15.454 | 37.222 | 1.00 | 65.80 | A |
| ATOM 1054 | O | LYS A 168 | -21.728 | -14.467 | 37.399 | 1.00 | 65.98 | A |
| ATOM 1055 | N | GLY A 169 | -21.000 | -16.502 | 38.045 | 1.00 | 65.48 | A |
| ATOM 1056 | CA | GLY A 169 | -21.851 | -16.528 | 39.220 | 1.00 | 62.40 | A |
| ATOM 1057 | C | GLY A 169 | -21.627 | -15.302 | 40.088 | 1.00 | 62.09 | A |
| ATOM 1058 | O | GLY A 169 | -22.241 | -15.164 | 41.141 | 1.00 | 64.40 | A |
| ATOM 1059 | N | GLN A 170 | -20.764 | -14.394 | 39.641 | 1.00 | 60.28 | A |
| ATOM 1060 | CA | GLN A 170 | -20.455 | -13.202 | 40.410 | 1.00 | 60.01 | A |
| ATOM 1061 | CB | GLN A 170 | -19.000 | -13.268 | 40.878 | 1.00 | 60.09 | A |
| ATOM 1062 | CG | GLN A 170 | -18.720 | -14.443 | 41.797 | 1.00 | 59.29 | A |
| ATOM 1063 | CD | GLN A 170 | -19.396 | -14.276 | 43.144 | 1.00 | 60.40 | A |
| ATOM 1064 | OE1 | GLN A 170 | -19.838 | -15.255 | 43.766 | 1.00 | 58.57 | A |
| ATOM 1065 | NE2 | GLN A 170 | -19.472 | -13.028 | 43.612 | 1.00 | 56.49 | A |
| ATOM 1066 | C | GLN A 170 | -20.688 | -11.920 | 39.631 | 1.00 | 61.05 | A |
| ATOM 1067 | O | GLN A 170 | -20.510 | -10.825 | 40.159 | 1.00 | 58.93 | A |
| ATOM 1068 | N | GLY A 171 | -21.085 | -12.059 | 38.373 | 1.00 | 64.02 | A |
| ATOM 1069 | CA | GLY A 171 | -21.325 | -10.884 | 37.560 | 1.00 | 69.34 | A |
| ATOM 1070 | C | GLY A 171 | -20.146 | -10.546 | 36.667 | 1.00 | 72.57 | A |
| ATOM 1071 | O | GLY A 171 | -19.940 | -9.388 | 36.293 | 1.00 | 73.44 | A |
| ATOM 1072 | N | LEU A 172 | -19.356 | -11.558 | 36.332 | 1.00 | 74.78 | A |
| ATOM 1073 | CA | LEU A 172 | -18.211 | -11.369 | 35.455 | 1.00 | 76.66 | A |
| ATOM 1074 | CB | LEU A 172 | -16.941 | -11.959 | 36.082 | 1.00 | 76.52 | A |
| ATOM 1075 | CG | LEU A 172 | -16.304 | -11.200 | 37.255 | 1.00 | 75.12 | A |
| ATOM 1076 | CD1 | LEU A 172 | -15.622 | -9.945 | 36.746 | 1.00 | 74.02 | A |
| ATOM 1077 | CD2 | LEU A 172 | -17.363 | -10.852 | 38.295 | 1.00 | 76.24 | A |
| ATOM 1078 | C | LEU A 172 | -18.551 | -12.095 | 34.166 | 1.00 | 77.94 | A |
| ATOM 1079 | O | LEU A 172 | -18.950 | -13.261 | 34.192 | 1.00 | 78.67 | A |
| ATOM 1080 | N | THR A 173 | -18.410 | -11.407 | 33.039 | 1.00 | 78.36 | A |
| ATOM 1081 | CA | TER A 173 | -18.727 | -12.017 | 31.755 | 1.00 | 79.91 | A |
| ATOM 1082 | CB | THR A 173 | -19.248 | -10.989 | 30.752 | 1.00 | 80.74 | A |
| ATOM 1083 | OG1 | THR A 173 | -18.138 | -10.416 | 30.051 | 1.00 | 82.51 | A |
| ATOM 1084 | CG2 | THR A 173 | -20.021 | -9.888 | 31.467 | 1.00 | 81.84 | A |
| ATOM 1085 | C | THR A 173 | -17.516 | -12.679 | 31.130 | 1.00 | 79.82 | A |
| ATOM 1086 | O | THR A 173 | -16.417 | -12.653 | 31.684 | 1.00 | 80.17 | A |
| ATOM 1087 | N | GLU A 174 | -17.732 | -13.257 | 29.955 | 1.00 | 80.54 | A |
| ATOM 1088 | CA | GLU A 174 | -16.681 | -13.945 | 29.222 | 1.00 | 82.10 | A |
| ATOM 1089 | CB | GLU A 174 | -17.261 | -14.552 | 27.940 | 1.00 | 85.56 | A |
| ATOM 1090 | CG | GLU A 174 | -16.541 | -15.799 | 27.443 | 1.00 | 89.66 | A |
| ATOM 1091 | CD | GLU A 174 | -16.562 | -16.933 | 28.460 | 1.00 | 92.42 | A |
| ATOM 1092 | OE1 | GLU A 174 | -15.909 | -16.798 | 29.520 | 1.00 | 94.51 | A |
| ATOM 1093 | OE2 | GLU A 174 | -17.235 | -17.957 | 28.204 | 1.00 | 93.50 | A |
| ATOM 1094 | C | GLU A 174 | -15.543 | -12.991 | 28.880 | 1.00 | 79.44 | A |
| ATOM 1095 | O | GLU A 174 | -14.371 | -13.358 | 28.932 | 1.00 | 77.18 | A |
| ATOM 1096 | N | GLU A 175 | -15.896 | -11.760 | 28.535 | 1.00 | 77.88 | A |
| ATOM 1097 | CA | GLU A 175 | -14.897 | -10.764 | 28.188 | 1.00 | 76.60 | A |
| ATOM 1098 | CB | GLU A 175 | -15.569 | -9.498 | 27.662 | 1.00 | 78.33 | A |
| ATOM 1099 | CG | GLU A 175 | -16.347 | -8.749 | 28.723 | 1.00 | 82.41 | A |
| ATOM 1100 | CD | GLU A 175 | -16.962 | -7.465 | 28.209 | 1.00 | 84.48 | A |
| ATOM 1101 | OE1 | GLU A 175 | -16.204 | -6.555 | 27.819 | 1.00 | 86.80 | A |
| ATOM 1102 | OE2 | GLU A 175 | -18.207 | -7.365 | 28.198 | 1.00 | 84.05 | A |
| ATOM 1103 | C | GLU A 175 | -14.047 | -10.412 | 29.399 | 1.00 | 74.57 | A |
| ATOM 1104 | O | GLU A 175 | -13.009 | -9.772 | 29.259 | 1.00 | 75.53 | A |
| ATOM 1105 | N | ASP A 176 | -14.486 | -10.818 | 30.587 | 1.00 | 72.84 | A |
| ATOM 1106 | CA | ASP A 176 | -13.737 | -10.525 | 31.808 | 1.00 | 71.42 | A |
| ATOM 1107 | CB | ASP A 176 | -14.680 | -10.222 | 32.974 | 1.00 | 72.57 | A |
| ATOM 1108 | CG | ASP A 176 | -15.839 | -9.341 | 32.576 | 1.00 | 74.87 | A |
| ATOM 1109 | OD1 | ASP A 176 | -16.734 | -9.846 | 31.873 | 1.00 | 74.74 | A |
| ATOM 1110 | OD2 | ASP A 176 | -15.853 | -8.150 | 32.963 | 1.00 | 75.76 | A |
| ATOM 1111 | C | ASP A 176 | -12.855 | -11.700 | 32.203 | 1.00 | 69.16 | A |
| ATOM 1112 | O | ASP A 176 | -11.823 | -11.530 | 32.841 | 1.00 | 69.29 | A |
| ATOM 1113 | N | ILE A 177 | -13.275 | -12.894 | 31.817 | 1.00 | 67.03 | A |
| ATOM 1114 | CA | ILE A 177 | -12.545 | -14.101 | 32.150 | 1.00 | 66.00 | A |
| ATOM 1115 | CB | ILE A 177 | -13.477 | -15.311 | 32.114 | 1.00 | 66.64 | A |
| ATOM 1116 | CG2 | ILE A 177 | -12.815 | -16.492 | 32.806 | 1.00 | 66.54 | A |
| ATOM 1117 | CG1 | ILE A 177 | -14.796 | -14.959 | 32.802 | 1.00 | 67.72 | A |
| ATOM 1118 | CD1 | ILE A 177 | -15.848 | -16.051 | 32.702 | 1.00 | 70.45 | A |
| ATOM 1119 | C | ILE A 177 | -11.370 | -14.364 | 31.218 | 1.00 | 65.75 | A |
| ATOM 1120 | O | ILE A 177 | -11.546 | -14.700 | 30.045 | 1.00 | 67.30 | A |
| ATOM 1121 | N | VAL A 178 | -10.166 | -14.224 | 31.756 | 1.00 | 63.32 | A |
| ATOM 1122 | CA | VAL A 178 | -8.955 | -14.447 | 30.985 | 1.00 | 59.20 | A |
| ATOM 1123 | CB | VAL A 178 | -8.006 | -13.243 | 31.082 | 1.00 | 58.03 | A |
| ATOM 1124 | CG1 | VAL A 178 | -6.856 | -13.419 | 30.114 | 1.00 | 60.86 | A |
| ATOM 1125 | CG2 | VAL A 178 | -8.755 | -11.962 | 30.803 | 1.00 | 58.82 | A |
| ATOM 1126 | C | VAL A 178 | -8.201 | -15.646 | 31.529 | 1.00 | 56.79 | A |
| ATOM 1127 | O | VAL A 178 | -8.326 | -15.993 | 32.698 | 1.00 | 59.10 | A |
| ATOM 1128 | N | PHE A 179 | -7.419 | -16.284 | 30.680 | 1.00 | 55.88 | A |
| ATOM 1129 | CA | PHE A 179 | -6.607 | -17.401 | 31.127 | 1.00 | 56.34 | A |
| ATOM 1130 | CB | PHE A 179 | -7.052 | -18.707 | 30.483 | 1.00 | 53.11 | A |
| ATOM 1131 | CG | PHE A 179 | -8.356 | -19.202 | 31.007 | 1.00 | 53.55 | A |
| ATOM 1132 | CD1 | PHE A 179 | -9.548 | -18.869 | 30.373 | 1.00 | 52.88 | A |
| ATOM 1133 | CD2 | PHE A 179 | -8.401 | -19.980 | 32.162 | 1.00 | 55.37 | A |
| ATOM 1134 | CE1 | PHE A 179 | -10.771 | -19.305 | 30.878 | 1.00 | 51.85 | A |
| ATOM 1135 | CE2 | PHE A 179 | -9.619 | -20.423 | 32.680 | 1.00 | 54.21 | A |
| ATOM 1136 | CZ | PRE A 179 | -10.809 | -20.084 | 32.034 | 1.00 | 54.72 | A |
| ATOM 1137 | C | PHE A 179 | -5.175 | -17.082 | 30.771 | 1.00 | 56.94 | A |
| ATOM 1138 | O | PHE A 179 | -4.750 | -17.224 | 29.629 | 1.00 | 58.94 | A |
| ATOM 1139 | N | LEU A 180 | -4.444 | -16.616 | 31.770 | 1.00 | 56.14 | A |
| ATOM 1140 | CA | LEU A 180 | -3.060 | -16.233 | 31.596 | 1.00 | 57.47 | A |
| ATOM 1141 | CB | LEU A 180 | -2.437 | -15.987 | 32.963 | 1.00 | 57.31 | A |
| ATOM 1142 | CG | LEU A 180 | -3.361 | -15.108 | 33.810 | 1.00 | 56.20 | A |
| ATOM 1143 | CD1 | LEU A 180 | -3.093 | -15.359 | 35.294 | 1.00 | 55.59 | A |
| ATOM 1144 | CD2 | LEU A 180 | -3.185 | -13.645 | 33.424 | 1.00 | 52.11 | A |
| ATOM 1145 | C | LEU A 180 | -2.274 | -17.288 | 30.843 | 1.00 | 58.94 | A |
| ATOM 1146 | O | LEU A 180 | -2.327 | -18.472 | 31.176 | 1.00 | 59.95 | A |
| ATOM 1147 | N | PRO A 181 | -1.549 | -16.867 | 29.799 | 1.00 | 60.40 | A |
| ATOM 1148 | CD | PRO A 181 | -1.633 | -15.518 | 29.208 | 1.00 | 59.15 | A |
| ATOM 1149 | CA | PRO A 181 | -0.727 | -17.741 | 28.961 | 1.00 | 63.62 | A |
| ATOM 1150 | CB | PRO A 181 | -0.619 | -16.953 | 27.666 | 1.00 | 61.46 | A |
| ATOM 1151 | CG | PRO A 181 | -0.533 | -15.549 | 28.166 | 1.00 | 57.89 | A |
| ATOM 1152 | C | PRO A 181 | 0.640 | -17.966 | 29.598 | 1.00 | 67.50 | A |
| ATOM 1153 | O | PRO A 181 | 1.154 | -17.086 | 30.284 | 1.00 | 68.06 | A |
| ATOM 1154 | N | GLN A 182 | 1.223 | -19.138 | 29.364 | 1.00 | 70.91 | A |
| ATOM 1155 | CA | GLN A 182 | 2.538 | -19.458 | 29.912 | 1.00 | 74.68 | A |
| ATOM 1156 | CB | GLN A 182 | 2.945 | -20.894 | 29.570 | 1.00 | 74.56 | A |
| ATOM 1157 | CG | GLN A 182 | 1.933 | -21.971 | 29.892 | 1.00 | 76.21 | A |
| ATOM 1158 | CD | GLN A 182 | 2.520 | -23.373 | 29.728 | 1.00 | 77.21 | A |
| ATOM 1159 | OE1 | GLN A 182 | 1.790 | -24.364 | 29.679 | 1.00 | 77.14 | A |
| ATOM 1160 | NE2 | GLN A 182 | 3.848 | -23.458 | 29.655 | 1.00 | 74.73 | A |
| ATOM 1161 | C | GLN A 182 | 3.599 | -18.527 | 29.329 | 1.00 | 77.11 | A |
| ATOM 1162 | O | GLN A 182 | 3.851 | -18.546 | 28.127 | 1.00 | 77.72 | A |
| ATOM 1163 | N | PRO A 183 | 4.232 | -17.696 | 30.169 | 1.00 | 79.26 | A |
| ATOM 1164 | CD | PRO A 183 | 3.959 | -17.395 | 31.585 | 1.00 | 78.43 | A |
| ATOM 1165 | CA | PRO A 183 | 5.260 | -16.800 | 29.637 | 1.00 | 82.07 | A |
| ATOM 1166 | CB | PRO A 183 | 5.334 | -15.706 | 30.691 | 1.00 | 79.37 | A |
| ATOM 1167 | CG | PRO A 183 | 5.106 | -16.472 | 31.944 | 1.00 | 78.09 | A |
| ATOM 1168 | C | PRO A 183 | 6.576 | -17.567 | 29.497 | 1.00 | 86.60 | A |
| ATOM 1169 | O | PRO A 183 | 7.309 | -17.743 | 30.474 | 1.00 | 88.58 | A |
| ATOM 1170 | N | ASP A 184 | 6.856 | -18.037 | 28.285 | 1.00 | 89.35 | A |
| ATOM 1171 | CA | ASP A 184 | 8.074 | -18.790 | 27.996 | 1.00 | 91.71 | A |
| ATOM 1172 | CB | ASP A 184 | 8.376 | -18.720 | 26.496 | 1.00 | 90.76 | A |
| ATOM 1173 | CG | ASP A 184 | 8.268 | -17.308 | 25.946 | 1.00 | 91.18 | A |
| ATOM 1174 | OD1 | ASP A 184 | 9.028 | -16.421 | 26.394 | 1.00 | 92.29 | A |
| ATOM 1175 | OD2 | ASP A 184 | 7.415 | -17.080 | 25.064 | 1.00 | 91.05 | A |
| ATOM 1176 | C | ASP A 184 | 9.287 | -18.298 | 28.786 | 1.00 | 93.40 | A |
| ATOM 1177 | O | ASP A 184 | 10.143 | -19.090 | 29.187 | 1.00 | 93.35 | A |
| ATOM 1178 | N | LYS A 185 | 9.347 | -16.989 | 29.014 | 1.00 | 95.42 | A |
| ATOM 1179 | CA | LYS A 185 | 10.451 | -16.376 | 29.744 | 1.00 | 95.83 | A |
| ATOM 1180 | CB | LYS A 185 | 10.661 | -14.943 | 29.239 | 1.00 | 96.46 | A |
| ATOM 1181 | CG | LYS A 185 | 11.974 | -14.287 | 29.659 | 1.00 | 98.21 | A |
| ATOM 1182 | CD | LYS A 185 | 12.077 | -12.872 | 29.092 | 1.00 | 100.88 | A |
| ATOM 1183 | CE | LYS A 185 | 13.448 | -12.250 | 29.335 | 1.00 | 102.05 | A |
| ATOM 1184 | NZ | LYS A 185 | 13.760 | -12.116 | 30.785 | 1.00 | 104.65 | A |
| ATOM 1185 | C | LYS A 185 | 10.192 | -16.366 | 31.250 | 1.00 | 95.82 | A |
| ATOM 1186 | O | LYS A 185 | 9.619 | -17.299 | 31.804 | 1.00 | 94.92 | A |
| ATOM 1187 | N | CYS A 186 | 10.627 | -15.291 | 31.890 | 1.00 | 96.28 | A |
| ATOM 1188 | CA | CYS A 186 | 10.492 | -15.074 | 33.321 | 1.00 | 97.57 | A |
| ATOM 1189 | C | CYS A 186 | 10.358 | -16.297 | 34.239 | 1.00 | 99.39 | A |
| ATOM 1190 | O | CYS A 186 | 11.328 | -16.685 | 34.887 | 1.00 | 100.13 | A |
| ATOM 1191 | CB | CYS A 186 | 9.352 | -14.098 | 33.575 | 1.00 | 95.95 | A |
| ATOM 1192 | SG | CYS A 186 | 9.759 | -13.012 | 34.968 | 1.00 | 98.27 | A |
| ATOM 1193 | N | ILE A 187 | 9.171 | -16.889 | 34.324 | 1.00 | 101.87 | A |
| ATOM 1194 | CA | ILE A 187 | 8.976 | -18.066 | 35.175 | 1.00 | 103.65 | A |
| ATOM 1195 | CB | ILE A 187 | 7.513 | -18.517 | 35.189 | 1.00 | 100.55 | A |
| ATOM 1196 | CG2 | ILE A 187 | 7.375 | -19.795 | 35.996 | 1.00 | 100.83 | A |
| ATOM 1197 | CG1 | ILE A 187 | 6.631 | -17.417 | 35.765 | 1.00 | 97.38 | A |
| ATOM 1198 | CD1 | ILE A 187 | 5.172 | -17.755 | 35.710 | 1.00 | 94.71 | A |
| ATOM 1199 | C | ILE A 187 | 9.805 | -19.235 | 34.659 | 1.00 | 107.87 | A |
| ATOM 1200 | O | ILE A 187 | 10.074 | -19.324 | 33.463 | 1.00 | 109.90 | A |
| ATOM 1201 | N | GLN A 188 | 10.193 | -20.138 | 35.554 | 1.00 | 111.72 | A |
| ATOM 1202 | CA | GLN A 188 | 10.998 | -21.296 | 35.168 | 1.00 | 116.09 | A |
| ATOM 1203 | CB | GLN A 188 | 11.467 | -22.053 | 36.412 | 1.00 | 117.84 | A |
| ATOM 1204 | CG | GLN A 188 | 12.347 | -21.230 | 37.335 | 1.00 | 120.37 | A |
| ATOM 1205 | CD | GLN A 188 | 13.626 | -20.764 | 36.665 | 1.00 | 120.88 | A |
| ATOM 1206 | OE1 | GLN A 188 | 13.592 | -20.088 | 35.637 | 1.00 | 121.37 | A |
| ATOM 1207 | NE2 | GLN A 188 | 14.763 | -21.121 | 37.250 | 1.00 | 120.87 | A |
| ATOM 1208 | C | GLN A 188 | 10.251 | -22.249 | 34.241 | 1.00 | 118.05 | A |
| ATOM 1209 | O | GLN A 188 | 9.512 | -23.125 | 34.696 | 1.00 | 118.11 | A |
| ATOM 1210 | N | GLU A 189 | 10.462 | -22.070 | 32.938 | 1.00 | 120.59 | A |
| ATOM 1211 | CA | GLU A 189 | 9.830 | -22.892 | 31.904 | 1.00 | 121.84 | A |
| ATOM 1212 | CB | GLU A 189 | 10.296 | -24.352 | 32.015 | 1.00 | 122.00 | A |
| ATOM 1213 | CG | GLU A 189 | 10.004 | -25.210 | 30.781 | 1.00 | 120.78 | A |
| ATOM 1214 | CD | GLU A 189 | 10.895 | -24.865 | 29.600 | 1.00 | 120.07 | A |
| ATOM 1215 | OE1 | GLU A 189 | 10.855 | -23.705 | 29.142 | 1.00 | 119.99 | A |
| ATOM 1216 | OE2 | GLU A 189 | 11.636 | -25.753 | 29.129 | 1.00 | 120.26 | A |
| ATOM 1217 | C | GLU A 189 | 8.308 | -22.830 | 32.001 | 1.00 | 122.03 | A |
| ATOM 1218 | O | GLU A 189 | 7.697 | -23.870 | 32.332 | 1.00 | 121.94 | A |
| ATOM 1219 | OT | GLU A 189 | 7.748 | -21.740 | 31.749 | 1.00 | 121.99 | A |
| ATOM 1220 | OH2 | WAT W 200 | 6.622 | -15.627 | 50.105 | 1.00 | 32.46 | W |
| ATOM 1221 | OH2 | WAT W 201 | -13.735 | -12.460 | 49.782 | 1.00 | 32.29 | W |
| ATOM 1222 | OH2 | WAT W 203 | -15.181 | -17.532 | 49.376 | 1.00 | 37.27 | W |
| ATOM 1223 | OH2 | WAT W 204 | -15.122 | -18.405 | 51.994 | 1.00 | 34.03 | W |
| ATOM 1224 | OH2 | WAT W 205 | -11.282 | -24.928 | 56.736 | 1.00 | 73.37 | W |
| ATOM 1225 | OH2 | WAT W 206 | 1.440 | -10.333 | 58.570 | 1.00 | 44.34 | W |
| ATOM 1226 | OH2 | WAT W 207 | -6.812 | -15.884 | 58.098 | 1.00 | 42.35 | W |
| ATOM 1227 | OH2 | WAT W 208 | -12.965 | -18.384 | 52.877 | 1.00 | 32.22 | W |
| ATOM 1228 | OH2 | WAT W 209 | 4.880 | -15.352 | 25.292 | 1.00 | 74.89 | W |
| ATOM 1229 | OH2 | WAT W 210 | -14.011 | 4.577 | 39.538 | 1.00 | 40.06 | W |
| ATOM 1230 | OH2 | WAT W 211 | -18.207 | -6.622 | 46.065 | 1.00 | 52.52 | W |
| ATOM 1231 | OH2 | WAT W 212 | -1.942 | -21.966 | 60.262 | 1.00 | 54.38 | W |
| ATOM 1232 | OH2 | WAT W 213 | -10.025 | 2.738 | 39.161 | 1.00 | 89.20 | W |
| ATOM 1233 | OH2 | WAT W 214 | -7.536 | 3.097 | 34.665 | 1.00 | 66.61 | W |
| ATOM 1234 | OH2 | WAT W 215 | -6.865 | 4.692 | 30.926 | 1.00 | 68.98 | W |
| ATOM 1235 | OH2 | WAT W 216 | -5.056 | 6.596 | 40.134 | 1.00 | 47.28 | W |
| ATOM 1236 | OH2 | WAT W 217 | -3.634 | 4.447 | 40.783 | 1.00 | 83.64 | W |
| ATOM 1237 | OH2 | WAT W 218 | 7.394 | -11.367 | 25.281 | 1.00 | 57.42 | W |
| ATOM 1238 | OH2 | WAT W 219 | 9.310 | -21.158 | 25.421 | 1.00 | 59.90 | W |
| ATOM 1239 | OH2 | WAT W 220 | 10.366 | -24.989 | 26.400 | 1.00 | 58.73 | W |
| ATOM 1240 | OH2 | WAT W 221 | -4.606 | 3.348 | 26.800 | 1.00 | 65.51 | W |
| ATOM 1241 | OH2 | WAT W 222 | -2.367 | 6.175 | 29.500 | 1.00 | 42.56 | W |
| ATOM 1242 | OH2 | WAT W 223 | -4.942 | 6.474 | 32.978 | 1.00 | 55.44 | W |
| ATOM 1243 | OH2 | WAT W 224 | -20.607 | -20.158 | 31.933 | 1.00 | 59.45 | W |
| ATOM 1244 | OH2 | WAT W 225 | -25.839 | -21.690 | 29.465 | 1.00 | 67.32 | W |
| ATOM 1245 | OH2 | WAT W 226 | -27.537 | -18.582 | 31.205 | 1.00 | 63.04 | W |
| ATOM 1246 | OH2 | WAT W 227 | 1.524 | -5.767 | 50.296 | 1.00 | 37.51 | W |
| ATOM 1247 | OH2 | WAT W 228 | -1.401 | -5.768 | 53.755 | 1.00 | 58.16 | W |
| ATOM 1248 | OH2 | WAT W 229 | 1.756 | 0.015 | 54.499 | 1.00 | 67.42 | W |
| ATOM 1249 | OH2 | WAT W 230 | 7.581 | -7.200 | 53.652 | 1.00 | 51.29 | W |
| ATOM 1250 | OH2 | WAT W 231 | 10.564 | -9.875 | 52.898 | 1.00 | 34.20 | W |
| ATOM 1251 | OH2 | WAT W 232 | -12.228 | 8.044 | 49.309 | 1.00 | 43.27 | W |
| ATOM 1252 | OH2 | WAT W 233 | 3.892 | -5.291 | 53.253 | 1.00 | 54.32 | W |
| ATOM 1253 | OH2 | WAT W 234 | -12.406 | -27.193 | 54.213 | 1.00 | 58.17 | W |
| ATOM 1254 | OH2 | WAT W 235 | -12.591 | -26.874 | 57.492 | 1.00 | 59.02 | W |
| ATOM 1255 | OH2 | WAT W 236 | -9.972 | -27.101 | 55.248 | 1.00 | 64.22 | W |
| ATOM 1256 | OH2 | WAT W 237 | -10.986 | -26.023 | 59.916 | 1.00 | 85.99 | W |
| ATOM 1257 | OH2 | WAT W 238 | -8.633 | -24.722 | 60.499 | 1.00 | 65.65 | W |
| ATOM 1258 | OH2 | WAT W 239 | -7.408 | -29.767 | 62.831 | 1.00 | 104.46 | W |
| ATOM 1259 | OH2 | WAT W 240 | -10.853 | -30.151 | 64.764 | 1.00 | 64.63 | W |
| ATOM 1260 | OH2 | WAT W 241 | -5.798 | -30.088 | 64.947 | 1.00 | 59.64 | W |
| ATOM 1261 | OH2 | WAT W 242 | -10.749 | -26.762 | 64.393 | 1.00 | 42.38 | W |
| ATOM 1262 | OH2 | WAT W 243 | -4.473 | -23.155 | 64.989 | 1.00 | 62.66 | W |
| ATOM 1263 | OH2 | WAT W 244 | -7.538 | -16.683 | 61.868 | 1.00 | 50.16 | W |
| ATOM 1264 | OH2 | WAT W 245 | -10.230 | -12.385 | 64.294 | 1.00 | 56.02 | W |
| ATOM 1265 | OH2 | WAT W 246 | -12.542 | -9.996 | 64.001 | 1.00 | 54.97 | W |
| ATOM 1266 | OH2 | WAT W 247 | -3.345 | -14.402 | 69.343 | 1.00 | 57.93 | W |
| ATOM 1267 | OH2 | WAT W 249 | -3.709 | -26.479 | 62.036 | 1.00 | 92.57 | W |
| ATOM 1268 | OH2 | WAT W 250 | -0.479 | -31.295 | 63.228 | 1.00 | 47.82 | W |
| ATOM 1269 | OH2 | WAT W 251 | -3.132 | -28.329 | 67.027 | 1.00 | 81.69 | W |
| ATOM 1270 | OH2 | WAT W 252 | 1.056 | -29.536 | 66.527 | 1.00 | 61.35 | W |
| ATOM 1271 | OH2 | WAT W 253 | -1.744 | -29.498 | 70.046 | 1.00 | 87.09 | W |
| ATOM 1272 | OH2 | WAT W 254 | -3.689 | -29.331 | 72.794 | 1.00 | 58.75 | W |
| ATOM 1273 | OH2 | WAT W 255 | -1.410 | -27.800 | 75.675 | 1.00 | 97.58 | W |
| ATOM 1274 | OH2 | WAT W 256 | -5.043 | -29.537 | 76.343 | 1.00 | 64.90 | W |
| ATOM 1275 | OH2 | WAT W 257 | -4.475 | -32.406 | 78.585 | 1.00 | 63.31 | W |
| ATOM 1276 | OH2 | WAT W 258 | -9.056 | -33.080 | 76.962 | 1.00 | 69.69 | W |
| ATOM 1277 | OH2 | WAT W 259 | -4.003 | -31.163 | 82.151 | 1.00 | 71.55 | W |
| ATOM 1278 | OH2 | WAT W 260 | -5.860 | -21.668 | 72.801 | 1.00 | 74.39 | W |
| ATOM 1279 | OH2 | WAT W 261 | -2.868 | -8.958 | 39.980 | 1.00 | 74.69 | W |
| ATOM 1280 | OH2 | WAT W 262 | -4.140 | -12.006 | 44.306 | 1.00 | 62.67 | W |
| ATOM 1281 | OH2 | WAT W 263 | -3.803 | -8.642 | 42.852 | 1.00 | 66.75 | W |
| ATOM 1282 | OH2 | WAT W 264 | 1.805 | -10.509 | 35.249 | 1.00 | 59.42 | W |
| ATOM 1283 | OH2 | WAT W 265 | -4.539 | -24.842 | 50.726 | 1.00 | 36.51 | W |
| ATOM 1284 | OH2 | WAT W 266 | -19.503 | -1.261 | 38.826 | 1.00 | 69.86 | W |
| ATOM 1285 | OH2 | WAT W 267 | 12.469 | -22.407 | 27.693 | 1.00 | 85.12 | W |

## Claims

1. A crystal of lipocalin-type prostaglandin D synthase derived from mouse.

2. A crystal as claimed in Claim 1 which has orthorhombic system space group P2₁2₁2₁ and in which the size of unit cell is a=46.2±0.5Å, b=66.8±0.7Å, and c=105.3±1.0Å

3. A crystal as claimed in Claim 1 which has orthorhombic system space group C222₁ and in which the size of unit cell is a=45.7±0.5Å, b=66.8±0.7Å, and c=104.5±1.0Å.

4. Lipocalin-type prostaglandin D synthase having a three dimensional structure represented by the structural coordinates in Table 2.

5. Lipocalin-type prostaglandin D synthase having a three dimensional structure represented by the structural coordinates in Table 3.

6. Use of the structural coordinates in Table 2 or 3 for the selection of a compound which inhibits lipocalin-type prostaglandin D synthase.

7. A method for selecting an inhibitor of lipocalin-type prostaglandin D synthase, which comprising
(a) providing the three dimensional structure coordinates in Table 2 or 3 representing three dimensional structure of lipocalin-type prostaglandin D synthase;
(b) providing three dimensional structures of candidate compounds; and
(c) selecting the candidate compound which fits to the substrate-binding site of lipocalin-type prostaglandin D synthase as inhibitor.

8. A method as claimed in Claim 7, further comprising
(d) contacting the inhibitor as selected above with lipocalin-type prostaglandin D synthase in the presence of prostaglandin H₂ to measure L-PGDS enzyme activity to confirm the inhibiting effect of the inhibitor selected.

9. An inhibitor for lipocalin-type prostaglandin D synthase selected by the method of Claim 7 or 8.

10. An inhibitor for lipocalin-type prostaglandin D synthase which is 4-dibenzo (a,d) cyclohepten-5-ylidene-1-(4-(2H-tetrazole-5-yl)butyl)piperidine.


<110> RIKEN
      Osaka Bioscience Institute

<120> THREE-DIMENSIONAL STRUCTURE OF LIPOCALIN-TYPE PROSTAGLANDIN D SYNTHASE
AND UTILIZATION OF THE SAME

<130> 664192

<160> 1

<210> 1
<211> 189
<212> PRT
<213> mouse
<400> 1
Met Ala Ala Leu Arg Met Leu Trp Met Gly Leu Val Leu Leu Gly Leu
 1               5                  10                  15
Leu Gly Phe Pro Gln Thr Pro Ala Gln Gly His Asp Thr Val Gln Pro
            20                  25                  30
Asn Phe Gln Gln Asp Lys Phe Leu Gly Arg Trp Tyr Ser Ala Gly Leu
        35                  40                  45
Ala Ser Asn Ser Ser Trp Phe Arg Glu Lys Lys Ala Val Leu Tyr Met
    50                  55                  60
Cys Lys Thr Val Val Ala Pro Ser Thr Glu Gly Gly Leu Asn Leu Thr
65                  70                  75                  80
Ser Thr Phe Leu Arg Lys Asn Gln Cys Glu Thr Lys Ile Met Val Leu
                85                  90                  95
Gln Pro Ala Gly Ala Pro Gly His Tyr Thr Tyr Ser Ser Pro His Ser
            100                 105                 110
Gly Ser Ile His Ser Val Ser Val Val Glu Ala Asn Tyr Asp Glu Tyr
        115                 120                 125
Ala Leu Leu Phe Ser Arg Gly Thr Lys Gly Pro Gly Gln Asp Phe Arg
    130                 135                 140
Met Ala Thr Leu Tyr Ser Arg Thr Gln Thr Leu Lys Asp Glu Leu Lys
145                 150                 155                 160
Glu Lys Phe Thr Thr Phe Ser Lys Ala Gln Gly Leu Thr Glu Glu Asp
                165                 170                 175
Ile Val Phe Leu Pro Gln Pro Asp Lys Cys Ile Gln Glu
            180                 185